(19) Europäisches Patentamt / European Patent Office / Office européen des brevets

(11) EP 2 214 693 B1

(12) EUROPEAN PATENT SPECIFICATION

(45) Date of publication and mention of the grant of the patent:
**09.09.2015 Bulletin 2015/37**

(21) Application number: **08846334.4**

(22) Date of filing: **05.11.2008**

(51) Int Cl.:
***A61K 38/00*** *(2006.01)*

(86) International application number:
**PCT/US2008/012490**

(87) International publication number:
**WO 2009/061411 (14.05.2009 Gazette 2009/20)**

# (54) USE OF MELANOCORTINS TO TREAT INSULIN SENSITIVITY

VERWENDUNG VON MELANOCORTINEN ZUR BEHANDLUNG VON INSULINSENSITIVITÄT

UTILISATION DE MÉLANOCORTINES POUR TRAITER UNE SENSIBILITÉ À L'INSULINE

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **05.11.2007 US 1933 P**

(43) Date of publication of application:
**11.08.2010 Bulletin 2010/32**

(60) Divisional application:
**15179111.8**

(73) Proprietors:
- **IPSEN PHARMA S.A.S.**
  **92100 Boulogne-Billancourt (FR)**
- **Board of Supervisors of Louisiana State University and Agriculture and Mechanical College**
  **Baton Rouge, LA 708008-4124 (US)**

(72) Inventors:
- **HALEM, Heather A.**
  **Westbouough, MA 01581 (US)**
- **CULLER, Michael Dewitt**
  **Hopkinton, MA 01748 (US)**
- **BUTLER, Andrew A.**
  **Jupiter, FL 33458 (US)**

(74) Representative: **Wells, Andrew et al**
**HGF Limited**
**4th Floor, Merchant Exchange**
**17-19 Whitworth Street West**
**Manchester M1 5WG (GB)**

(56) References cited:

| | |
|---|---|
| **WO-A2-2007/008684** | **WO-A2-2007/008704** |
| **WO-A2-2008/147556** | **WO-A2-2008/156677** |
| **US-A1- 2007 123 453** | **US-A1- 2007 244 054** |

- **W. FAN: "The Central Melanocortin System Can Directly Regulate Serum Insulin Levels", ENDOCRINOLOGY, vol. 141, no. 9, 1 September 2000 (2000-09-01), pages 3072-3079, XP55020467, ISSN: 0013-7227, DOI: 10.1210/en.141.9.3072**
- **HEIJBOER A C ET AL: "Intracerebroventricular administration of melanotan II increases insulin sensitivity of glucose disposal in mice", DIABETOLOGIA ; CLINICAL AND EXPERIMENTAL DIABETES AND METABOLISM, SPRINGER, BERLIN, DE, vol. 48, no. 8, 1 August 2005 (2005-08-01) , pages 1621-1626, XP019322647, ISSN: 1432-0428, DOI: 10.1007/S00125-005-1838-8**
- **BANNO R ET AL: "The melanocortin agonist melanotan II increases insulin sensitivity in OLETF rats", PEPTIDES, ELSEVIER, AMSTERDAM, vol. 25, no. 8, 1 August 2004 (2004-08-01) , pages 1279-1286, XP004551468, ISSN: 0196-9781, DOI: 10.1016/J.PEPTIDES. 2004.05.007**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> SOCIETE DE CONSEILS DE RECHERCHES ET D'APPLICATIONS SCIENTIFIQUES S.A.S. BOARD OF SUPERVISORS OF LOUISIANA STATE UNIVERSITY AND AGRICULTURE AND MECHANICAL COLLEGE Halem, Heather Culler, Michael Butler, Andrew

<120> USE OF MELANOCORTINS TO TREAT INSULIN SENSITIVITY

<130> 183P/PCT2

<150> US 61/001933
<151> 2007-11-05

<160> 293

<170> PatentIn version 3.5

<210> 1
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = beta-Ala or 1-amino-1-cyclohexanecarboxylic acid (Ac)

<220>
<221> MOD_RES
<222> (8)..(8)
<223> AMIDATION

<400> 1

```
Xaa Asp His Xaa Arg Trp Xaa Lys
1               5
```

<210> 2
<211> 8
<212> PRT
<213> Artificial Sequence

<220>
<223> Melanocortin receptor ligand to treat insulin sensitivity

<220>
<221> MISC_FEATURE

<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 6-aminohexanoic acid (Ahx) or 5-aminopentanoic acid (Apn)

<220>
<221> MOD_RES
<222> (8)..(8)
<223> AMIDATION

<400> 2

```
Xaa Cys His Xaa Arg Trp Xaa Cys
1               5
```

<210> 3
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = Ala, beta-Ala or 4-aminobutyric acid (Gaba)

<220>

<221> MISC_FEATURE
<222> (8)..(8)
<223> Xaa=D-Cys

<220>
<221> MOD_RES
<222> (9)..(9)
<223> AMIDATION

<400> 3

```
Xaa Cys His Xaa Arg Trp Xaa Xaa Thr
1               5
```

<210> 4
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MOD_RES
<222> (8)..(8)
<223> AMIDATION

<400> 4

```
Xaa Asp His Xaa Arg Trp Xaa Lys
1               5
```

<210> 5
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = 1-amino-1-cyclohexanecarboxylic acid (A6c) modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MOD_RES
<222> (8)..(8)
<223> AMIDATION

<400> 5

```
Xaa Asp His Xaa Arg Trp Xaa Lys
1               5
```

<210> 6
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = D-beta-(2-naphthyl)Ala (D-2-Nal), beta-cyclohexylAla (Cha) or Nle, all modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>

<221> MISC_FEATURE
<222> (7)..(7)
<223> Xaa = 4-aminobutyric acid (Gaba)

<220>
<221> MOD_RES
<222> (8)..(8)
<223> AMIDATION

<400> 6

```
Xaa Asp His Xaa Arg Trp Xaa Lys
1               5
```

<210> 7
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-Ala, beta-Ala, 4-aminobutyric acid (Gaba), alpha-aminoisobutyric acid (Aib) or Gly

<220>
<221> MISC_FEATURE
<222> (5)..(5)
<223> Xaa = D-Phe

<220>
<221> MOD_RES
<222> (8)..(8)
<223> AMIDATION

<400> 7

```
Xaa Cys Xaa His Xaa Arg Trp Cys
1               5
```

<210> 8
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (2)..(2)
<223> Xaa = D-Cys

<220>
<221> MISC_FEATURE
<222> (3)..(3)
<223> Xaa = Ala, D-Ala, beta-Ala, 4-aminobutyric acid (Gaba), alpha-aminoisobutyric acid (Aib) or Gly

<220>
<221> MISC_FEATURE
<222> (5)..(5)
<223> Xaa = D-Phe

<220>
<221> MOD_RES
<222> (8)..(8)
<223> AMIDATION

<400> 8

```
Xaa Xaa Xaa His Xaa Arg Trp Cys
1               5
```

<210> 9
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>

<221> MISC_FEATURE
<222> (3)..(3)
<223> Xaa = D-Ala, beta-Ala, 4-aminobutyric acid (Gaba), alpha-aminoisobutyric acid (Aib) or Gly

<220>
<221> MISC_FEATURE
<222> (5)..(5)
<223> Xaa = D-Phe

<220>
<221> MISC_FEATURE
<222> (8)..(8)
<223> Xaa = D-Cys

<220>
<221> MOD_RES
<222> (8)..(8)
<223> AMIDATION

<400> 9

```
Xaa Cys Xaa His Xaa Arg Trp Xaa
1               5
```

<210> 10
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (2)..(2)
<223> xaa = D-Cys

<220>
<221> MISC_FEATURE
<222> (3)..(3)
<223> Xaa = Ala, D-Ala, beta-Ala, 4-aminobutyric acid (Gaba) or alpha-aminoisobutyric acid (Aib)

<220>
<221> MISC_FEATURE
<222> (5)..(5)
<223> Xaa = D-Phe

<220>
<221> MISC_FEATURE
<222> (8)..(8)
<223> Xaa = D-Cys

<220>
<221> MOD_RES
<222> (8)..(8)
<223> AMIDATION

<400> 10

```
Xaa Xaa Xaa His Xaa Arg Trp Xaa
1               5
```

<210> 11
<211> 8
<212> PRT
<213> Artificial Sequence

<220>
<223> Melanocortin receptor ligand to treat insulin sensitivity

<220>
<221> MISC FEATURE
<222> (1)..(1)
<223> Xaa = octahydroindole-2-carboxylic acid (oic), cyclohexylGly (Chg), homo-cyclohexylAla (hcha), D-Cha,nipecotic acid (Nip), hPro, hLeu, Phe, D-Phe, D-Chg, hPhe, beta-homoMet, or 4-aminobutyric acid (Gaba), all modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MOD_RES
<222> (8)..(8)
<223> AMIDATION

<400> 11

```
Xaa Asp His Xaa Arg Trp Xaa Lys
1               5
```

<210> 12

<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = beta-cyclohexylAla (Cha) modified with n-butanoyl

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MOD_RES
<222> (8)..(8)
<223> AMIDATION

<400> 12

```
Xaa Asp His Xaa Arg Trp Xaa Lys
1               5
```

<210> 13
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = beta-cyclohexylAla (Cha), homo-cyclohexylAla (hCha), Leu, homo-Leu (hLeu) or Phe, all modified with acyl (Ac)

<220>
<221> DOMAIN
<222> (2)..(8)
<223> Cyclic

<220>

<221> MISC_FEATURE
<222> (4)..(4)
<223> Xaa = D-Phe

<220>
<221> MISC_FEATURE
<222> (6)..(6)
<223> Xaa = D-Trp

<220>
<221> MOD_RES
<222> (8)..(8)
<223> AMIDATION

<400> 13

```
Xaa Asp His Xaa Arg Xaa Ala Lys
1               5
```

<210> 14
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (6)..(6)
<223> Xaa = D-Trp

<220>
<221> MISC_FEATURE
<222> (7)..(7)
<223> Xaa = D-Ala, beta-Ala, 4-aminobutyric acid (Gaba), 7-aminoheptanoic acid (Aha) or 5-aminopentanoic acid (Apn)

<220>
<221> MOD_RES
<222> (8)..(8)
<223> AMIDATION

<400> 14

Xaa Asp His Xaa Arg Xaa Xaa Lys
1               5

<210> 15
<211> 8
<212> PRT
<213> Artificial sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (6)..(6)
<223> Xaa = D-Trp

<220>
<221> MISC_FEATURE
<222> (7)..(7)
<223> Xaa = 5-aminopentanoic acid (Apn), 4-aminobutyric acid (Gaba), 6-aminohexanoic acid (Ahx), beta-Ala, or D-Ala

<220>
<221> MOD_RES
<222> (8)..(8)
<223> AMIDATION

<400> 15

Xaa Cys His Xaa Arg Xaa Xaa Cys
1               5

<210> 16
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-beta-(2-naphthyl)Ala (D-2-Nal)

<220>
<221> MISC_FEATURE
<222> (7)..(7)
<223> Xaa = Trp, beta-(2-naphthyl)Ala (2-Nal) or beta-(1-naphthyl)Ala (1-Nal)

<220>
<221> MOD_RES
<222> (8)..(8)
<223> AMIDATION

<400> 16

```
Xaa Cys Xaa His Xaa Arg Xaa Cys
1               5
```

<210> 17
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with n-butanoyl

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (7)..(7)
<223> Xaa = beta-(2-naphthyl)Ala (2-Nal) or Trp

<220>
<221> MOD_RES
<222> (8)..(8)
<223> AMIDATION

<400> 17

```
Xaa Cys Xaa His Xaa Arg Xaa Cys
1               5
```

<210> 18
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (7)..(7)
<223> Xaa = beta-(2-naphthyl)Ala (2-Nal), beta-(1-naphthyl)Ala (1-Nal) or 3-benzothienylAla (Bal)

<220>
<221> MOD_RES
<222> (8)..(8)
<223> AMIDATION

<400> 18

Xaa Cys Xaa His Xaa Arg Xaa Cys
1               5

<210> 19
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = D-Ala

<220>
<221> MOD_RES
<222> (8)..(8)
<223> AMIDATION

<400> 19

Xaa Asp His Xaa Arg Trp Xaa Lys
1               5

<210> 20
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-beta-2-naphthylAla (D-2-Nal)

<220>
<221> MISC_FEATURE
<222> (7)..(7)
<223> Xaa = 3-benzothienylAla (Bal)

<220>
<221> MOD_RES
<222> (8)..(8)
<223> AMIDATION

<400> 20

```
Xaa Cys Xaa His Xaa Arg Xaa Cys
1               5
```

<210> 21
<211> 8
<212> PRT
<213> Artificial sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (2)..(2)
<223> Xaa = penicillamine (Pen)

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MOD_RES
<222> (8)..(8)
<223> AMIDATION

<400> 21

```
Xaa Xaa Xaa His Xaa Arg Trp Cys
1               5
```

<210> 22
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (2)..(2)
<223> Xaa = Cys or penicillamine (Pen)

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (8)..(8)
<223> Xaa = penicillamine (Pen)

<220>
<221> MOD_RES
<222> (8)..(8)
<223> AMIDATION

<400> 22

Xaa Xaa Xaa His Xaa Arg Trp Xaa
1 5

<210> 23
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (5)..(5)
<223> Xaa = homo-Arg (hArg)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MOD_RES
<222> (9)..(9)
<223> AMIDATION

<400> 23

Xaa Cys His Xaa Xaa Trp Xaa Xaa Thr
1 5

<210> 24
<211> 9
<212> PRT

<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> Cyclic

<220>
<221> MISC_FEATURE
<222> (4)..(4)
<223> Xaa = D-(Et)Tyr

<220>
<221> MISC_FEATURE
<222> (5)..(5)
<223> Xaa = Arg or homo-Arg (hArg)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MOD_RES
<222> (9)..(9)
<223> AMIDATION

<400> 24

```
Xaa Cys His Xaa Xaa Trp Xaa Xaa Thr
1               5
```

<210> 25
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (6)..(6)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MOD_RES
<222> (9)..(9)
<223> AMIDATION

<400> 25

```
Xaa Cys His Xaa Arg Xaa Xaa Xaa Thr
1               5
```

<210> 26
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> Cyclic

<220>
<221> MISC_FEATURE
<222> (4)..(4)
<223> Xaa = D-Phe or D-(Et)Tyr

<220>
<221> MISC_FEATURE
<222> (5)..(5)
<223> Xaa = homo-Arg (hArg)

<220>
<221> MISC_FEATURE
<222> (6)..(6)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MOD_RES
<222> (9)..(9)
<223> AMIDATION

<400> 26

```
Xaa Cys His Xaa Xaa Xaa Xaa Xaa Thr
1               5
```

<210> 27
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa= Nle

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MOD_RES
<222> (8)..(8)
<223> AMIDATION

<400> 27

Xaa Cys His Xaa Arg Trp Xaa Cys
1               5

<210> 28
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (7)..(7)
<223> Xaa = Trp or 3-benzothienylAla (Bal)

<220>
<221> MOD_RES
<222> (8)..(8)
<223> AMIDATION

<400> 28

Xaa Asp Xaa His Xaa Arg Xaa Lys
1               5

<210> 29
<211> 8
<212> PRT

<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (8)..(8)
<223> Xaa = penicillamine (Pen) or Cys

<220>
<221> MOD_RES
<222> (8)..(8)
<223> C-terminus is the free acid

<400> 29

```
Xaa Cys Xaa His Xaa Arg Trp Xaa
1               5
```

<210> 30
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-alpha-aminobutyric acid (D-Abu), D-val, D-Ile, D-Leu, D-tert-Leu (D-Tle) or D-beta-cyclohexylAla (D-Cha)

<220>
<221> MISC_FEATURE
<222> (5)..(5)
<223> Xaa = D-Phe

<220>
<221> MOD_RES
<222> (8)..(8)
<223> AMIDATION

<400> 30

```
Xaa Cys Xaa His Xaa Arg Trp Cys
1               5
```

<210> 31
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (2)..(2)
<223> Xaa = penicillamine (Pen)

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MOD_RES
<222> (8)..(8)

<223> AMIDATION

<400> 31

Xaa Xaa His Xaa Arg Trp Xaa Cys
1 5

<210> 32
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (2)..(2)
<223> Xaa = Cys or penicillamine (Pen)

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (8)..(8)
<223> Xaa = penicillamine (Pen)

<220>
<221> MOD_RES
<222> (8)..(8)
<223> AMIDATION

<400> 32

Xaa Xaa His Xaa Arg Trp Xaa Xaa
1 5

<210> 33
<211> 8

<212> PRT
<213> Artificial Sequence

<220>
<223> Melanocortin receptor ligand to treat insulin sensitivity

<220>
<221> MISC_FEATURE
<222> (1)..(1)
<223> Xaa = Leu, beta-cyclohexylAla (Cha), Ile, Phe, val or beta-(2-naphthyl)Ala (2-Nal), all modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MOD_RES
<222> (8)..(8)
<223> AMIDATION

<400> 33

```
Xaa Cys His Xaa Arg Trp Xaa Cys
1               5
```

<210> 34
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle or Phe

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MOD_RES
<222> (8)..(8)
<223> AMIDATION

<400> 34

```
Xaa Cys His Xaa Arg Trp Xaa Cys
1               5
```

<210> 35
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = beta-(3-pyridiyl)Ala (3-Pal)

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MOD_RES
<222> (8)..(8)
<223> AMIDATION

<400> 35

```
Xaa Cys Xaa Xaa Arg Trp Xaa Cys
1               5
```

<210> 36
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MOD_RES
<222> (8)..(8)
<223> C-terminus is the free acid

<400> 36

```
Xaa Cys Xaa His Xaa Arg Trp Cys
1               5
```

<210> 37
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>

<221> MISC_FEATURE
<222> (6)..(6)
<223> Xaa = D-Trp

<220>
<221> MISC_FEATURE
<222> (7)..(7)
<223> Xaa = 4-aminobutyric acid (Gaba)

<220>
<221> MOD_RES
<222> (8)..(8)
<223> AMIDATION

<400> 37

```
Xaa Cys His Phe Arg Xaa Xaa Cys
1               5
```

<210> 38
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-beta-(2-naphthyl)Ala (D-2-Nal)

<220>
<221> MISC_FEATURE
<222> (7)..(7)
<223> Xaa = Ala or beta-Ala

<220>
<221> MOD_RES
<222> (8)..(8)
<223> AMIDATION

<400> 38

```
Xaa Asp His Xaa Arg Trp Xaa Lys
1               5
```

<210> 39
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-beta-(2-naphthyl)Ala (D-2-Nal)

<220>
<221> MISC_FEATURE
<222> (7)..(7)
<223> Xaa = 4-aminobutyric acid (Gaba) or 6-aminohexanoic acid (Ahx)

<220>
<221> MOD_RES
<222> (8)..(8)
<223> AMIDATION

<400> 39

```
Xaa Cys His Xaa Arg Trp Xaa Cys
1               5
```

<210> 40
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = homo-Phe (hPhe) or beta-cyclohexylAla (Cha), both modified with acyl (Ac)

<220>
<221> DOMAIN
<222> (2)..(8)
<223> Cyclic

<220>

<221> MISC_FEATURE
<222> (4)..(4)
<223> Xaa = D-beta-(2-naphthyl)Ala (D-2-Nal)

<220>
<221> MISC_FEATURE
<222> (7)..(7)
<223> Xaa = 4-aminobutyric acid (Gaba)

<220>
<221> MOD_RES
<222> (8)..(8)
<223> AMIDATION

<400> 40

```
Xaa Asp His Xaa Arg Trp Xaa Lys
1               5
```

<210> 41
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = beta-Ala or 5-aminopentanoic acid (Apn)

<220>
<221> MOD_RES
<222> (8)..(8)
<223> C-terminus is the free acid

<400> 41

```
Xaa Asp His Xaa Arg Trp Xaa Lys
1               5
```

<210> 42
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 6-aminohexanoic acid (Ahx) or 5-aminopentanoic acid (Apn)

<220>
<221> MOD_RES
<222> (8)..(8)
<223> C-terminus is the free acid

<400> 42

```
Xaa Cys His Xaa Arg Trp Xaa Cys
1               5
```

<210> 43
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> Cyclic

<220>
<221> MISC_FEATURE

<222> (4)..(4)
<223> Xaa = D-phe

<220>
<221> MISC_FEATURE
<222> (7)..(7)
<223> Xaa = Ala, beta-Ala or 4-aminobutyric acid (Gaba)

<220>
<221> MISC_FEATURE
<222> (8)..(8)
<223> Xaa = D-Cys

<220>
<221> MOD_RES
<222> (9)..(9)
<223> C-terminus is the free acid

<400> 43

Xaa Cys His Xaa Arg Trp Xaa Xaa Thr
1 5

<210> 44
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = beta-cyclohexylAla (Cha), Nle, cyclohexylGly (chg), D-Cha, homo-cyclohexylAla (hcha), D-Chg or homo-Phe (hPhe), all modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MOD_RES
<222> (8)..(8)

<223> C-terminus is the free acid

<400> 44

Xaa Asp His Xaa Arg Trp Xaa Lys
1               5

<210> 45
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (6)..(6)
<223> Xaa = D-Trp

<220>
<221> MISC_FEATURE
<222> (7)..(7)
<223> Xaa = 4-aminobutyric acid (Gaba), 6-aminohexanoic acid (Ahx), beta-Ala or D-Ala

<220>
<221> MOD_RES
<222> (8)..(8)
<223> C-terminus is the free acid

<400> 45

Xaa Cys His Xaa Arg Xaa Xaa Cys
1               5

<210> 46
<211> 8
<212> PRT
<213> Artificial Sequence

<220>

<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-beta-(2-naphthyl)Ala (D-2-Nal)

<220>
<221> MISC_FEATURE
<222> (7)..(7)
<223> Xaa = Trp, beta-(2-naphthyl)Ala (2-Nal), beta-(1-naphthyl)Ala (1-Nal) or 3-benzothienylalanine (Bal)

<220>
<221> MOD_RES
<222> (8)..(8)
<223> C-terminus is the free acid

<400> 46

```
Xaa Cys Xaa His Xaa Arg Xaa Cys
1               5
```

<210> 47
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE

<222> (2)..(2)
<223> Xaa = penicillamine (Pen)

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MOD_RES
<222> (8)..(8)
<223> C-terminus is the free acid

<400> 47

```
Xaa Xaa Xaa His Xaa Arg Trp Cys
1               5
```

<210> 48
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (8)..(8)
<223> Xaa = penicillamine (Pen)

<220>

<221> MOD_RES
<222> (8)..(8)
<223> C-terminus is the free acid

<400> 48

Xaa Cys His Xaa Arg Trp Xaa Xaa
1 5

<210> 49
<211> 8
<212> PRT
<213> Artificial sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-beta-(2-naphthyl)Ala (D-2-Nal)

<220>
<221> MOD_RES
<222> (8)..(8)
<223> AMIDATION

<400> 49

Arg Cys Xaa His Xaa Arg Trp Cys
1 5

<210> 50
<211> 8
<212> PRT
<213> Artificial Sequence

<220>
<223> Melanocortin receptor ligand to treat insulin sensitivity

<220>

<221> MISC_FEATURE
<222> (1)..(1)
<223> Xaa = Arg or D-Arg, both modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MOD_RES
<222> (8)..(8)
<223> AMIDATION

<400> 50

```
Xaa Cys Xaa His Xaa Arg Trp Cys
1               5
```

<210> 51
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Arg or Arg, both modified with acyl (Ac)

<220>
<221> DOMAIN
<222> (2)..(8)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (8)..(8)
<223> Xaa = penicillamine (Pen)

<220>
<221> MOD_RES
<222> (8)..(8)
<223> AMIDATION

<400> 51

```
Xaa Cys Xaa His Xaa Arg Trp Xaa
1               5
```

<210> 52
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Arg or Arg, both modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (8)..(8)
<223> Xaa = penicillamine (Pen)

<220>
<221> MOD_RES
<222> (8)..(8)
<223> AMIDATION

<400> 52

Xaa Cys His Xaa Arg Trp Xaa Xaa
1               5

<210> 53
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Arg or Arg, both modified with acyl (Ac)

<220>
<221> DOMAIN
<222> (2)..(8)
<223> Cyclic

<220>
<221> MISC_FEATURE
<222> (4)..(4)
<223> Xaa = D-Phe

<220>
<221> MOD_RES
<222> (8)..(8)
<223> AMIDATION

<400> 53

Xaa Asp His Xaa Arg Trp Ala Lys
1               5

<210> 54
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(9)
<223> Cyclic

<220>
<221> MISC_FEATURE

<222> (3)..(3)
<223> Xaa = D-Ala

<220>
<221> MISC_FEATURE
<222> (5)..(5)
<223> Xaa = D-Phe

<220>
<221> MISC_FEATURE
<222> (8)..(8)
<223> Xaa = Gly, D-Ala, beta-Ala, 4-aminobutyric acid (Gaba) or 5-aminopentanoic acid (Apn)

<220>
<221> MOD_RES
<222> (9)..(9)
<223> AMIDATION

<400> 54

```
Xaa Cys Xaa His Xaa Arg Trp Xaa Cys
1               5
```

<210> 55
<211> 8
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> Cyclic

<220>
<221> MISC_FEATURE
<222> (4)..(4)
<223> Xaa = D-Phe

<220>
<221> MISC_FEATURE
<222> (6)..(6)
<223> Xaa = Trp or beta-(2-naphthyl)alanine (2-Nal)

<220>
<221> MOD_RES
<222> (8)..(8)
<223> AMIDATION

<400> 55

Cys Glu His Xaa Arg Xaa Ala Cys
1               5

<210> 56
<211> 8
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> Cyclic

<220>
<221> MISC_FEATURE
<222> (2)..(2)
<223> Xaa = D-Ala

<220>
<221> MISC_FEATURE
<222> (4)..(4)
<223> Xaa = D-Phe

<220>
<221> MISC_FEATURE
<222> (6)..(6)
<223> Xaa = Trp or beta-(2-naphthyl)Ala (2-Nal)

<220>
<221> MOD_RES
<222> (8)..(8)
<223> AMIDATION

<400> 56

Cys Xaa His Xaa Arg Xaa Ala Cys
1               5

<210> 57
<211> 9
<212> PRT
<213> Artificial Sequence

<220>
<223> Melanocortin receptor ligand to treat insulin sensitivity

<220>
<221> MISC_FEATURE

<222> (1)..(1)
<223> Xaa = Nle modified with acyl (Ac)

<220>
<221> DOMAIN
<222> (2)..(9)
<223> Cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (8)..(8)
<223> Xaa = Ala, beta-Ala or 4-aminobutyric acid (Gaba)

<220>
<221> MOD_RES
<222> (9)..(9)
<223> AMIDATION

<400> 57

```
Xaa Cys Xaa His Xaa Arg Trp Xaa Cys
1               5
```

<210> 58
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(9)
<223> Cyclic

<220>
<221> MISC_FEATURE
<222> (3)..(3)
<223> Xaa = D-Ala

<220>

<221> MISC_FEATURE
<222> (5)..(5)
<223> Xaa = D-Phe

<220>
<221> MISC_FEATURE
<222> (7)..(7)
<223> Xaa = 3-benzothienylAla (Bal)

<220>
<221> MOD_RES
<222> (9)..(9)
<223> AMIDATION

<400> 58

```
Xaa Asp Xaa His Xaa Arg Xaa Ala Lys
1               5
```

<210> 59
<211> 8
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> Cyclic

<220>
<221> MISC_FEATURE
<222> (3)..(3)
<223> Xaa = D-Glu

<220>
<221> MISC_FEATURE
<222> (5)..(5)
<223> Xaa = D-Phe

<220>
<221> MOD_RES
<222> (8)..(8)
<223> AMIDATION

<400> 59

```
Xaa Cys Xaa His Xaa Arg Trp Cys
1               5
```

<210> 60
<211> 18
<212> PRT
<213> Artificial sequence

<220>
<223> Melanocortin receptor ligand to treat insulin sensitivity

<220>
<221> MISC_FEATURE
<222> (12)..(12)
<223> Xaa = Nle

<220>
<221> DOMAIN
<222> (13)..(18)
<223> cyclic

<220>
<221> MISC_FEATURE
<222> (15)..(15)
<223> Xaa = D-beta-(2-naphthyl)Ala (D-2-Nal)

<220>
<221> MOD_RES
<222> (18)..(18)
<223> AMIDATION

<400> 60

```
Tyr Gly Arg Lys Lys Arg Arg Gln Arg Arg Arg Xaa Asp His Xaa Arg
1               5                   10                  15

Trp Lys
```

<210> 61
<211> 19
<212> PRT
<213> Artificial Sequence

<220>
<223> Melanocortin receptor ligand to treat insulin sensitivity

<220>
<221> MISC_FEATURE
<222> (12)..(12)
<223> Xaa = 8-amino-3,6-dioxaoctanoic acid (Doc)

<220>
<221> MISC_FEATURE
<222> (13)..(13)
<223> Xaa = Nle

<220>
<221> DOMAIN
<222> (14)..(19)
<223> cyclic

<220>
<221> MISC_FEATURE
<222> (16)..(16)
<223> Xaa = D-beta-(2-naphthyl)Ala (D-2-Nal)

<220>
<221> MOD_RES
<222> (19)..(19)
<223> AMIDATION

<400> 61

```
Tyr Gly Arg Lys Lys Arg Arg Gln Arg Arg Arg Xaa Xaa Asp His Xaa
1               5                   10                  15

Arg Trp Lys
```

<210> 62
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle or Nle modified with acyl (Ac)

<220>
<221> DOMAIN
<222> (2)..(7)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (8)..(8)
<223> Xaa = beta-Ala

<220>
<221> MOD_RES
<222> (19)..(19)
<223> AMIDATION

<400> 62

```
Xaa Asp His Xaa Arg Trp Lys Xaa Tyr Gly Arg Lys Lys Arg Arg Gln
1               5                   10                  15
```

Arg Arg Arg

<210> 63
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(7)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (8)..(9)
<223> Xaa = 8-amino-3,6-dioxaoctanoic acid (Doc)

<220>
<221> MOD_RES
<222> (20)..(20)
<223> AMIDATION

<400> 63

```
Xaa Asp His Xaa Arg Trp Lys Xaa Xaa Tyr Gly Arg Lys Lys Arg Arg
1               5                   10                  15

Gln Arg Arg Arg
            20
```

<210> 64
<211> 22
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-beta-(2-naphthyl)Ala (D-2-Nal)

<220>
<221> MOD_RES
<222> (22)..(22)
<223> AMIDATION

<400> 64

```
Xaa Asp His Xaa Arg Trp Lys Pro Pro Lys Asp Tyr Gly Arg Lys Lys
1               5                   10                  15

Arg Arg Gln Arg Arg Arg
            20
```

<210> 65
<211> 23
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MOD_RES
<222> (23)..(23)
<223> AMIDATION

<400> 65

Cys Glu His Xaa Arg Trp Gly Cys Pro Pro Lys Asp Tyr Gly Arg Lys
1 5 10 15

Lys Arg Arg Gln Arg Arg Arg
20

<210> 66
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (8)..(9)
<223> Xaa = beta-Ala

<220>
<221> MOD_RES
<222> (20)..(20)
<223> AMIDATION

<400> 66

Xaa Asp His Xaa Arg Trp Lys Xaa Xaa Tyr Gly Arg Lys Lys Arg Arg
1 5 10 15

Gln Arg Arg Arg
20

<210> 67
<211> 23
<212> PRT
<213> Artificial Sequence

<220>
<223> Melanocortin receptor ligand to treat insulin sensitivity

<220>

<221> MISC_FEATURE
<222> (1)..(1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (12)..(12)
<223> Xaa = 8-amino-3,6-dioxaoctanoic acid (Doc)

<220>
<221> MOD_RES
<222> (23)..(23)
<223> AMIDATION

<400> 67

```
Xaa Asp His Xaa Arg Trp Lys Pro Pro Lys Asp Xaa Tyr Gly Arg Lys
1               5                   10                  15

Lys Arg Arg Gln Arg Arg Arg
            20
```

<210> 68
<211> 24
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE

<222> (13)..(13)
<223> Xaa = 8-amino-3,6-dioxaoctanoic acid (Doc)

<220>
<221> MOD_RES
<222> (24)..(24)
<223> AMIDATION

<400> 68

```
Cys Glu His Xaa Arg Trp Gly Cys Pro Pro Lys Asp Xaa Tyr Gly Arg
1               5                   10                  15

Lys Lys Arg Arg Gln Arg Arg Arg
            20
```

<210> 69
<211> 24
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala or 8-amino-3,6-dioxaoctanoic acid (Doc)

<220>
<221> MOD_RES
<222> (24)..(24)
<223> AMIDATION

<400> 69

```
Cys Glu His Xaa Arg Trp Ala Cys Pro Pro Lys Asp Xaa Tyr Gly Arg
1               5                   10                  15

Lys Lys Arg Arg Gln Arg Arg Arg
            20
```

<210> 70
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (8)..(9)
<223> Xaa = 8-amino-3,6-dioxaoctanoic acid (Doc)

<220>
<221> MOD_RES
<222> (20)..(20)
<223> AMIDATION

<400> 70

```
Xaa Asp His Xaa Arg Trp Lys Xaa Xaa Tyr Gly Arg Lys Lys Arg Arg
1               5                   10                  15

Gln Arg Arg Arg
            20
```

<210> 71
<211> 24
<212> PRT
<213> Artificial sequence

<220>
<223> Melanocortin receptor ligand to treat insulin sensitivity

<220>
<221> DOMAIN
<222> (1)..(8)
<223> cyclic

<220>
<221> MOD_RES

<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal), beta-(1-naphthyl)Ala (1-Nal) or 3-benzothienylAla (Bal)

<220>
<221> MISC_FEATURE
<222> (13)..(13)
<223> Xaa = beta-Ala

<220>
<221> MOD_RES
<222> (24)..(24)
<223> AMIDATION

<400> 71

```
Cys Glu His Xaa Arg Xaa Ala Cys Pro Pro Lys Asp Xaa Tyr Gly Arg
1               5                   10                  15

Lys Lys Arg Arg Gln Arg Arg Arg
            20
```

<210> 72
<211> 22
<212> PRT
<213> Artificial sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)

<223> Xaa = 3-benzothienylAla (Bal)

<220>
<221> MISC_FEATURE
<222> (13)..(13)
<223> Xaa = beta-Ala

<220>
<221> MOD_RES
<222> (22)..(22)
<223> AMIDATION

<400> 72

```
Cys Glu His Xaa Arg Xaa Ala Cys Pro Pro Lys Asp Xaa Arg Arg Arg
1               5                   10                  15

Arg Arg Gln Arg Arg Arg
            20
```

<210> 73
<211> 23
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (13)..(13)
<223> Xaa = beta-Ala

<220>
<221> MOD_RES
<222> (23)..(23)
<223> AMIDATION

<400> 73

Cys Glu His Xaa Arg Xaa Ala Cys Pro Pro Lys Asp Xaa Gly Arg Arg
1 5 10 15

Arg Arg Arg Gln Arg Arg Arg
20

<210> 74
<211> 24
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (13)..(13)
<223> Xaa = beta-Ala

<220>
<221> MOD_RES
<222> (24)..(24)
<223> AMIDATION

<400> 74

Cys Glu His Xaa Arg Xaa Ala Cys Pro Pro Lys Asp Xaa Tyr Gly Arg
1 5 10 15

Arg Arg Arg Arg Gln Arg Arg Arg
20

<210> 75
<211> 24

<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 75

```
Cys Glu His Xaa Arg Trp Ala Cys Pro Pro Lys Asp Xaa Tyr Gly Arg
1               5                   10                  15

Lys Lys Arg Gln Arg Arg Arg Arg
            20
```

<210> 76
<211> 24
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 76

```
Cys Glu His Xaa Arg Trp Ala Cys Pro Pro Lys Asp Xaa Tyr Gly Arg
1               5                   10                  15

Lys Lys Gln Arg Arg Arg Arg Arg
            20
```

<210> 77
<211> 24
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 77

Cys Glu His Xaa Arg Trp Ala Cys Pro Pro Lys Asp Xaa Tyr Gly Arg
1 5 10 15

Lys Gln Lys Arg Arg Arg Arg Arg
20

<210> 78
<211> 24
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 78

Cys Glu His Xaa Arg Trp Ala Cys Pro Pro Lys Asp Xaa Tyr Gly Arg
1 5 10 15

Lys Lys Arg Arg Arg Arg Gln Arg
20

<210> 79
<211> 24
<212> PRT
<213> Artificial Sequence

<220>
<223> Melanocortin receptor ligand to treat insulin sensitivity

<220>

<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (13)..(13)
<223> Xaa = beta-Ala

<220>
<221> MISC_FEATURE
<222> (15)..(15)
<223> Xaa = alpha-aminoisobutyric acid (Aib)

<220>
<221> MOD_RES
<222> (24)..(24)
<223> AMIDATION

<400> 79

```
Cys Glu His Xaa Arg Xaa Ala Cys Pro Pro Lys Asp Xaa Tyr Xaa Arg
1               5                   10                  15

Lys Lys Arg Arg Gln Arg Arg Arg
            20
```

<210> 80
<211> 22
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES

<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> xaa = beta-(1-naphthyl)Ala (1-Nal)

<220>
<221> MISC_FEATURE
<222> (11)..(11)
<223> Xaa = Arg or Lys

<220>
<221> MISC_FEATURE
<222> (13)..(13)
<223> Xaa = beta-Ala

<220>
<221> MOD_RES
<222> (22)..(22)
<223> AMIDATION

<400> 80

```
Cys Glu His Xaa Arg Xaa Ala Cys Pro Pro Xaa Asp Xaa Arg Arg Arg
1               5                   10                  15

Arg Arg Gln Arg Arg Arg
            20
```

<210> 81
<211> 23
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)

<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal) or beta-(2-naphthyl)Ala (2-Nal)

<220>
<221> MISC_FEATURE
<222> (13)..(13)
<223> Xaa = beta-Ala

<220>
<221> MOD_RES
<222> (23)..(23)
<223> AMIDATION

<400> 81

```
Cys Glu His Xaa Arg Xaa Ala Cys Pro Pro Lys Asp Xaa Arg Arg Arg
1               5                   10                  15

Arg Arg Arg Gln Arg Arg Arg
            20
```

<210> 82
<211> 22
<212> PRT
<213> Artificial sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (11)..(11)
<223> Xaa = Arg or Lys

<220>
<221> MISC_FEATURE
<222> (13)..(13)
<223> Xaa = beta-Ala

<220>
<221> MOD_RES
<222> (22)..(22)
<223> AMIDATION

<400> 82

Cys Glu His Xaa Arg Xaa Ala Cys Pro Pro Xaa Asp Xaa Arg Arg Arg
1 5 10 15

Arg Arg Gln Arg Arg Arg
20

<210> 83
<211> 23
<212> PRT
<213> Artificial sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (11)..(11)
<223> Xaa = Arg or Lys

<220>
<221> MISC_FEATURE
<222> (13)..(13)
<223> Xaa = beta-Ala

<220>

<221> MOD_RES
<222> (23)..(23)
<223> AMIDATION

<400> 83

```
Cys Glu His Xaa Arg Xaa Ala Cys Pro Pro Xaa Asp Xaa Arg Arg Arg
1               5                   10                  15

Arg Arg Arg Gln Arg Arg Arg
            20
```

<210> 84
<211> 22
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (13)..(13)
<223> Xaa = beta-Ala

<220>
<221> MOD_RES
<222> (22)..(22)
<223> AMIDATION

<400> 84

Cys Glu His Xaa Arg Xaa Ala Cys Pro Pro Arg Asp Xaa Arg Arg Arg
1 5 10 15

Arg Arg Gln Arg Arg Arg
20

<210> 85
<211> 24
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 85

Cys Glu His Xaa Arg Trp Ala Cys Pro Pro Lys Asp Xaa Tyr Gly Arg
1 5 10 15

Lys Lys Arg Arg Arg Gln Arg Arg
20

<210> 86
<211> 24
<212> PRT
<213> Artificial Sequence

<220>
<223> Melanocortin receptor ligand to treat insulin sensitivity

<220>

<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 86

```
Cys Glu His Xaa Arg Trp Ala Cys Pro Pro Lys Asp Xaa Tyr Gly Arg
1               5                   10                  15

Gln Lys Lys Arg Arg Arg Arg Arg
            20
```

<210> 87
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE

<222> (13)..(13)
<223> xaa = beta-Ala

<220>
<221> MOD_RES
<222> (24)..(24)
<223> AMIDATION

<400> 87

Cys Glu His Xaa Arg Trp Ala Cys Pro Pro Lys Asp Xaa Tyr Gly Arg
1               5                   10                  15

Lys Lys Arg Arg Arg Arg Arg Gln
            20

<210> 88
<211> 22
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal) or beta-(2-naphthyl)Ala (2-Nal)

<220>
<221> MISC_FEATURE
<222> (13)..(13)
<223> xaa = beta-Ala

<220>
<221> MOD_RES
<222> (22)..(22)
<223> AMIDATION

<400> 88

Cys Glu His Xaa Arg Xaa Ala Cys Pro Pro Lys Asp Xaa Arg Arg Lys
1 5 10 15

Arg Arg Gln Arg Arg Arg
20

<210> 89
<211> 22
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (13)..(13)
<223> Xaa = beta-Ala

<220>
<221> MOD_RES
<222> (22)..(22)
<223> AMIDATION

<400> 89

Cys Glu His Xaa Arg Xaa Ala Cys Pro Pro Lys Asp Xaa Arg Lys Arg
1 5 10 15

Arg Arg Gln Arg Arg Arg
20

<210> 90
<211> 24
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (13)..(13)
<223> Xaa = beta-Ala

<220>
<221> MOD_RES
<222> (24)..(24)
<223> AMIDATION

<400> 90

```
Cys Glu His Xaa Arg Xaa Ala Cys Pro Pro Lys Asp Xaa Tyr Gly Arg
1               5                   10                  15

Arg Lys Arg Arg Gln Arg Arg Arg
            20
```

<210> 91
<211> 23
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)

<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (13)..(13)
<223> Xaa = beta-Ala

<220>
<221> MOD_RES
<222> (23)..(23)
<223> AMIDATION

<400> 91

Cys Glu His Xaa Arg Xaa Ala Cys Pro Pro Lys Asp Xaa Gly Arg Arg
1 5 10 15

Lys Arg Arg Gln Arg Arg Arg
20

<210> 92
<211> 23
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE

<222> (6)..(6)
<223> Xaa = beta-(2-naphthyl)Ala (2-Nal) or beta-(1-naphthyl)Ala (1-Nal)

<220>
<221> MISC_FEATURE
<222> (13)..(13)
<223> Xaa = beta-Ala

<220>
<221> MOD_RES
<222> (23)..(23)
<223> AMIDATION

<400> 92

```
Cys Glu His Xaa Arg Xaa Ala Cys Pro Pro Lys Asp Xaa Gly Arg Lys
1               5                   10                  15

Arg Arg Arg Gln Arg Arg Arg
            20
```

<210> 93
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (13)..(13)
<223> Xaa = beta-Ala

<220>
<221> MOD_RES
<222> (22)..(22)

<223> AMIDATION

<400> 93

Cys Glu His Xaa Arg Xaa Ala Cys Pro Pro Lys Asp Xaa Arg Arg Lys
1 5 10 15

Arg Arg Gln Arg Arg Arg
20

<210> 94
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (13)..(13)
<223> Xaa = beta-Ala

<220>
<221> MOD_RES
<222> (22)..(22)
<223> AMIDATION

<400> 94

Cys Glu His Xaa Arg Xaa Ala Cys Pro Pro Lys Asp Xaa Arg Lys Arg
1 5 10 15

Arg Arg Gln Arg Arg Arg
20

<210> 95
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (13)..(13)
<223> Xaa = beta-Ala

<220>
<221> MOD_RES
<222> (24)..(24)
<223> AMIDATION

<400> 95

```
Cys Glu His Xaa Arg Xaa Ala Cys Pro Pro Lys Asp Xaa Tyr Gly Arg
1               5                   10                  15

Arg Lys Arg Arg Gln Arg Arg Arg
            20
```

<210> 96
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (13)..(13)
<223> Xaa = beta-Ala

<220>
<221> MOD_RES
<222> (24)..(24)
<223> AMIDATION

<400> 96

```
Cys Glu His Xaa Arg Xaa Ala Cys Pro Pro Lys Asp Xaa Tyr Gly Arg
1               5                   10                  15

Lys Arg Arg Arg Gln Arg Arg Arg
            20
```

<210> 97
<211> 23
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>

<221> MISC_FEATURE
<222> (6)..(6)
<223> Xaa = beta-(1-naphthyl)Ala (1-Nal)

<220>
<221> MISC_FEATURE
<222> (13)..(13)
<223> Xaa = beta-Ala

<220>
<221> MOD_RES
<222> (23)..(23)
<223> AMIDATION

<400> 97

```
Cys Glu His Xaa Arg Xaa Ala Cys Pro Pro Lys Asp Xaa Gly Arg Arg
1               5                   10                  15

Lys Arg Arg Gln Arg Arg Arg
            20
```

<210> 98
<211> 22
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (13)..(13)
<223> Xaa = beta-Ala

<220>
<221> MOD_RES

<222> (22)..(22)
<223> AMIDATION

<400> 98

Cys Glu His Xaa Arg Xaa Ala Cys Pro Pro Lys Asp Xaa Arg Arg Lys
1 5 10 15

Arg Arg Gln Arg Arg Arg
20

<210> 99
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (13)..(13)
<223> Xaa = beta-Ala

<220>
<221> MOD_RES
<222> (22)..(22)
<223> AMIDATION

<400> 99

Cys Glu His Xaa Arg Xaa Ala Cys Pro Pro Lys Asp Xaa Arg Lys Arg
1               5                   10                  15

Arg Arg Gln Arg Arg Arg
            20

<210> 100
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal) or 3-benzothienylAla (Bal)

<220>
<221> MISC_FEATURE
<222> (13)..(13)
<223> Xaa = beta-Ala

<220>
<221> MOD_RES
<222> (24)..(24)
<223> AMIDATION

<400> 100

Cys Glu His Xaa Arg Xaa Ala Cys Pro Pro Lys Asp Xaa Tyr Gly Arg
1               5                   10                  15

Arg Lys Arg Arg Gln Arg Arg Arg
            20

<210> 101
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal) or 3-benzothienylAla (Bal)

<220>
<221> MISC_FEATURE
<222> (13)..(13)
<223> Xaa = beta-Ala

<220>
<221> MOD_RES
<222> (24)..(24)
<223> AMIDATION

<400> 101

```
Cys Glu His Xaa Arg Xaa Ala Cys Pro Pro Lys Asp Xaa Tyr Gly Arg
1               5                   10                  15

Lys Arg Arg Arg Gln Arg Arg Arg
            20
```

<210> 102
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (AC)

<220>
<221> DOMAIN
<222> (1)..(8)

<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal) or 3-benzothienylAla (Bal)

<220>
<221> MISC_FEATURE
<222> (13)..(13)
<223> Xaa = beta-Ala

<220>
<221> MOD_RES
<222> (23)..(23)
<223> AMIDATION

<400> 102

```
Cys Glu His Xaa Arg Xaa Ala Cys Pro Pro Lys Asp Xaa Gly Arg Arg
1               5                   10                  15

Lys Arg Arg Gln Arg Arg Arg
            20
```

<210> 103
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (AC)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal) or 3-benzothienylAla (Bal)

<220>
<221> MISC_FEATURE
<222> (13)..(13)
<223> Xaa = beta-Ala

<220>
<221> MOD_RES
<222> (23)..(23)
<223> AMIDATION

<400> 103

```
Cys Glu His Xaa Arg Xaa Ala Cys Pro Pro Lys Asp Xaa Gly Arg Lys
1               5                   10                  15

Arg Arg Arg Gln Arg Arg Arg
            20
```

<210> 104
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (AC)

<220>
<221> DOMAIN
<222> (1)..(8)
<223> cyclic

<220>
<221> MISC_FEATURE
<222> (4)..(4)
<223> xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (13)..(13)
<223> Xaa = beta-Ala

<220>
<221> MOD_RES
<222> (22)..(22)
<223> AMIDATION

<400> 104

Cys Glu His Xaa Arg Xaa Ala Cys Pro Pro Lys Asp Xaa Arg Arg Lys
1 5 10 15

Arg Arg Gln Arg Arg Arg
20

<210> 105
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (13)..(13)
<223> Xaa = beta-Ala

<220>
<221> MOD_RES
<222> (22)..(22)
<223> AMIDATION

<400> 105

Cys Glu His Xaa Arg Xaa Ala Cys Pro Pro Lys Asp Xaa Arg Lys Arg
1 5 10 15

Arg Arg Gln Arg Arg Arg
20

<210> 106
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 106

```
Cys Glu His Xaa Arg Trp Ala Cys Pro Pro Lys Asp Xaa Tyr Gly Arg
1               5                   10                  15

Lys Lys Arg Gln Arg Arg Arg Arg
            20
```

<210> 107
<211> 24
<212> PRT
<213> Artificial sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)

<223> xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 107

```
Cys Glu His Xaa Arg Trp Ala Cys Pro Pro Lys Asp Xaa Tyr Gly Arg
1               5                   10                  15

Lys Lys Gln Arg Arg Arg Arg Arg
            20
```

<210> 108
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 108

Cys Glu His Xaa Arg Trp Ala Cys Pro Pro Lys Asp Xaa Tyr Gly Arg
1 5 10 15

Lys Gln Lys Arg Arg Arg Arg Arg
20

<210> 109
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 109

Cys Glu His Xaa Arg Trp Ala Cys Pro Pro Lys Asp Xaa Tyr Gly Arg
1 5 10 15

Gln Lys Lys Arg Arg Arg Arg Arg
20

<210> 110
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 110

```
Cys Glu His Xaa Arg Trp Ala Cys Pro Pro Lys Asp Xaa Tyr Gly Arg
1               5                   10                  15

Lys Lys Arg Arg Arg Gln Arg Arg
            20
```

<210> 111
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>

<221> MISC_FEATURE
<222> (13)..(13)
<223> Xaa = beta-Ala

<220>
<221> MOD_RES
<222> (24)..(24)
<223> AMIDATION

<400> 111

```
Cys Glu His Xaa Arg Trp Ala Cys Pro Pro Lys Asp Xaa Tyr Gly Arg
1               5                   10                  15

Lys Lys Arg Arg Arg Arg Gln Arg
            20
```

<210> 112
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (13)..(13)
<223> Xaa = beta-Ala

<220>
<221> MOD_RES
<222> (24)..(24)
<223> AMIDATION

<400> 112

Cys Glu His Xaa Arg Trp Ala Cys Pro Pro Lys Asp Xaa Tyr Gly Arg
1 5 10 15

Lys Lys Arg Arg Arg Arg Arg Gln
20

<210> 113
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (22)..(22)
<223> AMIDATION

<400> 113

Cys Glu His Xaa Arg Trp Ala Cys Pro Pro Xaa Asp Xaa Arg Arg Arg
1 5 10 15

Arg Arg Gln Arg Arg Arg
20

<210> 114
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (24)..(24)
<223> AMIDATION

<400> 114

```
Cys Glu His Xaa Arg Trp Ala Cys Pro Pro Xaa Asp Xaa Tyr Gly Arg
1               5                   10                  15

Arg Arg Arg Arg Gln Arg Arg Arg
            20
```

<210> 115
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>

<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (23)..(23)
<223> AMIDATION

<400> 115

```
Cys Glu His Xaa Arg Trp Ala Cys Pro Pro Xaa Asp Xaa Arg Arg Arg
1               5                   10                  15

Arg Arg Gln Arg Arg Arg Arg
            20
```

<210> 116
<211> 25
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE

<222> (11)..(11)
<223> Xaa = Lys or Arg

<220>
<221> MISC_FEATURE
<222> (13)..(13)
<223> Xaa = beta-Ala

<220>
<221> MOD_RES
<222> (25)..(25)
<223> AMIDATION

<400> 116

```
Cys Glu His Xaa Arg Trp Ala Cys Pro Pro Xaa Asp Xaa Tyr Gly Arg
1               5                   10                  15

Arg Arg Arg Arg Gln Arg Arg Arg Arg
            20                  25
```

<210> 117
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> xaa = beta-(1-naphthyl)Ala (1-Nal)

<220>
<221> MISC_FEATURE
<222> (11)..(11)
<223> Xaa = Lys or Arg

<220>
<221> MISC_FEATURE
<222> (13)..(13)

<223> Xaa = beta-Ala

<220>
<221> MOD_RES
<222> (22)..(22)
<223> AMIDATION

<400> 117

```
Cys Glu His Xaa Arg Xaa Ala Cys Pro Pro Xaa Asp Xaa Arg Arg Arg
1               5                   10                  15

Arg Arg Gln Arg Arg Arg
            20
```

<210> 118
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (23)..(23)
<223> AMIDATION

<400> 118

Cys Glu His Xaa Arg Xaa Ala Cys Pro Pro Xaa Asp Xaa Arg Arg Arg
1 5 10 15

Arg Arg Gln Arg Arg Arg Arg
20

<210> 119
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (23)..(23)
<223> AMIDATION

<400> 119

Cys Glu His Xaa Arg Xaa Ala Cys Pro Pro Xaa Asp Xaa Arg Arg Arg
1               5                   10                  15

Arg Arg Arg Gln Arg Arg Arg
            20

<210> 120
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (24)..(24)
<223> AMIDATION

<400> 120

Cys Glu His Xaa Arg Xaa Ala Cys Pro Pro Xaa Asp Xaa Tyr Gly Arg
1               5                   10                  15

Arg Arg Arg Arg Gln Arg Arg Arg
            20

<210> 121
<211> 25
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (25)..(25)
<223> AMIDATION

<400> 121

```
Cys Glu His Xaa Arg Xaa Ala Cys Pro Pro Xaa Asp Xaa Tyr Gly Arg
1               5                   10                  15

Arg Arg Arg Arg Gln Arg Arg Arg Arg
            20                  25
```

<210> 122
<211> 25
<212> PRT
<213> Artificial Sequence

<220>

<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(1-naphthyl)Ala (1-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (25)..(25)
<223> AMIDATION

<400> 122

```
Cys Glu His Xaa Arg Xaa Ala Cys Pro Pro Xaa Asp Xaa Tyr Gly Arg
1               5                   10                  15

Arg Arg Arg Arg Arg Gln Arg Arg Arg
            20                  25
```

<210> 123
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (22)..(22)
<223> AMIDATION

<400> 123

Cys Glu His Xaa Arg Xaa Ala Cys Pro Pro Xaa Asp Xaa Arg Arg Arg
1 5 10 15

Arg Arg Gln Arg Arg Arg
20

<210> 124
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (23)..(23)
<223> AMIDATION

<400> 124

```
Cys Glu His Xaa Arg Xaa Ala Cys Pro Pro Xaa Asp Xaa Arg Arg Arg
1               5                   10                  15

Arg Arg Arg Gln Arg Arg Arg
            20
```

<210> 125
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>

<221> MISC_FEATURE
<222> (6)..(6)
<223> Xaa = beta-(2-naphthyl)Ala (2-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (23)..(23)
<223> AMIDATION

<400> 125

```
Cys Glu His Xaa Arg Xaa Ala Cys Pro Pro Xaa Asp Xaa Arg Arg Arg
1               5                   10                  15

Arg Arg Gln Arg Arg Arg Arg
            20
```

<210> 126
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE

<222> (11)..(11)
<223> Xaa = Lys or Arg

<220>
<221> MISC_FEATURE
<222> (13)..(13)
<223> Xaa = beta-Ala

<220>
<221> MOD_RES
<222> (24)..(24)
<223> AMIDATION

<400> 126

```
Cys Glu His Xaa Arg Xaa Ala Cys Pro Pro Xaa Asp Xaa Tyr Gly Arg
1               5                   10                  15

Arg Arg Arg Arg Gln Arg Arg Arg
            20
```

<210> 127
<211> 25
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (11)..(11)
<223> Xaa = Lys or Arg

<220>
<221> MISC_FEATURE
<222> (13)..(13)

<223> Xaa = beta-Ala

<220>
<221> MOD_RES
<222> (25)..(25)
<223> AMIDATION

<400> 127

```
Cys Glu His Xaa Arg Xaa Ala Cys Pro Pro Xaa Asp Xaa Tyr Gly Arg
1               5                   10                  15

Arg Arg Arg Arg Arg Gln Arg Arg Arg
            20                  25
```

<210> 128
<211> 25
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (25)..(25)
<223> AMIDATION

<400> 128

Cys Glu His Xaa Arg Xaa Ala Cys Pro Pro Xaa Asp Xaa Tyr Gly Arg
1               5                   10                  15

Arg Arg Arg Arg Gln Arg Arg Arg Arg
            20                  25

<210> 129
<211> 22
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (22)..(22)
<223> AMIDATION

<400> 129

Cys Glu His Xaa Arg Xaa Ala Cys Pro Pro Xaa Asp Xaa Arg Arg Arg
1 5 10 15

Arg Arg Gln Arg Arg Arg
20

<210> 130
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (23)..(23)
<223> AMIDATION

<400> 130

Cys Glu His Xaa Arg Xaa Ala Cys Pro Pro Xaa Asp Xaa Arg Arg Arg
1 5 10 15

Arg Arg Gln Arg Arg Arg Arg
20

<210> 131
<211> 23
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (11)..(11)
<223> Xaa = Lys or Arg

<220>
<221> MISC_FEATURE
<222> (13)..(13)
<223> xaa = beta-Ala

<220>
<221> MOD_RES
<222> (23)..(23)
<223> AMIDATION

<400> 131

```
Cys Glu His Xaa Arg Xaa Ala Cys Pro Pro Xaa Asp Xaa Arg Arg Arg
1               5                   10                  15

Arg Arg Arg Gln Arg Arg Arg
            20
```

<210> 132
<211> 23
<212> PRT
<213> Artificial Sequence

<220>

<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (13)..(13)
<223> Xaa = beta-Ala

<220>
<221> MOD_RES
<222> (23)..(23)
<223> AMIDATION

<400> 132

```
Cys Glu His Xaa Arg Xaa Ala Cys Pro Pro Lys Asp Xaa Gly Arg Arg
1               5                   10                  15

Arg Arg Arg Gln Arg Arg Arg
            20
```

<210> 133
<211> 24
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (24)..(24)
<223> AMIDATION

<400> 133

```
Cys Glu His Xaa Arg Xaa Ala Cys Pro Pro Xaa Asp Xaa Tyr Gly Arg
1               5                   10                  15

Arg Arg Arg Arg Gln Arg Arg Arg
            20
```

<210> 134
<211> 25
<212> PRT
<213> Artificial Sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>

<221> MISC_FEATURE
<222> (6)..(6)
<223> Xaa = 3-benzothienylAla (Bal)

<220>
<221> MISC_FEATURE
<222> (11)..(11)
<223> Xaa = Lys or Arg

<220>
<221> MISC_FEATURE
<222> (13)..(13)
<223> Xaa = beta-Ala

<220>
<221> MOD_RES
<222> (25)..(25)
<223> AMIDATION

<400> 134

```
Cys Glu His Xaa Arg Xaa Ala Cys Pro Pro Xaa Asp Xaa Tyr Gly Arg
1               5                   10                  15

Arg Arg Arg Arg Gln Arg Arg Arg Arg
            20                  25
```

<210> 135
<211> 25
<212> PRT
<213> Artificial sequence

<220>
<223> Melanocortin receptor ligand to treat insulin sensitivity

<220>
<221> MOD_RES
<222> (1)..(1)
<223> modified with acyl (Ac)

<220>
<221> DOMAIN
<222> (1)..(8)
<223> cyclic

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = 3-benzothienylAla (Bal)

<220>
<221> MISC_FEATURE

<222> (11)..(11)
<223> Xaa = Lys or Arg

<220>
<221> MISC_FEATURE
<222> (13)..(13)
<223> Xaa = beta-Ala

<220>
<221> MOD_RES
<222> (25)..(25)
<223> AMIDATION

<400> 135

```
Cys Glu His Xaa Arg Xaa Ala Cys Pro Pro Xaa Asp Xaa Tyr Gly Arg
1               5                   10                  15

Arg Arg Arg Arg Arg Gln Arg Arg Arg
            20                  25
```

<210> 136
<211> 21
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)

<223> Xaa = 8-amino-3,6-dioxaoctanoic acid (Doc) or absent

<220>
<221> MOD_RES
<222> (21)..(21)
<223> AMIDATION

<400> 136

```
Xaa Cys Xaa His Xaa Arg Trp Cys Xaa Xaa Tyr Gly Arg Lys Lys Arg
1               5                   10                  15

Arg Gln Arg Arg Arg
            20
```

<210> 137
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MOD_RES
<222> (20)..(20)
<223> AMIDATION

<400> 137

Xaa Cys Xaa His Xaa Arg Trp Cys Xaa Tyr Gly Arg Lys Lys Arg Gln
1 5 10 15

Arg Arg Arg Arg
20

<210> 138
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = Gly or absent

<220>
<221> MOD_RES
<222> (19)..(19)
<223> AMIDATION

<400> 138

Xaa Cys Xaa His Xaa Arg Trp Cys Xaa Xaa Arg Arg Arg Arg Arg Gln
1 5 10 15

Arg Arg Arg

<210> 139
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = Tyr or absent

<220>
<221> MOD_RES
<222> (20)..(20)
<223> AMIDATION

<400> 139

```
Xaa Cys Xaa His Xaa Arg Trp Cys Xaa Xaa Gly Arg Arg Arg Arg Arg
1               5                   10                  15

Gln Arg Arg Arg
            20
```

<210> 140
<211> 20
<212> PRT
<213> Artificial Sequence

<220>

<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MOD_RES
<222> (20)..(20)
<223> AMIDATION

<400> 140

```
Xaa Cys Xaa His Xaa Arg Trp Cys Xaa Gly Arg Arg Arg Arg Arg Gln
1               5                   10                  15

Arg Arg Arg Arg
            20
```

<210> 141
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = Tyr or absent

<220>
<221> MOD_RES
<222> (20)..(20)
<223> AMIDATION

<400> 141

```
Xaa Cys Xaa His Xaa Arg Trp Cys Xaa Xaa Gly Arg Arg Lys Arg Arg
1               5                   10                  15

Gln Arg Arg Arg
            20
```

<210> 142
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>

<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = Tyr or absent

<220>
<221> MOD_RES
<222> (20)..(20)
<223> AMIDATION

<400> 142

```
Xaa Cys Xaa His Xaa Arg Trp Cys Xaa Xaa Gly Arg Lys Arg Arg Arg
1               5                   10                  15

Gln Arg Arg Arg
            20
```

<210> 143
<211> 18
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE

<222> (9)..(9)
<223> Xaa = beta-Ala

<220>
<221> MOD_RES
<222> (18)..(18)
<223> AMIDATION

<400> 143

```
Xaa Cys Xaa His Xaa Arg Trp Cys Xaa Arg Arg Lys Arg Arg Gln Arg
1               5                   10                  15

Arg Arg
```

<210> 144
<211> 18
<212> PRT
<213> Artificial Sequence

<220>
<223> Melanocortin receptor ligand to treat insulin sensitivity

<220>
<221> MISC_FEATURE
<222> (1)..(1)
<223> xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MOD_RES
<222> (18)..(18)
<223> AMIDATION

<400> 144

Xaa Cys Xaa His Xaa Arg Trp Cys Xaa Arg Lys Arg Arg Arg Gln Arg
1 5 10 15

Arg Arg

<210> 145
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> xaa = beta-Ala

<220>
<221> MOD_RES
<222> (20)..(20)
<223> AMIDATION

<400> 145

Xaa Cys Xaa His Xaa Arg Trp Cys Xaa Tyr Gly Arg Lys Lys Arg Gln
1 5 10 15

Arg Arg Arg Arg
20

<210> 146
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MOD_RES
<222> (20)..(20)
<223> AMIDATION

<400> 146

```
Xaa Cys Xaa His Xaa Arg Trp Cys Xaa Tyr Gly Arg Lys Lys Arg Arg
1               5                   10                  15

Gln Arg Arg Arg
            20
```

<210> 147
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)

<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = Tyr or absent

<220>
<221> MOD_RES
<222> (20)..(20)
<223> AMIDATION

<400> 147

```
Xaa Cys Xaa His Xaa Arg Trp Cys Xaa Xaa Gly Arg Arg Arg Arg Arg
1               5                   10                  15

Gln Arg Arg Arg
            20
```

<210> 148
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (19)..(19)
<223> AMIDATION

<400> 148

```
Xaa Cys Xaa His Xaa Arg Trp Cys Xaa Xaa Arg Arg Arg Arg Arg Gln
1               5                   10                  15

Arg Arg Arg
```

<210> 149
<211> 21
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE

<222> (9)..(10)
<223> Xaa = beta-Ala

<220>
<221> MISC_FEATURE
<222> (11)..(11)
<223> Xaa = Tyr or absent

<220>
<221> MOD_RES
<222> (21)..(21)
<223> AMIDATION

<400> 149

```
Xaa Cys Xaa His Xaa Arg Trp Cys Xaa Xaa Xaa Gly Arg Arg Arg Arg
1               5                   10                  15

Arg Gln Arg Arg Arg
            20
```

<210> 150
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)

<223> Xaa = Tyr or absent

<220>
<221> MOD_RES
<222> (20)..(20)
<223> AMIDATION

<400> 150

Xaa Cys Xaa His Xaa Arg Trp Cys Xaa Xaa Gly Arg Arg Arg Arg Arg
1 5 10 15

Gln Arg Arg Arg
20

<210> 151
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (19)..(19)
<223> AMIDATION

<400> 151

Xaa Cys Xaa His Xaa Arg Trp Cys Xaa Xaa Arg Arg Arg Arg Arg Gln
1 5 10 15

Arg Arg Arg

<210> 152
<211> 21
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(10)
<223> Xaa = 8-amino-3,6-dioxaoctanoic acid (Doc)

<220>
<221> MISC_FEATURE
<222> (11)..(11)
<223> Xaa = Tyr or absent

<220>
<221> MOD_RES
<222> (21)..(21)
<223> AMIDATION

<400> 152

Xaa Cys Xaa His Xaa Arg Trp Cys Xaa Xaa Xaa Gly Arg Arg Arg Arg
1 5 10 15

Arg Gln Arg Arg Arg
20

<210> 153
<211> 21
<212> PRT
<213> Artificial sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = Tyr or absent

<220>
<221> MOD_RES
<222> (21)..(21)
<223> AMIDATION

<400> 153

Xaa Cys Xaa His Xaa Arg Trp Cys Xaa Xaa Gly Arg Arg Arg Arg Arg
1 5 10 15

Gln Arg Arg Arg Arg
20

<210> 154
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (20)..(20)
<223> AMIDATION

<400> 154

```
Xaa Cys Xaa His Xaa Arg Trp Cys Xaa Xaa Arg Arg Arg Arg Arg Gln
1               5                   10                  15

Arg Arg Arg Arg
            20
```

<210> 155
<211> 22
<212> PRT
<213> Artificial Sequence

<220>

<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (9)..(10)
<223> Xaa = beta-Ala

<220>
<221> MISC_FEATURE
<222> (11)..(11)
<223> Xaa = Tyr or absent

<220>
<221> MOD_RES
<222> (22)..(22)
<223> AMIDATION

<400> 155

```
Xaa Cys Xaa His Xaa Arg Trp Cys Xaa Xaa Xaa Gly Arg Arg Arg Arg
1               5                   10                  15

Arg Gln Arg Arg Arg Arg
            20
```

<210> 156
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (AC)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5) .. (5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = Tyr or absent

<220>
<221> MOD_RES
<222> (21)..(21)
<223> AMIDATION

<400> 156

```
Xaa Cys Xaa His Xaa Arg Trp Cys Xaa Xaa Gly Arg Arg Arg Arg Arg
1               5                   10                  15

Gln Arg Arg Arg Arg
            20
```

<210> 157
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2) .. (8)
<223> cyclic

<220>

<221> MISC_FEATURE
<222> (3)..(3)
<223> Xaa = D-Ala

<220>
<221> MISC_FEATURE
<222> (5) .. (5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (20)..(20)
<223> AMIDATION

<400> 157

```
Xaa Cys Xaa His Xaa Arg Trp Cys Xaa Xaa Arg Arg Arg Arg Arg Gln
1               5                   10                  15

Arg Arg Arg Arg
            20
```

<210> 158
<211> 22
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE

<222> (5)..(5)
<223> Xaa = D-Phe

<220>
<221> MISC_FEATURE
<222> (9) .. (10)
<223> Xaa = 8-amino-3,6-dioxaoctanoic acid (Doc)

<220>
<221> MISC_FEATURE
<222> (11)..(11)
<223> Xaa = Tyr or absent

<220>
<221> MOD_RES
<222> (22)..(22)
<223> AMIDATION

<400> 158

```
Xaa Cys Xaa His Xaa Arg Trp Cys Xaa Xaa Xaa Gly Arg Arg Arg Arg
1               5                   10                  15

Arg Gln Arg Arg Arg Arg
            20
```

<210> 159
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)

<223> xaa = beta-Ala

<220>
<221> MOD_RES
<222> (20)..(20)
<223> AMIDATION

<400> 159

Xaa Cys His Xaa Arg Trp Xaa Cys Xaa Tyr Gly Arg Arg Arg Arg Arg
1               5                   10                  15

Gln Arg Arg Arg
            20

<210> 160
<211> 18
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2) .. (8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9) .. (9)
<223> Xaa = beta-Ala

<220>
<221> MOD_RES
<222> (18)..(18)
<223> AMIDATION

<400> 160

Xaa Cys His Xaa Arg Trp Xaa Cys Xaa Arg Arg Arg Arg Arg Gln Arg
1 5 10 15

Arg Arg

<210> 161
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2) .. (8)
<223> cyclic

<220>
<221> MISC_FEATURE
<222> (4)..(4)
<223> Xaa = D-Phe

<220>
<221> MISC_FEATURE
<222> (9)..(9)
<223> Xaa = beta-Ala

<220>
<221> MOD_RES
<222> (20)..(20)
<223> AMIDATION

<400> 161

Xaa Asp His Xaa Arg Trp Ala Lys Xaa Tyr Gly Arg Arg Arg Arg Arg
1 5 10 15

Gln Arg Arg Arg
20

<210> 162
<211> 18
<212> PRT
<213> Artificial Sequence

<220>
<223> Melanocortin receptor ligand to treat insulin sensitivity

<220>
<221> MISC_FEATURE

<222> (1)..(1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (9)..(9)
<223> Xaa = beta-Ala

<220>
<221> MOD_RES
<222> (18)..(18)
<223> AMIDATION

<400> 162

```
Xaa Asp His Xaa Arg Trp Ala Lys Xaa Arg Arg Arg Arg Arg Gln Arg
1               5                   10                  15

Arg Arg
```

<210> 163
<211> 18
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(8)
<223> Xaa = beta-Ala

<220>
<221> MISC_FEATURE
<222> (9)..(9)
<223> Xaa = Gly or absent

<220>
<221> MOD_RES
<222> (18)..(18)
<223> AMIDATION

<400> 163

```
Xaa Asp His Xaa Arg Trp Lys Xaa Xaa Arg Arg Arg Arg Arg Gln Arg
1               5                   10                  15

Arg Arg
```

<210> 164
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(8)
<223> Xaa = beta-Ala

<220>
<221> MISC_FEATURE
<222> (9)..(9)
<223> Xaa = beta-Ala or absent

<220>
<221> MOD_RES
<222> (20)..(20)
<223> AMIDATION

<400> 164

Xaa Asp His Xaa Arg Trp Lys Xaa Xaa Tyr Gly Arg Arg Arg Arg Arg
1               5                   10                  15

Gln Arg Arg Arg
            20

<210> 165
<211> 19
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(10)
<223> Xaa = beta-Ala

<220>
<221> MISC_FEATURE
<222> (10) .. (10)
<223> Xaa = Gly or absent

<220>
<221> MOD_RES
<222> (19)..(19)
<223> AMIDATION

<400> 165

Xaa Asp His Xaa Arg Trp Lys Xaa Xaa Xaa Arg Arg Arg Arg Arg Gln
1               5                   10                  15

Arg Arg Arg

<210> 166
<211> 19
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(8)
<223> Xaa = 8-amino-3,6-dioxaoctanoic acid (Doc)

<220>
<221> MISC_FEATURE
<222> (9)..(9)
<223> Xaa = Tyr or absent

<220>
<221> MOD_RES
<222> (19)..(19)
<223> AMIDATION

<400> 166

```
Xaa Asp His Xaa Arg Trp Lys Xaa Xaa Gly Arg Arg Arg Arg Arg Gln
1               5                   10                  15

Arg Arg Arg
```

<210> 167
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(9)
<223> Xaa = 8-amino-3,6-dioxaoctanoic acid (Doc)

<220>
<221> MISC_FEATURE
<222> (10)..(10)
<223> Xaa = Tyr or absent

<220>
<221> MOD_RES
<222> (20)..(20)
<223> AMIDATION

<400> 167

```
Xaa Asp His Xaa Arg Trp Lys Xaa Xaa Xaa Gly Arg Arg Arg Arg Arg
1               5                   10                  15

Gln Arg Arg Arg
            20
```

<210> 168
<211> 18
<212> PRT
<213> Artificial sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(8)
<223> Xaa = 8-amino-3,6-dioxaoctanoic acid (Doc)

<220>

<221> MISC_FEATURE
<222> (9)..(9)
<223> Xaa = 8-amino-3,6-dioxaoctanoic acid (Doc) or absent

<220>
<221> MOD_RES
<222> (18)..(18)
<223> AMIDATION

<400> 168

```
Xaa Asp His Xaa Arg Trp Lys Xaa Xaa Arg Arg Arg Arg Arg Gln Arg
1               5                   10                  15

Arg Arg
```

<210> 169
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(8)
<223> Xaa = beta-Ala

<220>
<221> MISC_FEATURE
<222> (9)..(9)
<223> xaa = Gly or absent

<220>
<221> MOD_RES
<222> (19)..(19)
<223> AMIDATION

<400> 169

Xaa Asp His Xaa Arg Trp Lys Xaa Xaa Arg Arg Arg Arg Arg Gln Arg
1               5                   10                  15

Arg Arg Arg

<210> 170
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(8)
<223> Xaa = beta-Ala

<220>
<221> MISC_FEATURE
<222> (9)..(9)
<223> Xaa = beta-Ala or absent

<220>
<221> MOD_RES
<222> (21)..(21)
<223> AMIDATION

<400> 170

Xaa Asp His Xaa Arg Trp Lys Xaa Xaa Tyr Gly Arg Arg Arg Arg Arg
1               5                   10                  15

Gln Arg Arg Arg Arg
            20

<210> 171
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(9)
<223> Xaa = beta-Ala

<220>
<221> MISC_FEATURE
<222> (10)..(10)
<223> Xaa = Gly or absent

<220>
<221> MOD_RES
<222> (20)..(20)
<223> AMIDATION

<400> 171

```
Xaa Asp His Xaa Arg Trp Lys Xaa Xaa Xaa Arg Arg Arg Arg Arg Gln
1               5                   10                  15

Arg Arg Arg Arg
            20
```

<210> 172
<211> 19
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)

<223> cyclic

<220>
<221> MISC_FEATURE
<222> (4)..(4)
<223> Xaa = D-Phe

<220>
<221> MISC_FEATURE
<222> (8)..(8)
<223> Xaa = 8-amino-3,6-dioxaoctanoic acid (Doc)

<220>
<221> MISC_FEATURE
<222> (9)..(9)
<223> Xaa = Gly or absent

<220>
<221> MOD_RES
<222> (19)..(19)
<223> AMIDATION

<400> 172

```
Xaa Asp His Xaa Arg Trp Lys Xaa Xaa Arg Arg Arg Arg Arg Gln Arg
1               5                   10                  15

Arg Arg Arg
```

<210> 173
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(8)
<223> Xaa = 8-amino-3,6-dioxaoctanoic acid (Doc)

<220>
<221> MISC_FEATURE
<222> (9)..(9)
<223> Xaa = 8-amino-3,6-dioxaoctanoic acid (Doc) or absent

<220>
<221> MOD_RES
<222> (21)..(21)
<223> AMIDATION

<400> 173

Xaa Asp His Xaa Arg Trp Lys Xaa Xaa Tyr Gly Arg Arg Arg Arg Arg
1 5 10 15

Gln Arg Arg Arg Arg

20

<210> 174
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(7)
<223> cyclic

<220>
<221> MISC_FEATURE
<222> (4)..(4)
<223> Xaa = D-Phe

<220>
<221> MISC_FEATURE
<222> (8)..(9)
<223> Xaa = 8-amino-3,6-dioxaoctanoic acid (Doc)

<220>
<221> MISC_FEATURE
<222> (10)..(10)
<223> Xaa = Gly or absent

<220>
<221> MOD_RES
<222> (20)..(20)
<223> AMIDATION

<400> 174

Xaa Asp His Xaa Arg Trp Lys Xaa Xaa Xaa Arg Arg Arg Arg Arg Gln
1               5                   10                  15

Arg Arg Arg Arg
            20

<210> 175
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2) .. (8)
<223> cyclic

<220>
<221> MISC_FEATURE
<222> (4)..(4)
<223> Xaa = D-Phe

<220>
<221> MISC_FEATURE
<222> (7)..(7)
<223> Xaa = beta-Ala

<220>
<221> MISC_FEATURE
<222> (9)..(9)
<223> Xaa = beta-Ala

<220>
<221> MOD_RES
<222> (20)..(20)
<223> AMIDATION

<400> 175

Xaa Asp His Xaa Arg Trp Xaa Lys Xaa Tyr Gly Arg Arg Arg Arg Arg
1               5                   10                  15

Gln Arg Arg Arg
            20

<210> 176
<211> 18

<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = beta-Ala

<220>
<221> MISC_FEATURE
<222> (9)..(9)
<223> Xaa = beta-Ala

<220>
<221> MOD_RES
<222> (18)..(18)
<223> AMIDATION

<400> 176

```
Xaa Asp His Xaa Arg Trp Xaa Lys Xaa Arg Arg Arg Arg Arg Gln Arg
1               5                   10                  15

Arg Arg
```

<210> 177
<211> 20
<212> PRT
<213> Artificial sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>

<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 6-aminohexanoic acid (Ahx)

<220>
<221> MISC_FEATURE
<222> (9)..(9)
<223> Xaa = beta-Ala

<220>
<221> MOD_RES
<222> (20)..(20)
<223> AMIDATION

<400> 177

```
Xaa Cys His Xaa Arg Trp Xaa Cys Xaa Tyr Gly Arg Arg Arg Arg Arg
1               5                   10                  15

Gln Arg Arg Arg
            20
```

<210> 178
<211> 18
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE

<222> (7)..(7)
<223> Xaa = 6-aminohexanoic acid (Ahx)

<220>
<221> MISC_FEATURE
<222> (9)..(9)
<223> Xaa = beta-Ala

<220>
<221> MOD_RES
<222> (18)..(18)
<223> AMIDATION

<400> 178

Xaa Cys His Xaa Arg Trp Xaa Cys Xaa Arg Arg Arg Arg Arg Gln Arg
1 5 10 15

Arg Arg

<210> 179
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = beta-Ala

<220>
<221> MOD_RES
<222> (21)..(21)
<223> AMIDATION

<400> 179

```
Xaa Cys His Xaa Arg Trp Xaa Xaa Thr Xaa Tyr Gly Arg Arg Arg Arg
1               5                   10                  15

Arg Gln Arg Arg Arg
            20
```

<210> 180
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = beta-Ala

<220>
<221> MOD_RES
<222> (19)..(19)
<223> AMIDATION

<400> 180

Xaa Cys His Xaa Arg Trp Xaa Xaa Thr Xaa Arg Arg Arg Arg Arg Gln
1               5                   10                  15

Arg Arg Arg

<210> 181
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> Xaa = beta-Ala

<220>
<221> MOD_RES
<222> (20)..(20)
<223> AMIDATION

<400> 181

Xaa Cys His Xaa Arg Trp Xaa Cys Xaa Tyr Gly Arg Arg Arg Arg Arg
1               5                   10                  15

Gln Arg Arg Arg
            20

<210> 182
<211> 18
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2) .. (8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> Xaa = beta-Ala

<220>
<221> MOD_RES
<222> (18)..(18)
<223> AMIDATION

<400> 182

```
Xaa Cys His Xaa Arg Trp Xaa Cys Xaa Arg Arg Arg Arg Arg Gln Arg
1               5                   10                  15

Arg Arg
```

<210> 183
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = beta-cyclohexylAla (Cha), homo-cyclohexylAla (hcha), or Nle, all modified with acyl (Ac)

<220>
<221> DOMAIN
<222> (2)..(8)
<223> cyclic

<220>
<221> MISC_FEATURE
<222> (4)..(4)
<223> xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> xaa = beta-Ala

<220>
<221> MOD_RES
<222> (20)..(20)
<223> AMIDATION

<400> 183

```
Xaa Asp His Xaa Arg Trp Xaa Lys Xaa Tyr Gly Arg Arg Arg Arg Arg
1               5                   10                  15

Gln Arg Arg Arg
            20
```

<210> 184
<211> 18
<212> PRT
<213> Artificial Sequence

<220>
<223> Melanocortin receptor ligand to treat insulin sensitivity

<220>
<221> MISC_FEATURE
<222> (1) .. (1)
<223> Xaa = beta-cyclohexylAla (Cha) or homo-cyclohexylAla (hcha), both modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>

<221> MISC_FEATURE
<222> (9)..(9)
<223> Xaa = beta-Ala

<220>
<221> MOD_RES
<222> (18)..(18)
<223> AMIDATION

<400> 184

```
Xaa Asp His Xaa Arg Trp Xaa Lys Xaa Arg Arg Arg Arg Arg Gln Arg
1               5                   10                  15

Arg Arg
```

<210> 185
<211> 18
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle or cyclohexylGly (Chg), both modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MOD_RES
<222> (18)..(18)
<223> AMIDATION

<400> 185

Xaa Asp His Xaa Arg Trp Xaa Lys Xaa Arg Arg Arg Arg Arg Gln Arg
1 5 10 15

Arg Arg

<210> 186
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = cyclohexylGly (Chg) or homo-cyclohexylAla (hcha), both modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MOD_RES
<222> (20)..(20)
<223> AMIDATION

<400> 186

Xaa Asp His Xaa Arg Trp Xaa Lys Xaa Tyr Gly Arg Arg Arg Arg Arg
1 5 10 15

Gln Arg Arg Arg
20

<210> 187
<211> 21
<212> PRT
<213> Artificial Sequence

<220>
<223> Melanocortin receptor ligand to treat insulin sensitivity

<220>
<221> MISC_FEATURE
<222> (1)..(1)
<223> Xaa = homo-cyclohexylAla (hcha) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9) .. (10)
<223> Xaa = beta-Ala

<220>
<221> MOD_RES
<222> (21)..(21)
<223> AMIDATION

<400> 187

```
Xaa Asp His Xaa Arg Trp Xaa Lys Xaa Xaa Tyr Gly Arg Arg Arg Arg
1               5                   10                  15

Arg Gln Arg Arg Arg
            20
```

<210> 188
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = homo-cyclohexylAla (hcha) modified with acyl (Ac)

<220>
<221> DOMAIN
<222> (2)..(8)

<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (19)..(19)
<223> AMIDATION

<400> 188

```
Xaa Asp His Xaa Arg Trp Xaa Lys Xaa Xaa Arg Arg Arg Arg Arg Gln
1               5                   10                  15

Arg Arg Arg
```

<210> 189
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = homo-cyclohexylAla (hcha) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (21)..(21)
<223> AMIDATION

<400> 189

```
Xaa Asp His Xaa Arg Trp Xaa Lys Xaa Xaa Tyr Gly Arg Arg Arg Arg
1               5                   10                  15

Arg Gln Arg Arg Arg
            20
```

<210> 190
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = homo-cyclohexylAla (hCha) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>

<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (19)..(19)
<223> AMIDATION

<400> 190

```
Xaa Asp His Xaa Arg Trp Xaa Lys Xaa Xaa Arg Arg Arg Arg Arg Gln
1               5                   10                  15

Arg Arg Arg
```

<210> 191
<211> 22
<212> PRT
<213> Artificial Sequence

<220>
<223> Melanocortin receptor ligand to treat insulin sensitivity

<220>
<221> MISC_FEATURE
<222> (1)..(1)
<223> Xaa = homo-cyclohexylAla (hcha) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MISC_FEATURE
<222> (10)..(10)

<223> Xaa = beta-Ala or absent

<220>
<221> MOD_RES
<222> (22)..(22)
<223> AMIDATION

<400> 191

```
Xaa Asp His Xaa Arg Trp Xaa Lys Xaa Xaa Tyr Gly Arg Arg Arg Arg
1               5                   10                  15

Arg Gln Arg Arg Arg Arg
            20
```

<210> 192
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = homo-cyclohexylAla (hcha) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MISC_FEATURE
<222> (10) .. (10)
<223> Xaa = beta-Ala or absent

<220>
<221> MOD_RES
<222> (20)..(20)
<223> AMIDATION

<400> 192

Xaa Asp His Xaa Arg Trp Xaa Lys Xaa Xaa Arg Arg Arg Arg Arg Gln
1 5 10 15

Arg Arg Arg Arg
20

<210> 193
<211> 22
<212> PRT
<213> Artificial Sequence

<220>
<223> Melanocortin receptor ligand to treat insulin sensitivity

<220>
<221> MISC_FEATURE
<222> (1) .. (1)
<223> Xaa = homo-cyclohexylAla (hcha) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (22)..(22)
<223> AMIDATION

<400> 193

Xaa Asp His Xaa Arg Trp Xaa Lys Xaa Xaa Tyr Gly Arg Arg Arg Arg
1 5 10 15

Arg Gln Arg Arg Arg Arg
20

<210> 194
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = homo-cyclohexylAla (hcha) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = 8-amino-3,6-dioxaoctanoic acid (Doc)

<220>
<221> MISC_FEATURE
<222> (10) .. (10)
<223> Xaa = 8-amino-3,6-dioxaoctanoic acid (Doc) or absent

<220>
<221> MOD_RES
<222> (20)..(20)
<223> AMIDATION

<400> 194

Xaa Asp His Xaa Arg Trp Xaa Lys Xaa Xaa Arg Arg Arg Arg Arg Gln
1 5 10 15

Arg Arg Arg Arg
20

<210> 195
<211> 20

<212> PRT
<213> Artificial Sequence

<220>
<223> Melanocortin receptor ligand to treat insulin sensitivity

<220>
<221> MISC_FEATURE
<222> (1) .. (1)
<223> Xaa = D-cyclohexylGly (D-Chg) modified with acyl (Ac)

<220>
<221> DOMAIN
<222> (2) .. (8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MOD_RES
<222> (20)..(20)
<223> AMIDATION

<400> 195

```
Xaa Asp His Xaa Arg Trp Xaa Lys Xaa Tyr Gly Arg Arg Arg Arg Arg
1               5                   10                  15

Gln Arg Arg Arg
            20
```

<210> 196
<211> 18
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-cyclohexylGly (D-Chg) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC FEATURE
<222> (7) .. (7)
<223> Xaa = 4-aminobutyric acid (Gaba)

<220>
<221> MISC_FEATURE
<222> (9)..(9)
<223> Xaa = beta-Ala

<220>
<221> MOD_RES
<222> (18)..(18)
<223> AMIDATION

<400> 196

```
Xaa Asp His Xaa Arg Trp Xaa Lys Xaa Arg Arg Arg Arg Arg Gln Arg
1               5                   10                  15

Arg Arg
```

<210> 197
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = homo-phenylAla (hPhe) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE

<222> (7)..(7)
<223> Xaa = 4-aminobutyric acid (Gaba)

<220>
<221> MISC_FEATURE
<222> (9)..(9)
<223> Xaa = beta-Ala

<220>
<221> MOD_RES
<222> (20)..(20)
<223> AMIDATION

<400> 197

```
Xaa Asp His Xaa Arg Trp Xaa Lys Xaa Tyr Gly Arg Arg Arg Arg Arg
1               5                   10                  15

Gln Arg Arg Arg

            20
```

<210> 198
<211> 18
<212> PRT
<213> Artificial Sequence

<220>
<223> Melanocortin receptor ligand to treat insulin sensitivity

<220>
<221> MISC_FEATURE
<222> (1) .. (1)
<223> Xaa = homo-phenylAla (hPhe) modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>

<221> MOD_RES
<222> (18)..(18)
<223> AMIDATION

<400> 198

```
Xaa Asp His Xaa Arg Trp Xaa Lys Xaa Arg Arg Arg Arg Arg Gln Arg
1               5                   10                  15

Arg Arg
```

<210> 199
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2) .. (8)
<223> cyclic

<220>
<221> MISC_FEATURE
<222> (4)..(4)
<223> Xaa = D-Phe

<220>
<221> MISC_FEATURE
<222> (6)..(6)
<223> Xaa = D-Trp

<220>
<221> MISC_FEATURE
<222> (7)..(7)
<223> Xaa = 5-aminopentanoic acid (Apn) or 6-aminohexanoic acid (Ahx)

<220>
<221> MISC_FEATURE
<222> (9)..(9)
<223> Xaa = beta-Ala

<220>
<221> MOD_RES
<222> (20)..(20)
<223> AMIDATION

<400> 199

Xaa Cys His Xaa Arg Xaa Xaa Cys Xaa Tyr Gly Arg Arg Arg Arg Arg
1               5                   10                  15

Gln Arg Arg Arg
            20

<210> 200
<211> 18
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (6)..(6)
<223> Xaa = D-Trp

<220>
<221> MISC_FEATURE
<222> (7)..(7)
<223> Xaa = 5-aminopentanoic acid (Apn) or 6-aminohexanoic acid (Ahx)

<220>
<221> MISC_FEATURE
<222> (9)..(9)
<223> Xaa = beta-Ala

<220>
<221> MOD_RES
<222> (18)..(18)
<223> AMIDATION

<400> 200

Xaa Cys His Xaa Arg Xaa Xaa Cys Xaa Arg Arg Arg Arg Arg Gln Arg
1               5                   10                  15

Arg Arg

<210> 201
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (6)..(6)
<223> Xaa = D-Trp

<220>
<221> MISC_FEATURE
<222> (7)..(7)
<223> Xaa = beta-Ala

<220>
<221> MISC_FEATURE
<222> (9)..(9)
<223> Xaa = beta-Ala

<220>
<221> MOD_RES
<222> (20)..(20)
<223> AMIDATION

<400> 201

```
Xaa Cys His Xaa Arg Xaa Xaa Cys Xaa Tyr Gly Arg Arg Arg Arg Arg
1               5                   10                  15

Gln Arg Arg Arg
            20
```

<210> 202
<211> 18
<212> PRT
<213> Artificial Sequence

<220>

<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (6)..(6)
<223> Xaa = D-Trp

<220>
<221> MISC_FEATURE
<222> (7)..(7)
<223> Xaa = beta-Ala

<220>
<221> MISC_FEATURE
<222> (9)..(9)
<223> Xaa = beta-Ala

<220>
<221> MOD_RES
<222> (18)..(18)
<223> AMIDATION

<400> 202

```
Xaa Cys His Xaa Arg Xaa Xaa Cys Xaa Arg Arg Arg Arg Arg Gln Arg
1               5                   10                  15

Arg Arg
```

<210> 203
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (8)..(8)
<223> Xaa = penicillamine (Pen)

<220>
<221> MISC_FEATURE
<222> (9)..(9)
<223> Xaa = beta-Ala

<220>
<221> MISC_FEATURE
<222> (10)..(10)
<223> Xaa = Tyr or absent

<220>
<221> MOD_RES
<222> (20)..(20)
<223> AMIDATION

<400> 203

```
Xaa Cys Xaa His Xaa Arg Trp Xaa Xaa Xaa Gly Arg Arg Arg Arg Arg
1               5                   10                  15

Gln Arg Arg Arg
            20
```

<210> 204
<211> 21
<212> PRT
<213> Artificial sequence

<220>
<223> Melanocortin receptor ligand to treat insulin sensitivity

<220>
<221> MISC_FEATURE
<222> (1)..(1)
<223> Xaa = Nle modified with acyl (Ac)

<220>

<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5) .. (5)
<223> Xaa = D-Phe

<220>
<221> MISC_FEATURE
<222> (8)..(8)
<223> Xaa = penicillamine (Pen)

<220>
<221> MISC_FEATURE
<222> (9)..(10)
<223> Xaa = beta-Ala

<220>
<221> MISC_FEATURE
<222> (11)..(11)
<223> xaa = Tyr or absent

<220>
<221> MOD_RES
<222> (21)..(21)
<223> AMIDATION

<400> 204

```
Xaa Cys Xaa His Xaa Arg Trp Xaa Xaa Xaa Xaa Gly Arg Arg Arg Arg
1               5                   10                  15

Arg Gln Arg Arg Arg
            20
```

<210> 205
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN

<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (8)..(8)
<223> Xaa = penicillamine (Pen)

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (19)..(19)
<223> AMIDATION

<400> 205

```
Xaa Cys Xaa His Xaa Arg Trp Xaa Xaa Xaa Arg Arg Arg Arg Arg Gln
1               5                   10                  15

Arg Arg Arg
```

<210> 206
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MISC_FEATURE
<222> (8)..(8)
<223> xaa = penicillamine (Pen)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = Gly or absent

<220>
<221> MOD_RES
<222> (19)..(19)
<223> AMIDATION

<400> 206

```
Xaa Cys Xaa His Xaa Arg Trp Xaa Xaa Xaa Arg Arg Arg Arg Arg Gln
1               5                   10                  15

Arg Arg Arg
```

<210> 207
<211> 21
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE

<222> (3)..(3)
<223> Xaa = D-Ala

<220>
<221> MISC_FEATURE
<222> (5) .. (5)
<223> Xaa = D-Phe

<220>
<221> MISC_FEATURE
<222> (8)..(8)
<223> Xaa = penicillamine (Pen)

<220>
<221> MISC_FEATURE
<222> (9) .. (9)
<223> Xaa = 8-amino-3,6-dioxaoctanoic acid (Doc)

<220>
<221> MISC_FEATURE
<222> (10) .. (10)
<223> Xaa = 8-amino-3,6-dioxaoctanoic acid (Doc) or absent

<220>
<221> MOD_RES
<222> (21)..(21)
<223> AMIDATION

<400> 207

```
Xaa Cys Xaa His Xaa Arg Trp Xaa Xaa Xaa Tyr Gly Arg Arg Arg Arg
1               5                   10                  15

Arg Gln Arg Arg Arg
            20
```

<210> 208
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (AC)

<220>
<221> DOMAIN
<222> (2)..(8)
<223> cyclic

<220>
<221> MISC_FEATURE
<222> (3)..(3)

<223> Xaa = D-Ala

<220>
<221> MISC_FEATURE
<222> (5) .. (5)
<223> Xaa = D-Phe

<220>
<221> MISC_FEATURE
<222> (8)..(8)
<223> Xaa = penicillamine (Pen)

<220>
<221> MISC_FEATURE
<222> (9)..(10)
<223> Xaa = 8-amino-3,6-dioxaoctanoic acid (Doc)

<220>
<221> MISC_FEATURE
<222> (11)..(11)
<223> Xaa = Gly or absent

<220>
<221> MOD_RES
<222> (20)..(20)
<223> AMIDATION

<400> 208

```
Xaa Cys Xaa His Xaa Arg Trp Xaa Xaa Xaa Xaa Arg Arg Arg Arg Arg
1               5                   10                  15

Gln Arg Arg Arg
            20
```

<210> 209
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (9)..(9)
<223> Xaa = beta-Ala

<220>
<221> MISC_FEATURE
<222> (10) .. (10)
<223> Xaa = Tyr or absent

<220>
<221> MOD_RES
<222> (20)..(20)
<223> AMIDATION

<400> 209

```
Xaa Cys His Xaa Arg Trp Xaa Xaa Xaa Xaa Gly Arg Arg Arg Arg Arg
1               5                   10                  15

Gln Arg Arg Arg
            20
```

<210> 210
<211> 18
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>

<221> MISC_FEATURE
<222> (7)..(7)
<223> xaa = beta-Ala

<220>
<221> MISC_FEATURE
<222> (8)..(8)
<223> Xaa = D-Cys

<220>
<221> MISC_FEATURE
<222> (9)..(9)
<223> xaa = beta-Ala

<220>
<221> MOD_RES
<222> (18)..(18)
<223> AMIDATION

<400> 210

```
Xaa Cys His Xaa Arg Trp Xaa Xaa Xaa Arg Arg Arg Arg Arg Gln Arg
1               5                   10                  15

Arg Arg
```

<210> 211
<211> 19
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)

<223> Xaa = D-Cys

<220>
<221> MISC_FEATURE
<222> (9)..(9)
<223> xaa = beta-Ala

<220>
<221> MOD_RES
<222> (19)..(19)
<223> AMIDATION

<400> 211

```
Xaa Cys His Xaa Arg Trp Xaa Xaa Xaa Arg Arg Arg Arg Arg Gln Arg
1               5                   10                  15

Arg Arg Arg
```

<210> 212
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (9)..(10)
<223> Xaa = beta-Ala

<220>
<221> MOD_RES
<222> (21)..(21)
<223> AMIDATION

<400> 212

```
Xaa Cys His Xaa Arg Trp Xaa Xaa Xaa Xaa Tyr Gly Arg Arg Arg Arg
1               5                   10                  15

Arg Gln Arg Arg Arg
            20
```

<210> 213
<211> 20
<212> PRT
<213> Artificial Sequence

<220>
<223> Melanocortin receptor ligand to treat insulin sensitivity

<220>
<221> MISC_FEATURE
<222> (1)..(1)
<223> xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (9)..(10)
<223> Xaa = beta-Ala

<220>
<221> MISC_FEATURE
<222> (11)..(11)
<223> Xaa = Gly or absent

<220>

<221> MOD_RES
<222> (20)..(20)
<223> AMIDATION

<400> 213

```
Xaa Cys His Xaa Arg Trp Xaa Xaa Xaa Xaa Xaa Arg Arg Arg Arg Arg
1               5                   10                  15

Gln Arg Arg Arg
            20
```

<210> 214
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = Tyr or absent

<220>
<221> MOD_RES

<222> (20)..(20)
<223> AMIDATION

<400> 214

Xaa Cys His Xaa Arg Trp Xaa Xaa Xaa Xaa Gly Arg Arg Arg Arg Arg
1 5 10 15

Gln Arg Arg Arg
20

<210> 215
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (9)..(10)
<223> Xaa = 8-amino-3,6-dioxaoctanoic acid (Doc)

<220>
<221> MISC_FEATURE
<222> (11)..(11)
<223> Xaa = Tyr or absent

<220>
<221> MOD_RES
<222> (21)..(21)

<223> AMIDATION

<400> 215

```
Xaa Cys His Xaa Arg Trp Xaa Xaa Xaa Xaa Xaa Gly Arg Arg Arg Arg
1               5                   10                  15

Arg Gln Arg Arg Arg
            20
```

<210> 216
<211> 19
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2) .. (8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (19)..(19)
<223> AMIDATION

<400> 216

Xaa Cys His Xaa Arg Trp Xaa Xaa Xaa Xaa Arg Arg Arg Arg Arg Gln
1 5 10 15

Arg Arg Arg

<210> 217
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (20)..(20)
<223> AMIDATION

<400> 217

Xaa Cys His Xaa Arg Trp Xaa Xaa Xaa Xaa Arg Arg Arg Arg Arg Gln
1 5 10 15

Arg Arg Arg Arg
20

<210> 218
<211> 22
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5)..(5)
<223> Xaa = homo-Arg (hArg)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = beta-Ala

<220>
<221> MISC_FEATURE
<222> (11)..(11)
<223> Xaa = beta-Ala or absent

<220>
<221> MOD_RES
<222> (22)..(22)

<223> AMIDATION

<400> 218

Xaa Cys His Xaa Xaa Trp Xaa Xaa Thr Xaa Xaa Tyr Gly Arg Arg Arg
1 5 10 15

Arg Arg Gln Arg Arg Arg
20

<210> 219
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5) .. (5)
<223> Xaa = homo-Arg (hArg)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = beta-Ala

<220>
<221> MISC_FEATURE
<222> (11)..(11)
<223> Xaa = beta-Ala or absent

<220>
<221> MOD_RES
<222> (20)..(20)
<223> AMIDATION

<400> 219

```
Xaa Cys His Xaa Xaa Trp Xaa Xaa Thr Xaa Xaa Arg Arg Arg Arg Arg
1               5                   10                  15

Gln Arg Arg Arg
            20
```

<210> 220
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5) .. (5)
<223> Xaa = homo-Arg (hArg)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = 8-amino-3,6-dioxaoctanoic acid (Doc)

<220>

<221> MOD_RES
<222> (19)..(19)
<223> AMIDATION

<400> 220

```
Xaa Cys His Xaa Xaa Trp Xaa Xaa Thr Xaa Arg Arg Arg Arg Arg Gln
1               5                   10                  15

Arg Arg Arg
```

<210> 221
<211> 22
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5) .. (5)
<223> Xaa = homo-Arg (hArg)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = 8-amino-3,6-dioxaoctanoic acid (Doc)

<220>
<221> MISC_FEATURE
<222> (11)..(11)

<223> Xaa = 8-amino-3,6-dioxaoctanoic acid (Doc) or absent

<220>
<221> MOD_RES
<222> (22)..(22)
<223> AMIDATION

<400> 221

Xaa Cys His Xaa Xaa Trp Xaa Xaa Thr Xaa Xaa Tyr Gly Arg Arg Arg
1 5 10 15

Arg Arg Gln Arg Arg Arg
20

<210> 222
<211> 23
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5) .. (5)
<223> Xaa = homo-Arg (hArg)

<220>
<221> MISC_FEATURE
<222> (7)..(7)
<223> xaa = beta-Ala

<220>
<221> MISC_FEATURE
<222> (8)..(8)
<223> Xaa = D-Cys

<220>
<221> MISC_FEATURE
<222> (10)..(10)
<223> Xaa = beta-Ala

<220>
<221> MISC_FEATURE
<222> (11)..(11)
<223> Xaa = beta-Ala or absent

<220>
<221> MOD_RES
<222> (23)..(23)
<223> AMIDATION

<400> 222

```
Xaa Cys His Xaa Xaa Trp Xaa Xaa Thr Xaa Xaa Tyr Gly Arg Arg Arg
1               5                   10                  15

Arg Arg Gln Arg Arg Arg Arg
            20
```

<210> 223
<211> 21
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5) .. (5)
<223> Xaa = homo-Arg (hArg)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>

<221> MISC_FEATURE
<222> (10) .. (10)
<223> Xaa = beta-Ala

<220>
<221> MISC_FEATURE
<222> (11)..(11)
<223> Xaa = beta-Ala or absent

<220>
<221> MOD_RES
<222> (21)..(21)
<223> AMIDATION

<400> 223

```
Xaa Cys His Xaa Xaa Trp Xaa Xaa Thr Xaa Xaa Arg Arg Arg Arg Arg
1               5                   10                  15

Gln Arg Arg Arg Arg
            20
```

<210> 224
<211> 23
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5)..(5)
<223> Xaa = homo-Arg (hArg)

<220>
<221> MISC_FEATURE
<222> (7)..(7)
<223> Xaa = beta-Ala

<220>
<221> MISC_FEATURE

<222> (8)..(8)
<223> Xaa = D-Cys

<220>
<221> MISC_FEATURE
<222> (10)..(10)
<223> Xaa = 8-amino-3,6-dioxaoctanoic acid (Doc)

<220>
<221> MISC_FEATURE
<222> (11)..(11)
<223> Xaa = 8-amino-3,6-dioxaoctanoic acid (Doc) or absent

<220>
<221> MOD_RES
<222> (23)..(23)
<223> AMIDATION

<400> 224

```
Xaa Cys His Xaa Xaa Trp Xaa Xaa Thr Xaa Xaa Tyr Gly Arg Arg Arg
1               5                   10                  15

Arg Arg Gln Arg Arg Arg Arg
            20
```

<210> 225
<211> 21
<212> PRT
<213> Artificial sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5)..(5)
<223> Xaa = homo-Arg (hArg)

<220>
<221> MISC_FEATURE
<222> (7)..(7)

<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = 8-amino-3,6-dioxaoctanoic acid (Doc)

<220>
<221> MISC_FEATURE
<222> (11)..(11)
<223> Xaa = 8-amino-3,6-dioxaoctanoic acid (Doc) or absent

<220>
<221> MOD_RES
<222> (21)..(21)
<223> AMIDATION

<400> 225

```
Xaa Cys His Xaa Xaa Trp Xaa Xaa Thr Xaa Xaa Arg Arg Arg Arg Arg
1               5                   10                  15

Gln Arg Arg Arg Arg
            20
```

<210> 226
<211> 22
<212> PRT
<213> Artificial Sequence

<220>
<223> Melanocortin receptor ligand to treat insulin sensitivity

<220>
<221> MISC_FEATURE
<222> (1)..(1)
<223> xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5)..(5)
<223> Xaa = homo-Arg (hArg)

<220>
<221> MISC_FEATURE
<222> (6)..(6)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = beta-Ala

<220>
<221> MOD_RES
<222> (22)..(22)
<223> AMIDATION

<400> 226

```
Xaa Cys His Xaa Xaa Xaa Xaa Xaa Thr Xaa Tyr Gly Arg Arg Arg Arg
1               5                   10                  15

Arg Gln Arg Arg Arg Arg
            20
```

<210> 227
<211> 22
<212> PRT
<213> Artificial sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>

<221> MISC_FEATURE
<222> (5) .. (5)
<223> Xaa = homo-Arg (hArg)

<220>
<221> MISC_FEATURE
<222> (6)..(6)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = beta-Ala

<220>
<221> MISC_FEATURE
<222> (11)..(11)
<223> Xaa = beta-Ala or absent

<220>
<221> MOD_RES
<222> (22)..(22)
<223> AMIDATION

<400> 227

```
Xaa Cys His Xaa Xaa Xaa Xaa Xaa Thr Xaa Xaa Tyr Gly Arg Arg Arg
1               5                   10                  15

Arg Arg Gln Arg Arg Arg
            20
```

<210> 228
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN

<222> (2)..(8)
<223> cyclic

<220>
<221> MISC_FEATURE
<222> (4)..(4)
<223> Xaa = D-(Et)Tyr

<220>
<221> MISC_FEATURE
<222> (5)..(5)
<223> Xaa = homo-Arg (hArg)

<220>
<221> MISC_FEATURE
<222> (6)..(6)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> xaa = D-Cys

<220>
<221> MISC_FEATURE
<222> (10)..(10)
<223> Xaa = beta-Ala

<220>
<221> MISC_FEATURE
<222> (11)..(11)
<223> Xaa = beta-Ala or absent

<220>
<221> MOD_RES
<222> (20)..(20)
<223> AMIDATION

<400> 228

```
Xaa Cys His Xaa Xaa Xaa Xaa Xaa Thr Xaa Xaa Arg Arg Arg Arg Arg
1               5                   10                  15

Gln Arg Arg Arg
            20
```

<210> 229
<211> 21
<212> PRT
<213> Artificial Sequence

<220>

<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5)..(5)
<223> xaa = homo-Arg (hArg)

<220>
<221> MISC_FEATURE
<222> (5)..(5)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = 8-amino-3,6-dioxaoctanoic acid (Doc)

<220>
<221> MOD_RES
<222> (21)..(21)
<223> AMIDATION

<400> 229

```
Xaa Cys His Xaa Xaa Xaa Xaa Xaa Thr Xaa Tyr Gly Arg Arg Arg Arg
1               5                   10                  15

Arg Gln Arg Arg Arg
            20
```

<210> 230
<211> 22
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2) .. (8)
<223> cyclic

<220>
<221> MISC_FEATURE
<222> (4)..(4)
<223> Xaa = D-(Et)Tyr

<220>
<221> MISC_FEATURE
<222> (5)..(5)
<223> Xaa = homo-Arg (hArg)

<220>
<221> MISC_FEATURE
<222> (6)..(6)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10)..(10)
<223> Xaa = 8-amino-3,6-dioxaoctanoic acid (Doc)

<220>
<221> MOD_RES
<222> (22)..(22)
<223> AMIDATION

<400> 230

Xaa Cys His Xaa Xaa Xaa Xaa Xaa Thr Xaa Tyr Gly Arg Arg Arg Arg
1               5                   10                  15

Arg Gln Arg Arg Arg Arg
            20

<210> 231
<211> 22
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5)..(5)
<223> Xaa = homo-Arg (hArg)

<220>
<221> MISC_FEATURE
<222> (6)..(6)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE
<222> (10) .. (11)
<223> Xaa = 8-amino-3,6-dioxaoctanoic acid (Doc)

<220>

<221> MOD_RES
<222> (22)..(22)
<223> AMIDATION

<400> 231

```
Xaa Cys His Xaa Xaa Xaa Xaa Xaa Thr Xaa Xaa Tyr Gly Arg Arg Arg
1               5                   10                  15

Arg Arg Gln Arg Arg Arg
            20
```

<210> 232
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (5) .. (5)
<223> Xaa = homo-Arg (hArg)

<220>
<221> MISC_FEATURE
<222> (6)..(6)
<223> Xaa = 4,4'-biphenylAla (Bip)

<220>
<221> MISC_FEATURE
<222> (7)..(7)
<223> Xaa = beta-Ala

<220>
<221> MISC_FEATURE
<222> (8)..(8)
<223> Xaa = D-Cys

<220>
<221> MISC_FEATURE

<222> (10)..(10)
<223> Xaa = 8-amino-3,6-dioxaoctanoic acid (Doc)

<220>
<221> MISC_FEATURE
<222> (11)..(11)
<223> Xaa = 8-amino-3,6-dioxaoctanoic acid (Doc) or absent

<220>
<221> MOD_RES
<222> (20)..(20)
<223> AMIDATION

<400> 232

```
Xaa Cys His Xaa Xaa Xaa Xaa Xaa Thr Xaa Xaa Arg Arg Arg Arg Arg
1               5                   10                  15

Gln Arg Arg Arg
            20
```

<210> 233
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(9)
<223> cyclic

<220>
<221> MISC_FEATURE
<222> (3)..(3)
<223> Xaa = D-Ala

<220>
<221> MISC_FEATURE
<222> (5) .. (5)
<223> Xaa = D-Phe

<220>
<221> MISC_FEATURE
<222> (10)..(10)
<223> Xaa = beta-Ala

<220>
<221> MOD_RES
<222> (21)..(21)

<223> AMIDATION

<400> 233

```
Xaa Cys Xaa His Xaa Arg Trp Gly Cys Xaa Tyr Gly Arg Arg Arg Arg
1               5                   10                  15

Arg Gln Arg Arg Arg
            20
```

<210> 234
<211> 19
<212> PRT
<213> Artificial sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(9)
<223> cyclic

<220>
<221> MISC_FEATURE
<222> (3) .. (3)
<223> Xaa = D-Ala

<220>
<221> MISC_FEATURE
<222> (5) .. (5)
<223> Xaa = D-Phe

<220>
<221> MISC_FEATURE
<222> (10)..(10)
<223> Xaa = beta-Ala

<220>
<221> MOD_RES
<222> (19)..(19)
<223> AMIDATION

<400> 234

```
Xaa Cys Xaa His Xaa Arg Trp Gly Cys Xaa Arg Arg Arg Arg Arg Gln
1               5                   10                  15

Arg Arg Arg
```

<210> 235

<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle

<220>
<221> DOMAIN
<222> (2) .. (8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> xaa = beta-Ala

<220>
<221> MISC_FEATURE
<222> (10)..(10)
<223> Xaa = beta-Ala or absent

<220>
<221> MOD_RES
<222> (21)..(21)
<223> AMIDATION

<400> 235

```
Xaa Cys His Xaa Arg Trp Xaa Cys Xaa Xaa Tyr Gly Arg Arg Arg Arg
1               5                   10                  15

Arg Gln Arg Arg Arg
            20
```

<210> 236
<211> 19
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle

<220>
<221> DOMAIN
<222> (2)..(8)
<223> cyclic

<220>
<221> MISC_FEATURE
<222> (4)..(4)
<223> xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (19)..(19)
<223> AMIDATION

<400> 236

```
Xaa Cys His Xaa Arg Trp Xaa Cys Xaa Xaa Arg Arg Arg Arg Arg Gln
1               5                   10                  15

Arg Arg Arg
```

<210> 237
<211> 22
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN

<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9) .. (9)
<223> Xaa = beta-Ala

<220>
<221> MISC_FEATURE
<222> (10)..(10)
<223> Xaa = beta-Ala or absent

<220>
<221> MOD_RES
<222> (22)..(22)
<223> AMIDATION

<400> 237

```
Xaa Cys His Xaa Arg Trp Xaa Cys Xaa Xaa Tyr Gly Arg Arg Arg Arg
1               5                   10                  15

Arg Gln Arg Arg Arg Arg
            20
```

<210> 238
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)

<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (20)..(20)
<223> AMIDATION

<400> 238

```
Xaa Cys His Xaa Arg Trp Xaa Cys Xaa Xaa Arg Arg Arg Arg Arg Gln
1               5                   10                  15

Arg Arg Arg Arg
            20
```

<210> 239
<211> 21
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (21)..(21)
<223> AMIDATION

<400> 239

```
Xaa Cys His Xaa Arg Trp Xaa Cys Xaa Xaa Tyr Gly Arg Arg Arg Arg
1               5                   10                  15

Arg Gln Arg Arg Arg
            20
```

<210> 240
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> Xaa = 8-amino-3,6-dioxaoctanoic acid (Doc)

<220>

<221> MISC_FEATURE
<222> (10)..(10)
<223> Xaa = 8-amino-3,6-dioxaoctanoic acid (Doc) or absent

<220>
<221> MOD_RES
<222> (19)..(19)
<223> AMIDATION

<400> 240

```
Xaa Cys His Xaa Arg Trp Xaa Cys Xaa Xaa Arg Arg Arg Arg Arg Gln
1               5                   10                  15

Arg Arg Arg
```

<210> 241
<211> 22
<212> PRT
<213> Artificial sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (22)..(22)

<223> AMIDATION

<400> 241

Xaa Cys His Xaa Arg Trp Xaa Cys Xaa Xaa Tyr Gly Arg Arg Arg Arg
1 5 10 15

Arg Gln Arg Arg Arg Arg
20

<210> 242
<211> 20
<212> PRT
<213> Artificial sequence

<220>
<223> Melanocortin receptor ligand to treat insulin sensitivity

<220>
<221> MISC_FEATURE
<222> (1) .. (1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 5-aminopentanoic acid (Apn)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (20)..(20)
<223> AMIDATION

<400> 242

Xaa Cys His Xaa Arg Trp Xaa Cys Xaa Xaa Arg Arg Arg Arg Arg Gln
1 5 10 15

Arg Arg Arg Arg
20

<210> 243
<211> 21
<212> PRT
<213> Artificial Sequence

<220>
<223> Melanocortin receptor ligand to treat insulin sensitivity

<220>
<221> MISC_FEATURE
<222> (1) .. (1)
<223> xaa = Nle modified with acyl (Ac)

<220>
<221> DOMAIN
<222> (2) .. (8)
<223> cyclic

<220>
<221> MISC_FEATURE
<222> (3)..(3)
<223> Xaa = D-Leu

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10) .. (10)
<223> Xaa = beta-Ala or absent

<220>
<221> MOD_RES
<222> (21)..(21)
<223> AMIDATION

<400> 243

Xaa Cys Xaa His Xaa Arg Trp Cys Xaa Xaa Tyr Gly Arg Arg Arg Arg
1 5 10 15

Arg Gln Arg Arg Arg
20

<210> 244
<211> 19
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Leu

<220>
<221> MISC_FEATURE
<222> (5) .. (5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (19)..(19)
<223> AMIDATION

<400> 244

```
Xaa Cys Xaa His Xaa Arg Trp Cys Xaa Xaa Arg Arg Arg Arg Arg Gln
1               5                   10                  15

Arg Arg Arg
```

<210> 245
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Leu

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (21)..(21)
<223> AMIDATION

<400> 245

```
Xaa Cys Xaa His Xaa Arg Trp Cys Xaa Xaa Tyr Gly Arg Arg Arg Arg
1               5                   10                  15

Arg Gln Arg Arg Arg
            20
```

<210> 246
<211> 19
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>

<221> DOMAIN
<222> (2)..(8)
<223> cyclic

<220>
<221> MISC_FEATURE
<222> (3)..(3)
<223> Xaa = D-Leu

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (19)..(19)
<223> AMIDATION

<400> 246

```
Xaa Cys Xaa His Xaa Arg Trp Cys Xaa Xaa Arg Arg Arg Arg Arg Gln
1               5                   10                  15

Arg Arg Arg
```

<210> 247
<211> 22
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (3)..(3)

<223> Xaa = D-Leu

<220>
<221> MISC_FEATURE
<222> (5) .. (5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (22)..(22)
<223> AMIDATION

<400> 247

```
Xaa Cys Xaa His Xaa Arg Trp Cys Xaa Xaa Tyr Gly Arg Arg Arg Arg
1               5                   10                  15

Arg Gln Arg Arg Arg Arg
            20
```

<210> 248
<211> 20
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Leu

<220>
<221> MISC_FEATURE
<222> (5) .. (5)
<223> Xaa = D-Phe

<220>
<221> MISC_FEATURE
<222> (9)..(9)
<223> Xaa = beta-Ala

<220>
<221> MISC_FEATURE
<222> (10) .. (10)
<223> Xaa = beta-Ala or absent

<220>
<221> MOD_RES
<222> (20)..(20)
<223> AMIDATION

<400> 248

```
Xaa Cys Xaa His Xaa Arg Trp Cys Xaa Xaa Arg Arg Arg Arg Arg Gln
1               5                   10                  15

Arg Arg Arg Arg
            20
```

<210> 249
<211> 22
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Leu

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>

<221> MISC_FEATURE
<222> (10)..(10)
<223> Xaa = 8-amino-3,6-dioxaoctanoic acid (Doc) or absent

<220>
<221> MOD_RES
<222> (22)..(22)
<223> AMIDATION

<400> 249

```
Xaa Cys Xaa His Xaa Arg Trp Cys Xaa Xaa Tyr Gly Arg Arg Arg Arg
1               5                   10                  15

Arg Gln Arg Arg Arg Arg
            20
```

<210> 250
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-Leu

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES

<222> (20)..(20)
<223> AMIDATION

<400> 250

Xaa Cys Xaa His Xaa Arg Trp Cys Xaa Xaa Arg Arg Arg Arg Arg Gln
1 5 10 15

Arg Arg Arg Arg
20

<210> 251
<211> 21
<212> PRT
<213> Artificial Sequence

<220>
<223> Melanocortin receptor ligand to treat insulin sensitivity

<220>
<221> MISC_FEATURE
<222> (1) .. (1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-beta-cyclohexylAla (D-Cha)

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (21)..(21)
<223> AMIDATION

<400> 251

Xaa Cys Xaa His Xaa Arg Trp Cys Xaa Xaa Tyr Gly Arg Arg Arg Arg
1 5 10 15

Arg Gln Arg Arg Arg
20

<210> 252
<211> 19
<212> PRT
<213> Artificial sequence

<220>
<223> Melanocortin receptor ligand to treat insulin sensitivity

<220>
<221> MISC_FEATURE
<222> (1)..(1)
<223> Xaa = Nle modified with acyl (AC)

<220>
<221> DOMAIN
<222> (2)..(8)
<223> cyclic

<220>
<221> MISC_FEATURE
<222> (3)..(3)
<223> Xaa = D-beta-cyclohexylAla (D-cha)

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (19)..(19)
<223> AMIDATION

<400> 252

Xaa Cys Xaa His Xaa Arg Trp Cys Xaa Xaa Arg Arg Arg Arg Arg Gln
1 5 10 15

Arg Arg Arg

<210> 253
<211> 21
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-beta-cyclohexylAla (D-Cha)

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (21)..(21)
<223> AMIDATION

<400> 253

```
Xaa Cys Xaa His Xaa Arg Trp Cys Xaa Xaa Tyr Gly Arg Arg Arg Arg
1               5                   10                  15

Arg Gln Arg Arg Arg
            20
```

<210> 254
<211> 19
<212> PRT
<213> Artificial Sequence

<220>

<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = beta-cyclohexylAla (D-Cha)

<220>
<221> MISC_FEATURE
<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (19)..(19)
<223> AMIDATION

<400> 254

```
Xaa Cys Xaa His Xaa Arg Trp Cys Xaa Xaa Arg Arg Arg Arg Arg Gln
1               5                   10                  15

Arg Arg Arg
```

<210> 255
<211> 22
<212> PRT
<213> Artificial Sequence

<220>
<223> Melanocortin receptor ligand to treat insulin sensitivity

<220>
<221> MISC_FEATURE
<222> (1)..(1)
<223> xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-beta-cyclohexylAla (D-Cha)

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (22)..(22)
<223> AMIDATION

<400> 255

```
Xaa Cys Xaa His Xaa Arg Trp Cys Xaa Xaa Tyr Gly Arg Arg Arg Arg
1               5                   10                  15

Arg Gln Arg Arg Arg Arg
            20
```

<210> 256
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>

<221> MISC_FEATURE
<222> (3)..(3)
<223> Xaa = D-beta-cyclohexylAla (D-Cha)

<220>
<221> MISC_FEATURE
<222> (5)..(5)
<223> Xaa = D-Phe

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (20)..(20)
<223> AMIDATION

<400> 256

```
Xaa Cys Xaa His Xaa Arg Trp Cys Xaa Xaa Arg Arg Arg Arg Arg Gln
1               5                   10                  15

Arg Arg Arg Arg
            20
```

<210> 257
<211> 22
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-beta-cyclohexylAla (D-Cha)

<220>
<221> MISC_FEATURE

<222> (5)..(5)
<223> Xaa = D-Phe

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (22)..(22)
<223> AMIDATION

<400> 257

```
Xaa Cys Xaa His Xaa Arg Trp Cys Xaa Xaa Tyr Gly Arg Arg Arg Arg
1               5                   10                  15

Arg Gln Arg Arg Arg Arg
            20
```

<210> 258
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle modified with acyl (Ac)

<220>
<221> DOMAIN
<222> (2)..(8)
<223> cyclic

<220>
<221> MISC_FEATURE
<222> (3)..(3)
<223> Xaa = D-beta-cyclohexylAla (D-Cha)

<220>
<221> MISC_FEATURE
<222> (5)..(5)
<223> xaa = D-Phe

<220>
<221> MISC_FEATURE
<222> (9)..(9)

<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (20)..(20)
<223> AMIDATION

<400> 258

```
Xaa Cys Xaa His Xaa Arg Trp Cys Xaa Xaa Arg Arg Arg Arg Arg Gln
1               5                   10                  15

Arg Arg Arg Arg
            20
```

<210> 259
<211> 21
<212> PRT
<213> Artificial sequence

<220>
<223> Melanocortin receptor ligand to treat insulin sensitivity

<220>
<221> MISC_FEATURE
<222> (1)..(1)
<223> xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (21)..(21)
<223> AMIDATION

<400> 259

Xaa Cys His Xaa Arg Trp Xaa Cys Xaa Xaa Tyr Gly Arg Arg Arg Arg
1               5                   10                  15

Arg Gln Arg Arg Arg
            20

<210> 260
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (19)..(19)
<223> AMIDATION

<400> 260

Xaa Cys His Xaa Arg Trp Xaa Cys Xaa Xaa Arg Arg Arg Arg Arg Gln
1 5 10 15

Arg Arg Arg

<210> 261
<211> 22
<212> PRT
<213> Artificial Sequence

<220>
<223> Melanocortin receptor ligand to treat insulin sensitivity

<220>
<221> MISC_FEATURE
<222> (1)..(1)
<223> xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (22)..(22)
<223> AMIDATION

<400> 261

Xaa Cys His Xaa Arg Trp Xaa Cys Xaa Xaa Tyr Gly Arg Arg Arg Arg
1 5 10 15

Arg Gln Arg Arg Arg Arg
20

<210> 262
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = beta-Ala

<220>
<221> MISC_FEATURE
<222> (10)..(10)
<223> Xaa = beta-Ala or absent

<220>
<221> MOD_RES
<222> (20)..(20)
<223> AMIDATION

<400> 262

```
Xaa Cys His Xaa Arg Trp Xaa Cys Xaa Xaa Arg Arg Arg Arg Arg Gln
1               5                   10                  15

Arg Arg Arg Arg
            20
```

<210> 263
<211> 21
<212> PRT
<213> Artificial Sequence

<220>

<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (21)..(21)
<223> AMIDATION

<400> 263

```
Xaa Cys His Xaa Arg Trp Xaa Cys Xaa Xaa Tyr Gly Arg Arg Arg Arg
1               5                   10                  15

Arg Gln Arg Arg Arg
            20
```

<210> 264
<211> 19
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (19)..(19)
<223> AMIDATION

<400> 264

```
Xaa Cys His Xaa Arg Trp Xaa Cys Xaa Xaa Arg Arg Arg Arg Arg Gln
1               5                   10                  15

Arg Arg Arg
```

<210> 265
<211> 22
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE

<222> (4)..(4)
<223> Xaa = D-Phe

<220>
<221> MISC_FEATURE
<222> (7)..(7)
<223> Xaa = 4-aminobutyric acid (Gaba)

<220>
<221> MISC_FEATURE
<222> (9)..(9)
<223> Xaa = 8-amino-3,6-dioxaoctanoic acid (DOC)

<220>
<221> MISC_FEATURE
<222> (10)..(10)
<223> Xaa = 8-amino-3,6-dioxaoctanoic acid (Doc) or absent

<220>
<221> MOD_RES
<222> (22)..(22)
<223> AMIDATION

<400> 265

```
Xaa Cys His Xaa Arg Trp Xaa Cys Xaa Xaa Tyr Gly Arg Arg Arg Arg
1               5                   10                  15

Arg Gln Arg Arg Arg Arg
            20
```

<210> 266
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = Nle

<220>
<221> DOMAIN
<222> (2)..(8)
<223> cyclic

<220>
<221> MISC_FEATURE
<222> (4)..(4)
<223> Xaa = D-Phe

<220>
<221> MISC_FEATURE

<222> (7)..(7)
<223> Xaa = 4-aminobutyric acid (Gaba)

<220>
<221> MISC_FEATURE
<222> (9)..(9)
<223> Xaa = 8-amino-3,6-dioxaoctanoic acid (Doc)

<220>
<221> MISC_FEATURE
<222> (10)..(10)
<223> Xaa = 8-amino-3,6-dioxaoctanoic acid (Doc) or absent

<220>
<221> MOD_RES
<222> (20)..(20)
<223> AMIDATION

<400> 266

```
Xaa Cys His Xaa Arg Trp Xaa Cys Xaa Xaa Arg Arg Arg Arg Arg Gln
1               5                   10                  15

Arg Arg Arg Arg
            20
```

<210> 267
<211> 20
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Phe

<220>
<221> DOMAIN
<222> (2)..(8)
<223> cyclic

<220>
<221> MISC_FEATURE
<222> (4)..(4)
<223> Xaa = D-(Et)Tyr

<220>
<221> MISC_FEATURE
<222> (7)..(7)
<223> Xaa = beta-Ala

<220>
<221> MISC_FEATURE
<222> (8)..(8)

<223> Xaa = D-Cys

<220>
<221> MISC_FEATURE
<222> (9)..(10)
<223> Xaa = beta-Ala

<220>
<221> MOD_RES
<222> (20)..(20)
<223> AMIDATION

<400> 267

Xaa Cys His Xaa Arg Trp Xaa Xaa Xaa Xaa Arg Arg Arg Arg Arg Gln
1 5 10 15

Arg Arg Arg Arg
20

<210> 268
<211> 12
<212> PRT
<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (AC)

<220>
<221> MISC_FEATURE
<222> (4)..(4)
<223> Xaa = D-4-bromo-phenylAla (D-4-Br-Phe)

<220>
<221> MOD_RES
<222> (12)..(12)
<223> AMIDATION

<400> 268

Cys Glu His Xaa Arg Trp Gly Cys Pro Pro Lys Asp
1 5 10

<210> 269
<211> 12
<212> PRT

<213> Artificial Sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (Ac)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = Trp, beta-(2-naphthyl)Ala (2-Nal), beta-(1-naphthyl)Ala (1-Nal) or 3-benzothienylAla (Bal)

<220>
<221> MOD_RES
<222> (12)..(12)
<223> AMIDATION

<400> 269

```
Cys Glu His Xaa Arg Xaa Ala Cys Pro Pro Lys Asp
1               5                   10
```

<210> 270
<211> 12
<212> PRT
<213> Artificial sequence

<220>
<223> Melanocortin receptor ligand to treat insulin sensitivity

<220>
<221> DOMAIN
<222> (1)..(8)
<223> cyclic

<220>
<221> MOD_RES
<222> (1)..(1)
<223> modified with acyl (AC)

<220>
<221> MISC_FEATURE
<222> (4)..(4)
<223> Xaa = D-beta-(2-naphthyl)Ala (D-2-Nal)

<220>
<221> MISC_FEATURE
<222> (6)..(6)
<223> Xaa = beta-(2-naphthyl)Ala (2-Nal)

<220>
<221> MISC_FEATURE
<222> (7)..(7)
<223> Xaa = beta-Ala or alpha-aminoisobutyric acid (Aib)

<220>
<221> MOD_RES
<222> (12)..(12)
<223> AMIDATION

<400> 270

```
Cys Glu His Xaa Arg Xaa Xaa Cys Pro Pro Lys Asp
1               5                   10
```

<210> 271
<211> 7
<212> PRT
<213> Artificial Sequence

<220>
<223> Melanocortin receptor ligand to treat insulin sensitivity

<220>
<221> DOMAIN
<222> (1)..(7)
<223> cyclic

<220>
<221> MOD_RES
<222> (1)..(1)
<223> modified with hydantoin(CO)

<220>
<221> MISC_FEATURE
<222> (1)..(1)
<223> Xaa = Cys or homocyteine (hCys)

<220>
<221> MISC_FEATURE
<222> (2)..(2)
<223> Xaa = D-Ala

<220>
<221> MISC_FEATURE
<222> (4)..(4)
<223> Xaa = D-Phe

<220>
<221> MOD_RES
<222> (7)..(7)
<223> AMIDATION

<400> 271

Xaa Xaa His Xaa Arg Trp Cys
1               5

<210> 272
<211> 7
<212> PRT
<213> Artificial Sequence

<220>
<223> Melanocortin receptor ligand to treat insulin sensitivity

<220>
<221> DOMAIN
<222> (1)..(7)
<223> Cyclic

<220>
<221> MOD_RES
<222> (1)..(1)
<223> modified with hydantoin(CO)

<220>
<221> MISC_FEATURE
<222> (1)..(1)
<223> Xaa = Cys or homocysteine (hCys)

<220>
<221> MISC_FEATURE
<222> (2)..(2)
<223> Xaa = D-Ala

<220>
<221> MISC_FEATURE
<222> (4)..(4)
<223> Xaa = D-beta-(2-naphythyl)alanine (D-2-Nal)

<220>
<221> MOD_RES
<222> (7)..(7)
<223> AMIDATION

<400> 272

Xaa Xaa His Xaa Arg Trp Cys
1               5

<210> 273
<211> 7
<212> PRT
<213> Artificial Sequence

<220>
<223> Melanocortin receptor ligand to treat insulin sensitivity

<220>
<221> DOMAIN
<222> (1)..(7)
<223> cyclic

<220>
<221> MOD_RES
<222> (1)..(1)
<223> modified with hydantoin(CO)

<220>
<221> MISC_FEATURE
<222> (2)..(2)
<223> Xaa = D-Ala

<220>
<221> MISC_FEATURE
<222> (4)..(4)
<223> Xaa = D-Phe

<220>
<221> MISC_FEATURE
<222> (7)..(7)
<223> Xaa = Lys, ornithine (Orn), 2,4-diaminobutyric acid (Dab), or 2,3-diaminopropionic acid (Dap)

<220>
<221> MOD_RES
<222> (7)..(7)
<223> AMIDATION

<400> 273

```
Asp Xaa His Xaa Arg Trp Xaa
1               5
```

<210> 274
<211> 6
<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> DOMAIN
<222> (1)..(6)
<223> cyclic

<220>
<221> MISC_FEATURE
<222> (2)..(2)
<223> Xaa = His, 1-amino-1-cyclopropanecarboxylic acid (A3c), 1-amino-1cyclopentanecarboxylic acid (A5c), 1-amino-1-cyclohexanecarboxylic acid (A6c), 2-aminoindan-2-carboxylic acid (Aic), or Apc

<220>
<221> MISC_FEATURE
<222> (3)..(3)
<223> Xaa = D-Phe

<220>
<221> MOD_RES
<222> (6)..(6)
<223> AMIDATION

<400> 274

```
Asp Xaa Xaa Arg Trp Lys
1               5
```

<210> 275
<211> 6
<212> PRT
<213> Artificial Sequence

<220>
<223> Melanocortin receptor ligand to treat insulin sensitivity

<220>
<221> DOMAIN
<222> (1)..(6)
<223> cyclic

<220>
<221> MOD_RES
<222> (1)..(1)
<223> modified with hydantoin(CO)

<220>
<221> MISC_FEATURE
<222> (2)..(2)
<223> Xaa = His, 1-amino-1-cyclopropanecarboxylic acid (A3C), 1-amino-lcyclopentanecarboxylic acid (A5C), 1-amino-1-cyclohexanecarboxylic acid (A6C), 2-aminoindan-2-carboxylic acid (AiC), or ApC

<220>
<221> MISC_FEATURE
<222> (3)..(3)
<223> Xaa = D-beta-(2-naphythyl)alanine (D-2-Nal)

<220>
<221> MOD_RES
<222> (6)..(6)
<223> AMIDATION

<400> 275

```
Asp Xaa Xaa Arg Trp Lys
1               5
```

<210> 276
<211> 7

<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> DOMAIN
<222> (1)..(7)
<223> cyclic

<220>
<221> MISC_FEATURE
<222> (2)..(2)
<223> Xaa = D-Ala

<220>
<221> MISC_FEATURE
<222> (4)..(4)
<223> Xaa = D-Phe

<220>
<221> MISC_FEATURE
<222> (7)..(7)
<223> Xaa = Lys, ornithine (Orn), 2,4-diaminobutyric acid (Dab), or 2,3-diaminopropionic acid (Dap)

<220>
<221> MOD_RES
<222> (7)..(7)
<223> AMIDATION

<400> 276

```
Glu Xaa His Xaa Arg Trp Xaa
1               5
```

<210> 277
<211> 6
<212> PRT
<213> Artificial Sequence

<220>
<223> Melanocortin receptor ligand to treat insulin sensitivity

<220>
<221> DOMAIN
<222> (1)..(6)
<223> cyclic

<220>
<221> MOD_RES
<222> (1)..(1)

<223> modified with hydantoin(CO)

<220>
<221> MISC_FEATURE
<222> (3)..(3)
<223> Xaa = D-Phe

<220>
<221> MOD_RES
<222> (6)..(6)
<223> AMIDATION

<220>
<221> MISC_FEATURE
<222> (6)..(6)
<223> Xaa = Lys or 2,3-diaminopropionic acid (Dap)

<400> 277

```
Glu His Xaa Arg Trp Xaa
1               5
```

<210> 278
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = Arg, norleucine (Nle), Gly, or D-Arg

<220>
<221> MOD_RES
<222> (1)..(1)
<223> modified with hydantoin(CO)

<220>
<221> DOMAIN
<222> (3)..(9)
<223> cyclic

<220>
<221> MISC_FEATURE
<222> (6)..(6)
<223> Xaa = D-Phe

<220>
<221> MOD_RES
<222> (9)..(9)
<223> AMIDATION

<400> 278

Xaa Gly Cys Glu His Xaa Arg Trp Cys
1 5

<210> 279
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = norleucine (Nle), Gly, Ala, D-Ala, alpha-aminoisobutyric acid (Aib), Val, Ile, Leu, D-Arg, or Arg

<220>
<221> MOD_RES
<222> (1)..(1)
<223> modified with hydantoin(CO)

<220>
<221> DOMAIN
<222> (3)..(9)
<223> cyclic

<220>
<221> MISC_FEATURE
<222> (4)..(4)
<223> Xaa = D-Ala

<220>
<221> MISC_FEATURE
<222> (6)..(6)
<223> Xaa = D-Phe

<220>
<221> MOD_RES
<222> (9)..(9)
<223> AMIDATION

<400> 279

Xaa Gly Cys Xaa His Xaa Arg Trp Cys
1 5

<210> 280
<211> 9
<212> PRT
<213> Artificial sequence

<220>
<223> Melanocortin receptor ligand to treat insulin sensitivity

<220>
<221> MISC_FEATURE

<222> (1)..(1)
<223> Xaa = norleucine (Nle) or Gly

<220>
<221> MOD_RES
<222> (1)..(1)
<223> modified with hydantoin(CO)

<220>
<221> DOMAIN
<222> (3)..(9)
<223> cyclic

<220>
<221> MISC_FEATURE
<222> (4)..(4)
<223> Xaa = D-Ala

<220>
<221> MISC_FEATURE
<222> (6)..(6)
<223> Xaa = D-Phe

<220>
<221> MISC_FEATURE
<222> (9)..(9)
<223> Xaa = penicillamine (Pen)

<220>
<221> MOD_RES
<222> (9)..(9)
<223> AMIDATION

<400> 280

```
Xaa Gly Cys Xaa His Xaa Arg Trp Xaa
1               5
```

<210> 281
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = norleucine (Nle) or Gly

<220>
<221> MOD_RES
<222> (1)..(1)
<223> modified with hydantoin(CO)

<220>

<221> DOMAIN
<222> (3)..(9)
<223> cyclic

<220>
<221> MISC_FEATURE
<222> (6)..(6)
<223> Xaa = D-beta-(2-naphythyl)alanine (D-2-Nal)

<220>
<221> MOD_RES
<222> (9)..(9)
<223> AMIDATION

<400> 281

```
Xaa Gly Cys Glu His Xaa Arg Trp Cys
1               5
```

<210> 282
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = D-Arg or Arg

<220>
<221> MOD_RES
<222> (1)..(1)
<223> modified with hydantoin(CO)

<220>
<221> DOMAIN
<222> (3)..(9)
<223> cyclic

<220>
<221> MISC_FEATURE
<222> (4)..(4)
<223> Xaa = D-Ala

<220>
<221> MISC_FEATURE
<222> (6)..(6)
<223> Xaa = D-beta-(2-naphythyl)alanine (D-2-Nal)

<220>
<221> MOD_RES
<222> (9)..(9)
<223> AMIDATION

<400> 282

Xaa Gly Cys Xaa His Xaa Arg Trp Cys
1 5

<210> 283
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = Ala, Val, or Gly

<220>
<221> MOD_RES
<222> (1)..(1)
<223> modified with hydantoin(CO)

<220>
<221> MISC_FEATURE
<222> (2)..(2)
<223> Xaa = norleucine (Nle)

<220>
<221> DOMAIN
<222> (3)..(9)
<223> cyclic

<220>
<221> MISC_FEATURE
<222> (6)..(6)
<223> Xaa = D-Phe

<220>
<221> MOD_RES
<222> (9)..(9)
<223> AMIDATION

<400> 283

Xaa Xaa Cys Glu His Xaa Arg Trp Cys
1 5

<210> 284
<211> 9
<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> MISC_FEATURE
<222> (1)..(1)
<223> Xaa = 1-amino-1-cyclohexanecarboxylic acid (A6C), Gly, Ala, D-Ala, Val, Leu, beta-cyclohexylalanine (Cha), or alpha-aminoisobutyric acid (Aib)

<220>
<221> MISC_FEATURE
<222> (2)..(2)
<223> Xaa = norleucine (Nle)

<220>
<221> DOMAIN
<222> (3)..(9)
<223> cyclic

<220>
<221> MISC_FEATURE
<222> (4)..(4)
<223> xaa = D-Ala

<220>
<221> MISC_FEATURE
<222> (6)..(6)
<223> Xaa = D-Phe

<220>
<221> MOD_RES
<222> (9)..(9)
<223> AMIDATION

<400> 284

```
Xaa Xaa Cys Xaa His Xaa Arg Trp Cys
1               5
```

<210> 285
<211> 9
<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> DOMAIN
<222> (3)..(9)

<223> cyclic

<220>
<221> MISC_FEATURE
<222> (6)..(6)
<223> xaa = D-Phe or D-beta-(2-naphythyl)alanine (D-2-Nal)

<220>
<221> MOD_RES
<222> (9)..(9)
<223> AMIDATION

<400> 285

```
Gly Arg Cys Glu His Xaa Arg Trp Cys
1               5
```

<210> 286
<211> 9
<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> DOMAIN
<222> (3)..(9)
<223> cyclic

<220>
<221> MISC_FEATURE
<222> (4)..(4)
<223> Xaa = D-Ala

<220>
<221> MISC_FEATURE
<222> (6)..(6)
<223> Xaa = D-Phe or D-beta-(2-naphythyl)alanine (D-2-Nal)

<220>
<221> MOD_RES
<222> (9)..(9)
<223> AMIDATION

<400> 286

```
Gly Arg Cys Xaa His Xaa Arg Trp Cys
1               5
```

<210> 287

<211> 9
<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> MISC_FEATURE
<222> (2)..(2)
<223> Xaa = D-Arg

<220>
<221> DOMAIN
<222> (3)..(9)
<223> cyclic

<220>
<221> MISC_FEATURE
<222> (6)..(6)
<223> Xaa = D-Phe

<220>
<221> MOD_RES
<222> (9)..(9)
<223> AMIDATION

<400> 287

```
Gly Xaa Cys Glu His Xaa Arg Trp Cys
1               5
```

<210> 288
<211> 9
<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> MISC_FEATURE
<222> (2)..(2)
<223> Xaa = D-Arg

<220>
<221> DOMAIN

<222> (3)..(9)
<223> cyclic

<220>
<221> MISC_FEATURE
<222> (4)..(4)
<223> Xaa = D-Ala

<220>
<221> MISC_FEATURE
<222> (6)..(6)
<223> Xaa = D-Phe or D-beta-(2-naphythyl)alanine (D-2-Nal)

<220>
<221> MOD_RES
<222> (9)..(9)
<223> AMIDATION

<400> 288

```
Gly Xaa Cys Xaa His Xaa Arg Trp Cys
1               5
```

<210> 289
<211> 9
<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> MISC_FEATURE
<222> (1)..(1)
<223> Xaa = norleucine (Nle)

<220>
<221> DOMAIN
<222> (3)..(9)
<223> cyclic

<220>
<221> MISC_FEATURE
<222> (6)..(6)
<223> Xaa = D-Phe

<220>
<221> MOD_RES
<222> (9)..(9)
<223> AMIDATION

<400> 289

Xaa Ala Cys Glu His Xaa Arg Trp Cys
1               5

<210> 290
<211> 8
<212> PRT
<213> Artificial Sequence

<220>
<223> Melanocortin receptor ligand to treat insulin sensitivity

<220>
<221> MOD_RES
<222> (1)..(1)
<223> modified with hydantoin(CO)

<220>
<221> MISC_FEATURE
<222> (1)..(1)
<223> Xaa = norleucine (Nle), Ala, D-Ala, alpha-aminoisobutyric acid (Aib), Val, alpha-aminobutyric acid (Abu), Leu, Ile, beta-cyclohexylalanine (Cha), 1-amino-1-cyclohexanecarboxylic acid (A6C), Phe, or Gly

<220>
<221> DOMAIN
<222> (1)..(8)
<223> cyclic

<220>
<221> MISC_FEATURE
<222> (3)..(3)
<223> Xaa = D-Ala

<220>
<221> MISC_FEATURE
<222> (5)..(5)
<223> Xaa = D-Phe

<220>
<221> MOD_RES
<222> (8)..(8)
<223> AMIDATION

<400> 290

Xaa Cys Xaa His Xaa Arg Trp Cys
1               5

<210> 291
<211> 8
<212> PRT
<213> Artificial sequence

<220>
<223> Melanocortin receptor ligand to treat insulin sensitivity

<220>

<221> DOMAIN
<222> (1)..(8)
<223> cyclic

<220>
<221> MOD_RES
<222> (1)..(1)
<223> modified with hydantoin(CO)

<220>
<221> MISC_FEATURE
<222> (5)..(5)
<223> Xaa = D-Phe

<220>
<221> MOD_RES
<222> (8)..(8)
<223> AMIDATION

<400> 291

```
Gly Cys Glu His Xaa Arg Trp Cys
1               5
```

<210> 292
<211> 9
<212> PRT
<213> Artificial Sequence

<220>
<223> Melanocortin receptor ligand to treat insulin sensitivity

<220>
<221> MISC_FEATURE
<222> (1)..(1)
<223> Xaa = Tyr, beta-(2-naphthyl)alanine (2-Nal), beta-(1-naphthyl)alanine (1-Nal), Phe, Trp, (S)-pentatluorophenylalanine (Pff), or His, all modified with acyl group (AC)

<220>
<221> DOMAIN
<222> (3)..(9)
<223> cylic

<220>
<221> MISC_FEATURE
<222> (4)..(4)
<223> Xaa = D-Ala

<220>
<221> MISC_FEATURE
<222> (6)..(6)
<223> Xaa = D-Phe

<220>
<221> MOD_RES
<222> (9)..(9)
<223> AMIDATION

<400> 292

```
Xaa Arg Cys Xaa His Xaa Arg Trp Cys
1               5
```

<210> 293
<211> 9
<212> PRT
<213> Artificial Sequence

<220>
<223> Melanocortin receptor ligand to treat insulin sensitivity

<220>
<221> DOMAIN
<222> (3)..(9)
<223> cyclic

<220>
<221> MISC_FEATURE
<222> (4)..(4)
<223> Xaa = D-Ala

<220>
<221> MISC_FEATURE
<222> (6)..(6)
<223> Xaa = D-Phe

<220>
<221> MOD_RES
<222> (9)..(9)
<223> AMIDATION

<400> 293

```
His Arg Cys Xaa His Xaa Arg Trp Cys
1               5
```

## Description

BACKGROUND OF THE INVENTION

Melanocortins are a family of regulatory peptides which are formed by post-translational processing of prohormone pro-opiomelanocortin (POMC; 131 amino acids in length). POMC is processed into three classes of hormones; the melanocortins, adrenocorticotropin hormone, and various endorphins (e.g. lipotropin) (Cone et al., Recent Prog. Horm. Res., 51:287-317, (1996); Cone et al., Ann. N.Y. Acad. Sci., 31:342-363, (1993)).
Five melanocortin receptors (MC-R) have been characterized to date. These include melanocyte-specific receptor (MC1-R), corticoadrenal-specific ACTH receptor (MC2-R), melacortin-3 (MC3-R), melanocortin-4 (MC4-R) and melanocortin-5 receptor (MC5-R). All of the melanocortin receptors respond to the peptide hormone class of melanocyte stimulating hormones (MSH) (Cone et al., Ann. N.Y. Acad. Sci., 680:342-363 (1993); Cone et al., Recent Prog. Horm. Res., 51:287-318 (1996)).
There has been great interest in melanocortin (MC-R) receptors as targets for the design of novel therapeutics to treat disorders of body weight such as obesity and cachexia. One of the receptors, MC4-R, is a 332 amino acid transmembrane protein expressed in brain as well as placental and gut tissues (Cone et al., Ann. N.Y. Acad. Sci., 680:342-363 (1993); Cone et al., Recent Prog. Horm. Res., 51:287-318 (1996)). Recent pharmacological confirmation has established that central MC4-R receptors are the prime mediators of the anorexic and orexigenic effects reported for melanocortin agonists and antagonists, respectively (Giraudo et al., Brain Res., 809:302-306 (1998); Farooqi et al., NE J Med., 348:1085-1095 (2003); MacNeil et al., Eu. J. Pharm., 44:141-157 (2002); MacNeil et al., Eu. J. Pharm., 450:93-109 (2002); Kask et al., NeuroReport, 10:707-711 (1999); Chen et al.,. Transgenic Res., 9:145-54, (2000); Marsh et al., Nat Genet., 21:119-22, (1999); Balthasar et al., Cell, 123:493-505 (2005)).
Complications of body weight disorders commonly include an inability to produce and utilize insulin, often resulting in faulty glucose regulation. The consequence of failure to properly control glucose metabolism affects many aspects of overall health including energy metabolism, neuropathy and heart disease. Current progress with receptor-selective melanocortin receptor ligands evidences the therapeutic potential of melanocortin receptor activation, particularly MC4-R, in the treatment of glucose regulation, including insulin metabolism.

SUMMARY OF THE INVENTION

Described herein is the use of peptides which are ligands of one or more of the melanocortin receptors (MC-R), or the pharmaceutically-acceptable salts thereof, to treat mammals suffering from insulin resistance. The ligands may be agonists to the melanocortin 4 receptor. The melanocortin receptor ligands may be selected from particular peptides described herein.
The insulin resistant subject mammals may be obese or overweight and may lose weight as a result of the administration of the peptides herein. The insulin resistant subject mammals may also be normal weight or lean. The insulin resistant condition of the subject mammals may be treated independent of weight loss. In addition, the subject mammals may be human subjects of any age, such as an infant, a child, an adult or an elderly adult.
Accordingly the present invention provides a therapeutically effective amount of a melanocortin receptor 4 agonist for use in the treatment of insulin resistance in a subject by peripheral administration wherein said melanocortin receptor 4 agonist is:

Ac-Arg-c(Cys-D-Ala-His-D-Phe-Arg-Trp-Cys)-$NH_2$; SEQ ID NO:50;

or a pharmaceutically acceptable salt thereof.
The subject may be obese, overweight, of normal weight or lean, and the obese, overweight, normal weight or lean subject may suffer from type II diabetes. The peripheral administration may be oral, subcutaneous, intraperitoneal, intramuscular, intravenous, rectal, transdermal or intranasal. The oral, subcutaneous, intraperitoneal, intramuscular, intravenous, rectal, transdermal or intranasal peripheral administration may be continuous, hourly, four times daily, three time daily, twice daily, once daily, once every other day, twice weekly, once weekly, once every two weeks, once a month, or once every two months. The peripheral administration to treat insulin resistance may also reduce the body weight of the subject.
Also described herein is a method to treat insulin resistance in a mammalian subject, with or without weight loss, by the administration of a therapeutically effective amount of a melanocortin receptor 4 ligand described herein and pharmaceutically acceptable salts, hydrates, solvates or prodrugs thereof, wherein the ligand is a compound of the formula:

Ac-Nle-c(Asp-His-D-Phe-Arg-Trp-β-Ala-Lys)-$NH_2$; SEQ ID NO:1

Ac-Nle-c(Asp-His-D-Phe-Arg-Trp-A6c-Lys)-$NH_2$; SEQ ID NO:1
Ac-Nle-c(Cys-His-D-Phe-Arg-Trp-Ahx-Cys)-$NH_2$; SEQ ID NO:2
D-Phe-c(Cys-His-D-Phe-Arg-Trp-Ala-D-Cys)-Thr-$NH_2$; SEQ ID NO:3
D-Phe-c(Cys-His-D-Phe-Arg-Trp-β-Ala-D-Cys)-Thr-$NH_2$; SEQ ID NO:3
D-Phe-c(Cys-His-D-Phe-Arg-Trp-Gaba-D-Cys)-Thr-$NH_2$; SEQ ID NO:3
Ac-Nle-c(Cys-His-D-Phe-Arg-Trp-Apn-Cys)-$NH_2$; SEQ ID NO:2
Ac-Nle-c(Asp-His-D-Phe-Arg-Trp-Apn-Lys)-$NH_2$; SEQ ID NO:4
Ac-A6c-c(Asp-His-D-Phe-Arg-Trp-Gaba-Lys)-$NH_2$; SEQ ID NO:5
Ac-D-2-Nal-c(Asp-His-D-Phe-Arg-Trp-Gaba-Lys)-$NH_2$; SEQ ID NO:6
Ac-Cha-c(Asp-His-D-Phe-Arg-Trp-Gaba-Lys)-$NH_2$; SEQ ID NO:6
Ac-Nle-c(Asp-His-D-Phe-Arg-Trp-Gaba-Lys)-$NH_2$; SEQ ID NO:6
Ac-Nle-c(Cys-D-Ala-His-D-Phe-Arg-Trp-Cys)-$NH_2$; SEQ ID NO:7
Ac-Nle-c(Cys-β-Ala-His-D-Phe-Arg-Trp-Cys)-$NH_2$; SEQ ID NO:7
Ac-Nle-c(Cys-Gaba-His-D-Phe-Arg-Trp-Cys)-$NH_2$; SEQ ID NO:7
Ac-Nle-c(Cys-Aib-His-D-Phe-Arg-Trp-Cys)-$NH_2$; SEQ ID NO:7
Ac-Nle-c(Cys-Gly-His-D-Phe-Arg-Trp-Cys)-$NH_2$; SEQ ID NO:7
Ac-Nle-c(D-Cys-Ala-His-D-Phe-Arg-Trp-Cys)-$NH_2$; SEQ ID NO:8
Ac-Nle-c(D-Cys-D-Ala-His-D-Phe-Arg-Trp-Cys)-$NH_2$; SEQ ID NO:8
Ac-Nle-c(D-Cys-β-Ala-His-D-Phe-Arg-Trp-Cys)-$NH_2$; SEQ ID NO:8
Ac-Nle-c(D-Cys-Gaba-His-D-Phe-Arg-Trp-Cys)-$NH_2$; SEQ ID NO:8
Ac-Nle-c(D-Cys-Aib-His-D-Phe-Arg-Trp-Cys)-$NH_2$; SEQ ID NO:8
Ac-Nle-c(D-Cys-Gly-His-D-Phe-Arg-Trp-Cys)-$NH_2$; SEQ ID NO:8
Ac-Nle-c(Cys-D-Ala-His-D-Phe-Arg-Trp-D-Cys)-$NH_2$; SEQ ID NO:9
Ac-Nle-c(Cys-β-Ala-His-D-Phe-Arg-Trp-D-Cys)-$NH_2$; SEQ ID NO:9
Ac-Nle-c(Cys-Gaba-His-D-Phe-Arg-Trp-D-Cys)-$NH_2$; SEQ ID NO:9
Ac-Nle-c(Cys-Aib-His-D-Phe-Arg-Trp-D-Cys)-$NH_2$; SEQ ID NO:9
Ac-Nle-c(Cys-Gly-His-D-Phe-Arg-Trp-D-Cys)-$NH_2$; SEQ ID NO:9
Ac-Nle-c(D-Cys-Ala-His-D-Phe-Arg-Trp-D-Cys)-$NH_2$; SEQ ID NO:10
Ac-Nle-c(D-Cys-D-Ala-His-D-Phe-Arg-Trp-D-Cys)-$NH_2$; SEQ ID NO:10
Ac-Nle-c(D-Cys-β-Ala-His-D-Phe-Arg-Trp-D-Cys)-$NH_2$; SEQ ID NO:10
Ac-Nle-c(D-Cys-Gaba-His-D-Phe-Arg-Trp-D-Cys)-$NH_2$; SEQ ID NO:10
Ac-Nle-c(D-Cys-Aib-His-D-Phe-Arg-Trp-D-Cys)-$NH_2$; SEQ ID NO:10
Ac-Oic-c(Asp-His-D-Phe-Arg-Trp-Gaba-Lys)-$NH_2$; SEQ ID NO:11
Ac-Chg-c(Asp-His-D-Phe-Arg-Trp-Gaba-Lys)-$NH_2$; SEQ ID NO:11
Ac-hCha-c(Asp-His-D-Phe-Arg-Trp-Gaba-Lys)-$NH_2$; SEQ ID NO:11
Ac-D-Cha-c(Asp-His-D-Phe-Arg-Trp-Gaba-Lys)-$NH_2$; SEQ ID NO:11
Ac-Nip-c(Asp-His-D-Phe-Arg-Trp-Gaba-Lys)-$NH_2$; SEQ ID NO:11
Ac-hPro-c(Asp-His-D-Phe-Arg-Trp-Gaba-Lys)-$NH_2$; SEQ ID NO:11
Ac-hLeu-c(Asp-His-D-Phe-Arg-Trp-Gaba-Lys)-$NH_2$; SEQ ID NO:11
Ac-hCha-c(Asp-His-D-Phe-Arg-Trp-Gaba-Lys)-$NH_2$; SEQ ID NO:11
Ac-Phe-c(Asp-His-D-Phe-Arg-Trp-Gaba-Lys)-$NH_2$; SEQ ID NO:11
Ac-D-Phe-c(Asp-His-D-Phe-Arg-Trp-Gaba-Lys)-$NH_2$; SEQ ID NO:11
Ac-D-Chg-c(Asp-His-D-Phe-Arg-Trp-Gaba-Lys)-$NH_2$; SEQ ID NO:11
n-butanoyl-Cha-c(Asp-His-D-Phe-Arg-Trp-Gaba-Lys)-$NH_2$; SEQ ID NO:12
Ac-hPhe-c(Asp-His-D-Phe-Arg-Trp-Gaba-Lys)-$NH_2$; SEQ ID NO:11
Ac-β-hMet-c(Asp-His-D-Phe-Arg-Trp-Gaba-Lys)-$NH_2$; SEQ ID NO:11
Ac-Gaba-c(Asp-His-D-Phe-Arg-Trp-Gaba-Lys)-$NH_2$; SEQ ID NO:11
Ac-Cha-c(Asp-His-D-Phe-Arg-D-Trp-Ala-Lys)-$NH_2$; SEQ ID NO:13
Ac-hCha-c(Asp-His-D-Phe-Arg-D-Trp-Ala-Lys)-$NH_2$; SEQ ID NO:13
Ac-Leu-c(Asp-His-D-Phe-Arg-D-Trp-Ala-Lys)-$NH_2$; SEQ ID NO:13
Ac-hLeu-c(Asp-His-D-Phe-Arg-D-Trp-Ala-Lys)-$NH_2$; SEQ ID NO:13
Ac-Phe-c(Asp-His-D-Phe-Arg-D-Trp-Ala-Lys)-$NH_2$; SEQ ID NO:13
Ac-Nle-c(Asp-His-D-Phe-Arg-D-Trp-D-Ala-Lys)-$NH_2$; SEQ ID NO:14
Ac-Nle-c(Asp-His-D-Phe-Arg-D-Trp-β-Ala-Lys)-$NH_2$; SEQ ID NO:14
Ac-Nle-c(Asp-His-D-Phe-Arg-D-Trp-Gaba-Lys)-$NH_2$; SEQ ID NO:14
Ac-Nle-c(Asp-His-D-Phe-Arg-D-Trp-Aha-Lys)-$NH_2$; SEQ ID NO:14
Ac-Nle-c(Asp-His-D-Phe-Arg-D-Trp-Apn-Lys)-$NH_2$; SEQ ID NO:14
Ac-Nle-c(Cys-His-D-Phe-Arg-D-Trp-Apn-Cys)-$NH_2$; SEQ ID NO:15

Ac-Nle-c(Cys-His-D-Phe-Arg-D-Trp-Gaba-Cys)-$NH_2$; SEQ ID NO:15
Ac-Nle-c(Cys-His-D-Phe-Arg-D-Trp-Ahx-Cys)-$NH_2$; SEQ ID NO:15
Ac-Nle-c(Cys-His-D-Phe-Arg-D-Trp-β-Ala-Cys)-$NH_2$; SEQ ID NO:15
Ac-Nle-c(Cys-His-D-Phe-Arg-D-Trp-D-Ala-Cys)-$NH_2$; SEQ ID NO:15
Ac-Nle-c(Cys-D-Ala-His-D-2-Nal-Arg-Trp-Cys)-$NH_2$; SEQ ID NO:16
Ac-Nle-c(Cys-D-Ala-His-D-2-Nal-Arg-2-Nal-Cys)-$NH_2$; SEQ ID NO:16
Ac-Nle-c(Cys-D-Ala-His-D-2-Nal-Arg-1-Nal-Cys)-$NH_2$; SEQ ID NO:16
n-butanoyl-Nle-c(Cys-D-Ala-His-D-Phe-Arg-2-Nal-Cys)-$NH_2$; SEQ ID NO:17
n-butanoyl-Nle-c(Cys-D-Ala-His-D-Phe-Arg-Trp-Cys)-$NH_2$; SEQ ID NO:17
Ac-Nle-c(Cys-D-Ala-His-D-Phe-Arg-2-Nal-Cys)-$NH_2$; SEQ ID NO:18
Ac-Nle-c(Cys-D-Ala-His-D-Phe-Arg-1-Nal-Cys)-$NH_2$; SEQ ID NO:18
Ac-Nle-c(Cys-D-Ala-His-D-Phe-Arg-Bal-Cys)-$NH_2$; SEQ ID NO:18
Ac-Nle-c(Cys-D-Glu-His-D-Phe-Arg-Trp-Cys)-$NH_2$; SEQ ID NO:61
Ac-Nle-c(Asp-His-D-Phe-Arg-Trp-D-Ala-Lys)-$NH_2$; SEQ ID NO:19
Ac-Nle-c(Cys-D-Ala-His-D-2-Nal-Arg-Bal-Cys)-$NH_2$; SEQ ID NO:20
Ac-Nle-c(Pen-D-Ala-His-D-Phe-Arg-Trp-Cys)-$NH_2$; SEQ ID NO:21
Ac-Nle-c(Cys-D-Ala-His-D-Phe-Arg-Trp-Pen)-$NH_2$; SEQ ID NO:22
Ac-Nle-c(Pen-D-Ala-His-D-Phe-Arg-Trp-Pen)-$NH_2$; SEQ ID NO:22
D-Phe-c(Cys-His-D-Phe-hArg-Trp-β-Ala-D-Cys)-Thr-$NH_2$; SEQ ID NO:23
D-Phe-c(Cys-His-D-(Et)Tyr-Arg-Trp-β-Ala-D-Cys)-Thr-$NH_2$; SEQ ID NO:24
D-Phe-c(Cys-His-D-Phe-Arg-Bip-β-Ala-D-Cys)-Thr-$NH_2$; SEQ ID NO:25
D-Phe-c(Cys-His-D-(Et)Tyr-hArg-Trp-β-Ala-D-Cys)-Thr-$NH_2$; SEQ ID NO:24
D-Phe-c(Cys-His-D-Phe-hArg-Bip-β-Ala-D-Cys)-Thr-$NH_2$; SEQ ID NO:26
D-Phe-c(Cys-His-D-(Et)Tyr-hArg-Bip-β-Ala-D-Cys)-Thr-$NH_2$; SEQ ID NO:26
Nle-c(Cys-His-D-Phe-Arg-Trp-Apn-Cys)-$NH_2$; SEQ ID NO:27
Ac-Nle-c(Asp-D-Ala-His-D-Phe-Arg-Trp-Lys)-$NH_2$; SEQ ID NO:28
Ac-Nle-c(Asp-D-Ala-His-D-Phe-Arg-Bal-Lys)-$NH_2$; SEQ ID NO:28
Ac-Nle-c(Cys-D-Ala-His-D-Phe-Arg-Trp-Pen)-OH; SEQ ID NO:29
Ac-Nle-c(Cys-D-Abu-His-D-Phe-Arg-Trp-Cys)-$NH_2$; SEQ ID NO:30
Ac-Nle-c(Cys-D-Val-His-D-Phe-Arg-Trp-Cys)-$NH_2$; SEQ ID NO:30
Ac-Nle-c(Cys-D-Ile-His-D-Phe-Arg-Trp-Cys)-$NH_2$; SEQ ID NO:30
Ac-Nle-c(Cys-D-Leu-His-D-Phe-Arg-Trp-Cys)-$NH_2$; SEQ ID NO:30
Ac-Nle-c(Cys-D-Tle-His-D-Phe-Arg-Trp-Cys)-$NH_2$; SEQ ID NO:30
Ac-Nle-c(Cys-D-Cha-His-D-Phe-Arg-Trp-Cys)-$NH_2$; SEQ ID NO:30
Ac-Nle-c(Pen-His-D-Phe-Arg-Trp-Gaba-Cys)-$NH_2$; SEQ ID NO:31
Ac-Nle-c(Cys-His-D-Phe-Arg-Trp-Gaba-Pen)-$NH_2$; SEQ ID NO:32
Ac-Nle-c(Pen-His-D-Phe-Arg-Trp-Gaba-Pen)-$NH_2$; SEQ ID NO:32
Ac-Leu-c(Cys-His-D-Phe-Arg-Trp-Gaba-Cys)-$NH_2$; SEQ ID NO:33
Ac-Cha-c(Cys-His-D-Phe-Arg-Trp-Gaba-Cys)-$NH_2$; SEQ ID NO:33
Ac-Ile-c(Cys-His-D-Phe-Arg-Trp-Gaba-Cys)-$NH_2$; SEQ ID NO:33
Ac-Phe-c(Cys-His-D-Phe-Arg-Trp-Gaba-Cys)-$NH_2$; SEQ ID NO:33
Ac-Val-c(Cys-His-D-Phe-Arg-Trp-Gaba-Cys)-$NH_2$; SEQ ID NO:33
Ac-2-Nal-c(Cys-His-D-Phe-Arg-Trp-Gaba-Cys)-$NH_2$; SEQ ID NO:33
Nle-c(Cys-His-D-Phe-Arg-Trp-Gaba-Cys)-$NH_2$; SEQ ID NO:34
Phe-c(Cys-His-D-Phe-Arg-Trp-Gaba-Cys)-$NH_2$; SEQ ID NO:34
Ac-Nle-c(Cys-3-Pal-D-Phe-Arg-Trp-Gaba-Cys)-$NH_2$; SEQ ID NO:35
Ac-Nle-c(Cys-D-Ala-His-D-Phe-Arg-Trp-Cys)-OH; SEQ ID NO:36
Ac-Nle-c(Cys-His-Phe-Arg-D-Trp-Gaba-Cys)-$NH_2$; SEQ ID NO:37
Ac-Nle-c(Asp-His-D-2-Nal-Arg-Trp-Ala-Lys)-$NH_2$; SEQ ID NO:38
Ac-Nle-c(Asp-His-D-2-Nal-Arg-Trp-β-Ala-Lys)-$NH_2$; SEQ ID NO:38
Ac-Nle-c(Cys-His-D-2-Nal-Arg-Trp-Gaba-Cys)-$NH_2$; SEQ ID NO:39
Ac-Nle-c(Cys-His-D-2-Nal-Arg-Trp-Ahx-Cys)-$NH_2$; SEQ ID NO:39
Ac-hPhe-c(Asp-His-D-2-Nal-Arg-Trp-Gaba-Lys)-$NH_2$; SEQ ID NO:40
Ac-Cha-c(Asp-His-D-2-Nal-Arg-Trp-Gaba-Lys)-$NH_2$; SEQ ID NO:40
Ac-Nle-c(Asp-His-D-Phe-Arg-Trp-β-Ala-Lys)-OH; SEQ ID NO:41
Ac-Nle-c(Cys-His-D-Phe-Arg-Trp-Ahx-Cys)-OH; SEQ ID NO:42
D-Phe-c(Cys-His-D-Phe-Arg-Trp-Ala-D-Cys)-Thr-OH; SEQ ID NO:43
D-Phe-c(Cys-His-D-Phe-Arg-Trp-β-Ala-D-Cys)-Thr-OH; SEQ ID NO:43

D-Phe-c(Cys-His-D-Phe-Arg-Trp-Gaba-D-Cys)-Thr-OH; SEQ ID NO:43
Ac-Nle-c(Cys-His-D-Phe-Arg-Trp-Apn-Cys)-OH; SEQ ID NO:42
Ac-Nle-c(Asp-His-D-Phe-Arg-Trp-Apn-Lys)-OH; SEQ ID NO:41
Ac-Cha-c(Asp-His-D-Phe-Arg-Trp-Gaba-Lys)-OH; SEQ ID NO:44
Ac-Nle-c(Asp-His-D-Phe-Arg-Trp-Gaba-Lys)-OH; SEQ ID NO:44
Ac-Nle-c(Cys-D-Ala-His-D-Phe-Arg-Trp-Cys)-OH; SEQ ID NO:29
Ac-Chg-c(Asp-His-D-Phe-Arg-Trp-Gaba-Lys)-OH; SEQ ID NO:44
Ac-D-Cha-c(Asp-His-D-Phe-Arg-Trp-Gaba-Lys)-OH; SEQ ID NO:44
Ac-hCha-c(Asp-His-D-Phe-Arg-Trp-Gaba-Lys)-OH; SEQ ID NO:44
Ac-D-Chg-c(Asp-His-D-Phe-Arg-Trp-Gaba-Lys)-OH; SEQ ID NO:44
Ac-hPhe-c(Asp-His-D-Phe-Arg-Trp-Gaba-Lys)-OH; SEQ ID NO:44
Ac-Nle-c(Cys-His-D-Phe-Arg-D-Trp-Gaba-Cys)-OH; SEQ ID NO:45
Ac-Nle-c(Cys-His-D-Phe-Arg-D-Trp-Ahx-Cys)-OH; SEQ ID NO:45
Ac-Nle-c(Cys-His-D-Phe-Arg-D-Trp-β-Ala-Cys)-OH; SEQ ID NO:45
Ac-Nle-c(Cys-His-D-Phe-Arg-D-Trp-D-Ala-Cys)-OH; SEQ ID NO:45
Ac-Nle-c(Cys-D-Ala-His-D-2-Nal-Arg-Trp-Cys)-OH; SEQ ID NO:46
Ac-Nle-c(Cys-D-Ala-His-D-2-Nal-Arg-2-Nal-Cys)-OH; SEQ ID NO:46
Ac-Nle-c(Cys-D-Ala-His-D-2-Nal-Arg-1-Nal-Cys)-OH; SEQ ID NO:46
Ac-Nle-c(Cys-D-Ala-His-D-2-Nal-Arg-Bal-Cys)-OH; SEQ ID NO:46
Ac-Nle-c(Pen-D-Ala-His-D-Phe-Arg-Trp-Cys)-OH; SEQ ID NO:47
Ac-Nle-c(Cys-D-Ala-His-D-Phe-Arg-Trp-Pen)-OH; SEQ ID NO:29
Ac-Nle-c(Cys-His-D-Phe-Arg-Trp-Gaba-Pen)-OH; SEQ ID NO:48
Ac-Arg-c(Cys-D-Ala-His-D-2-Nal-Arg-Trp-Cys)-$NH_2$; SEQ ID NO:49
Ac-Arg-c(Cys-D-Ala-His-D-Phe-Arg-Trp-Cys)-$NH_2$; SEQ ID NO:50
Ac-D-Arg-c(Cys-D-Ala-His-D-Phe-Arg-Trp-Cys)-$NH_2$; SEQ ID NO:50
Ac-D-Arg-c(Cys-D-Ala-His-D-Phe-Arg-Trp-Pen)-$NH_2$; SEQ ID NO:51
Ac-D-Arg-c(Cys-His-D-Phe-Arg-Trp-Gaba-Pen)-$NH_2$; SEQ ID NO:52
Ac-Arg-c(Cys-His-D-Phe-Arg-Trp-Gaba-Pen)-$NH_2$; SEQ ID NO:52
Ac-Arg-c(Cys-D-Ala-His-D-Phe-Arg-Trp-Pen)-$NH_2$; SEQ ID NO:51
Ac-D-Arg-c(Asp-His-D-Phe-Arg-Trp-Ala-Lys)-$NH_2$; SEQ ID NO:53
Ac-Arg-c(Asp-His-D-Phe-Arg-Trp-Ala-Lys)-$NH_2$; SEQ ID NO:53
Ac-Nle-c(Cys-D-Ala-His-D-Phe-Arg-Trp-Cys)-$NH_2$; SEQ ID NO:7
D-Phe-c(Cys-His-D-(Et)Tyr-Arg-Trp-β-Ala-D-Cys)-Thr-$NH_2$; SEQ ID NO:24
Nle-c(Cys-His-D-Phe-Arg-Trp-Apn-Cys)-$NH_2$; SEQ ID NO:27
Ac-Nle-c(Cys-His-D-Phe-Arg-Trp-Gaba-Pen)-$NH_2$; SEQ ID NO:32
Nle-c(Cys-His-D-Phe-Arg-Trp-Gaba-Cys)-$NH_2$; SEQ ID NO:34
Ac-Nle-c(Asp-His-D-Phe-Arg-Trp-β-Ala-Lys)-$NH_2$; SEQ ID NO:1
Ac-Nle-c(Cys-His-D-Phe-Arg-Trp-Ahx-Cys)-$NH_2$; SEQ ID NO:2
D-Phe-c(Cys-His-D-Phe-Arg-Trp-β-Ala-D-Cys)-Thr-$NH_2$; SEQ ID NO:3
D-Phe-c(Cys-His-D-Phe-Arg-Trp-Gaba-D-Cys)-Thr-$NH_2$; SEQ ID NO:3
Ac-Nle-c(Cys-His-D-Phe-Arg-Trp-Apn-Cys)-$NH_2$; SEQ ID NO:2
Ac-Nle-c(Asp-His-D-Phe-Arg-Trp-Apn-Lys)-$NH_2$; SEQ ID NO:4
Ac-Cha-c(Asp-His-D-Phe-Arg-Trp-Gaba-Lys)-$NH_2$; SEQ ID NO:6
Ac-Nle-c(Asp-His-D-Phe-Arg-Trp-Gaba-Lys)-$NH_2$; SEQ ID NO:6
Ac-Chg-c(Asp-His-D-Phe-Arg-Trp-Gaba-Lys)-$NH_2$; SEQ ID NO:11
Ac-hCha-c(Asp-His-D-Phe-Arg-Trp-Gaba-Lys)-$NH_2$; SEQ ID NO:11
Ac-D-Chg-c(Asp-His-D-Phe-Arg-Trp-Gaba-Lys)-$NH_2$; SEQ ID NO:11
Ac-hPhe-c(Asp-His-D-Phe-Arg-Trp-Gaba-Lys)-$NH_2$; SEQ ID NO:11
Ac-Nle-c(Cys-His-D-Phe-Arg-D-Trp-β-Ala-Cys)-$NH_2$; SEQ ID NO:15
Ac-Nle-c(Pen-D-Ala-His-D-Phe-Arg-Trp-Cys)-$NH_2$; SEQ ID NO:21
Ac-Nle-c(Cys-D-Ala-His-D-Phe-Arg-Trp-Pen)-$NH_2$; SEQ ID NO:22
D-Phe-c(Cys-His-D-Phe-hArg-Trp-β-Ala-D-Cys)-Thr-$NH_2$; SEQ ID NO:23
D-Phe-c(Cys-His-D-Phe-Arg-Bip-β-Ala-D-Cys)-Thr-$NH_2$; SEQ ID NO:25
D-Phe-c(Cys-His-D-(Et)Tyr-hArg-Trp-β-Ala-D-Cys)-Thr-$NH_2$; SEQ ID NO:24
D-Phe-c(Cys-His-D-Phe-hArg-Bip-β-Ala-D-Cys)-Thr-$NH_2$; SEQ ID NO:26
D-Phe-c(Cys-His-D-(Et)Tyr-hArg-Bip-β-Ala-D-Cys)-Thr-$NH_2$; SEQ ID NO:26
Ac-Nle-c(Asp-D-Ala-His-D-Phe-Arg-Trp-Lys)-$NH_2$; SEQ ID NO:28
Ac-Nle-c(Asp-D-Ala-His-D-Phe-Arg-Bal-Lys)-$NH_2$; SEQ ID NO:28

Ac-Nle-c(Cys-D-Ala-His-D-Phe-Arg-Trp-Pen)-OH; SEQ ID NO:29
Ac-Nle-c(Cys-D-Abu-His-D-Phe-Arg-Trp-Cys)-$NH_2$; SEQ ID NO:30
Ac-Nle-c(Cys-D-Val-His-D-Phe-Arg-Trp-Cys)-$NH_2$; SEQ ID NO:30
Ac-Nle-c(Cys-D-Ile-His-D-Phe-Arg-Trp-Cys)-$NH_2$; SEQ ID NO:30
Ac-Nle-c(Cys-D-Leu-His-D-Phe-Arg-Trp-Cys)-$NH_2$; SEQ ID NO:30
Ac-Nle-c(Cys-D-Tle-His-D-Phe-Arg-Trp-Cys)-$NH_2$; SEQ ID NO:30
Ac-Nle-c(Cys-D-Cha-His-D-Phe-Arg-Trp-Cys)-$NH_2$; SEQ ID NO:30
Ac-Nle-c(Pen-His-D-Phe-Arg-Trp-Gaba-Cys)-$NH_2$; SEQ ID NO:31
Ac-Nle-c(Pen-His-D-Phe-Arg-Trp-Gaba-Pen)-$NH_2$; SEQ ID NO:32
Ac-Leu-c(Cys-His-D-Phe-Arg-Trp-Gaba-Cys)-$NH_2$; SEQ ID NO:33
Ac-Cha-c(Cys-His-D-Phe-Arg-Trp-Gaba-Cys)-$NH_2$; SEQ ID NO:33
Ac-Ile-c(Cys-His-D-Phe-Arg-Trp-Gaba-Cys)-$NH_2$; SEQ ID NO:33
Ac-Phe-c(Cys-His-D-Phe-Arg-Trp-Gaba-Cys)-$NH_2$; SEQ ID NO:33
Ac-Val-c(Cys-His-D-Phe-Arg-Trp-Gaba-Cys)-$NH_2$; SEQ ID NO:33
Ac-2-Nal-c(Cys-His-D-Phe-Arg-Trp-Gaba-Cys)-$NH_2$; SEQ ID NO:33
Phe-c(Cys-His-D-Phe-Arg-Trp-Gaba-Cys)-$NH_2$; SEQ ID NO:34
Ac-Nle-c(Cys-3-Pal-D-Phe-Arg-Trp-Gaba-Cys)-$NH_2$; SEQ ID NO:35
Ac-Nle-c(Cys-D-Ala-His-D-Phe-Arg-Trp-Cys)-OH; SEQ ID NO:36
Ac-Nle-c(Cys-His-Phe-Arg-D-Trp-Gaba-Cys)-$NH_2$; SEQ ID NO:37
Ac-Nle-c(Cys-D-Ala-His-D-2-Nal-Arg-Trp-Cys)-$NH_2$; SEQ ID NO:16
Ac-Nle-c(Cys-D-Ala-His-D-2-Nal-Arg-2-Nal-Cys)-$NH_2$; SEQ ID NO:16
Ac-Nle-c(Cys-D-Ala-His-D-2-Nal-Arg-Bal-Cys)-$NH_2$; SEQ ID NO:20
Ac-Nle-c(Asp-His-D-2-Nal-Arg-Trp-Ala-Lys)-$NH_2$; SEQ ID NO:38
Ac-Nle-c(Asp-His-D-2-Nal-Arg-Trp-β-Ala-Lys)-$NH_2$; SEQ ID NO:38
Ac-Nle-c(Cys-His-D-2-Nal-Arg-Trp-Gaba-Cys)-$NH_2$; SEQ ID NO:39
Ac-Nle-c(Cys-His-D-2-Nal-Arg-Trp-Ahx-Cys)-$NH_2$; SEQ ID NO:39
Ac-hPhe-c(Asp-His-D-2-Nal-Arg-Trp-Gaba-Lys)-$NH_2$; SEQ ID NO:40
Ac-Cha-c(Asp-His-D-2-Nal-Arg-Trp-Gaba-Lys)-$NH_2$; SEQ ID NO:40 or
Ac-Arg-c(Cys-D-Ala-His-D-2-Nal-Arg-Trp-Cys)-$NH_2$; SEQ ID NO:49

or pharmaceutically acceptable salts thereof.

Also described herein is a method to treat insulin resistance in a mammalian subject, with or without weight loss, by the administration of a therapeutically effective amount of a melanocortin receptor ligand described herein and pharmaceutically acceptable salts, hydrates, solvates or prodrugs thereof, wherein the ligand is a compound of the formula:

Ac-Nle-c(Cys-D-Ala-His-D-Phe-Arg-Trp-Gly-Cys)-$NH_2$; SEQ ID NO:54
Ac-Nle-c(Cys-D-Ala-His-D-Phe-Arg-Trp-D-Ala-Cys)-$NH_2$; SEQ ID NO:54
Ac-Nle-c(Cys-D-Ala-His-D-Phe-Arg-Trp-β-Ala-Cys)-$NH_2$; SEQ ID NO:54
Ac-Nle-c(Cys-D-Ala-His-D-Phe-Arg-Trp-Gaba-Cys)-$NH_2$; SEQ ID NO:54
Ac-Nle-c(Cys-D-Ala-His-D-Phe-Arg-Trp-Apn-Cys)-$NH_2$; SEQ ID NO:54
Ac-c(Cys-Glu-His-D-Phe-Arg-Trp-Ala-Cys)-$NH_2$; SEQ ID NO:55
Ac-c(Cys-Glu-His-D-Phe-Arg-2-Nal-Ala-Cys)-$NH_2$; SEQ ID NO:55
Ac-c(Cys-D-Ala-His-D-Phe-Arg-Trp-Ala-Cys)-$NH_2$; SEQ ID NO:56
Ac-c(Cys-D-Ala-His-D-Phe-Arg-2-Nal-Ala-Cys)-$NH_2$; SEQ ID NO:56
Ac-Nle-c(Cys-D-Ala-His-D-Phe-Arg-Trp-Ala-Cys)-$NH_2$; SEQ ID NO:57
Ac-Nle-c(Cys-D-Ala-His-D-Phe-Arg-Trp-β-Ala-Cys)-$NH_2$; SEQ ID NO:57
Ac-Nle-c(Cys-D-Ala-His-D-Phe-Arg-Trp-Gaba-Cys)-$NH_2$; SEQ ID NO:57 or
Ac-Nle-c(Asp-D-Ala-His-D-Phe-Arg-Bal-Ala-Lys)-$NH_2$; SEQ ID NO:58

or a pharmaceutically acceptable salt thereof.

Further described herein is a method to treat insulin resistance in a mammalian subject, with or without weight loss, by the administration of a therapeutically effective amount of a melanocortin receptor compound described herein, and pharmaceutically acceptable salts, hydrates, solvates or prodrugs thereof wherein the compound is a compound of the formula:

Tyr-Gly-Arg-$(Lys)_2$-$(Arg)_2$-Gln-$(Arg)_3$-Nle-c(Asp-His-D-2-Nal-Arg-Trp-Lys)-$NH_2$; (SEQ ID NO:60)

Tyr-Gly-Arg-$(Lys)_2$-$(Arg)_2$-Gln-$(Arg)_3$-Doc-Nle-c(Asp-His-D-2-Nal-Arg-Trp-Lys)-$NH_2$; (SEQ ID NO:61)

Nle-c(Asp-His-D-2-Nal-Arg-Trp-Lys)-*ß*-Ala-Tyr-Gly-Arg-$(Lys)_2$-$(Arg)_2$-Gln-$(Arg)_3$-$NH_2$; (SEQ ID NO:62)

Ac-Nle-c(Asp-His-D-2-Nal-Arg-Trp-Lys)-*ß*-Ala-Tyr-Gly-Arg-$(Lys)_2$-$(Arg)_2$-Gln-$(Arg)_3$-$NH_2$; (SEQ ID NO:62)

Nle-c(Asp-His-D-2-Nal-Arg-Trp-Lys)-$(Doc)_2$-Tyr-Gly-Arg-$(Lys)_2$-$(Arg)_2$-Gln-$(Arg)_3$-$NH_2$; (SEQ ID NO:63)

Ac-Nle-c(Asp-His-D-2-Nal-Arg-Trp-Lys)-$(Pro)_2$-Lys-Asp-Tyr-Gly-Arg-$(Lys)_2$-$(Arg)_2$-Gln-$(Arg)_3$-$NH_2$; (SEQ ID NO:64)

Ac-c(Cys-Glu-His-D-2-Nal-Arg-Trp-Gly-Cys)-$(Pro)_2$-Lys-Asp-Tyr-Gly-Arg-$(Lys)_2$-$(Arg)_2$-Gln-$(Arg)_3$-$NH_2$; (SEQ ID NO:65)

Ac-Nle-c(Asp-His-D-2-Nal-Arg-Trp-Lys)-(*ß*-Ala)$_2$-Tyr-Gly-Arg-$(Lys)_2$-$(Arg)_2$-Gln-$(Arg)_3$-$NH_2$; (SEQ ID NO:66)

Ac-Nle-c(Asp-His-D-2-Nal-Arg-Trp-Lys)-$(Pro)_2$-Lys-Asp-Doc-Tyr-Gly-Arg-$(Lys)_2$-$(Arg)_2$-Gln-$(Arg)_3$-$NH_2$; (SEQ ID NO:67)

Ac-c(Cys-Glu-His-D-2-Nal-Arg-Trp-Gly-Cys)-$(Pro)_2$-Lys-Asp-Doc-Tyr-Gly-Arg-$(Lys)_2$-$(Arg)_2$-Gln-$(Arg)_3$-$NH_2$; (SEQ ID NO:68)

Ac-c(Cys-Glu-His-D-2-Nal-Arg-Trp-Ala-Cys)-$(Pro)_2$-Lys-Asp-*ß*-Ala-Tyr-Gly-Arg-$(Lys)_2$-$(Arg)_2$-Gln-$(Arg)_3$-$NH_2$; (SEQ ID NO:69)

Ac-c(Cys-Glu-His-D-2-Nal-Arg-Trp-Ala-Cys)-$(Pro)_2$-Lys-Asp-Doc-Tyr-Gly-Arg-$(Lys)_2$-$(Arg)_2$-Gln-$(Arg)_3$-$NH_2$; (SEQ ID NO:69)

Ac-Nle-c(Asp-His-D-2-Nal-Arg-Trp-Lys)-$(Doc)_2$-Tyr-Gly-Arg-$(Lys)_2$-$(Arg)_2$-Gln-$(Arg)_3$-$NH_2$; (SEQ ID NO:70)

Ac-c(Cys-Glu-His-D-2-Nal-Arg-2-Nal-Ala-Cys)-$(Pro)_2$-Lys-Asp-*ß*-Ala-Tyr-Gly-Arg-$(Lys)_2$-$(Arg)_2$-Gln-$(Arg)_3$-$NH_2$; (SEQ ID NO:71)

Ac-c(Cys-Glu-His-D-2-Nal-Arg-Bal-Ala-Cys)-$(Pro)_2$-Lys-Asp-*ß*-Ala-$(Arg)_5$-Gln-$(Arg)_3$-$NH_2$; (SEQ ID NO:72)

Ac-c(Cys-Glu-His-D-2-Nal-Arg-Bal-Ala-Cys)-$(Pro)_2$-Lys-Asp-*ß*-Ala-Gly-$(Arg)_5$-Gln-$(Arg)_3$-$NH_2$; (SEQ ID NO:73)

Ac-c(Cys-Glu-His-D-2-Nal-Arg-Bal-Ala-Cys)-$(Pro)_2$-Lys-Asp-*ß*-Ala-Tyr-Gly-$(Arg)_5$-Gln-$(Arg)_3$-$NH_2$; (SEQ ID NO:74)

Ac-c(Cys-Glu-His-D-2-Nal-Arg-Trp-Ala-Cys)-$(Pro)_2$-Lys-Asp-*ß*-Ala-Tyr-Gly-Arg-$(Lys)_2$-Arg-Gln-$(Arg)_4$-$NH_2$; (SEQ ID NO:75)

Ac-c(Cys-Glu-His-D-2-Nal-Arg-Trp-Ala-Cys)-$(Pro)_2$-Lys-Asp-*ß*-Ala-Tyr-Gly-Arg-$(Lys)_2$-Gln-$(Arg)_5$-$NH_2$; (SEQ ID NO:76)

Ac-c(Cys-Glu-His-D-2-Nal-Arg-Trp-Ala-Cys)-$(Pro)_2$-Lys-Asp-*ß*-Ala-Tyr-Gly-Arg-Lys-Gln-Lys-$(Arg)_5$-$NH_2$; (SEQ ID NO:77)

Ac-c(Cys-Glu-His-D-2-Nal-Arg-Trp-Ala-Cys)-$(Pro)_2$-Lys-Asp-*ß*-Ala-Tyr-Gly-Arg-$(Lys)_2$-$(Arg)_4$-Gln-Arg-$NH_2$; (SEQ ID NO:78)

Ac-c(Cys-Glu-His-D-2-Nal-Arg-Bal-Ala-Cys)-$(Pro)_2$-Lys-Asp-*ß*-Ala-Tyr-Aib-Arg-$(Lys)_2$-$(Arg)_2$-Gln-$(Arg)_3$-$NH_2$; (SEQ ID NO:79)

Ac-c(Cys-Glu-His-D-2-Nal-Arg-1-Nal-Ala-Cys)-$(Pro)_2$-Arg-Asp-*ß*-Ala-$(Arg)_5$-Gln-$(Arg)_3$-$NH_2$; (SEQ ID NO:80)

Ac-c(Cys-Glu-His-D-2-Nal-Arg-1-Nal-Ala-Cys)-(Pro)$_2$-Lys-Asp-ß-Ala-(Arg)$_5$-Gln-(Arg)$_3$-NH$_2$; (SEQ ID NO:80)

Ac-c(Cys-Glu-His-D-2-Nal-Arg-1-Nal-Ala-Cys)-(Pro)$_2$-Lys-Asp-ß-Ala-(Arg)$_6$-Gln-(Arg)$_3$-NH$_2$; (SEQ ID NO:81)

Ac-c(Cys-Glu-His-D-2-Nal-Arg-2-Nal-Ala-Cys)-(Pro)$_2$-Arg-Asp-ß-Ala-(Arg)$_5$-Gln-(Arg)$_3$-NH$_2$; (SEQ ID NO:82)

Ac-c(Cys-Glu-His-D-2-Nal-Arg-2-Nal-Ala-Cys)-(Pro)$_2$-Lys-Asp-ß-Ala-(Arg)$_5$-Gln-(Arg)$_3$-NH$_2$; (SEQ ID NO:82)

Ac-c(Cys-Glu-His-D-2-Nal-Arg-2-Nal-Ala-Cys)-(Pro)$_2$-Lys-Asp-ß-Ala-(Arg)$_6$-Gln-(Arg)$_3$-NH$_2$; (SEQ ID NO:81)

Ac-c(Cys-Glu-His-D-2-Nal-Arg-Bal-Ala-Cys)-(Pro)$_2$-Arg-Asp-ß-Ala-(Arg)$_6$-Gln-(Arg)$_3$-NH$_2$; (SEQ ID NO:83)

Ac-c(Cys-Glu-His-D-2-Nal-Arg-Bal-Ala-Cys)-(Pro)$_2$-Arg-Asp-ß-Ala-(Arg)$_5$-Gln-(Arg)$_3$-NH$_2$; (SEQ ID NO:84)

Ac-c(Cys-Glu-His-D-2-Nal-Arg-Bal-Ala-Cys)-(Pro)$_2$-Lys-Asp-ß-Ala-(Arg)$_6$-Gln-(Arg)$_3$-NH$_2$; (SEQ ID NO:83)

Ac-c(Cys-Glu-His-D-2-Nal-Arg-Trp-Ala-Cys)-(Pro)$_2$-Lys-Asp-ß-Ala-Tyr-Gly-Arg-(Lys)$_2$-(Arg)$_3$-Gln-(Arg)$_2$-NH$_2$; (SEQ ID NO:85)

Ac-c(Cys-Glu-His-D-2-Nal-Arg-Trp-Ala-Cys)-(Pro)$_2$-Lys-Asp-ß-Ala-Tyr-Gly-Arg-Gln-(Lys)$_2$-(Arg)$_5$-NH$_2$; (SEQ ID NO:86)

Ac-c(Cys-Glu-His-D-2-Nal-Arg-Trp-Ala-Cys)-(Pro)$_2$-Lys-Asp-ß-Ala-Tyr-Gly-Arg-(Lys)$_2$-(Arg)$_5$-Gln-NH$_2$; (SEQ ID NO:87)

Ac-c(Cys-Glu-His-D-2-Nal-Arg-1-Nal-Ala-Cys)-$(Pro)_2$-Lys-Asp-ß-Ala-Tyr-Gly-Arg-$(Lys)_2$-$(Arg)_2$-Gln-$(Arg)_3$-$NH_2$; (SEQ ID NO:71)

Ac-c(Cys-Glu-His-D-2-Nal-Arg-Bal-Ala-Cys)-$(Pro)_2$-Lys-Asp-ß-Ala-Tyr-Gly-Arg-$(Lys)_2$-$(Arg)_2$-Gln-$(Arg)_3$-$NH_2$; (SEQ ID NO:71)

Ac-c(Cys-Glu-His-D-2-Nal-Arg-1-Nal-Ala-Cys)-$(Pro)_2$-Lys-Asp-ß-Ala- $(Arg)_2$-Lys-$(Arg)_2$-Gln-$(Arg)_3$-$NH_2$; (SEQ ID NO:88)

Ac-c(Cys-Glu-His-D-2-Nal-Arg-1-Nal-Ala-Cys)-$(Pro)_2$-Lys-Asp-ß-Ala-Arg-Lys-$(Arg)_3$-Gln-$(Arg)_3$-$NH_2$; (SEQ ID NO:89)

Ac-c(Cys-Glu-His-D-2-Nal-Arg-2-Nal-Ala-Cys)-$(Pro)_2$-Lys-Asp-ß-Ala- $(Arg)_2$-Lys-$(Arg)_2$-Gln-$(Arg)_3$-$NH_2$; (SEQ ID NO:88)

Ac-c(Cys-Glu-His-D-2-Nal-Arg-2-Nal-Ala-Cys)-$(Pro)_2$-Lys-Asp-ß-Ala-Tyr-Gly-$(Arg)_2$-Lys-$(Arg)_2$-Gln-$(Arg)_3$-$NH_2$; (SEQ ID NO:90)

Ac-c(Cys-Glu-His-D-2-Nal-Arg-2-Nal-Ala-Cys)-$(Pro)_2$-Lys-Asp-ß-Ala-Gly-$(Arg)_2$-Lys-$(Arg)_2$-Gln-$(Arg)_3$-$NH_2$; (SEQ ID NO:91)

Ac-c(Cys-Glu-His-D-2-Nal-Arg-2-Nal-Ala-Cys)-$(Pro)_2$-Lys-Asp-ß-Ala-Gly-Arg-Lys-$(Arg)_3$-Gln-$(Arg)_3$-$NH_2$; (SEQ ID NO:92)

Ac-c(Cys-Glu-His-D-2-Nal-Arg-1-Nal-Ala-Cys)-$(Pro)_2$-Lys-Asp-ß-Ala- $(Arg)_2$-Lys-$(Arg)_2$-Gln-$(Arg)_3$-$NH_2$; (SEQ ID NO:93)

Ac-c(Cys-Glu-His-D-2-Nal-Arg-1-Nal-Ala-Cys)-$(Pro)_2$-Lys-Asp-ß-Ala-Arg-Lys-$(Arg)_3$-Gln-$(Arg)_3$-$NH_2$; (SEQ ID NO:94)

Ac-c(Cys-Glu-His-D-2-Nal-Arg-1-Nal-Ala-Cys)-$(Pro)_2$-Lys-Asp-ß-Ala-Tyr-Gly-$(Arg)_2$-Lys-$(Arg)_2$-Gln-$(Arg)_3$-$NH_2$; (SEQ ID NO:95)

Ac-c(Cys-Glu-His-D-2-Nal-Arg-1-Nal-Ala-Cys)-$(Pro)_2$-Lys-Asp-ß-Ala-Tyr-Gly-Arg-Lys-$(Arg)_3$-Gln-$(Arg)_3$-$NH_2$; (SEQ ID NO:96)

Ac-c(Cys-Glu-His-D-2-Nal-Arg-1-Nal-Ala-Cys)-$(Pro)_2$-Lys-Asp-ß-Ala-Gly-$(Arg)_2$-Lys-$(Arg)_2$-Gln-$(Arg)_3$-$NH_2$; (SEQ ID NO:97)

Ac-c(Cys-Glu-His-D-2-Nal-Arg-1-Nal-Ala-Cys)-$(Pro)_2$-Lys-Asp-ß-Ala-Gly-Arg-Lys-$(Arg)_3$-Gln-$(Arg)_3$-$NH_2$; (SEQ ID NO:92)

Ac-c(Cys-Glu-His-D-2-Nal-Arg-2-Nal-Ala-Cys)-$(Pro)_2$-Lys-Asp-ß-Ala-$(Arg)_2$-Lys-$(Arg)_2$-Gln-$(Arg)_3$-$NH_2$; (SEQ ID NO:98)

Ac-c(Cys-Glu-His-D-2-Nal-Arg-2-Nal-Ala-Cys)-$(Pro)_2$-Lys-Asp-ß-Ala-Arg-Lys-$(Arg)_3$-Gln-$(Arg)_3$-$NH_2$; (SEQ ID NO:99)

Ac-c(Cys-Glu-His-D-2-Nal-Arg-2-Nal-Ala-Cys)-$(Pro)_2$-Lys-Asp-ß-Ala-Tyr-Gly-$(Arg)_2$-Lys-$(Arg)_2$-Gln-$(Arg)_3$-$NH_2$; (SEQ ID NO:100)

Ac-c(Cys-Glu-His-D-2-Nal-Arg-2-Nal-Ala-Cys)-$(Pro)_2$-Lys-Asp-ß-Ala-Tyr-Gly-Arg-Lys-$(Arg)_3$-Gln-$(Arg)_3$-$NH_2$; (SEQ ID NO:101)

Ac-c(Cys-Glu-His-D-2-Nal-Arg-2-Nal-Ala-Cys)-$(Pro)_2$-Lys-Asp-ß-Ala-Gly-$(Arg)_2$-Lys-$(Arg)_2$-Gln-$(Arg)_3$-$NH_2$; (SEQ ID NO:102)

Ac-c(Cys-Glu-His-D-2-Nal-Arg-2-Nal-Ala-Cys)-$(Pro)_2$-Lys-Asp-ß-Ala-Gly-Arg-Lys-$(Arg)_3$-Gln-$(Arg)_3$-$NH_2$; (SEQ ID NO:103)

Ac-c(Cys-Glu-His-D-2-Nal-Arg-Bal-Ala-Cys)-$(Pro)_2$-Lys-Asp-ß-Ala- $(Arg)_2$-Lys-$(Arg)_2$-Gln-$(Arg)_3$-$NH_2$; (SEQ ID NO:104)

Ac-c(Cys-Glu-His-D-2-Nal-Arg-Bal-Ala-Cys)-$(Pro)_2$-Lys-Asp-ß-Ala-Arg-Lys-$(Arg)_3$-Gln-$(Arg)_3$-$NH_2$; (SEQ ID NO:105)

Ac-c(Cys-Glu-His-D-2-Nal-Arg-Bal-Ala-Cys)-$(Pro)_2$-Lys-Asp-ß-Ala-Tyr-Gly-$(Arg)_2$-Lys-$(Arg)_2$-Gln-$(Arg)_3$-$NH_2$; (SEQ ID NO:100)

Ac-c(Cys-Glu-His-D-2-Nal-Arg-Bal-Ala-Cys)-$(Pro)_2$-Lys-Asp-ß-Ala-Tyr-Gly-Arg-Lys-$(Arg)_3$-Gln-$(Arg)_3$-$NH_2$; (SEQ ID NO:101)

Ac-c(Cys-Glu-His-D-2-Nal-Arg-Bal-Ala-Cys)-$(Pro)_2$-Lys-Asp-ß-Ala-Gly-$(Arg)_2$-Lys-$(Arg)_2$-Gln-$(Arg)_3$-$NH_2$; (SEQ ID NO:102)

Ac-c(Cys-Glu-His-D-2-Nal-Arg-Bal-Ala-Cys)-$(Pro)_2$-Lys-Asp-ß-Ala-Gly-Arg-Lys-$(Arg)_3$-Gln-$(Arg)_3$-$NH_2$; (SEQ ID NO:103)

Ac-c(Cys-Glu-His-D-2-Nal-Arg-Trp-Ala-Cys)-$(Pro)_2$-Lys-Asp-ß-Ala-Tyr-Gly-Arg-$(Lys)_2$-Arg-Gln-$(Arg)_4$-$NH_2$; (SEQ ID NO:106)

Ac-c(Cys-Glu-His-D-2-Nal-Arg-Trp-Ala-Cys)-$(Pro)_2$-Lys-Asp-ß-Ala-Tyr-Gly-Arg-$(Lys)_2$-Gln-$(Arg)_5$-$NH_2$; (SEQ ID NO:107)

Ac-c(Cys-Glu-His-D-2-Nal-Arg-Trp-Ala-Cys)-$(Pro)_2$-Lys-Asp-ß-Ala-Tyr-Gly-Arg-Lys-Gln-Lys-$(Arg)_5$-$NH_2$; (SEQ ID NO:108)

Ac-c(Cys-Glu-His-D-2-Nal-Arg-Trp-Ala-Cys)-$(Pro)_2$-Lys-Asp-ß-Ala-Tyr-Gly-Arg-Gln-$(Lys)_2$-$(Arg)_5$-$NH_2$; (SEQ ID NO:109)

Ac-c(Cys-Glu-His-D-2-Nal-Arg-Trp-Ala-Cys)-$(Pro)_2$-Lys-Asp-ß-Ala-Tyr-Gly-Arg-$(Lys)_2$-$(Arg)_3$-Gln-$(Arg)_2$-$NH_2$; (SEQ ID NO:110)

Ac-c(Cys-Glu-His-D-2-Nal-Arg-Trp-Ala-Cys)-$(Pro)_2$-Lys-Asp-ß-Ala-Tyr-Gly-Arg-$(Lys)_2$-$(Arg)_4$-Gln-Arg-$NH_2$; (SEQ ID NO:111)

Ac-c(Cys-Glu-His-D-2-Nal-Arg-Trp-Ala-Cys)-$(Pro)_2$-Lys-Asp-ß-Ala-Tyr-Gly-Arg-$(Lys)_2$-$(Arg)_5$-Gln-$NH_2$; (SEQ ID NO:112)

Ac-c(Cys-Glu-His-D-2-Nal-Arg-Trp-Ala-Cys)-$(Pro)_2$-Lys-Asp-*ß*-Ala-$(Arg)_5$-Gln-$(Arg)_3$-$NH_2$; (SEQ ID NO:113)

Ac-c(Cys-Glu-His-D-2-Nal-Arg-Trp-Ala-Cys)-$(Pro)_2$-Arg-Asp-*ß*-Ala-$(Arg)_5$-Gln-$(Arg)_3$-$NH_2$; (SEQ ID NO:113)

Ac-c(Cys-Glu-His-D-2-Nal-Arg-Trp-Ala-Cys)-$(Pro)_2$-Lys-Asp-*ß*-Ala-Tyr-Gly-$(Arg)_5$-Gln-$(Arg)_3$-$NH_2$; (SEQ ID NO:114)

Ac-c(Cys-Glu-His-D-2-Nal-Arg-Trp-Ala-Cys)-$(Pro)_2$-Arg-Asp-*ß*-Ala-Tyr-Gly-$(Arg)_5$-Gln-$(Arg)_3$-$NH_2$; (SEQ ID NO:114)

Ac-c(Cys-Glu-His-D-2-Nal-Arg-Trp-Ala-Cys)-$(Pro)_2$-Lys-Asp-*ß*-Ala-$(Arg)_5$-Gln-$(Arg)_4$-$NH_2$; (SEQ ID NO:115)

Ac-c(Cys-Glu-His-D-2-Nal-Arg-Trp-Ala-Cys)-$(Pro)_2$-Arg-Asp-*ß*-Ala-$(Arg)_5$-Gln-$(Arg)_4$-$NH_2$; (SEQ ID NO:115)

Ac-c(Cys-Glu-His-D-2-Nal-Arg-Trp-Ala-Cys)-$(Pro)_2$-Lys-Asp-*ß*-Ala-Tyr-Gly-$(Arg)_5$-Gln-$(Arg)_4$-$NH_2$; (SEQ ID NO:116)

Ac-c(Cys-Glu-His-D-2-Nal-Arg-Trp-Ala-Cys)-$(Pro)_2$-Arg-Asp-*ß*-Ala-Tyr-Gly-$(Arg)_5$-Gln-$(Arg)_4$-$NH_2$; (SEQ ID NO:116)

Ac-c(Cys-Glu-His-D-2-Nal-Arg-1-Nal-Ala-Cys)-$(Pro)_2$-Lys-Asp-*ß*-Ala-$(Arg)_5$-Gln-$(Arg)_3$-$NH_2$; (SEQ ID NO:117)

Ac-c(Cys-Glu-His-D-2-Nal-Arg-1-Nal-Ala-Cys)-$(Pro)_2$-Arg-Asp-*ß*-Ala-$(Arg)_5$-Gln-$(Arg)_3$-$NH_2$; (SEQ ID NO:117)

Ac-c(Cys-Glu-His-D-2-Nal-Arg-1-Nal-Ala-Cys)-$(Pro)_2$-Lys-Asp-*ß*-Ala-$(Arg)_5$-Gln-$(Arg)_4$-$NH_2$; (SEQ ID NO:118)

Ac-c(Cys-Glu-His-D-2-Nal-Arg-1-Nal-Ala-Cys)-$(Pro)_2$-Arg-Asp-*ß*-Ala-$(Arg)_5$-Gln-$(Arg)_4$-$NH_2$; (SEQ ID NO:118)

Ac-c(Cys-Glu-His-D-2-Nal-Arg-1-Nal-Ala-Cys)-$(Pro)_2$-Lys-Asp-*ß*-Ala-$(Arg)_6$-Gln-$(Arg)_3$-$NH_2$; (SEQ ID NO:119)

Ac-c(Cys-Glu-His-D-2-Nal-Arg-1-Nal-Ala-Cys)-$(Pro)_2$-Arg-Asp-*ß*-Ala-$(Arg)_6$-Gln-$(Arg)_3$-$NH_2$; (SEQ ID NO:119)

Ac-c(Cys-Glu-His-D-2-Nal-Arg-1-Nal-Ala-Cys)-$(Pro)_2$-Lys-Asp-*ß*-Ala-Tyr-Gly-$(Arg)_5$-Gln-$(Arg)_3$-$NH_2$; (SEQ ID NO:120)

Ac-c(Cys-Glu-His-D-2-Nal-Arg-1-Nal-Ala-Cys)-$(Pro)_2$-Arg-Asp-*ß*-Ala-Tyr-Gly-$(Arg)_5$-Gln-$(Arg)_3$-$NH_2$; (SEQ ID NO:120)

Ac-c(Cys-Glu-His-D-2-Nal-Arg-1-Nal-Ala-Cys)-$(Pro)_2$-Lys-Asp-*ß*-Ala-Tyr-Gly-$(Arg)_5$-Gln-$(Arg)_4$-$NH_2$; (SEQ ID NO:121)

Ac-c(Cys-Glu-His-D-2-Nal-Arg-1-Nal-Ala-Cys)-$(Pro)_2$-Arg-Asp-*ß*-Ala-Tyr-Gly-$(Arg)_5$-Gln-$(Arg)_4$-$NH_2$; (SEQ ID NO:121)

Ac-c(Cys-Glu-His-D-2-Nal-Arg-1-Nal-Ala-Cys)-$(Pro)_2$-Lys-Asp-*ß*-Ala-Tyr-Gly-$(Arg)_6$-Gln-$(Arg)_3$-$NH_2$; (SEQ ID NO:122)

Ac-c(Cys-Glu-His-D-2-Nal-Arg-1-Nal-Ala-Cys)-$(Pro)_2$-Arg-Asp-*ß*-Ala-Tyr-Gly-$(Arg)_6$-Gln-$(Arg)_3$-$NH_2$; (SEQ ID NO:122)

Ac-c(Cys-Glu-His-D-2-Nal-Arg-2-Nal-Ala-Cys)-$(Pro)_2$-Lys-Asp-*ß*-Ala-$(Arg)_5$-Gln-$(Arg)_3$-$NH_2$; (SEQ ID NO:123)

Ac-c(Cys-Glu-His-D-2-Nal-Arg-2-Nal-Ala-Cys)-$(Pro)_2$-Arg-Asp-*ß*-Ala-$(Arg)_5$-Gln-$(Arg)_3$-$NH_2$; (SEQ ID NO:123)

Ac-c(Cys-Glu-His-D-2-Nal-Arg-2-Nal-Ala-Cys)-$(Pro)_2$-Lys-Asp-*ß*-Ala-$(Arg)_6$-Gln-$(Arg)_3$-$NH_2$; (SEQ ID NO:124)

Ac-c(Cys-Glu-His-D-2-Nal-Arg-2-Nal-Ala-Cys)-$(Pro)_2$-Arg-Asp-*ß*-Ala-$(Arg)_6$-Gln-$(Arg)_3$-$NH_2$; (SEQ ID NO:124)

Ac-c(Cys-Glu-His-D-2-Nal-Arg-2-Nal-Ala-Cys)-$(Pro)_2$-Lys-Asp-*ß*-Ala-$(Arg)_5$-Gln-$(Arg)_4$-$NH_2$; (SEQ ID NO:125)

Ac-c(Cys-Glu-His-D-2-Nal-Arg-2-Nal-Ala-Cys)-$(Pro)_2$-Arg-Asp-*ß*-Ala-$(Arg)_5$-Gln-$(Arg)_4$-$NH_2$; (SEQ ID NO:125)

Ac-c(Cys-Glu-His-D-2-Nal-Arg-2-Nal-Ala-Cys)-$(Pro)_2$-Lys-Asp-*ß*-Ala-Tyr-Gly-$(Arg)_5$-Gln-$(Arg)_3$-$NH_2$; (SEQ ID NO:126)

Ac-c(Cys-Glu-His-D-2-Nal-Arg-2-Nal-Ala-Cys)-$(Pro)_2$-Arg-Asp-*ß*-Ala-Tyr-Gly-$(Arg)_5$-Gln-$(Arg)_3$-$NH_2$; (SEQ ID NO:126)

Ac-c(Cys-Glu-His-D-2-Nal-Arg-2-Nal-Ala-Cys)-$(Pro)_2$-Lys-Asp-*ß*-Ala-Tyr-Gly-$(Arg)_6$-Gln-$(Arg)_3$-$NH_2$; (SEQ ID NO:127)

Ac-c(Cys-Glu-His-D-2-Nal-Arg-2-Nal-Ala-Cys)-$(Pro)_2$-Arg-Asp-*ß*-Ala-Tyr-Gly-$(Arg)_6$-Gln-$(Arg)_3$-$NH_2$; (SEQ ID NO:127)

Ac-c(Cys-Glu-His-D-2-Nal-Arg-2-Nal-Ala-Cys)-$(Pro)_2$-Lys-Asp-*ß*-Ala-Tyr-Gly-$(Arg)_5$-Gln-$(Arg)_4$-$NH_2$; (SEQ ID NO:128)

Ac-c(Cys-Glu-His-D-2-Nal-Arg-2-Nal-Ala-Cys)-$(Pro)_2$-Arg-Asp-*ß*-Ala-Tyr-Gly-$(Arg)_5$-Gln-$(Arg)_4$-$NH_2$; (SEQ ID NO:128)

Ac-c(Cys-Glu-His-D-2-Nal-Arg-Bal-Ala-Cys)-$(Pro)_2$-Lys-Asp-*ß*-Ala-$(Arg)_5$-Gln-$(Arg)_3$-$NH_2$; (SEQ ID NO:129)

Ac-c(Cys-Glu-His-D-2-Nal-Arg-Bal-Ala-Cys)-$(Pro)_2$-Arg-Asp-*ß*-Ala-$(Arg)_5$-Gln-$(Arg)_3$-$NH_2$; (SEQ ID NO:129)

Ac-c(Cys-Glu-His-D-2-Nal-Arg-Bal-Ala-Cys)-$(Pro)_2$-Lys-Asp-*ß*-Ala-$(Arg)_5$-Gln-$(Arg)_4$-$NH_2$; (SEQ ID NO:130)

Ac-c(Cys-Glu-His-D-2-Nal-Arg-Bal-Ala-Cys)-$(Pro)_2$-Arg-Asp-*ß*-Ala-$(Arg)_5$-Gln-$(Arg)_4$-$NH_2$; (SEQ ID NO:130)

Ac-c(Cys-Glu-His-D-2-Nal-Arg-Bal-Ala-Cys)-$(Pro)_2$-Lys-Asp-*ß*-Ala-$(Arg)_6$-Gln-$(Arg)_3$-$NH_2$; (SEQ ID NO:131)

Ac-c(Cys-Glu-His-D-2-Nal-Arg-Bal-Ala-Cys)-$(Pro)_2$-Arg-Asp-*ß*-Ala-$(Arg)_6$-Gln-$(Arg)_3$-$NH_2$; (SEQ ID NO:131)

Ac-c(Cys-Glu-His-D-2-Nal-Arg-Bal-Ala-Cys)-$(Pro)_2$-Lys-Asp-*ß*-Ala-Gly-$(Arg)_5$-Gln-$(Arg)_3$-$NH_2$; (SEQ ID NO:132)

Ac-c(Cys-Glu-His-D-2-Nal-Arg-Bal-Ala-Cys)-$(Pro)_2$-Lys-Asp-*ß*-Ala-Tyr-Gly-$(Arg)_5$-Gln-$(Arg)_3$-$NH_2$; (SEQ ID NO:133)

Ac-c(Cys-Glu-His-D-2-Nal-Arg-Bal-Ala-Cys)-$(Pro)_2$-Arg-Asp-*ß*-Ala-Tyr-Gly-$(Arg)_5$-Gln-$(Arg)_3$-$NH_2$; (SEQ ID NO:133)

Ac-c(Cys-Glu-His-D-2-Nal-Arg-Bal-Ala-Cys)-$(Pro)_2$-Lys-Asp-*ß*-Ala-Tyr-Gly-$(Arg)_5$-Gln-$(Arg)_4$-$NH_2$; (SEQ ID NO:134)

Ac-c(Cys-Glu-His-D-2-Nal-Arg-Bal-Ala-Cys)-$(Pro)_2$-Arg-Asp-*ß*-Ala-Tyr-Gly-$(Arg)_5$-Gln-$(Arg)_4$-$NH_2$; (SEQ ID NO:134)

Ac-c(Cys-Glu-His-D-2-Nal-Arg-Bal-Ala-Cys)-$(Pro)_2$-Lys-Asp-*ß*-Ala-Tyr-Gly-$(Arg)_6$-Gln-$(Arg)_3$-$NH_2$; (SEQ ID NO:135)

Ac-c(Cys-Glu-His-D-2-Nal-Arg-Bal-Ala-Cys)-$(Pro)_2$-Arg-Asp-ß-Ala-Tyr-Gly-$(Arg)_6$-Gln-$(Arg)_3$-$NH_2$; (SEQ ID NO:135)

Ac-Nle-c(Cys-D-Ala-His-D-Phe-Arg-Trp-Cys)-$(Doc)_2$-Tyr-Gly-Arg-$(Lys)_2$-$(Arg)_2$-Gln-$(Arg)_3$-$NH_2$; (SEQ ID NO:136)

Ac-Nle-c(Cys-D-Ala-His-D-Phe-Arg-Trp-Cys)-ß-Ala-Tyr-Gly-Arg-$(Lys)_2$-Arg-Gln-$(Arg)_4$-$NH_2$; (SEQ ID NO:137)

Ac-Nle-c(Cys-D-Ala-His-D-Phe-Arg-Trp-Cys)-Doc-Tyr-Gly-Arg-$(Lys)_2$-$(Arg)_2$-Gln-$(Arg)_3$-$NH_2$; (SEQ ID NO:136)

Ac-Nle-c(Cys-D-Ala-His-D-Phe-Arg-Trp-Cys)-ß-Ala-$(Arg)_5$-Gln-$(Arg)_3$-$NH_2$; (SEQ ID NO:138)

Ac-Nle-c(Cys-D-Ala-His-D-Phe-Arg-Trp-Cys)-ß-Ala-Gly-$(Arg)_5$-Gln-$(Arg)_3$-$NH_2$; (SEQ ID NO:138)

Ac-Nle-c(Cys-D-Ala-His-D-Phe-Arg-Trp-Cys)-ß-Ala-Gly-$(Arg)_5$-Gln-$(Arg)_3$-$NH_2$; (SEQ ID NO:138)

Ac-Nle-c(Cys-D-Ala-His-D-Phe-Arg-Trp-Cys)-ß-Ala-Tyr-Gly-$(Arg)_5$-Gln-$(Arg)_3$-$NH_2$; (SEQ ID NO:139)

Ac-Nle-c(Cys-D-Ala-His-D-Phe-Arg-Trp-Cys)-ß-Ala-Gly-$(Arg)_5$-Gln-$(Arg)_4$-$NH_2$; (SEQ ID NO:140)

Ac-Nle-c(Cys-D-Ala-His-D-Phe-Arg-Trp-Cys)-ß-Ala-Tyr-Gly-$(Arg)_2$-Lys-$(Arg)_2$-Gln-$(Arg)_3$-$NH_2$; (SEQ ID NO:141)

Ac-Nle-c(Cys-D-Ala-His-D-Phe-Arg-Trp-Cys)-ß-Ala-Tyr-Gly-Arg-Lys-$(Arg)_3$-Gln-$(Arg)_3$-$NH_2$; (SEQ ID NO:142)

Ac-Nle-c(Cys-D-Ala-His-D-Phe-Arg-Trp-Cys)-ß-Ala-Gly-$(Arg)_2$-Lys-$(Arg)_2$-Gln-$(Arg)_3$-$NH_2$; (SEQ ID NO:141)

Ac-Nle-c(Cys-D-Ala-His-D-Phe-Arg-Trp-Cys)-ß-Ala-Gly-Arg-Lys-$(Arg)_3$-Gln-$(Arg)_3$-$NH_2$; (SEQ ID NO:142)

Ac-Nle-c(Cys-D-Ala-His-D-Phe-Arg-Trp-Cys)-ß-Ala- $(Arg)_2$-Lys-$(Arg)_2$-Gln-$(Arg)_3$-$NH_2$; (SEQ ID NO:143)

Ac-Nle-c(Cys-D-Ala-His-D-Phe-Arg-Trp-Cys)-ß-Ala-Arg-Lys-$(Arg)_3$-Gln-$(Arg)_3$-$NH_2$; (SEQ ID NO:144)

Ac-Nle-c(Cys-D-Ala-His-D-Phe-Arg-Trp-Cys)-ß-Ala-Tyr-Gly-Arg-$(Lys)_2$-Arg-Gln-$(Arg)_4$-$NH_2$; (SEQ ID NO:145)

Ac-Nle-c(Cys-D-Ala-His-D-Phe-Arg-Trp-Cys)-Doc-Tyr-Gly-Arg-$(Lys)_2$-$(Arg)_2$-Gln-$(Arg)_3$-$NH_2$; (SEQ ID NO:146)

Ac-Nle-c(Cys-D-Ala-His-D-Phe-Arg-Trp-Cys)-ß-Ala-$(Arg)_5$-Gln-$(Arg)_3$-$NH_2$; (SEQ ID NO:148)

Ac-Nle-c(Cys-D-Ala-His-D-Phe-Arg-Trp-Cys)-β-Ala-Gly-$(Arg)_5$-Gln-$(Arg)_3$-$NH_2$; (SEQ ID NO:147)
Ac-Nle-c(Cys-D-Ala-His-D-Phe-Arg-Trp-Cys)-β-Ala-Gly-$(Arg)_5$-Gln-$(Arg)_3$-$NH_2$; (SEQ ID NO:147)
Ac-Nle-c(Cys-D-Ala-His-D-Phe-Arg-Trp-Cys)-$(\beta\text{-Ala})_2$-$(Arg)_5$-Gln-$(Arg)_3$-$NH_2$; (SEQ ID NO:148)
Ac-Nle-c(Cys-D-Ala-His-D-Phe-Arg-Trp-Cys)-$(\beta\text{-Ala})_2$-Gly-$(Arg)_5$-Gln-$(Arg)_3$-$NH_2$; (SEQ ID NO:149)

Ac-Nle-c(Cys-D-Ala-His-D-Phe-Arg-Trp-Cys)-(ß-Ala)$_2$-Tyr-Gly-$(Arg)_5$-Gln-$(Arg)_3$-$NH_2$; (SEQ ID NO:149)

Ac-Nle-c(Cys-D-Ala-His-D-Phe-Arg-Trp-Cys)-Doc-$(Arg)_5$-Gln-$(Arg)_3$-$NH_2$; (SEQ ID NO:151)
Ac-Nle-c(Cys-D-Ala-His-D-Phe-Arg-Trp-Cys)-Doc-Gly-$(Arg)_5$-Gln-$(Arg)_3$-$NH_2$; (SEQ ID NO:150)
Ac-Nle-c(Cys-D-Ala-His-D-Phe-Arg-Trp-Cys)-Doc-Tyr-Gly-$(Arg)_5$-Gln-$(Arg)_3$-$NH_2$; (SEQ ID NO:150)
Ac-Nle-c(Cys-D-Ala-His-D-Phe-Arg-Trp-Cys)-$(Doc)_2$-$(Arg)_5$-Gln-$(Arg)_3$-$NH_2$; (SEQ ID NO:151)
Ac-Nle-c(Cys-D-Ala-His-D-Phe-Arg-Trp-Cys)-$(Doc)_2$-Gly-$(Arg)_5$-Gln-$(Arg)_3$-$NH_2$; (SEQ ID NO:152)
Ac-Nle-c(Cys-D-Ala-His-D-Phe-Arg-Trp-Cys)-$(Doc)_2$-Tyr-Gly-$(Arg)_5$-Gln-$(Arg)_3$-$NH_2$; (SEQ ID NO:152)
Ac-Nle-c(Cys-D-Ala-His-D-Phe-Arg-Trp-Cys)-β-Ala-$(Arg)_5$-Gln-$(Arg)_4$-$NH_2$; (SEQ ID NO:154)
Ac-Nle-c(Cys-D-Ala-His-D-Phe-Arg-Trp-Cys)-β-Ala-Gly-$(Arg)_5$-Gln-$(Arg)_4$-$NH_2$; (SEQ ID NO:153)
Ac-Nle-c(Cys-D-Ala-His-D-Phe-Arg-Trp-Cys)-β-Ala-Tyr-Gly-$(Arg)_5$-Gln-$(Arg)_4$-$NH_2$; (SEQ ID NO:153)
Ac-Nle-c(Cys-D-Ala-His-D-Phe-Arg-Trp-Cys)-$(\beta\text{-Ala})_2$-$(Arg)_5$-Gln-$(Arg)_4$-$NH_2$; (SEQ ID NO:154)
Ac-Nle-c(Cys-D-Ala-His-D-Phe-Arg-Trp-Cys)-$(\beta\text{-Ala})_2$-Gly-$(Arg)_5$-Gln-$(Arg)_4$-$NH_2$; (SEQ ID NO:155)

Ac-Nle-c(Cys-D-Ala-His-D-Phe-Arg-Trp-Cys)-(β-Ala)$_2$-Tyr-Gly-(Arg)$_5$-Gln-(Arg)$_4$-NH$_2$; (SEQ ID NO:155)

Ac-Nle-c(Cys-D-Ala-His-D-Phe-Arg-Trp-Cys)-Doc-(Arg)$_5$-Gln-(Arg)$_4$-NH$_2$; (SEQ ID NO:157)
Ac-Nle-c(Cys-D-Ala-His-D-Phe-Arg-Trp-Cys)-Doc-Gly-(Arg)$_5$-Gln-(Arg)$_4$-NH$_2$; (SEQ ID NO:156)
Ac-Nle-c(Cys-D-Ala-His-D-Phe-Arg-Trp-Cys)-Doc-Tyr-Gly-(Arg)$_5$-Gln-(Arg)$_4$-NH$_2$; (SEQ ID NO:156)
Ac-Nle-c(Cys-D-Ala-His-D-Phe-Arg-Trp-Cys)-(Doc)$_2$-(Arg)$_5$-Gln-(Arg)$_4$-NH$_2$; (SEQ ID NO:157)
Ac-Nle-c(Cys-D-Ala-His-D-Phe-Arg-Trp-Cys)-(Doc)$_2$-Gly-(Arg)$_5$-Gln-(Arg)$_4$-NH$_2$; (SEQ ID NO:158)
Ac-Nle-c(Cys-D-Ala-His-D-Phe-Arg-Trp-Cys)-(Doc)$_2$-Tyr-Gly-(Arg)$_5$-Gln-(Arg)$_4$-NH$_2$; (SEQ ID NO:158)
Ac-Nle-c(Cys-His-D-Phe-Arg-Trp-Gaba-Cys)-β-Ala-Tyr-Gly-(Arg)$_5$-Gln-(Arg)$_3$-NH$_2$; (SEQ ID NO:159)
Ac-Nle-c(Cys-His-D-Phe-Arg-Trp-Gaba-Cys)-β-Ala-(Arg)$_5$-Gln-(Arg)$_3$-NH$_2$; (SEQ ID NO:160)
Ac-Nle-c(Asp-His-D-Phe-Arg-Trp-Ala-Lys)-β-Ala-Tyr-Gly-(Arg)$_5$-Gln-(Arg)$_3$-NH$_2$; (SEQ ID NO:161)
Ac-Nle-c(Asp-His-D-Phe-Arg-Trp-Ala-Lys)-β-Ala-(Arg)$_5$-Gln-(Arg)$_3$-NH$_2$; (SEQ ID NO:162)
Ac-Nle-c(Asp-His-D-Phe-Arg-Trp-Lys)-β-Ala-Tyr-Gly-(Arg)$_5$-Gln-(Arg)$_3$-NH$_2$; (SEQ ID NO:164)
Ac-Nle-c(Asp-His-D-Phe-Arg-Trp-Lys)-β-Ala-Gly-(Arg)$_5$-Gln-(Arg)$_3$-NH$_2$; (SEQ ID NO:163)
Ac-Nle-c(Asp-His-D-Phe-Arg-Trp-Lys)-β-Ala-(Arg)$_5$-Gln-(Arg)$_3$-NH$_2$; (SEQ ID NO:163)
Ac-Nle-c(Asp-His-D-Phe-Arg-Trp-Lys)-(β-Ala)$_2$-Tyr-Gly-(Arg)$_5$-Gln-(Arg)$_3$-NH$_2$; (SEQ ID NO:164)
Ac-Nle-c(Asp-His-D-Phe-Arg-Trp-Lys)-(β-Ala)$_2$-Gly-(Arg)$_5$-Gln-(Arg)$_3$-NH$_2$; (SEQ ID NO:165)
Ac-Nle-c(Asp-His-D-Phe-Arg-Trp-Lys)-(β-Ala)$_2$-(Arg)$_5$-Gln-(Arg)$_3$-NH$_2$; (SEQ ID NO:165)
Ac-Nle-c(Asp-His-D-Phe-Arg-Trp-Lys)-Doc-Tyr-Gly-(Arg)$_5$-Gln-(Arg)$_3$-NH$_2$; (SEQ ID NO:166)
Ac-Nle-c(Asp-His-D-Phe-Arg-Trp-Lys)-Doc-Gly-(Arg)$_5$-Gln-(Arg)$_3$-NH$_2$; (SEQ ID NO:166)
Ac-Nle-c(Asp-His-D-Phe-Arg-Trp-Lys)-Doc-(Arg)$_5$-Gln-(Arg)$_3$-NH$_2$; (SEQ ID NO:168)
Ac-Nle-c(Asp-His-D-Phe-Arg-Trp-Lys)-(Doc)$_2$-Tyr-Gly-(Arg)$_5$-Gln-(Arg)$_3$-NH$_2$; (SEQ ID NO:167)
Ac-Nle-c(Asp-His-D-Phe-Arg-Trp-Lys)-(Doc)$_2$-Gly-(Arg)$_5$-Gln-(Arg)$_3$-NH$_2$; (SEQ ID NO:167)
Ac-Nle-c(Asp-His-D-Phe-Arg-Trp-Lys)-(Doc)$_2$-(Arg)$_5$-Gln-(Arg)$_3$-NH$_2$; (SEQ ID NO:168)
Ac-Nle-c(Asp-His-D-Phe-Arg-Trp-Lys)-β-Ala-Tyr-Gly-(Arg)$_5$-Gln-(Arg)$_4$-NH$_2$; (SEQ ID NO:170)
Ac-Nle-c(Asp-His-D-Phe-Arg-Trp-Lys)-β-Ala-Gly-(Arg)$_5$-Gln-(Arg)$_4$-NH$_2$; (SEQ ID NO:169)
Ac-Nle-c(Asp-His-D-Phe-Arg-Trp-Lys)-β-Ala-(Arg)$_5$-Gln-(Arg)$_4$-NH$_2$; (SEQ ID NO:169)
Ac-Nle-c(Asp-His-D-Phe-Arg-Trp-Lys)-(β-Ala)$_2$-Tyr-Gly-(Arg)$_5$-Gln-(Arg)$_4$-NH$_2$; (SEQ ID NO:170)
Ac-Nle-c(Asp-His-D-Phe-Arg-Trp-Lys)-(β-Ala)$_2$-Gly-(Arg)$_5$-Gln-(Arg)$_4$-NH$_2$; (SEQ ID NO:171)
Ac-Nle-c(Asp-His-D-Phe-Arg-Trp-Lys)-(β-Ala)$_2$-(Arg)$_5$-Gln-(Arg)$_4$-NH$_2$; (SEQ ID NO:171)
Ac-Nle-c(Asp-His-D-Phe-Arg-Trp-Lys)-Doc-Tyr-Gly-(Arg)$_5$-Gln-(Arg)$_4$-NH$_2$; (SEQ ID NO:173)
Ac-Nle-c(Asp-His-D-Phe-Arg-Trp-Lys)-Doc-Gly-(Arg)$_5$-Gln-(Arg)$_4$-NH$_2$; (SEQ ID NO:172)
Ac-Nle-c(Asp-His-D-Phe-Arg-Trp-Lys)-Doc-(Arg)$_5$-Gln-(Arg)$_4$-NH$_2$; (SEQ ID NO:172)
Ac-Nle-c(Asp-His-D-Phe-Arg-Trp-Lys)-(Doc)$_2$-Tyr-Gly-(Arg)$_5$-Gln-(Arg)$_4$-NH$_2$; (SEQ ID NO:173)
Ac-Nle-c(Asp-His-D-Phe-Arg-Trp-Lys)-(Doc)$_2$-Gly-(Arg)$_5$-Gln-(Arg)$_4$-NH$_2$; (SEQ ID NO:174)
Ac-Nle-c(Asp-His-D-Phe-Arg-Trp-Lys)-(Doc)$_2$-(Arg)$_5$-Gln-(Arg)$_4$-NH$_2$; (SEQ ID NO:174)
Ac-Nle-c(Asp-His-D-Phe-Arg-Trp-β-Ala-Lys)-β-Ala-Tyr-Gly-(Arg)$_5$-Gln-(Arg)$_3$-NH$_2$; (SEQ ID NO:175)
Ac-Nle-c(Asp-His-D-Phe-Arg-Trp-β-Ala-Lys)-β-Ala-(Arg)$_5$-Gln-(Arg)$_3$-NH$_2$; (SEQ ID NO:176)
Ac-Nle-c(Cys-His-D-Phe-Arg-Trp-Ahx-Cys)-β-Ala-Tyr-Gly-(Arg)$_5$-Gln-(Arg)$_3$-NH$_2$; (SEQ ID NO:177)
Ac-Nle-c(Cys-His-D-Phe-Arg-Trp-Ahx-Cys)-β-Ala-(Arg)$_5$-Gln-(Arg)$_3$-NH$_2$; (SEQ ID NO:178)
D-Phe-c(Cys-His-D-Phe-Arg-Trp-β-Ala-D-Cys)-Thr-β-Ala-Tyr-Gly-(Arg)$_5$-Gln-(Arg)$_3$-NH$_2$; (SEQ ID NO:179)
D-Phe-c(Cys-His-D-Phe-Arg-Trp-β-Ala-D-Cys)-Thr-β-Ala-(Arg)$_5$-Gln-(Arg)$_3$-NH$_2$; (SEQ ID NO:180)
Ac-Nle-c(Cys-His-D-Phe-Arg-Trp-Apn-Cys)-β-Ala-Tyr-Gly-(Arg)$_5$-Gln-(Arg)$_3$-NH$_2$; (SEQ ID NO:181)
Ac-Nle-c(Cys-His-D-Phe-Arg-Trp-Apn-Cys)-β-Ala-(Arg)$_5$-Gln-(Arg)$_3$-NH$_2$; (SEQ ID NO:182)
Ac-Cha-c(Asp-His-D-Phe-Arg-Trp-Gaba-Lys)-β-Ala-Tyr-Gly-(Arg)$_5$-Gln-(Arg)$_3$-NH$_2$; (SEQ ID NO:183)
Ac-Cha-c(Asp-His-D-Phe-Arg-Trp-Gaba-Lys)-β-Ala-(Arg)$_5$-Gln-(Arg)$_3$-NH$_2$; (SEQ ID NO:184)
Ac-Nle-c(Asp-His-D-Phe-Arg-Trp-Gaba-Lys)-β-Ala-Tyr-Gly-(Arg)$_5$-Gln-(Arg)$_3$-NH$_2$; (SEQ ID NO:183)
Ac-Nle-c(Asp-His-D-Phe-Arg-Trp-Gaba-Lys)-β-Ala-(Arg)$_5$-Gln-(Arg)$_3$-NH$_2$; (SEQ ID NO:185)
Ac-Chg-c(Asp-His-D-Phe-Arg-Trp-Gaba-Lys)-β-Ala-Tyr-Gly-(Arg)$_5$-Gln-(Arg)$_3$-NH$_2$; (SEQ ID NO:186)
Ac-Chg-c(Asp-His-D-Phe-Arg-Trp-Gaba-Lys)-β-Ala-(Arg)$_5$-Gln-(Arg)$_3$-NH$_2$; (SEQ ID NO:185)
Ac-hCha-c(Asp-His-D-Phe-Arg-Trp-Gaba-Lys)-β-Ala-Tyr-Gly-(Arg)$_5$-Gln-(Arg)$_3$-NH$_2$; (SEQ ID NO:186)
Ac-hCha-c(Asp-His-D-Phe-Arg-Trp-Gaba-Lys)-β-Ala-(Arg)$_5$-Gln-(Arg)$_3$-NH$_2$; (SEQ ID NO:188)
Ac-hCha-c(Asp-His-D-Phe-Arg-Trp-Gaba-Lys)-β-Ala)$_2$-Tyr-Gly-(Arg)$_5$-Gln-(Arg)$_3$-NH$_2$; (SEQ ID NO:187)
Ac-hCha-c(Asp-His-D-Phe-Arg-Trp-Gaba-Lys)-(β-Ala)$_2$-(Arg)$_5$-Gln-(Arg)$_3$-NH$_2$; (SEQ ID NO:188)
Ac-hCha-c(Asp-His-D-Phe-Arg-Trp-Gaba-Lys)-Doc-Tyr-Gly-(Arg)$_5$-Gln-(Arg)$_3$-NH$_2$; (SEQ ID NO:189)

Ac-hCha-c(Asp-His-D-Phe-Arg-Trp-Gaba-Lys)-Doc-$(Arg)_5$-Gln-$(Arg)_3$-$NH_2$; (SEQ ID NO:190)
Ac-hCha-c(Asp-His-D-Phe-Arg-Trp-Gaba-Lys)-$(Doc)_2$-Tyr-Gly-$(Arg)_5$-Gln-$(Arg)_3$-$NH_2$; (SEQ ID NO:189)
Ac-hCha-c(Asp-His-D-Phe-Arg-Trp-Gaba-Lys)-$(Doc)_2$-$(Arg)_5$-Gln-$(Arg)_3$-$NH_2$; (SEQ ID NO:190)
Ac-hCha-c(Asp-His-D-Phe-Arg-Trp-Gaba-Lys)-β-Ala-Tyr-Gly-$(Arg)_5$-Gln-$(Arg)_4$-$NH_2$; (SEQ ID NO:191)
Ac-hCha-c(Asp-His-D-Phe-Arg-Trp-Gaba-Lys)-β-Ala-$(Arg)_5$-Gln-$(Arg)_4$-$NH_2$; (SEQ ID NO:192)

Ac-hCha-c(Asp-His-D-Phe-Arg-Trp-Gaba-Lys)-(*β*-Ala)$_2$-Tyr-Gly-$(Arg)_5$-Gln-$(Arg)_4$-$NH_2$; (SEQ ID NO:191)

Ac-hCha-c(Asp-His-D-Phe-Arg-Trp-Gaba-Lys)-(β-Ala)$_2$-$(Arg)_5$-Gln-$(Arg)_4$-$NH_2$; (SEQ ID NO:192)
Ac-hCha-c(Asp-His-D-Phe-Arg-Trp-Gaba-Lys)-Doc-Tyr-Gly-$(Arg)_5$-Gln-$(Arg)_4$-$NH_2$; (SEQ ID NO:193)
Ac-hCha-c(Asp-His-D-Phe-Arg-Trp-Gaba-Lys)-Doc-$(Arg)_5$-Gln-$(Arg)_4$-$NH_2$; (SEQ ID NO:194)

Ac-hCha-c(Asp-His-D-Phe-Arg-Trp-Gaba-Lys)-$(Doc)_2$-Tyr-Gly-$(Arg)_5$-Gln-$(Arg)_4$-$NH_2$; (SEQ ID NO:193)

Ac-hCha-c(Asp-His-D-Phe-Arg-Trp-Gaba-Lys)-$(Doc)_2$-$(Arg)_5$-Gln-$(Arg)_4$-$NH_2$; (SEQ ID NO:194)

Ac-D-Chg-c(Asp-His-D-Phe-Arg-Trp-Gaba-Lys)-*β*-Ala-Tyr-Gly-$(Arg)_5$-Gln-$(Arg)_3$-$NH_2$; (SEQ ID NO:195)

Ac-D-Chg-c(Asp-His-D-Phe-Arg-Trp-Gaba-Lys)-β-Ala-$(Arg)_5$-Gln-$(Arg)_3$-$NH_2$; (SEQ ID NO:196)
Ac-hPhe-c(Asp-His-D-Phe-Arg-Trp-Gaba-Lys)-β-Ala-Tyr-Gly-$(Arg)_5$-Gln-$(Arg)_3$-$NH_2$; (SEQ ID NO:197)
Ac-hPhe-c(Asp-His-D-Phe-Arg-Trp-Gaba-Lys)-β-Ala-$(Arg)_5$-Gln-$(Arg)_3$-$NH_2$; (SEQ ID NO:198)
Ac-Nle-c(Cys-His-D-Phe-Arg-D-Trp-Apn-Cys)-β-Ala-Tyr-Gly-$(Arg)_5$-Gln-$(Arg)_3$-$NH_2$; (SEQ ID NO:199)
Ac-Nle-c(Cys-His-D-Phe-Arg-D-Trp-Apn-Cys)-β-Ala-$(Arg)_5$-Gln-$(Arg)_3$-$NH_2$; (SEQ ID NO:200)
Ac-Nle-c(Cys-His-D-Phe-Arg-D-Trp-Ahx-Cys)-β-Ala-Tyr-Gly-$(Arg)_5$-Gln-$(Arg)_3$-$NH_2$; (SEQ ID NO:199)
Ac-Nle-c(Cys-His-D-Phe-Arg-D-Trp-Ahx-Cys)-β-Ala-$(Arg)_5$-Gln-$(Arg)_3$-$NH_2$; (SEQ ID NO:200)

Ac-Nle-c(Cys-His-D-Phe-Arg-D-Trp-*β*-Ala-Cys)-*β*-Ala-Tyr-Gly-$(Arg)_5$-Gln-$(Arg)_3$-$NH_2$; (SEQ ID NO:201)

Ac-Nle-c(Cys-His-D-Phe-Arg-D-Trp-β-Ala-Cys)-β-Ala-$(Arg)_5$-Gln-$(Arg)_3$-$NH_2$; (SEQ ID NO:202)
Ac-Nle-c(Cys-D-Ala-His-D-Phe-Arg-Trp-Pen)-β-Ala-Tyr-Gly-$(Arg)_5$-Gln-$(Arg)_3$-$NH_2$; (SEQ ID NO:203)
Ac-Nle-c(Cys-D-Ala-His-D-Phe-Arg-Trp-Pen)-β-Ala-Gly-$(Arg)_5$-Gln-$(Arg)_3$-$NH_2$; (SEQ ID NO:203)
Ac-Nle-c(Cys-D-Ala-His-D-Phe-Arg-Trp-Pen)-β-Ala-$(Arg)_5$-Gln-$(Arg)_3$-$NH_2$; (SEQ ID NO:205)

Ac-Nle-c(Cys-D-Ala-His-D-Phe-Arg-Trp-Pen)-(*β*-Ala)$_2$-Tyr-Gly-$(Arg)_5$-Gln-$(Arg)_3$-$NH_2$; (SEQ ID NO:204)

Ac-Nle-c(Cys-D-Ala-His-D-Phe-Arg-Trp-Pen)-(β-Ala)$_2$-Gly-$(Arg)_5$-Gln-$(Arg)_3$-$NH_2$; (SEQ ID NO:204)
Ac-Nle-c(Cys-D-Ala-His-D-Phe-Arg-Trp-Pen)-(β-Ala)$_2$-$(Arg)_5$-Gln-$(Arg)_3$-$NH_2$; (SEQ ID NO:205)
Ac-Nle-c(Cys-D-Ala-His-D-Phe-Arg-Trp-Pen)-Doc-Tyr-Gly-(Arg)5$_5$s-Gln-$(Arg)_3$-$NH_2$; (SEQ ID NO:207)
Ac-Nle-c(Cys-D-Ala-His-D-Phe-Arg-Trp-Pen)-Doc-Gly-$(Arg)_5$-Gln-$(Arg)_3$-$NH_2$; (SEQ ID NO:206)
Ac-Nle-c(Cys-D-Ala-His-D-Phe-Arg-Trp-Pen)-Doc-$(Arg)_5$-Gln-$(Arg)_3$-$NH_2$; (SEQ ID NO:206)
Ac-Nle-c(Cys-D-Ala-His-D-Phe-Arg-Trp-Pen)-$(Doc)_2$-Tyr-Gly-$(Arg)_5$-Gln-$(Arg)_3$-$NH_2$; (SEQ ID NO:207)
Ac-Nle-c(Cys-D-Ala-His-D-Phe-Arg-Trp-Pen)-$(Doc)_2$-Gly-$(Arg)_5$-Gln-$(Arg)_3$-$NH_2$; (SEQ ID NO:208)
Ac-Nle-c(Cys-D-Ala-His-D-Phe-Arg-Trp-Pen)-$(Doc)_2$-$(Arg)_5$-Gln-$(Arg)_3$-$NH_2$; (SEQ ID NO:208)

D-Phe-c(Cys-His-D-(Et)Tyr-Arg-Trp-ß-Ala-D-Cys)-ß-Ala-Tyr-Gly-$(Arg)_5$-Gln-$(Arg)_3$-$NH_2$; (SEQ ID NO:209)

D-Phe-c(Cys-His-D-(Et)Tyr-Arg-Trp-β-Ala-D-Cys)-β-Ala-$(Arg)_5$-Gln-$(Arg)_3$-$NH_2$; (SEQ ID NO:210)
D-Phe-c(Cys-His-D-(Et)Tyr-Arg-Trp-β-Ala-D-Cys)-β-Ala-Gly-$(Arg)_5$-Gln-$(Arg)_3$-$NH_2$; (SEQ ID NO:209)
D-Phe-c(Cys-His-D-(Et)Tyr-Arg-Trp-β-Ala-D-Cys)-β-Ala-$(Arg)_5$-Gln-$(Arg)_4$-$NH_2$; (SEQ ID NO:211)

D-Phe-c(Cys-His-D-(Et)Tyr-Arg-Trp-ß-Ala-D-Cys)-$(ß\text{-}Ala)_2$-Tyr-Gly-$(Arg)_5$-Gln-$(Arg)_3$-$NH_2$; (SEQ ID NO:212)

D-Phe-c(Cys-His-D-(Et)Tyr-Arg-Trp-β-Ala-D-Cys)-$(β\text{-}Ala)_2$-$(Arg)_5$-Gln-$(Arg)_3$-$NH_2$; (SEQ ID NO:213)

D-Phe-c(Cys-His-D-(Et)Tyr-Arg-Trp-ß-Ala-D-Cys)-$(ß\text{-}Ala)_2$-Gly-$(Arg)_5$-Gln-$(Arg)_3$-$NH_2$; (SEQ ID NO:213)

D-Phe-c(Cys-His-D-(Et)Tyr-Arg-Trp-β-Ala-D-Cys)-$(β\text{-}Ala)_2$-$(Arg)_5$-Gln-$(Arg)_4$-$NH_2$; (SEQ ID NO:267)

D-Phe-c(Cys-His-D-(Et)Tyr-Arg-Trp-ß-Ala-D-Cys)-Doc-Tyr-Gly-$(Arg)_5$-Gln-$(Arg)_3$-$NH_2$; (SEQ ID NO:214)

D-Phe-c(Cys-His-D-(Et)Tyr-Arg-Trp-β-Ala-D-Cys)-Doc-$(Arg)_5$-Gln-$(Arg)_3$-$NH_2$; (SEQ ID NO:216)
D-Phe-c(Cys-His-D-(Et)Tyr-Arg-Trp-β-Ala-D-Cys)-Doc-Gly-$(Arg)_5$-Gln-$(Arg)_3$-$NH_2$; (SEQ ID NO:214)
D-Phe-c(Cys-His-D-(Et)Tyr-Arg-Trp-β-Ala-D-Cys)-Doc-$(Arg)_5$-Gln-$(Arg)_4$-$NH_2$; (SEQ ID NO:217)

D-Phe-c(Cys-His-D-(Et)Tyr-Arg-Trp-ß-Ala-D-Cys)-$(Doc)_2$-Tyr-Gly-$(Arg)_5$-Gln-$(Arg)_3$-$NH_2$; (SEQ ID NO:215)

D-Phe-c(Cys-His-D-(Et)Tyr-Arg-Trp-β-Ala-D-Cys)-$(Doc)_2$-$(Arg)_5$-Gln-$(Arg)_3$-$NH_2$; (SEQ ID NO:216)

D-Phe-c(Cys-His-D-(Et)Tyr-Arg-Trp-ß-Ala-D-Cys)-$(Doc)_2$-Gly-$(Arg)_5$-Gln-$(Arg)_3$-$NH_2$; (SEQ ID NO:215)

D-Phe-c(Cys-His-D-(Et)Tyr-Arg-Trp-β-Ala-D-Cys)-$(Doc)_2$-$(Arg)_5$-Gln-$(Arg)_4$-$NH_2$; (SEQ ID NO:217)

D-Phe-c(Cys-His-D-(Et)Tyr-hArg-Trp-ß-Ala-D-Cys)-Thr-ß-Ala-Tyr-Gly-$(Arg)_5$-Gln-$(Arg)_3$-$NH_2$; (SEQ ID NO:218)

D-Phe-c(Cys-His-D-(Et)Tyr-hArg-Trp-ß-Ala-D-Cys)-Thr-ß-Ala-$(Arg)_5$-Gln-$(Arg)_3$-$NH_2$; (SEQ ID NO:219)

D-Phe-c(Cys-His-D-(Et)Tyr-hArg-Trp-ß-Ala-D-Cys)-Thr-(ß-Ala)$_2$-Tyr-Gly-(Arg)$_5$-Gln-(Arg)$_3$-$NH_2$; (SEQ ID NO:218)

D-Phe-c(Cys-His-D-(Et)Tyr-hArg-Trp-ß-Ala-D-Cys)-Thr-(ß-Ala)$_2$-(Arg)$_5$-Gln-(Arg)$_3$-$NH_2$; (SEQ ID NO:219)

D-Phe-c(Cys-His-D-(Et)Tyr-hArg-Trp-ß-Ala-D-Cys)-Thr-Doc-Tyr-Gly-(Arg)$_5$-Gln-(Arg)$_3$-$NH_2$; (SEQ ID NO:221)

D-Phe-c(Cys-His-D-(Et)Tyr-hArg-Trp-β-Ala-D-Cys)-Thr-Doc-(Arg)$_5$-Gln-(Arg)$_3$-$NH_2$; (SEQ ID NO:220)

D-Phe-c(Cys-His-D-(Et)Tyr-hArg-Trp-ß-Ala-D-Cys)-Thr-(Doc)$_2$-Tyr-Gly-(Arg)$_5$-Gln-(Arg)$_3$-$NH_2$; (SEQ ID NO:221)

D-Phe-c(Cys-His-D-(Et)Tyr-hArg-Trp-ß-Ala-D-Cys)-Thr-ß-Ala-Tyr-Gly-(Arg)$_5$-Gln-(Arg)$_4$-$NH_2$; (SEQ ID NO:222)

D-Phe-c(Cys-His-D-(Et)Tyr-hArg-Trp-ß-Ala-D-Cys)-Thr-ß-Ala-(Arg)$_5$-Gln-(Arg)$_4$-$NH_2$; (SEQ ID NO:223)

D-Phe-c(Cys-His-D-(Et)Tyr-hArg-Trp-ß-Ala-D-Cys)-Thr-(ß-Ala)$_2$-Tyr-Gly-(Arg)$_5$-Gln-(Arg)$_4$-$NH_2$; (SEQ ID NO:222)

D-Phe-c(Cys-His-D-(Et)Tyr-hArg-Trp-ß-Ala-D-Cys)-Thr-(ß-Ala)$_2$-(Arg)$_5$-Gln-(Arg)$_4$-$NH_2$; (SEQ ID NO:223)

D-Phe-c(Cys-His-D-(Et)Tyr-hArg-Trp-ß-Ala-D-Cys)-Thr-Doc-Tyr-Gly-(Arg)$_5$-Gln-(Arg)$_4$-$NH_2$; (SEQ ID NO:224)

D-Phe-c(Cys-His-D-(Et)Tyr-hArg-Trp-β-Ala-D-Cys)-Thr-Doc-(Arg)$_5$-Gln-(Arg)$_4$-$NH_2$; (SEQ ID NO:225)

D-Phe-c(Cys-His-D-(Et)Tyr-hArg-Trp-ß-Ala-D-Cys)-Thr-(Doc)$_2$-Tyr-Gly-(Arg)$_5$-Gln-(Arg)$_4$-$NH_2$; (SEQ ID NO:224)

D-Phe-c(Cys-His-D-(Et)Tyr-hArg-Trp-ß-Ala-D-Cys)-Thr-(Doc)$_2$-(Arg)$_5$-Gln-(Arg)$_4$-$NH_2$; (SEQ ID NO:225)

D-Phe-c(Cys-His-D-(Et)Tyr-hArg-Bip-ß-Ala-D-Cys)-Thr-ß-Ala-Tyr-Gly-$(Arg)_5$-Gln-$(Arg)_3$-$NH_2$; (SEQ ID NO:227)

D-Phe-c(Cys-His-D-(Et)Tyr-hArg-Bip-ß-Ala-D-Cys)-Thr-ß-Ala-Tyr-Gly-$(Arg)_5$-Gln-$(Arg)_4$-$NH_2$; (SEQ ID NO:226)

D-Phe-c(Cys-His-D-(Et)Tyr-hArg-Bip-ß-Ala-D-Cys)-Thr-ß-Ala-$(Arg)_5$-Gln-$(Arg)_3$-$NH_2$; (SEQ ID NO:228)

D-Phe-c(Cys-His-D-(Et)Tyr-hArg-Bip-ß-Ala-D-Cys)-Thr-$(ß\text{-Ala})_2$-Tyr-Gly-$(Arg)_5$-Gln-$(Arg)_3$-$NH_2$; (SEQ ID NO:227)

D-Phe-c(Cys-His-D-(Et)Tyr-hArg-Bip-ß-Ala-D-Cys)-Thr-$(ß\text{-Ala})_2$-$(Arg)_5$-Gln-$(Arg)_3$-$NH_2$; (SEQ ID NO:228)

D-Phe-c(Cys-His-D-(Et)Tyr-hArg-Bip-ß-Ala-D-Cys)-Thr-Doc-Tyr-Gly-$(Arg)_5$-Gln-$(Arg)_3$-$NH_2$; (SEQ ID NO:229)

D-Phe-c(Cys-His-D-(Et)Tyr-hArg-Bip-ß-Ala-D-Cys)-Thr-Doc-Tyr-Gly-$(Arg)_5$-Gln-$(Arg)_4$-$NH_2$; (SEQ ID NO:230)

D-Phe-c(Cys-His-D-(Et)Tyr-hArg-Bip-β-Ala-D-Cys)-Thr-Doc-$(Arg)_5$-Gln-$(Arg)_3$-$NH_2$; (SEQ ID NO:232)

D-Phe-c(Cys-His-D-(Et)Tyr-hArg-Bip-ß-Ala-D-Cys)-Thr-$(Doc)_2$-Tyr-Gly-$(Arg)_5$-Gln-$(Arg)_3$-$NH_2$; (SEQ ID NO:231)

D-Phe-c(Cys-His-D-(Et)Tyr-hArg-Bip-ß-Ala-D-Cys)-Thr-$(Doc)_2$-$(Arg)_5$-Gln-$(Arg)_3$-$NH_2$; (SEQ ID NO:232)

Ac-Nle-c(Cys-D-Ala-His-D-Phe-Arg-Trp-Gly-Cys)-ß-Ala-Tyr-Gly-$(Arg)_5$-Gln-$(Arg)_3$-$NH_2$; (SEQ ID NO:233)

Ac-Nle-c(Cys-D-Ala-His-D-Phe-Arg-Trp-Gly-Cys)-β-Ala-$(Arg)_5$-Gln-$(Arg)_3$-$NH_2$; (SEQ ID NO:234)
Nle-c(Cys-His-D-Phe-Arg-Trp-Apn-Cys)-β-Ala- Tyr-Gly-$(Arg)_5$-Gln-$(Arg)_3$-$NH_2$; (SEQ ID NO:235)
Nle-c(Cys-His-D-Phe-Arg-Trp-Apn-Cys)-β-Ala-$(Arg)_5$-Gln-$(Arg)_3$-$NH_2$; (SEQ ID NO:236)
Nle-c(Cys-His-D-Phe-Arg-Trp-Apn-Cys)-$(β\text{-Ala})_2$-Tyr-Gly-$(Arg)_5$-Gln-$(Arg)_3$-$NH_2$; (SEQ ID NO:235)
Nle-c(Cys-His-D-Phe-Arg-Trp-Apn-Cys)-$(β\text{-Ala})_2$-$(Arg)_5$-Gln-$(Arg)_3$-$NH_2$; (SEQ ID NO:236)
Nle-c(Cys-His-D-Phe-Arg-Trp-Apn-Cys)-β-Ala-Tyr-Gly-$(Arg)_5$-Gln-$(Arg)_4$-$NH_2$; (SEQ ID NO:237)

Nle-c(Cys-His-D-Phe-Arg-Trp-Apn-Cys)-β-Ala-$(Arg)_5$-Gln-$(Arg)_4$-$NH_2$; (SEQ ID NO:238)
Nle-c(Cys-His-D-Phe-Arg-Trp-Apn-Cys)-$(β-Ala)_2$-Tyr-Gly-$(Arg)_5$-Gln-$(Arg)_4$-$NH_2$; (SEQ ID NO:237)
Nle-c(Cys-His-D-Phe-Arg-Trp-Apn-Cys)-$(β-Ala)_2$-$(Arg)_5$-Gln-$(Arg)_4$-$NH_2$; (SEQ ID NO:238)
Nle-c(Cys-His-D-Phe-Arg-Trp-Apn-Cys)-Doc-Tyr-Gly-$(Arg)_5$-Gln-$(Arg)_3$-$NH_2$; (SEQ ID NO:239)
Nle-c(Cys-His-D-Phe-Arg-Trp-Apn-Cys)-Doc-$(Arg)_5$-Gln-$(Arg)_3$-$NH_2$; (SEQ ID NO:240)
Nle-c(Cys-His-D-Phe-Arg-Trp-Apn-Cys)-$(Doc)_2$-Tyr-Gly-$(Arg)_5$-Gln-$(Arg)_3$-$NH_2$; (SEQ ID NO:239)
Nle-c(Cys-His-D-Phe-Arg-Trp-Apn-Cys)-$(Doc)_2$-$(Arg)_5$-Gln-$(Arg)_3$-$NH_2$; (SEQ ID NO:240)
Nle-c(Cys-His-D-Phe-Arg-Trp-Apn-Cys)-Doc-Tyr-Gly-$(Arg)_5$-Gln-$(Arg)_4$-$NH_2$; (SEQ ID NO:241)
Nle-c(Cys-His-D-Phe-Arg-Trp-Apn-Cys)-Doc-$(Arg)_5$-Gln-$(Arg)_4$-$NH_2$; (SEQ ID NO:242)
Nle-c(Cys-His-D-Phe-Arg-Trp-Apn-Cys)-$(Doc)_2$-Tyr-Gly-$(Arg)_5$-Gln-$(Arg)_4$-$NH_2$; (SEQ ID NO:241)
Nle-c(Cys-His-D-Phe-Arg-Trp-Apn-Cys)-$(Doc)_2$-$(Arg)_5$-Gln-$(Arg)_4$-$NH_2$; (SEQ ID NO:242)
Ac-Nle-c(Cys-D-Leu-His-D-Phe-Arg-Trp-Cys)-β-Ala-Tyr-Gly-$(Arg)_5$-Gln-$(Arg)_3$-$NH_2$; (SEQ ID NO:243)
Ac-Nle-c(Cys-D-Leu-His-D-Phe-Arg-Trp-Cys)-β-Ala-$(Arg)_5$-Gln-$(Arg)_3$-$NH_2$; (SEQ ID NO:244)

Ac-Nle-c(Cys-D-Leu-His-D-Phe-Arg-Trp-Cys)-$(ß-Ala)_2$-Tyr-Gly-$(Arg)_5$-Gln-$(Arg)_3$-$NH_2$; (SEQ ID NO:243)

Ac-Nle-c(Cys-D-Leu-His-D-Phe-Arg-Trp-Cys)-$(β-Ala)_2$-$(Arg)_5$-Gln-$(Arg)_3$-$NH_2$; (SEQ ID NO:244)
Ac-Nle-c(Cys-D-Leu-His-D-Phe-Arg-Trp-Cys)-Doc-Tyr-Gly-$(Arg)_5$-Gln-$(Arg)_3$-$NH_2$; (SEQ ID NO:245)
Ac-Nle-c(Cys-D-Leu-His-D-Phe-Arg-Trp-Cys)-Doc-$(Arg)_5$-Gln-$(Arg)_3$-$NH_2$; (SEQ ID NO:246)

Ac-Nle-c(Cys-D-Leu-His-D-Phe-Arg-Trp-Cys)-$(Doc)_2$-Tyr-Gly-$(Arg)_5$-Gln-$(Arg)_3$-$NH_2$; (SEQ ID NO:245)

Ac-Nle-c(Cys-D-Leu-His-D-Phe-Arg-Trp-Cys)-$(Doc)_2$-$(Arg)_5$-Gln-$(Arg)_3$-$NH_2$; (SEQ ID NO:246)
Ac-Nle-c(Cys-D-Leu-His-D-Phe-Arg-Trp-Cys)-β-Ala-Tyr-Gly-$(Arg)_5$-Gln-$(Arg)_4$-$NH_2$; (SEQ ID NO:247)
Ac-Nle-c(Cys-D-Leu-His-D-Phe-Arg-Trp-Cys)-β-Ala-$(Arg)_5$-Gln-$(Arg)_4$-$NH_2$; (SEQ ID NO:248)

Ac-Nle-c(Cys-D-Leu-His-D-Phe-Arg-Trp-Cys)-$(ß-Ala)_2$-Tyr-Gly-$(Arg)_5$-Gln-$(Arg)_4$-$NH_2$; (SEQ ID NO:247)

Ac-Nle-c(Cys-D-Leu-His-D-Phe-Arg-Trp-Cys)-$(β-Ala)_2$-$(Arg)_5$-Gln-$(Arg)_4$-$NH_2$; (SEQ ID NO:248)
Ac-Nle-c(Cys-D-Leu-His-D-Phe-Arg-Trp-Cys)-Doc-Tyr-Gly-$(Arg)_5$-Gln-$(Arg)_4$-$NH_2$; (SEQ ID NO:249) Ac-Nle-c(Cys-D-Leu-His-D-Phe-Arg-Trp-Cys)-Doc-$(Arg)_5$-Gln-$(Arg)_4$-$NH_2$; (SEQ ID NO:250)

Ac-Nle-c(Cys-D-Leu-His-D-Phe-Arg-Trp-Cys)-$(Doc)_2$-Tyr-Gly-$(Arg)_5$-Gln-$(Arg)_4$-$NH_2$; (SEQ ID NO:249)

Ac-Nle-c(Cys-D-Leu-His-D-Phe-Arg-Trp-Cys)-(Doc)2-$(Arg)_5$-Gln-$(Arg)_4$-$NH_2$; (SEQ ID NO:250)
Ac-Nle-c(Cys-D-Cha-His-D-Phe-Arg-Trp-Cys)-β-Ala-Tyr-Gly-$(Arg)_5$-Gln-$(Arg)_3$-$NH_2$; (SEQ ID NO:251)
Ac-Nle-c(Cys-D-Cha-His-D-Phe-Arg-Trp-Cys)-β-Ala-$(Arg)_5$-Gln-$(Arg)_3$-$NH_2$; (SEQ ID NO:252)

Ac-Nle-c(Cys-D-Cha-His-D-Phe-Arg-Trp-Cys)-$(ß-Ala)_2$-Tyr-Gly-$(Arg)_5$-Gln-$(Arg)_3$-$NH_2$; (SEQ ID NO:251)

Ac-Nle-c(Cys-D-Cha-His-D-Phe-Arg-Trp-Cys)-$(β-Ala)_2$-$(Arg)_2$-Gln-$(Arg)_5$-$NH_2$; (SEQ ID NO:252)
Ac-Nle-c(Cys-D-Cha-His-D-Phe-Arg-Trp-Cys)-Doc-Tyr-Gly-$(Arg)_5$-Gln-$(Arg)_3$-$NH_2$; (SEQ ID NO:253)
Ac-Nle-c(Cys-D-Cha-His-D-Phe-Arg-Trp-Cys)-Doc-$(Arg)_5$-Gln-$(Arg)_3$-$NH_2$; (SEQ ID NO:254)

Ac-Nle-c(Cys-D-Cha-His-D-Phe-Arg-Trp-Cys)-(Doc)$_2$-Tyr-Gly-(Arg)$_5$-Gln-(Arg)$_3$-NH$_2$; (SEQ ID NO:253)

Ac-Nle-c(Cys-D-Cha-His-D-Phe-Arg-Trp-Cys)-(Doc)$_2$-(Arg)$_5$-Gln-(Arg)$_3$-NH$_2$; (SEQ ID NO:254)
Ac-Nle-c(Cys-D-Cha-His-D-Phe-Arg-Trp-Cys)-β-Ala-Tyr-Gly-(Arg)$_5$-Gln-(Arg)$_4$-NH$_2$; (SEQ ID NO:255)
Ac-Nle-c(Cys-D-Cha-His-D-Phe-Arg-Trp-Cys)-β-Ala-(Arg)$_5$-Gln-(Arg)$_4$-NH$_2$; (SEQ ID NO:256)

Ac-Nle-c(Cys-D-Cha-His-D-Phe-Arg-Trp-Cys)-(*ß*-Ala)$_2$-Tyr-Gly-(Arg)$_5$-Gln-(Arg)$_4$-NH$_2$; (SEQ ID NO:255)

Ac-Nle-c(Cys-D-Cha-His-D-Phe-Arg-Trp-Cys)-(β-Ala)$_2$-(Arg)$_5$-Gln-(Arg)$_4$-NH$_2$; (SEQ ID NO:256)
Ac-Nle-c(Cys-D-Cha-His-D-Phe-Arg-Trp-Cys)-Doc-Tyr-Gly-(Arg)$_5$-Gln-(Arg)$_4$-NH$_2$; (SEQ ID NO:257)
Ac-Nle-c(Cys-D-Cha-His-D-Phe-Arg-Trp-Cys)-Doc-(Arg)$_5$-Gln-(Arg)$_4$-NH$_2$; (SEQ ID NO:258)

Ac-Nle-c(Cys-D-Cha-His-D-Phe-Arg-Trp-Cys)-(Doc)$_2$-Tyr-Gly-(Arg)$_5$-Gln-(Arg)$_4$-NH$_2$; (SEQ ID NO:257)

Ac-Nle-c(Cys-D-Cha-His-D-Phe-Arg-Trp-Cys)-(Doc)$_2$-(Arg)$_5$-Gln-(Arg)$_4$-NH$_2$; (SEQ ID NO:258)
Nle-c(Cys-His-D-Phe-Arg-Trp-Gaba-Cys)-β-Ala-Tyr-Gly-(Arg)$_5$-Gln-(Arg)$_3$-NH$_2$; (SEQ ID NO:259)
Nle-c(Cys-His-D-Phe-Arg-Trp-Gaba-Cys)-β-Ala-(Arg)$_5$-Gln-(Arg)$_3$-NH$_2$; (SEQ ID NO:260)
Nle-c(Cys-His-D-Phe-Arg-Trp-Gaba-Cys)-(β-Ala)$_2$-Tyr-Gly-(Arg)$_5$-Gln-(Arg)$_3$-NH$_2$; (SEQ ID NO:259)
Nle-c(Cys-His-D-Phe-Arg-Trp-Gaba-Cys)-(β-Ala)$_2$-(Arg)$_5$-Gln-(Arg)$_3$-NH$_2$; (SEQ ID NO:260)
Nle-c(Cys-His-D-Phe-Arg-Trp-Gaba-Cys)-β-Ala-Tyr-Gly-(Arg)$_5$-Gln-(Arg)$_4$-NH$_2$; (SEQ ID NO:261)
Nle-c(Cys-His-D-Phe-Arg-Trp-Gaba-Cys)-β-Ala-(Arg)$_5$-Gln-(Arg)$_4$-NH$_2$; (SEQ ID NO:262)
Nle-c(Cys-His-D-Phe-Arg-Trp-Gaba-Cys)-(β-Ala)$_2$-Tyr-Gly-(Arg)$_5$-Gln-(Arg)$_4$-NH$_2$; (SEQ ID NO:261)
Nle-c(Cys-His-D-Phe-Arg-Trp-Gaba-Cys)-(β-Ala)$_2$-(Arg)$_5$-Gln-(Arg)$_4$-NH$_2$; (SEQ ID NO:262)
Nle-c(Cys-His-D-Phe-Arg-Trp-Gaba-Cys)-Doc-Tyr-Gly-(Arg)$_5$-Gln-(Arg)$_3$-NH$_2$; (SEQ ID NO:263)
Nle-c(Cys-His-D-Phe-Arg-Trp-Gaba-Cys)-Doc-(Arg)$_5$-Gln-(Arg)$_3$-NH$_2$; (SEQ ID NO:264)
Nle-c(Cys-His-D-Phe-Arg-Trp-Gaba-Cys)-(Doc)$_2$-Tyr-Gly-(Arg)$_5$-Gln-(Arg)$_3$-NH$_2$; (SEQ ID NO:263)
Nle-c(Cys-His-D-Phe-Arg-Trp-Gaba-Cys)-(Doc)$_2$-(Arg)$_5$-Gln-(Arg)$_3$-NH$_2$; (SEQ ID NO:264)
Nle-c(Cys-His-D-Phe-Arg-Trp-Gaba-Cys)-Doc-Tyr-Gly-(Arg)$_5$-Gln-(Arg)$_4$-NH$_2$; (SEQ ID NO:265)
Nle-c(Cys-His-D-Phe-Arg-Trp-Gaba-Cys)-Doc-(Arg)$_5$-Gln-(Arg)$_4$-NH$_2$; (SEQ ID NO:266)
Nle-c(Cys-His-D-Phe-Arg-Trp-Gaba-Cys)-(Doc)$_2$-Tyr-Gly-(Arg)$_5$-Gln-(Arg)$_4$-NH$_2$; (SEQ ID NO:265) or
Nle-c(Cys-His-D-Phe-Arg-Trp-Gaba-Cys)-(Doc)$_2$-(Arg)$_5$-Gln-(Arg)$_4$-NH$_2$; (SEQ ID NO:266);

or pharmaceutically acceptable salts thereof.

Also described herein is a method to treat insulin resistance in a mammalian subject, with or without weight loss, by the administration of a therapeutically effective amount of a melanocortin receptor compound described herein, and pharmaceutically acceptable salts, hydrates, solvates and prodrugs thereof, wherein the compounds discovered to treat insulin resistance in a mammalian subject, with or without weight loss, include the following:

Ac-c(Cys-Glu-His-D-4-Br-Phe-Arg-Trp-Gly-Cys)-(Pro)$_2$-Lys-Asp-NH$_2$; (SEQ ID NO:268)
Ac-c(Cys-Glu-His-D-2-Nal-Arg-Trp-Ala-Cys)-(Pro)$_2$-Lys-Asp-NH$_2$; (SEQ ID NO:269)
Ac-c(Cys-Glu-His-D-2-Nal-Arg-2-Nal-Ala-Cys)-(Pro)$_2$-Lys-Asp-NH$_2$; (SEQ ID NO:269)
Ac-c(Cys-Glu-His-D-2-Nal-Arg-1-Nal-Ala-Cys)-(Pro)$_2$-Lys-Asp-NH$_2$; (SEQ ID NO:269)
Ac-c(Cys-Glu-His-D-2-Nal-Arg-Bal-Ala-Cys)-(Pro)$_2$-Lys-Asp-NH$_2$; (SEQ ID NO:2690)
Ac-c(Cys-Glu-His-D-2-Nal-Arg-2-Nal-β-Ala-Cys)-(Pro)$_2$-Lys-Asp-NH$_2$; (SEQ ID NO:270) or
Ac-c(Cys-Glu-His-D-2-Nal-Arg-2-Nal-Aib-Cys)-(Pro)$_2$-Lys-Asp-NH$_2$; (SEQ ID NO:270)

or pharmaceutically acceptable salts thereof.

Further described is a method to treat insulin resistance in a mammalian subject, with or without weight loss, by the administration of a therapeutically effective amount of a melanocortin receptor compound modified with a hydantoin

moiety as described herein, and pharmaceutically acceptable salts, hydrates, solvates or prodrugs thereof.

Described is a method to treat insulin resistance in a mammalian subject, with or without weight loss, by the administration of a therapeutically effective amount of a melanocortin receptor ligand described herein, pharmaceutically-acceptable salts, hydrates, solvates and/or prodrugs thereof, wherein the ligand is a compound as follows:

c[Hydantoin(C(O)-(Cys-D-Ala))-His-D-Phe-Arg-Trp-Cys]-$NH_2$; (SEQ ID NO: 271)
c[Hydantoin(C(O)-(hCys-D-Ala))-His-D-Phe-Arg-Trp-Cys]-$NH_2$;(SEQ ID NO:271)
c[Hydantoin(C(O)-(Cys-D-Ala))-His-D-2-Nal-Arg-Trp-Cys]-$NH_2$; (SEQ ID NO:272) or
c[Hydantoin(C(O)-(hCys-D-Ala))-His-D-2-Nal-Arg-Trp-Cys]-$NH_2$; (SEQ ID NO:272)
c[Hydantoin(C(O)-(Asp-D-Ala))-His-D-Phe-Arg-Trp-Lys]-$NH_2$; (SEQ ID NO:273)
c[Hydantoin(C(O)-(Asp-D-Ala))-His-D-Phe-Arg-Trp-Orn]-$NH_2$; (SEQ ID NO:273)
c[Hydantoin(C(O)-(Asp-D-Ala))-His-D-Phe-Arg-Trp-Dab]-$NH_2$; (SEQ ID NO:273) or
c[Hydantoin(C(O)-(Asp-D-Ala))-His-D-Phe-Arg-Trp-Dap]-$NH_2$; (SEQ ID NO:273)
c[Hydantoin(C(O)-(Asp-His))-D-2-Nal-Arg-Trp-Lys]-$NH_2$; (SEQ ID NO:275)
c[Hydantoin(C(O)-(Asp-His))-D-Phe-Arg-Trp-Lys]-$NH_2$ (SEQ ID NO: 274)
c[Hydantoin(C(O)-(Asp-A3c))-D-Phe-Arg-Trp-Lys]-$NH_2$; (SEQ ID NO:274)
c[Hydantoin(C(O)-(Asp-A5c))-D-Phe-Arg-Trp-Lys]-$NH_2$; (SEQ ID NO:274)
c[Hydantoin(C(O)-(Asp-A6c))-D-Phe-Arg-Trp-Lys]-$NH_2$; (SEQ ID NO:274)
c[Hydantoin(C(O)-(Asp-A3c))-D-2-Nal-Arg-Trp-Lys]-$NH_2$; (SEQ ID NO:275)
c[Hydantoin(C(O)-(Asp-A5c))-D-2-Nal-Arg-Trp-Lys]-$NH_2$; (SEQ ID NO:275)
c[Hydantoin(C(O)-(Asp-A6c))-D-2-Nal-Arg-Trp-Lys]-$NH_2$; (SEQ ID NO:275)
c[Hydantoin(C(O)-(Asp-Aic))-D-Phe-Arg-Trp-Lys]-$NH_2$; (SEQ ID NO:274)
c[Hydantoin(C(O)-(Asp-Apc))-D-Phe-Arg-Trp-Lys]-$NH_2$; (SEQ ID NO:274)
c[Hydantoin(C(O)-(Asp-Aic))-D-2-Nal-Arg-Trp-Lys]-$NH_2$; (SEQ ID NO:275)
c[Hydantoin(C(O)-(Asp-Apc))-D-2-Nal-Arg-Trp-Lys]-$NH_2$; (SEQ ID NO:275)
c[Hydantoin-(C(O)-(Asp-Aic))-D-2-Nal-Arg-Trp-Lys]-$NH_2$(SEQ ID NO:275)
c[Hydantoin-(C(O)-(Asp-Apc))-D-2-Nal-Arg-Trp-Lys]-$NH_2$ (SEQ ID NO:275)
c[Hydantoin(C(O)-(Glu-D-Ala))-His-D-Phe-Arg-Trp-Orn]-$NH_2$; (SEQ ID NO:276)
c[Hydantoin(C(O)-(Glu-D-Ala))-His-D-Phe-Arg-Trp-Dab]-$NH_2$; (SEQ ID NO:276) or
c[Hydantoin(C(O)-(Glu-D-Ala))-His-D-Phe-Arg-Trp-Dap]-$NH_2$, (SEQ ID NO:276)
c[Hydantoin(C(O)-(Glu-His))-D-Phe-Arg-Trp-Dap]-$NH_2$ (SEQ ID NO:277)

Also described is a method to treat insulin resistance in a mammalian subject, with or without weight loss, by the administration of a therapeutically effective amount of a melanocortin receptor compound described herein, pharmaceutically-acceptable salts, hydrates, solvates and/or prodrugs thereof, wherein the compounds useful to treat insulin resistance in a mammalian subject, with or without weight loss, include:

Hydantoin(C(O)-(Arg-Gly))-c(Cys-Glu-His-D-Phe-Arg-Trp-Cys)-$NH_2$; (SEQ ID NO:278)
Hydantoin(C(O)-(Nle-Gly))-c(Cys-Glu-His-D-Phe-Arg-Trp-Cys)-$NH_2$; (SEQ ID NO:278)
Hydantoin(C(O)-(Gly-Gly))-c(Cys-Glu-His-D-Phe-Arg-Trp-Cys)-$NH_2$; (SEQ ID NO:278)
Hydantoin(C(O)-(Nle-Gly))-c(Cys-D-Ala-His-D-Phe-Arg-Trp-Cys)-$NH_2$; (SEQ ID NO:279)
Hydantoin(C(O)-(Gly-Gly))-c(Cys-D-Ala-His-D-Phe-Arg-Trp-Cys)-$NH_2$; (SEQ ID NO:279)
Hydantoin(C(O)-(Nle-Gly))-c(Cys-D-Ala-His-D-Phe-Arg-Trp-Pen)-$NH_2$; (SEQ ID NO:280)
Hydantoin(C(O)-(Gly-Gly))-c(Cys-D-Ala-His-D-Phe-Arg-Trp-Pen)-$NH_2$; (SEQ ID NO:280)
Hydantoin(C(O)-(Ala-Gly))-c(Cys-D-Ala-His-D-Phe-Arg-Trp-Cys)-$NH_2$; (SEQ ID NO:279)
Hydantoin(C(O)-(D-Ala-Gly))-c(Cys-D-Ala-His-D-Phe-Arg-Trp-Cys)-$NH_2$;(SEQ ID NO:279)
Hydantoin(C(O)-(Aib-Gly))-c(Cys-D-Ala-His-D-Phe-Arg-Trp-Cys)-$NH_2$; (SEQ ID NO:279)
Hydantoin(C(O)-(Val-Gly))-c(Cys-D-Ala-His-D-Phe-Arg-Trp-Cys)-$NH_2$;(SEQ ID NO:279)
Hydantoin(C(O)-(Ile-Gly))-c(Cys-D-Ala-His-D-Phe-Arg-Trp-Cys)-$NH_2$; (SEQ ID NO:279)
Hydantoin(C(O)-(Leu-Gly))-c(Cys-D-Ala-His-D-Phe-Arg-Trp-Cys)-$NH_2$; (SEQ ID NO:279)
Hydantoin(C(O)-(Gly-Gly))-c(Cys-Glu-His-D-2-Nal-Arg-Trp-Cys)-$NH_2$; (SEQ ID NO:281)
Hydantoin(C(O)-(Nle-Gly))-c(Cys-Glu-His-D-2-Nal-Arg-Trp-Cys)-$NH_2$; (SEQ ID NO:281)
Hydantoin(C(O)-(D-Arg-Gly))-c(Cys-Glu-His-D-Phe-Arg-Trp-Cys)-$NH_2$; (SEQ ID NO:278)
Hydantoin(C(O)-(Arg-Gly))-c(Cys-Glu-His-D-Phe-Arg-Trp-Cys)-$NH_2$; (SEQ ID NO:278)
Hydantoin(C(O)-(D-Arg-Gly))-c(Cys-D-Ala-His-D-Phe-Arg-Trp-Cys)-$NH_2$; (SEQ ID NO:279)
Hydantoin(C(O)-(Arg-Gly))-c(Cys-D-Ala-His-D-Phe-Arg-Trp-Cys)-$NH_2$; (SEQ ID NO:279)
Hydantoin(C(O)-(D-Arg-Gly))-c(Cys-D-Ala-His-D-2-Nal-Arg-Trp-Cys)-$NH_2$; (SEQ ID NO:282)
Hydantoin(C(O)-(Arg-Gly))-c(Cys-D-Ala-His-D-2-Nal-Arg-Trp-Cys)-$NH_2$; (SEQ ID NO:282)

Hydantoin(C(O)-(Nle-Gly))-c(Cys-Glu-His-D-Phe-Arg-Trp-Cys)-$NH_2$; (SEQ ID NO:278)
Hydantoin(C(O)-(Gly-Gly))-c(Cys-Glu-His-D-Phe-Arg-Trp-Cys)-$NH_2$; (SEQ ID NO:278)
Hydantoin(C(O)-(Nle-Gly))-c(Cys-D-Ala-His-D-Phe-Arg-Trp-Cys)-$NH_2$;(SEQ ID NO:279)
Hydantoin(C(O)-(Gly-Gly))-c(Cys-D-Ala-His-D-Phe-Arg-Trp-Cys)-$NH_2$; (SEQ ID NO:279)
Hydantoin(C(O)-(Nle-Gly))-c(Cys-D-Ala-His-D-Phe-Arg-Trp-Pen)-$NH_2$;(SEQ ID NO:280)
Hydantoin(C(O)-(Gly-Gly))-c(Cys-D-Ala-His-D-Phe-Arg-Trp-Pen)-$NH_2$;(SEQ ID NO:280)
Hydantoin(C(O)-(Ala-Gly))-c(Cys-D-Ala-His-D-Phe-Arg-Trp-Cys)-$NH_2$; (SEQ ID NO:279)
Hydantoin(C(O)-(D-Ala-Gly))-c(Cys-D-Ala-His-D-Phe-Arg-Trp-Cys)-$NH_2$; (SEQ ID NO:279)
Hydantoin(C(O)-(Aib-Gly))-c(Cys-D-Ala-His-D-Phe-Arg-Trp-Cys)-$NH_2$; (SEQ ID NO:279)
Hydantoin(C(O)-(Val-Gly))-c(Cys-D-Ala-His-D-Phe-Arg-Trp-Cys)-$NH_2$; (SEQ ID NO:279)
Hydantoin(C(O)-(Ile-Gly))-c(Cys-D-Ala-His-D-Phe-Arg-Trp-Cys)-$NH_2$; (SEQ ID NO:279)
Hydantoin(C(O)-(Leu-Gly))-c(Cys-D-Ala-His-D-Phe-Arg-Trp-Cys)-$NH_2$; (SEQ ID NO:279)
Hydantoin(C(O)-(D-Arg-Gly))-c(Cys-Glu-His-D-Phe-Arg-Trp-Cys)-$NH_2$; (SEQ ID NO:278)
Hydantoin(C(O)-(Arg-Gly))-c(Cys-Glu-His-D-Phe-Arg-Trp-Cys)-$NH_2$; (SEQ ID NO:278)
Hydantoin(C(O)-(Arg-Gly))-c(Cys-D-Ala-His-D-Phe-Arg-Trp-Cys)-$NH_2$; (SEQ ID NO:279) or
Hydantoin(C(O)-(D-Arg-Gly))-c(Cys-D-Ala-His-D-Phe-Arg-Trp-Cys)-$NH_2$; (SEQ ID NO:279)
Hydantoin(C(O)-(Ala-Nle))-c(Cys-Glu-His-D-Phe-Arg-Trp-Cys)-$NH_2$;(SEQ ID NO:283)
Hydantoin(C(O)-(Val-Nle))-c(Cys-Glu-His-D-Phe-Arg-Trp-Cys)-$NH_2$; (SEQ ID NO:283)
Hydantoin(C(O)-(Gly-Nle))-c(Cys-Glu-His-D-Phe-Arg-Trp-Cys)-$NH_2$;(SEQ ID NO:283)
Hydantoin(C(O)-(A6c-Nle))-c(Cys-D-Ala-His-D-Phe-Arg-Trp-Cys)-$NH_2$; (SEQ ID NO:284)
Hydantoin(C(O))-(Gly-Nle))-c(Cys-D-Ala-His-D-Phe-Arg-Trp-Cys)-$NH_2$;(SEQ ID NO:284)
Hydantoin(C(O)-(Ala-Nle))-c(Cys-D-Ala-His-D-Phe-Arg-Trp-Cys)-$NH_2$;(SEQ ID NO:284)
Hydantoin(C(O)-(D-Ala-Nle))-c(Cys-D-Ala-His-D-Phe-Arg-Trp-Cys)-$NH_2$; (SEQ ID NO:284)
Hydantoin(C(O)-(Val-Nle))-c(Cys-D-Ala-His-D-Phe-Arg-Trp-Cys)-$NH_2$; (SEQ ID NO:284)
Hydantoin(C(O)-(Leu-Nle))-c(Cys-D-Ala-His-D-Phe-Arg-Trp-Cys)-$NH_2$; (SEQ ID NO:284)
Hydantoin(C(O)-(Cha-Nle))-c(Cys-D-Ala-His-D-Phe-Arg-Trp-Cys)-$NH_2$; (SEQ ID NO:284)or
Hydantoin(C(O)-(Aib-Nle))-c(Cys-D-Ala-His-D-Phe-Arg-Trp-Cys)-$NH_2$; (SEQ ID NO:284)
Hydantoin(C(O)-(Gly-Arg))-c(Cys-Glu-His-D-Phe-Arg-Trp-Cys)-$NH_2$; (SEQ ID NO:285)
Hydantoin(C(O)-(Gly-Arg))-c(Cys-Glu-His-D-2-Nal-Arg-Trp-Cys)-$NH_2$; (SEQ ID NO:285)
Hydantoin(C(O)-(Gly-Arg))-c(Cys-D-Ala-His-D-Phe-Arg-Trp-Cys)-$NH_2$; (SEQ ID NO:286)
Hydantoin(C(O)-(Gly-Arg))-c(Cys-D-Ala-His-D-2-Nal-Arg-Trp-Cys)-$NH_2$; (SEQ ID NO:286)
Hydantoin(C(O)-(Gly-D-Arg))-c(Cys-Glu-His-D-Phe-Arg-Trp-Cys)-$NH_2$; (SEQ ID NO:287)
Hydantoin(C(O)-(Gly-D-Arg))-c(Cys-Glu-His-D-Phe-Arg-Trp-Cys)-$NH_2$;(SEQ ID NO:287)
Hydantoin(C(O)-(Gly-D-Arg))-c(Cys-D-Ala-His-D-Phe-Arg-Trp-Cys)-$NH_2$; (SEQ ID NO:288) and
Hydantoin(C(O)-(Gly-D-Arg))-c(Cys-D-Ala-His-D-2-Nal-Arg-Trp-Cys)-$NH_2$; (SEQ ID NO:288)
Hydantoin(C(O)-(Nle-Ala))-c(Cys-Glu-His-D-Phe-Arg-Trp-Cys)-$NH_2$ (SEQ ID NO:289);

or pharmaceutically acceptable salts thereof.

Also described is a method to treat insulin resistance in a mammalian subject, with or without weight loss, by the administration of a therapeutically effective amount of a melanocortin receptor ligand belonging to a class of cyclic peptide analogs that are ligands for the melanocortin receptors as described herein, wherein the compounds useful in the treatment of insulin resistance in a mammalian subject, include the following compounds:

c[Hydantoin(C(O)-(Nle-Cys))-D-Ala-His-D-Phe-Arg-Trp-Cys]-$NH_2$; (SEQ ID NO:290)
c[Hydantoin(C(O)-(Ala-Cys))-D-Ala-His-D-Phe-Arg-Trp-Cys]-$NH_2$; (SEQ ID NO:290)
c[Hydantoin(C(O)-(D-Ala-Cys))-D-Ala-His-D-Phe-Arg-Trp-Cys]-$NH_2$; (SEQ ID NO:290)
c[Hydantoin(C(O)-(Aib-Cys))-D-Ala-His-D-Phe-Arg-Trp-Cys]-$NH_2$; (SEQ ID NO:290)
c[Hydantoin(C(O)-(Val-Cys))-D-Ala-His-D-Phe-Arg-Trp-Cys]-$NH_2$; (SEQ ID NO:290)
c[Hydantoin(C(O)-(Abu-Cys))-D-Ala-His-D-Phe-Arg-Trp-Cys]-$NH_2$; (SEQ ID NO:290)
c[Hydantoin(C(O)-(Leu-Cys))-D-Ala-His-D-Phe-Arg-Trp-Cys]-$NH_2$; (SEQ ID NO:290)
c[Hydantoin(C(O)-(Ile-Cys))-D-Ala-His-D-Phe-Arg-Trp-Cys]-$NH_2$;(SEQ ID NO:290)
c[Hydantoin(C(O)-(Cha-Cys))-D-Ala-His-D-Phe-Arg-Trp-Cys]-$NH_2$; (SEQ ID NO:290)
c[Hydantoin(C(O)-(A6c-Cys))-D-Ala-His-D-Phe-Arg-Trp-Cys]-$NH_2$; (SEQ ID NO:290)
c[Hydantoin(C(O)-(Phe-Cys))-D-Ala-His-D-Phe-Arg-Trp-Cys]-$NH_2$;(SEQ ID NO:290)
c[Hydantoin(C(O)-(Gly-Cys))-D-Ala-His-D-Phe-Arg-Trp-Cys]-$NH_2$; (SEQ ID NO:290) or
c[Hydantoin(C(O)-(Gly-Cys))-Glu-His-D-Phe-Arg-Trp-Cys]-$NH_2$; (SEQ ID NO:291) or
c[Hydantoin(C(O)-(Gly-Cys))-Glu-His-D-Phe-Arg-Trp-Cys]-$NH_2$ (SEQ ID NO:291);

or pharmaceutically acceptable salts thereof.

Also described is a method to treat insulin resistance in a mammalian subject, with or without weight loss, by the administration of a therapeutically effective amount of a melanocortin receptor ligand described herein, wherein the ligand is a compound of the formula:

Ac-Tyr-Arg-c(Cys-D-Ala-His-D-Phe-Arg-Trp-Cys)-$NH_2$; (SEQ ID NO:292)
Ac-2-Nal-Arg-c(Cys-D-Ala-His-D-Phe-Arg-Trp-Cys)-$NH_2$; (SEQ ID NO:292)
Ac-1-Nal-Arg-c(Cys-D-Ala-His-DPhe-Arg-Trp-Cys)-$NH_2$; (SEQ ID NO:292)
Ac-Phe-Arg-c(Cys-D-Ala-His-D-Phe-Arg-Trp-Cys)-$NH_2$; (SEQ ID NO:292)
Ac-Trp-Arg-c(Cys-D-Ala-His-D-Phe-Arg-Trp-Cys)-$NH_2$; (SEQ ID NO:292)
Ac-Pff-Arg-c(Cys-D-Ala-His-D-Phe-Arg-Trp-Cys)-$NH_2$; (SEQ ID NO:292)
H-His-Arg-c(Cys-D-Ala-His-D-Phe-Arg-Trp-Cys)-$NH_2$; (SEQ ID NO:293) or
Ac-His-Arg-c(Cys-D-Ala-His-D-Phe-Arg-Trp-Cys)-$NH_2$; (SEQ ID NO:292)

or pharmaceutically acceptable salts thereof.

In one instance, the compound or compounds as defined hereinabove, which are useful to treat insulin resistance in a mammalian subject, with or without weight loss, or a pharmaceutically acceptable salt thereof, are provided to said subject in need in a composition with a pharmaceutically acceptable carrier or diluent.

In one instance, the method of treating insulin resistance in a subject in need thereof, comprises peripheral administration of an effective amount of a melanocortin receptor 4 agonist to treat the insulin resistance in the subject in need thereof.

According to the invention, the melanocortin receptor 4 agonist useful to treat insulin resistance in the subject in need thereof, is Ac-Arg-c(Cys-D-Ala-His-D-Phe-Arg-Trp-Cys)-$NH_2$ (SEQ ID NO:50) or a pharmaceutically acceptable salt thereof. In another instance described herein, the melanocortin receptor 4 agonist useful to treat insulin resistance in the subject in need thereof, is Hydantoin(C(O)-(Arg-Gly))-c(Cys-Glu-His-D-Phe-Arg-Trp-Cys)-$NH_2$ (SEQ ID NO:278) or a pharmaceutically acceptable salt thereof.

Administration of a compound or compostion comprising a compound or pharmaceutical salt of a compound described herein useful to treat insulin resistance, may be continuous, hourly, four times daily, three time daily, twice daily, once daily, once every other day, twice weekly, once weekly, once every two weeks, once a month, or once every two months, or longer.

The subject in need of treatment may be obese, overweight, of normal weight or lean. The obese, overweight, normal weight or lean subject may suffer from type II diabetes. Administration of a compound or compostion comprising a compound or pharmaceutical salt of a compound described herein useful to treat insulin resistance, may be peripheral administration. Examples of peripheral administration include oral, subcutaneous, intraperitoneal, intramuscular, intravenous, rectal, transdermal or intranasal forms of administration.

## BRIEF DESCRIPTION OF THE DRAWINGS:

Figure 1: Food consumed in fasted rats 6 hours after administration of 100 nmole/Kg of selected compounds.
Figure 2. Effect of subcutaneous administration of 75, 300 or 600 nmole/kg/day of Compound A upon (A) body weight, (B) cumulative food intake, (C) insulin levels and (D) glucose levels in rats.
Figure 3. Effect of subcutaneous administration of 75, 300 or 600 nmole/kg/day of Compound B upon (A) body weight, (B) cumulative food intake, (C) insulin levels and (D) glucose levels in rats.
Figure 4. Effect of subcutaneous administration of 200, 600 or 1800 nmole/kg/day of Compound A upon blood glucose levels in mice.
Figure 5. Effect of intraperitoneal administration of 6.4 μmole/kg of Compound A upon blood glucose levels in obese mice.

## DETAILED DESCRIPTION OF THE INVENTION

Recent studies have reported that staggering numbers of people world wide are overweight and suffering a wide variety of serious and expensive health problems. According the World Health Organization (as reported in Kouris-Blazos et al., Asia Pac. J. Clin. Nutr., 2007, 16:329-338), an estimated 1 billion people throughout the world are overweight and an estimated 300 million of these are obese. An estimated 22 million children under the age of 5 are severely overweight and in the European Union alone, the number of children who are overweight is expected to rise by 1.3 million children per year (Kosti et al., 2006, Cent. Eur. J. Public Health, 14:151-159). Obesity, as defined by the Statistical

Bulletin provided by the Metropolitan Life Insurance Co., (1959, 40:1), is a condition in which a person is approximately 20-25% over normal body weight. Alternatively, an individual is considered obese if the person has a body mass index of greater than 25% over normal or greater than 30% over normal with risk factors (see Bray et al., Diabetes/Metabolism Review, 1988, 4:653-679 or Flynn et al., Proc. Nutritional Society, 1991, 50:413). One of the main causes for obesity is the consumption of a high caloric diet (Riccardi et al., Clin. Nutr., 2004, 23:447-456).

Diabetes is a chronic, debilitating disease afflicting many overweight and obese people. It is estimated that 20.8 million people in the United States alone have diabetes and more than 6 million more additional cases remain undiagnosed (Cornell, Manag. Care Pharm., 2007, 13:S11-5). Type 2 diabetes (also referred to herein as type II diabetes) is a chronic disease characterized by insulin resistance, impaired insulin secretion and hyperglycemia. Worldwide, type II diabetes is believed to affect approximately 171 million people, imparting numerous microvascular and macrovascular complications resulting in morbidity and mortality (Mudaliar, Indian J. Med. Res., 2007, 125:275-296). Mudaliar further notes that despite the availability of anti-hyperglycaemic agents available, control of glucose remains elusive in many patients.

Insulin resistance, also referred to as reduced insulin sensitivity, is a condition in which the amount of insulin needed to clear glucose from the blood of a subject is increased as compared to the amount of insulin needed to clear the same amount of glucose from the blood of a normal, non-insulin sensitive subject. Insulin resistance is regarded as the main link between obesity and type II diabetes (see Obici et al., J. Clin. Inv., 2001, 108:1079-1085 and references therein). It is known that rats fed a high fat diet show an increase in body weight (diet-induced obesity or DIO) and a decrease in insulin sensitivity. Such DIO rats provide an animal model in which to study the mechanisms of insulin resistance due to obesity (see for example Banno et al., FEBS letters, 2007, 581:1131-1136). The size and weight of adipose tissues are increased in DIO rats and it is thought that the accompanying hypertrophy of adipocytes leads to changes in the release of adipocytokines such as leptin and adiponectin which are known to regulate insulin sensitivity; it is thought that morphological changes in adipose tissue as well as changes in plasma levels of adipocytokines are among the causes of insulin resistance in DIO rats (summarized in Banno, et al., FEBS letters, 2007, 581:1131-1136 and references therein).

Melanocortins are proposed to play a large role in energy metabolism and homeostasis. Melanocortins cleaved from the POMC precursor exert their effects by binding to members of the melanocortin receptor family located in the brain. The major effect of melanocortin in the brain is to reduce food intake however, it has also been shown that melanocortin agonists or antagonists injected directly into the cerebral ventricle affect insulin actions in the periphery while food was withdrawn or while food intake was kept constant (see Schwartz et al., Nature, 2000, 404:661-671; Seeley et al., Ann. Rev. Nutr., 2004, 24:133-149; Cone et al., Recent Prog. Horm. Res., 1996, 51:287-317; Heijbor et al., Diabetologia, 2005, 48:1621-1626; Obici et al., J. Clin. Inv., 2001, 108:1079-1085). Taken together, these data suggest that central administration of melanocortins affects insulin sensitivity and may do so independently of energy balance. Banno et al., (FEBS letters, 2007, 581:1131-1136) demonstrated that intracerebral injections of a melanocortin agonist to DIO rats ameliorated insulin sensitivity in the periphery, decreased the size of and increased the number of adipocytes in white adipose tissue and decreased triglycerides content in the liver.

Considering the large numbers of overweight and/or insulin resistant subjects in need of treatment, intracerebral administration is an unlikely means to disperse medicaments to patients. There is a need in the art, therefore, to identify melanocortin agonists and antagonists suitable for peripheral administration to affect parameters of insulin action and energy metabolism such as insulin sensitivity, cellular characteristics of white adipose tissue, triglyceride levels and the like.

### *Nomenclature and Abbreviations*

As used herein, an "obese subject" or mammal is characterized as having a body weight approximately 20% or greater than the normal body weight for said subject. Normal body weight may be determined by a comparison of the weight of the subject at a prior point in time, such as when insulin metabolism was normal, or by a comparison of the weight of the subject as compared to averages of other subjects of a similar age and/or condition.

As used herein, an "overweight subject" or mammal is characterized as having a body weight approximately 5% greater to approximately 20% greater than the normal body weight for said subject. Normal body weight may be determined by a comparison of the weight of the subject at a prior point in time, such as when insulin metabolism was normal, or by a comparison of the weight of the subject as compared to averages of other subjects of a similar age and/or condition.

As used herein, a "normal subject" or mammal is characterized as having a body weight up to approximately 5% greater than to approximately 5% less than the normal body weight for said subject. Normal body weight may be determined by a comparison of the weight of the subject at a prior point in time, such as when insulin metabolism was normal, or by a comparison of the weight of the subject as compared to averages of other subjects of a similar age and/or condition.

As used herein, a "lean subject" or mammal is characterized as having a body weight approximately 5% to 30% or even to 50% less than the normal body weight for said subject. Normal body weight may be determined by a comparison of the weight of the subject at a prior point in time, such as when insulin metabolism was normal, or by a comparison of the weight of the subject as compared to averages of other subjects of a similar age and/or condition.

As used herein, the terms "treat", "treating" and "treatment" include palliative, curative and prophylactic treatment.

As used herein, "measurable" means the biologic effect is both reproducible and significantly different from the baseline variability of the assay.

As used herein, peripherial administration includes all forms of administration of a compound or a composition comprising a compound of the instant invention which excludes intracranial administration. Examples of peripheral administration include, but are not limited to, oral, parenteral *(e.g.,* intramuscular, intraperitoneal, intravenous or subcutaneous injection, implant and the like), nasal, vaginal, rectal, sublingual or topical routes of administration, including transdermal patch applications and the like.

A "subject", as used herein and throughout this application, refers to a mammalian or non-mammalian animal including, for example and without limitation, a human, a rat, a mouse or farm animal. Reference to a subject does not necessarily indicate the presence of a disease or disorder. The term "subject" includes, for example, a mammalian or non-mammalian animal being dosed with a melanocortin analog as part of an experiment, a mammalian or non-mammalian animal being treated to help alleviate a disease or disorder, and a mammalian or non-mammalian animal being treated prophylactically to retard or prevent the onset of a disease or disorder. Subject mammals may be human subjects of any age, such as an infant, a child, an adult or an elderly adult.

A "therapeutically acceptable amount" of a compound or composition of the invention, regardless of the formulation or route of administration, is that amount which elicits a desired biological response in a subject. The biological effect of the therapeutic amount may occur at and be measured at many levels in an organism. For example, the biological effect of the therapeutic amount may occur at and be measured at the cellular level by measuring the response at a receptor which binds melanocortin and/or a melanocortin analog, or the biological effect of the therapeutic amount may occur at and be measured at the system level, such as effecting an increase/decrease in the levels of insulin. The biological effect of the therapeutic amount may occur at and be measured at the organism level, such as the alleviation of a symptom(s) or progression of a disease or condition in a subject. A therapeutically acceptable amount of a compound or composition of the invention, regardless of the formulation or route of administration, may result in one or more biological responses in a subject. In the event that the compound or composition of the invention is subject to testing in an *in nitro* system, a therapeutically acceptable amount of the compound or composition may be viewed as that amount which gives a measurable response in the *in vitro* system of choice.

The nomenclature used to define the peptides is that typically used in the art wherein the amino group at the N-terminus appears to the left and the carboxyl group at the C-terminus appears to the right. Where the amino acid has D and L isomeric forms, it is the L form of the amino acid that is represented unless otherwise explicitly indicated.

The compounds herein useful for the treatment of insulin resistance, with or without weight loss, may possess one or more chiral centers and so exist in a number of stereoisomeric forms. All stereoisomers and mixtures thereof are included in the scope of the present disclosure. Racemic compounds may either be separated using preparative HPLC and a column with a chiral stationary phase or resolved to yield individual enantiomers utilizing methods known to those skilled in the art. In addition, chiral intermediate compounds may be resolved and used to prepare chiral compounds.

The compounds herein useful for the treatment of insulin resistance, with or without weight loss, may exist in one or more tautomeric forms. All tautomers and mixtures thereof are included in the scope of the present disclosure. For example, 2-hydroxypyridinyl would also cover its tautomeric form, α-pyridonyl.

Unless defined otherwise, all technical and scientific terms used herein have the same meaning as commonly understood by one of ordinary skill in the art to which this invention belongs. Also, all publications, patent applications, patents and other references mentioned herein are incorporated by reference in their entirety.

| Symbol | Meaning |
|---|---|
| Abu | α-aminobutyric acid |
| Ac | acyl group |
| Acc | 1-amino-1-cyclo($C_3$-$C_9$)alkyl carboxylic acid |
| A3c | 1-amino-1-cyclopropanecarboxylic acid |
| A4c | 1-amino-1-cyclobutanecarboxylic acid |
| A5c | 1-amino-1-cyclopentanecarboxylic acid |
| A6c | 1-amino-1-cyclohexanecarboxylic acid |
| Aha | 7-aminoheptanoic acid |

(continued)

| Symbol | Meaning |
|---|---|
| Ahx | 6-aminohexanoic acid |
| Aib | α-aminoisobutyric acid |
| Aic | 2-aminoindan-2-carboxylic acid |
| Ala or A | alanine |
| β-Ala | β-alanine |
| Apc | denotes the structure: 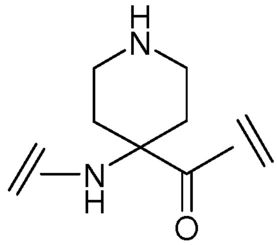 |
| Apn | 5-aminopentanoic acid $(HN-(CH2)_4-C(O)$ |
| Arg or R | arginine |
| hArg | homoarginine |
| Asn or N | asparagine |
| Asp or D | aspartic acid |
| Bal | 3-benzothienylalanine |
| Bip | 4,4'-biphenylalanine, represented by the structure 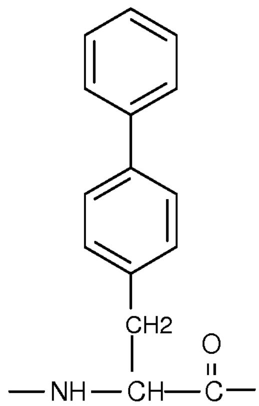 |
| Bpa | 4-benzoylphenylalanine |
| 4-Br-Phe | 4-bromo-phenylalanine |
| Cha | ß-cyclohexylalanine |
| hCha | homo-cyclohexylalanine |
| Chg | cyclohexylglycine |
| Cys or C | cysteine |
| hCys | homocysteine |
| Dab | 2,4-diaminobutyric acid |
| Dap | 2,3-diaminopropionic acid |
| Dip | β,β-diphenylalanine |
| Doc | 8-amino-3,6-dioxaoctanoic acid with the structure of: 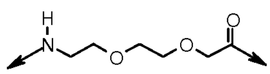 |
| 2-Fua | β-(2-furyl)-alanine |
| Gaba | 4-aminobutyric acid |
| Gln or Q | glutamine |
| Glu or E | glutamic acid |

(continued)

| Symbol | Meaning |
|---|---|
| Gly or G | glycine |
| His or H | histidine |
| 3-Hyp | trans-3-hydroxy-L-proline, *i.e.*, (2S, 3S)-3-hydroxypyrrolidine-2-carboxylic acid |
| 4-Hyp | 4-hydroxyproline, *i.e.*, (2S, 4R)-4-hydroxypyrrolidine-2-carboxylic acid |
| Ile or I | isoleucine |
| Leu or L | leucine |
| hLeu | homoleucine |
| Lys or K | lysine |
| Met or M | methionine |
| β-hMet | β-homomethionine |
| 1-Nal | β-(1-naphthyl)alanine: |
| 2-Nal | β-(2-naphthyl)alanine |
| Nip | nipecotic acid |
| Nle | norleucine |
| Oic | octahydroindole-2-carboxylic acid |
| Orn | ornithine |
| 2-Pal | β-(2-pyridiyl)alanine |
| 3-Pal | β-(3-pyridiyl)alanine |
| 4-Pal | β-(4-pyridiyl)alanine |
| Pen | penicillamine |
| Pff | (S)-pentafluorophenylalanine |
| Phe or F | phenylalanine |
| hPhe | homophenylalanine |
| Pro or P | proline |
| hPro | homoproline |
| Ser or S | serine |
| Tle | tert-Leucine |
| Taz | ß-(4-thiazolyl)alanine |
| 2-Thi | ß-(2-thienyl)alanine |
| 3-Thi | ß-(3-thienyl)alanine |
| Thr or T | threonine |
| Trp or W | tryptophan |
| Tyr or Y | tyrosine |
| D-(Et)Tyr | has a structure of 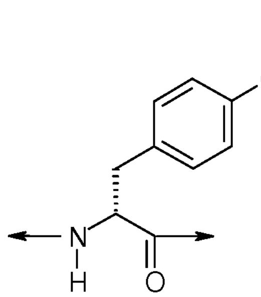 |
| Val or V | valine |

Certain other abbreviations used herein are defined as follows:

| | |
|---|---|
| Boc Bzl: | *tert*-butyloxycarbonyl |
| Bzl: | benzyl |
| DCM: | dichloromethane |
| DIC: | N, N-diisopropylcarbodiimide |

(continued)

| Certain other abbreviations used herein are defined as follows: | |
|---|---|
| DIEA: | diisopropylethyl amine |
| Dmab: | 4-{N-(1-(4,4-dimethyl-2,6-dioxocyclohexylidene)-3-methylbutyl)-amino} benzyl |
| DMAP: | 4-(dimethylamino)pyridine |
| DMF | dimethylformamide |
| DNP: | 2,4-dinitrophenyl |
| Fm: | fluorenylmethyl |
| Fmoc: | fluorenylmethyloxycarbonyl |
| For: | formyl |
| HBTU: | 2-(1H-benzotriazole-1-yl)-1,1,3,3-tetramethyluronium hexafluorophosphate |
| cHex | cyclohexyl |
| HOAT: | O-(7-azabenzotriazol-1-yl)-1,1,3,3-tetramethyluronium hexafluorophosphate |
| HOBt: | 1-hydroxy-benzotriazole |
| MBHA | 4-methylbenzhydrylamine |
| Mmt: | 4-methoxytrityl |
| NMP: | N-methylpyrrolidone |
| O-tBu | oxy-tert-butyl |
| Pbf: | 2,2,4,6,7-pentamethyldihydrobenzofuran-5-sulfonyl |
| PyBroP | bromo-tris-pyrrolidino-phosphonium hexafluorophosphate |
| tBu: | tert-butyl |
| TIS: | triisopropylsilane |
| TOS: | tosyl |
| Trt | trityl |
| TFA: | trifluoro acetic acid |
| TFFH: | tetramethylfluoroforamidinium hexafluorophosphate |
| Z: | benzyloxycarbonyl |

Unless otherwise indicated, with the exception of the N-terminal amino acid, all abbreviations (e.g. Ala) of amino acids in this disclosure stand for the structure of -NH-C(R)(R')-CO-, wherein R and R' each is, independently, hydrogen or the side chain of an amino acid (e.g., R = $CH_3$ and R' = H for Ala), or R and R' may be joined to form a ring system.

For the N-terminal amino acid, the abbreviation stands for the structure of:

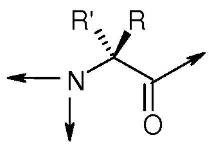

The designation "$NH_2$" in e.g., Ac-Nle-c(Cys-D-Ala-His-D-Phe-Arg-Trp-Cys)-$NH_2$ (SEQ ID NO:7), indicates that the C-terminus of the peptide is amidated. Ac-Nle-c(Cys-D-Ala-His-D-Phe-Arg-Trp-Cys) (SEQ ID NO:36), or alternatively Ac-Nle-c(Cys-D-Ala-His-D-Phe-Arg-Trp-Cys)- OH (SEQ ID NO:36), indicates that the C-terminus is the free acid.

"-c(Cys-Cys)-" or "-cyclo(Cys-Cys)-" denotes the structure:

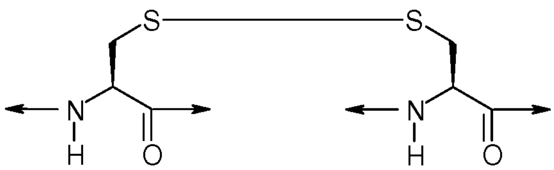

"-c(Cys-Pen)-" or "-cyclo(Cys-Pen)-" denotes the structure:

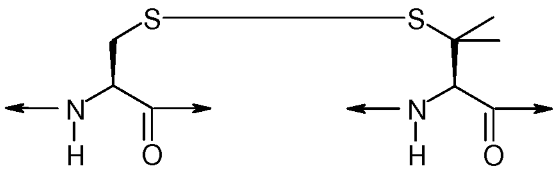

"-c(Asp-Lys)-" or "-cyclo(Asp-Lys)-" denotes the structure:

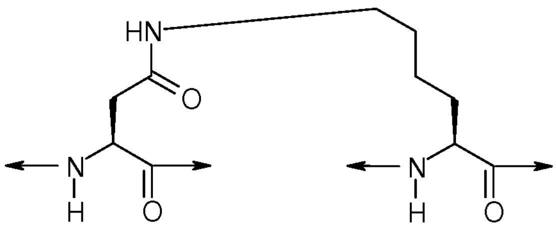

Applicants have devised the following shorthand used in naming the specific embodiments and/or species:

"HydantoinC(O)-($A^a$-$A^b$)" denotes the structure:

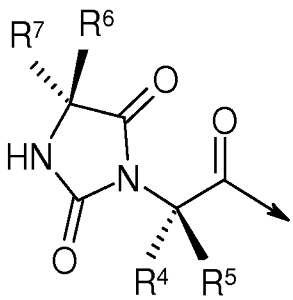

wherein amino acid "$A^a$" has the structure:

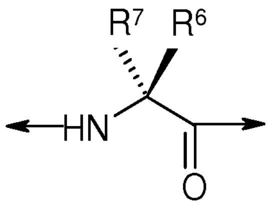

and

amino acid "$A^b$" the structure:

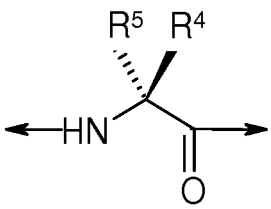

.

For example, a compound represented as "c[Hydantoin(C(O)-(Cys-$A^b$))-$A^1$-$A^2$-$A^3$-$A^4$-Cys]-" would have the following the structure:

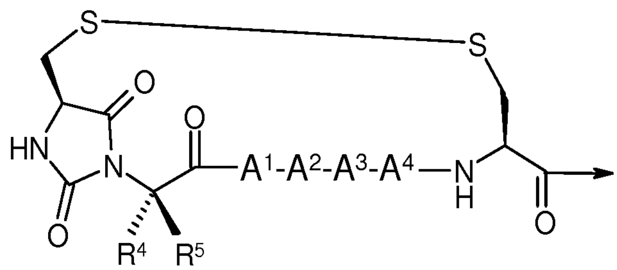

,

whereas a compound represented as "c[Hydantoin(C(O)-($A^b$-Cys))-$A^1$-$A^2$-$A^3$ -$A^4$-Cys]-" would have the structure:

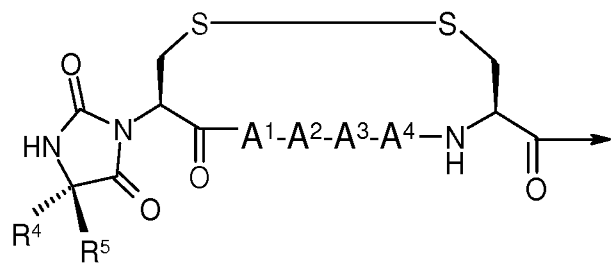

.

For further guidance, "c[Hydantoin(C(O)-(Asp-$A^b$))-$A^1$-$A^2$-$A^3$-$A^4$-Lys]-" represents the following compound:

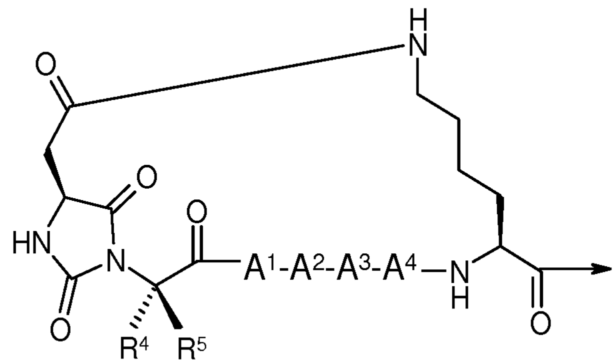

,

whereas "c[Hydantoin(C(O)-(Dap-$A^b$))-$A^1$-$A^2$-$A^3$-$A^4$-Asp]-" has the following formula:

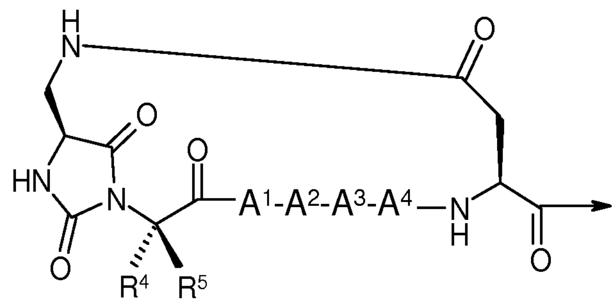

.

"Acyl" refers to R"-C(O)-, where R" is H, alkyl, substituted alkyl, heteroalkyl, substituted heteroalkyl, alkenyl, substituted alkenyl, aryl, alkylaryl, or substituted alklyaryl, and is indicated in the general formula of a particular embodiment as "Ac".

"Alkyl" refers to a hydrocarbon group containing one or more carbon atoms, where multiple carbon atoms if present are joined by single bonds. The alkyl hydrocarbon group may be straight-chain or contain one or more branches or cyclic groups.

"Hydroxyalkyl" refers to an alkyl group wherein one or more hydrogen atoms of the hydrocarbon group are substituted with one or more hydroxy radicals, such as hydroxymethyl, hydroxyethyl, hydroxypropyl, hydroxybutyl, hydroxypentyl, hydroxyhexyl and the like.

"Substituted alkyl" refers to an alkyl wherein one or more hydrogen atoms of the hydrocarbon group are replaced with one or more substituents selected from the group consisting of halogen, (i.e., fluorine, chlorine, bromine, and iodine), -OH, -CN, -SH, $-NH_2$, $-NHCH_3$, $-NO_2$, and $-C_{1-20}$ alkyl, wherein said $-C_{1-20}$ alkyl optionally may be substituted with one or more substituents selected, independently for each occurrence, from the group consisting of halogens, $-CF_3$, $-OCH_3$, $-OCF_3$, and $-(CH_2)_{0-20}$-COOH. In different embodiments 1, 2, 3 or 4 substituents are present. The presence of $-(CH_2)_{0-20}$-COOH results in the production of an alkyl acid. Non-limiting examples of alkyl acids containing, or consisting of, $-(CH_2)_{0-20}$-COOH include 2-norbornane acetic acid, tert-butyric acid, 3-cyclopentyl propionic acid, and the like.

The term "halo" encompasses fluoro, chloro, bromo and iodo.

"Heteroalkyl" refers to an alkyl wherein one of more of the carbon atoms in the hydrocarbon group is replaced with one or more of the following groups: amino, amido, -O-, -S- or carbonyl. In different embodiments 1 or 2 heteroatoms are present.

"Substituted heteroalkyl" refers to a heteroalkyl wherein one or more hydrogen atoms of the hydrocarbon group are replaced with one or more substituents selected from the group consisting of halogen, (i.e., fluorine, chlorine, bromine, and iodine), -OH, -CN, -SH, $-NH_2$, $-NHCH_3$, $-NO_2$, and $-C_{1-20}$ alkyl, wherein said $-C_{1-20}$ alkyl optionally may be substituted with one or more substituents selected, independently for each occurrence, from the group consisting of halogens, $-CF_3$, $-OCH_3$, $-OCF_3$, and $-(CH_2)_{0-20}$-COOH. In different embodiments 1, 2, 3 or 4 substituents are present.

"Alkenyl" refers to a hydrocarbon group made up of two or more carbons where one or more carbon-carbon double bonds are present. The alkenyl hydrocarbon group may be straight-chain or contain one or more branches or cyclic groups.

"Substituted alkenyl" refers to an alkenyl wherein one or more hydrogens are replaced with one or more substituents selected from the group consisting of halogen (i.e., fluorine, chlorine, bromine, and iodine), -OH, -CN, -SH, $-NH_2$, $-NHCH_3$, $-NO_2$, and $-C_{1-20}$ alkyl, wherein said $-C_{1-20}$ alkyl optionally may be substituted with one or more substituents selected, independently for each occurrence, from the group consisting of halogens, $-CF_3$, $-OCH_3$, $-OCF_3$, and $-(CH_2)_{0-20}$-COOH. In different embodiments 1, 2, 3 or 4 substituents are present.

"Aryl" refers to an optionally substituted aromatic group with at least one ring having a conjugated pi-electron system, containing up to three conjugated or fused ring systems. Aryl includes carbocyclic aryl, heterocyclic aryl and biaryl groups. Preferably, the aryl is a 5- or 6-membered ring. Preferred atoms for a heterocyclic aryl are one or more sulfur, oxygen, and/or nitrogen. Non-limiting examples of aryl include phenyl, 1-naphthyl, 2-naphthyl, indole, quinoline, 2-imidazole, 9-anthracene, and the like. Aryl substituents are selected from the group consisting of $-C_{1-20}$ alkyl, $-C_{1-20}$ alkoxy, halogen (i.e., fluorine, chlorine, bromine, and iodine), -OH, -CN, -SH, $-NH_2$, $-NO_2$, $-C_{1-20}$ alkyl substituted with halogens, $-CF_3$, $-OCF_3$, and $-(CH_2)_{0-20}$-COOH. In different embodiments the aryl contains 0, 1, 2, 3, or 4 substituents.

"Alkylaryl" refers to an "alkyl" joined to an "aryl".

The term "($C_1$-$C_{12}$)hydrocarbon moiety" encompasses alkyl, alkenyl and alkynyl and in the case of alkenyl and alkynyl there is $C_2$-$C_{12}$.

For the avoidance of doubt, unless otherwise indicated, the term substituted means substituted by one or more defined groups. In the case where groups may be selected from a number of alternative groups, the selected groups may be the same or different. For the avoidance of doubt, the term independently means that where more than one substituent is selected from a number of possible substituents, those substituents may be the same or different.

The pharmaceutically acceptable salts of the compounds herein which contain a basic centre are, for example, non-toxic acid addition salts formed with inorganic acids such as hydrochloric, hydrobromic, hydroiodic, sulfuric and phosphoric acid, with carboxylic acids or with organo-sulfonic acids. Examples include the HCl, HBr, HI, sulfate or bisulfate, nitrate, phosphate or hydrogen phosphate, acetate, benzoate, succinate, saccharate, fumarate, maleate, lactate, citrate, tartrate, gluconate, camsylate, methanesulfonate, ethanesulfonate, benzene sulfonate, p-toluenesulfonate and pamoate salts. Compounds can also provide pharmaceutically acceptable metal salts, in particular non-toxic alkali and alkaline earth metal salts, with bases. Examples include the sodium, potassium, aluminum, calcium, magnesium, zinc and diethanolamine salts (Berge, S. M. et al., J. Pharm. Sci., 66:1-19 (1977); Gould, P.L., Int'l J. Pharmaceutics, 33:201-17 (1986); and Bighley, L.D. et al., Encyclo. Parma. Tech., Marcel Dekker Inc, New York, 13:453-97 (1996).

The pharmaceutically acceptable solvates of the compounds herein include the hydrates thereof. Also included within the scope of the disclosure and various salts herein are polymorphs thereof. Compounds of the disclosure therefore include compounds, their pharmaceutically acceptable salts, their solvates or polymorphs, defined in any aspect (except intermediate compounds in chemical processes).

*In vitro* studies

Compounds herein can be and were tested for activity as ligands of one or more of the melanocortin receptors

according to the following procedures. One skilled in the art would know that procedures similar to those described herein may be used to assay the binding activities of the compounds to melanocortin receptor molecules.

***Radioligand Binding Assays***

Cellular membranes used for the *in vitro* receptor binding assays were obtained from transgenic CHO-K1 cells stably expressing hMC-R receptor subtypes 1, 3, 4 or 5. The CHO-K1 cells expressing the desired hMC-R receptor type were sonicated (Branson® setting 7, approximately 30 sec) in ice-cold 50 mM Tris-HCl at pH 7.4 and then centrifuged at 39,000 g for 10 minutes at approximately 4°C. The pellets were resuspended in the same buffer and centrifuged at 50,000 g for 10 minutes at approximately 4°C. The washed pellets containing the cellular membranes were stored at approximately - 80°C.

Competitive inhibition of [$^{125}$I](Tyr$^2$)-(Nle$^4$-D-Phe$^7$)α-MSH ([$^{125}$I]-NDP-α-MSH, Amersham Biosciences®) binding was carried out in polypropylene 96 well plates. Cell membranes (1-10 μg protein/well) prepared as described above were incubated in 50 mM Tris-HCl at pH 7.4 containing 0.2% bovine serum albumin (BSA), 5 mM MgCh, 1 mM $CaCl_2$ and 0.1 mg/mL bacitracin, with increasing concentrations of the test compound and 0.1-0.3 nM [$^{125}$I]-NDP-α-MSH for approximately 90-120 minutes at approximately 37°C. Bound [$^{125}$I]-NDP-α-MSH ligand was separated from free [$^{125}$I] -NDP-α-MSH by filtration through GF/C glass fiber filter plates (Unifilter®; Packard) presoaked with 0.1 % (w/v) polyethylenimine (PEI), using a Packard Filtermate® harvester. Filters were washed three times with 50 mM Tris-HCl at pH 7.4 at a temperature of approximately 0-4°C and then assayed for radioactivity using a Packard Topcount® scintillation counter. Binding data were analyzed by computer-assisted non-linear regression analysis (XL fit; IDBS). A selection of the compounds herein was tested using the above-discussed assay and the binding constants (Ki in nM) are reported in Tables 5, 6, 7 and 8.

TABLE 5 - Radioligand Binding Assay Data for Selected Compounds

Table 5A

| Compound | Ki hMC 1-R | Ki hMC 3-R | Ki hMC4-R | Ki hMC5-R | Ki hMC1-R/ MC4-R | SEQ ID NO: |
|---|---|---|---|---|---|---|
| Ac-Arg-c(Cys-D-Ala-His-D-Phe-Arg-Trp-Cys)-$NH_2$ | 3.87 | 10.1 | 2.09 | 430 | 1.9 | SEQ ID NO:50 |
| Ac-D-Arg-c(Cys-D-Ala-His-D-Phe-Arg-Trp-Cys)-$NH_2$ | 4.01 | 12.1 | 1.76 | 352 | 2.3 | SEQ ID NO:50 |
| Ac-D-Arg-c(Cys-D-Ala-His-D-Phe-Arg-Trp-Pen)-$NH_2$ | 8.29 | 13.3 | 2.78 | 816 | 3.0 | SEQ ID NO:51 |
| Ac-D-Arg-c(Cys-His-D-Phe-Arg-Trp-Gaba-Pen)-$NH_2$ | 3.93 | 172 | 11.0 | 538 | 0.36 | SEQ ID NO:52 |
| Ac-Arg-c(Cys-His-D-Phe-Arg-Trp-Gaba-Pen)-$NH_2$ | 1.81 | 20.5 | 4.57 | 502 | 0.4 | SEQ ID NO:52 |
| Ac-Arg-c(Cys-D-Ala-His-D-Phe-Arg-Trp-Pen)-$NH_2$ | 9.67 | 22.0 | 4.2 | 1900 | 2.3 | SEQ ID NO:51 |
| Ac-D-Arg-c(Asp-His-D-Phe-Arg-Trp-Ala-Lys)-$NH_2$ | 0.79 | 45.5 | 1.21 | 493 | 0.6 | SEQ ID NO:53 |
| Ac-Arg-c(Asp-His-D-Phe-Arg-Trp-Ala-Lys)-$NH_2$ | 0.68 | 20.7 | 1.01 | 783 | 0.7 | SEQ ID NO:53 |

Table 5B

| Compound | Ki hMC1-R | Ki hMC 3-R | Ki hMC4-R | Ki hMC 5-R | Ki hMC1-R /MC4-R | SEQ ID NO: |
|---|---|---|---|---|---|---|
| Ac-Nle-c(Cys-D-Ala-His-D-2-Nal-Arg-1-Nal-Cys)-$NH_2$ | 114 | 63.9 | 3.07 | 1657 | 37.1 | SEQ ID NO:16 |
| Ac-Nle-c(Cys-D-Ala-His-D-Phe-Arg-Trp-Cys)-$NH_2$ | 11 | 26 | 7.6 | 1800 | 1.4 | SEQ ID NO:7 |
| D-Phe-c(Cys-His-D-(Et)Tyr-Arg-Trp-β-Ala-D-Cys)-Thr-$NH_2$ | 0.05 | 9.3 | 1.1 | 2.9 | 0.0 | SEQ ID NO:24 |
| Nle-c(Cys-His-D-Phe-Arg-Trp-Apn-Cys)-$NH_2$ | 0.07 | 4.1 | 0.85 | 8.8 | 0.1 | SEQ ID NO:27 |
| Ac-Nle-c(Cys-His-D-Phe-Arg-Trp-Gaba-Pen)-$NH_2$ | 0.12 | 10 | 0.43 | 0.42 | 0.3 | SEQ ID NO:32 |
| Nle-c(Cys-His-D-Phe-Arg-Trp-Gaba-Cys)-$NH_2$ | 0.05 | 1.3 | 0.47 | 0.2 | 0.1 | SEQ ID NO:34 |
| Ac-Nle-c(Asp-His-D-Phe-Arg-Trp-β-Ala-Lys)-$NH_2$ | 0.0996 | 9318 | 0.617 | 10.9 | 0.16 | SEQ ID NO:1 |
| Ac-Nle-c(Cys-His-D-Phe-Arg-Trp-Ahx-Cys)-$NH_2$ | .0132 | 16.1 | 1.23 | 0.359 | 0.11 | SEQ ID NO:2 |
| D-Phe-c(Cys-His-D-Phe-Arg-Trp-β-Ala-D-Cys)-Thr-$NH_2$ | 0.207 | 43.2 | 2.58 | 344 | 0.08 | SEQ ID NO:3 |
| D-Phe-c(Cys-His-D-Phe-Arg-Trp-Gaba-D-Cys)-Thr-$NH_2$ | 0.420 | 106 | 4.75 | 1260 | 0.09 | SEQ ID NO:3 |
| Ac-Nle-c(Cys-His-D-Phe-Arg-Trp-Apn-Cys)-$NH_2$ | 0.0951 | 9.33 | 0.894 | 13.4 | 0.11 | SEQ ID NO:2 |
| Ac-Nle-c(Asp-His-D-Phe-Arg-Trp-Apn-Lys)-$NH_2$ | 0.999 | 300 | 11.1 | 431 | 0.09 | SEQ ID NO:4 |
| Ac-Cha-c(Asp-His-D-Phe-Arg-Trp-Gaba-Lys)-$NH_2$ | 0.106 | 11.8 | 1.49 | 110 | 0.07 | SEQ ID NO:6 |
| Ac-Nle-c(Asp-His-D-Phe-Arg-Trp-Gaba-Lys)-$NH_2$ | 0.0506 | 9.89 | 1.04 | 16.3 | 0.05 | SEQ ID NO:6 |
| Ac-Chg-c(Asp-His-D-Phe-Arg-Trp-Gaba-Lys)-$NH_2$ | 0.884 | 223 | 22.5 | 609 | 0.04 | SEQ ID NO:11 |
| Ac-hCha-c(Asp-His-D-Phe-Arg-Trp-Gaba-Lys)-$NH_2$ | 0.721 | 93.5 | 56.0 | 747 | 0.01 | SEQ ID NO:11 |

(continued)

| Compound | Ki hMC1-R | Ki hMC 3-R | Ki hMC4-R | Ki hMC 5-R | Ki hMC1-R /MC4-R | SEQ ID NO: |
|---|---|---|---|---|---|---|
| Ac-D-Chg-c(Asp-His-D-Phe-Arg-Trp-Gaba-Lys)-$NH_2$ | 0.227 | 14.5 | 2.99 | 164 | 0.08 | SEQ ID NO:11 |
| Ac-hPhe-c(Asp-His-D-Phe-Arg-Trp-Gaba-Lys)-$NH_2$ | 0.277 | 25.2 | 3.37 | 203 | 0.08 | SEQ ID NO:11 |
| Ac-Nle-c(Cys-His-D-Phe-Arg-D-Trp-β-Ala-Cys)-$NH_2$ | 0.323 | 14.1 | 1.96 | 24.0 | 0.16 | SEQ ID NO:15 |
| Ac-Nle-c(Pen-D-Ala-His-D-Phe-Arg-Trp-Cys)-$NH_2$ | 34.1 | 118 | 17.0 | 5560 | 2.01 | SEQ ID NO:21 |
| Ac-Nle-c(Cys-D-Ala-His-D-Phe-Arg-Trp-Pen)-$NH_2$ | 29.1 | 22.8 | 3.84 | 2550 | 7.58 | SEQ ID NO:22 |
| D-Phe-c(Cys-His-D-Phe-hArg-Trp-β-Ala-D-Cys)-Thr-$NH_2$ | 0.442 | 123 | 10.3 | 521 | 0.04 | SEQ ID NO:23 |
| D-Phe-c(Cys-His-D-Phe-Arg-Bip-β-Ala-D-Cys)-Thr-$NH_2$ | 5.80 | 3370 | 583 | 1130 | 0.01 | SEQ ID NO:25 |
| D-Phe-c(Cys-His-D-(Et)Tyr-hArg-Trp-β-Ala-D-Cys)-Thr-$NH_2$ | 0.0567 | 31.4 | 14.7 | 9.27 | 0 | SEQ ID NO:24 |
| D-Phe-c(Cys-His-D-Phe-hArg-Bip-β-Ala-D-Cys)-Thr-$NH_2$ | 1.68 | 1260 | 172 | 1220 | 0.01 | SEQ ID NO:26 |
| D-Phe-c(Cys-His-D-(Et)Tyr-hArg-Bip-β-Ala-D-Cys)-Thr-$NH_2$ | 0.128 | 85.6 | 36.9 | 38.0 | 0 | SEQ ID NO:26 |
| Ac-Nle-c(Cys-D-Ala-His-D-Phe-Arg-Trp-Gly-Cys)-$NH_2$ | 0.352 | 149 | 3.01 | 339 | 0.12 | SEQ ID NO:54 |
| Ac-Nle-c(Cys-D-Ala-His-D-Phe-Arg-Trp-D-Ala-Cys)-$NH_2$ | 3.93 | 876 | 48.0 | 4940 | 0.08 | SEQ ID NO:54 |
| Ac-Nle-c(Cys-D-Ala-His-D-Phe-Arg-Trp-β-Ala-Cys)-$NH_2$ | 0.995 | 287 | 4.80 | 766 | 0.21 | SEQ ID NO:54 |
| Ac-Nle-c(Cys-D-Ala-His-D-Phe-Arg-Trp-Gaba-Cys)-$NH_2$ | 0.848 | 184 | 3.76 | 956 | 0.23 | SEQ ID NO:54 |

(continued)

| Compound | Ki hMC1-R | Ki hMC 3-R | Ki hMC4-R | Ki hMC 5-R | Ki hMC1-R /MC4-R | SEQ ID NO: |
|---|---|---|---|---|---|---|
| Ac-Nle-c(Cys-D-Ala-His-D-Phe-Arg-Trp-Apn-Cys)-$NH_2$ | 1.10 | 228 | 7.58 | 859 | 0.15 | SEQ ID NO:54 |
| Ac-Nle-c(Asp-D-Ala-His-D-Phe-Arg-Trp-Lys)-$NH_2$ | 0.659 | 98.9 | 2.55 | 4.19 | 0.26 | SEQ ID NO:28 |
| Ac-Nle-c(Asp-D-Ala-His-D-Phe-Arg-Bal-Lys)-$NH_2$ | 4.12 | 445 | 50.6 | 4300 | 0.08 | SEQ ID NO:28 |
| Ac-c(Cys-Glu-His-D-Phe-Arg-Trp-Ala-Cys)-$NH_2$ | 111 | 1710 | 47.7 | 694 | 2.33 | SEQ ID NO:55 |
| Ac-c(Cys-Glu-His-D-Phe-Arg-2-Nal-Ala-Cys)-$NH_2$ | 262 | 2500 | 96.4 | 1460 | 2.72 | SEQ ID NO:55 |
| Ac-c(Cys-D-Ala-His-D-Phe-Arg-Trp-Ala-Cys)-$NH_2$ | 199 | 5990 | 96.7 | > 10000 | 2.06 | SEQ ID NO:56 |
| Ac-c(Cys-D-Ala-His-D-Phe-Arg-2-Nal-Ala-Cys)-$NH_2$ | 132 | 4560 | 40.7 | 8810 | 3.24 | SEQ ID NO:56 |
| Ac-Nle-c(Cys-D-Ala-His-D-Phe-Arg-Trp-Ala-Cys)-$NH_2$ | 9.12 | 1130 | 22.1 | 2860 | 0.41 | SEQ ID NO:57 |
| Ac-Nle-c(Cys-D-Ala-His-D-Phe-Arg-Trp-β-Ala-Cys)-$NH_2$ | 1.00 | 227 | 5.55 | 496 | 0.18 | SEQ ID NO:57 |
| Ac-Nle-c(Cys-D-Ala-His-D-Phe-Arg-Trp-Gaba-Cys)-$NH_2$ | 0.536 | 169 | 3.12 | 358 | 0.17 | SEQ ID NO:57 |
| Ac-Nle-c(Cys-D-Ala-His-D-Phe-Arg-Trp-Pen)-OH | 32.1 | 330 | 17.4 | 165 | 1.84 | SEQ ID NO:29 |
| Ac-Nle-c(Cys-D-Abu-His-D-Phe-Arg-Trp-Cys)-$NH_2$ | 10.6 | 41.1 | 7.69 | 54.9 | 1.38 | SEQ ID NO:30 |
| Ac-Nle-c(Cys-D-Val-His-D-Phe-Arg-Trp-Cys)-$NH_2$ | 13.0 | 104 | 10.1 | 40 | 1.29 | SEQ ID NO:30 |
| Ac-Nle-c(Cys-D-Ile-His-D-Phe-Arg-Trp-Cys)-$NH_2$ | 4.28 | 38.5 | 9.0 | 12.5 | 0.48 | SEQ ID NO:30 |
| Ac-Nle-c(Cys-D-Leu-His-D-Phe-Arg-Trp-Cys)-$NH_2$ | 1.60 | 6.82 | 4.13 | 5.57 | 0.39 | SEQ ID NO:30 |
| Ac-Nle-c(Cys-D-Tle-His-D-Phe-Arg-Trp-Cys)-$NH_2$ | 12.0 | 85.8 | 11.2 | 40 | 1.07 | SEQ ID NO:30 |

(continued)

| Compound | Ki hMC1-R | Ki hMC 3-R | Ki hMC4-R | Ki hMC 5-R | Ki hMC1-R /MC4-R | SEQ ID NO: |
|---|---|---|---|---|---|---|
| Ac-Nle-c(Cys-D-Cha-His-D-Phe-Arg-Trp-Cys)-$NH_2$ | 0.353 | 2.08 | 1.41 | 0.857 | 0.25 | SEQ ID NO:30 |
| Ac-Nle-c(Pen-His-D-Phe-Arg-Trp-Gaba-Cys)-$NH_2$ | 0.537 | 86.1 | 5.89 | 2.56 | 0.09 | SEQ ID NO:31 |
| Ac-Nle-c(Pen-His-D-Phe-Arg-Trp-Gaba-Pen)-$NH_2$ | 0.744 | 178 | 3.51 | 2.69 | 0.21 | SEQ ID NO:32 |
| Ac-Leu-c(Cys-His-D-Phe-Arg-Trp-Gaba-Cys)-$NH_2$ | 0.216 | 17.4 | 0.995 | 0.486 | 0.22 | SEQ ID NO:33 |
| Ac-Cha-c(Cys-His-D-Phe-Arg-Trp-Gaba-Cys)-$NH_2$ | 0.107 | 9.11 | 0.884 | 0.354 | 0.12 | SEQ ID NO:33 |
| Ac-Ile-c(Cys-His-D-Phe-Arg-Trp-Gaba-Cys)-$NH_2$ | 0.148 | 13.9 | 1.06 | 0.423 | 0.14 | SEQ ID NO:33 |
| Ac-Phe-c(Cys-His-D-Phe-Arg-Trp-Gaba-Cys)-$NH_2$ | 0.254 | 18.5 | 2.13 | 0.714 | 0.12 | SEQ ID NO:33 |
| Ac-Val-c(Cys-His-D-Phe-Arg-Trp-Gaba-Cys)-$NH_2$ | 0.256 | 29.9 | 1.98 | 0.864 | 0.13 | SEQ ID NO:33 |
| Ac-2-Nal-c(Cys-His-D-Phe-Arg-Trp-Gaba-Cys)-$NH_2$ | 0.560 | 39.2 | 2.94 | 2.73 | 0.19 | SEQ ID NO:33 |
| Phe-c(Cys-His-D-Phe-Arg-Trp-Gaba-Cys)-$NH_2$ | 0.186 | 15.2 | 4.93 | 0.537 | 0.04 | SEQ ID NO:34 |
| Ac-Nle-c(Cys-3-Pal-D-Phe-Arg-Trp-Gaba-Cys)-$NH_2$ | 21.1 | 151 | 10.4 | 92.6 | 2.03 | SEQ ID NO:35 |
| Ac-Nle-c(Cys-D-Ala-His-D-Phe-Arg-Trp-Cys)-OH | 30.7 | 152 | 15.6 | 114 | 1.97 | SEQ ID NO:36 |
| Ac-Nle-c(Cys-His-Phe-Arg-D-Trp-Gaba-Cys)-$NH_2$ | 5.20 | 150 | 138 | 20.3 | 0.04 | SEQ ID NO:37 |
| Ac-Nle-c(Asp-D-Ala-His-D-Phe-Arg-Bal-Ala-Lys)-$NH_2$ | 4.89 | 290 | 21.3 | 11.1 | 0.23 | SEQ ID NO:58 |
| Ac-Nle-c(Cys-D-Ala-His-D-2-Nal-Arg-Trp-Cys)-$NH_2$ | 25.5 | 3.82 | 7.61 | 102 | 3.35 | SEQ ID NO:16 |
| Ac-Nle-c(Cys-D-Ala-His-D-2-Nal-Arg-2-Nal-Cys)-$NH_2$ | 32.5 | 5.85 | 2.53 | 94.6 | 12.85 | SEQ ID NO:16 |
| Ac-Nle-c(Cys-D-Ala-His-D-2-Nal-Arg-Bal-Cys)-$NH_2$ | 22.2 | 12.7 | 16.6 | 125 | 1.34 | SEQ ID NO:20 |

(continued)

| Compound | Ki hMC1-R | Ki hMC 3-R | Ki hMC4-R | Ki hMC 5-R | Ki hMC1-R /MC4-R | SEQ ID NO: |
|---|---|---|---|---|---|---|
| Ac-Nle-c(Asp-His-D-2-Nal-Arg-Trp-Ala-Lys)-$NH_2$ | 1.17 | 1.56 | 0.277 | 3.24 | 4.22 | SEQ ID NO:38 |
| Ac-Nle-c(Asp-His-D-2-Nal-Arg-Trp-β-Ala-Lys)-$NH_2$ | 0.648 | 2.78 | 0.329 | 1.4 | 1.97 | SEQ ID NO:38 |
| Ac-Nle-c(Cys-His-D-2-Nal-Arg-Trp-Gaba-Cys)-$NH_2$ | 0.393 | 1.86 | 0.375 | 1.11 | 1.05 | SEQ ID NO:39 |
| Ac-Nle-c(Cys-His-D-2-Nal-Arg-Trp-Ahx-Cys)-$NH_2$ | 0.333 | 2.91 | 0.998 | 0.366 | 0.33 | SEQ ID NO:39 |
| Ac-hPhe-c(Asp-His-D-2-Nal-Arg-Trp-Gaba-Lys)-$NH_2$ | 0.461 | 2.45 | 0.931 | 1.37 | 0.50 | SEQ ID NO:40 |
| Ac-Cha-c(Asp-His-D-2-Nal-Arg-Trp-Gaba-Lys)-$NH_2$ | 0.576 | 3.98 | 2.82 | 3.91 | 0.20 | SEQ ID NO:40 |

Table 5C

| Compound | Ki hMC1-R | Ki hMC 3-R | Ki hMC4-R | Ki hMC 5-R | Ki hMC1-R /MC4-R | SEQ ID NO: |
|---|---|---|---|---|---|---|
| Ac-Arg-c(Cys-D-Ala-His-D-2-Nal-Arg-Trp-Cys)-$NH_2$ | 17.9 | 1.68 | 0.256 | 23.4 | 69.9 | SEQ ID NO:49 |

TABLE 6 - Radioligand Binding Assay Data for Selected Compounds

Table 6A

| Compound | Ki hMC1-R | Ki hMC3-R | Ki hMC4-R | Ki hMC5-R |
|---|---|---|---|---|
| Ac-c(Cys-Glu-His-D-2-Nal-Arg-Trp-Ala-Cys)-Pro-Pro-Lys-Asp-$NH_2$ (SEQ ID NO:269) | 49.9 | 9.00 | 0.569 | 218 |
| Tyr-Gly-Arg-Lys-Lys-Arg-Arg-Gln-Arg-Arg-Arg-Nle-c(Asp-His-D-2-Nal-Arg-Trp-Lys)-$NH_2$ (SEQ ID NO:60) | 11.9 | 38.1 | 5.70 | 11.8 |
| Tyr-Gly-Arg-Lys-Lys-Arg-Arg-Gln-Arg-Arg-Arg-Doc-Nle-c(Asp-His-D-2-Nal-Arg-Trp-Lys)-$NH_2$ (SEQ ID NO:61) | 3.46 | 16.6 | 6.65 | 4.88 |
| Nle-c(Asp-His-D-2-Nal-Arg-Trp-Lys)-*ß*-Ala-Tyr-Gly-Arg-Lys-Lys-Arg-Arg-Gln-Arg-Arg-Arg-$NH_2$ (SEQ ID NO:62) | 0.614 | 5.09 | 2.31 | 3.23 |

(continued)

| Compound | Ki hMC1-R | Ki hMC3-R | Ki hMC4-R | Ki hMC5-R |
|---|---|---|---|---|
| Ac-Nle-c(Asp-His-D-2-Nal-Arg-Trp-Lys)-ß-Ala-Tyr-Gly-Arg-Lys-Lys-Arg-Arg-Gln-Arg-Arg-Arg-$NH_2$ (SEQ ID NO:62) | 1.56 | 14.1 | 5.17 | 7.12 |
| H-Nle-c(Asp-His-D-2-Nal-Arg-Trp-Lys)-Doc-Doc-Tyr-Gly-Arg-Lys-Lys-Arg-Arg-Gln-Arg-Arg-Arg-$NH_2$ (SEQ ID NO:63) | 1.10 | 1.58 | 6.00 | 0.629 |
| Ac-Nle-c(Asp-His-D-2-Nal-Arg-Trp-Lys)-Pro-Pro-Lys-Asp-Tyr-Gly-Arg-Lys-Lys-Arg-Arg-Gln-Arg-Arg-Arg-$NH_2$ (SEQ ID NO:64) | 0.0868 | 0.751 | 0.0944 | 0.147 |
| Ac-c(Cys-Glu-His-D-2-Nal-Arg-Trp-Gly-Cys)-Pro-Pro-Lys-Asp-Tyr-Gly-Arg-Lys-Lys-Arg-Arg-Gln-Arg-Arg-Arg-$NH_2$ (SEQ ID NO:65) | 1.66 | 4.80 | 0.250 | 9.62 |
| Ac-Nle-c(Asp-His-D-2-Nal-Arg-Trp-Lys)-ß-Ala-ß-Ala-Tyr-Gly-Arg-Lys-Lys-Arg-Arg-Gln-Arg-Arg-Arg-$NH_2$ (SEQ ID NO:66) | 0.0452 | 0.298 | 0.169 | 0.386 |
| Ac-Nle-c(Asp-His-D-2-Nal-Arg-Trp-Lys)-Pro-Pro-Lys-Asp-Doc-Tyr-Gly-Arg-Lys-Lys-Arg-Arg-Gln-Arg-Arg-Arg-$NH_2$ (SEQ ID NO:67) | 0.0808 | 0.396 | 0.0747 | 0.311 |
| Ac-c(Cys-Glu-His-D-2-Nal-Arg-Trp-Gly-Cys)-Pro-Pro-Lys-Asp-Doc-Tyr-Gly-Arg-Lys-Lys-Arg-Arg-Gln-Arg-Arg-Arg-$NH_2$ (SEQ ID NO:68) | 4.41 | 4.23 | 0.455 | 12.9 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Arg-Arg-Gln-Arg-Arg-Arg-$NH_2$ (SEQ ID NO:71) | 1.25 | 0.661 | 0.292 | 5.94 |
| Ac-c(Cys-Glu-His-D-2-Nal-Arg-Trp-Ala-Cys)-Pro-Pro-Lys-Asp-Doc-Tyr-Gly-Arg-Lys-Lys-Arg-Arg-Gln-Arg-Arg-Arg-$NH_2$ (SEQ ID NO:71) | 1.89 | 0.546 | 0.166 | 6.06 |
| Ac-c(Cys-Glu-His-D-2-Nal-Arg-2-Nal-Ala-Cys)-Pro-Pro-Lys-Asp-$NH_2$ (SEQ ID NO:269) | 87.8 | 9.08 | 1.20 | 359 |
| Ac-c(Cys-Glu-His-D-2-Nal-Arg-1-Nal-Ala-Cys)-Pro-Pro-Lys-Asp-$NH_2$ (SEQ ID NO:269) | 124 | 17.8 | 1.11 | 348 |
| Ac-c(Cys-Glu-His-D-2-Nal-Arg-Bal-Ala-Cys)-Pro-Pro-Lys-Asp-$NH_2$ (SEQ ID NO:269) | 163 | 23.0 | 0.586 | 844 |

(continued)

| Compound | Ki hMC1-R | Ki hMC3-R | Ki hMC4-R | Ki hMC5-R |
|---|---|---|---|---|
| Ac-Nle-c(Asp-His-D-2-Nal-Arg-Trp-Lys)-Doc-Doc-Tyr-Gly-Arg-Lys-Lys-Arg-Arg-Gln-Arg-Arg-Arg-$NH_2$ (SEQ ID NO:701) | 0.144 | 0.352 | 0.0845 | 0.415 |
| Ac-c(Cys-Glu-His-D-2-Nal-Arg-2-Nal-Ala-Cys)-Pro-Pro-Lys-Asp-ß-Ala-Tyr-Gly-Arg-Lys-Lys-Arg-Arg-Gln-Arg-Arg-Arg-$NH_2$ (SEQ ID NO:71) | 1.74 | 0.590 | 0.170 | 4.38 |
| Ac-c(Cys-Glu-His-D-2-Nal-Arg-Bal-Ala-Cys)-Pro-Pro-Lys-Asp-ß-Ala-Arg-Arg-Arg-Arg-Arg-Gln-Arg-Arg-Arg-$NH_2$ (SEQ ID NO:83) | 3.86 | 4.97 | 0.192 | 38.3 |
| Ac-c(Cys-Glu-His-D-2-Nal-Arg-Bal-Ala-Cys)-Pro-Pro-Lys-Asp-ß-Ala-Gly-Arg-Arg-Arg-Arg-Arg-Gln-Arg-Arg-Arg-$NH_2$ (SEQ ID NO:73) | 12.8 | 15.9 | 0.950 | 165 |
| Ac-c(Cys-Glu-His-D-2-Nal-Arg-Bal-Ala-Cys)-Pro-Pro-Lys-Asp-ß-Ala-Tyr-Gly-Arg-Arg-Arg-Arg-Arg-Gln-Arg-Arg-Arg-$NH_2$ (SEQ ID NO:74) | 3.07 | 4.05 | 0.498 | 31.1 |
| Ac-c(Cys-Glu-His-D-2-Nal-Arg-Trp-Ala-Cys)-Pro-Pro-Lys-Asp-βAla-Tyr-Gly-Arg-Lys-Lys-Arg-Gln-Arg-Arg-Arg-Arg-$NH_2$ (SEQ ID NO:75) | 0.792 | 0.570 | 0.162 | 4.18 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Gln-Arg-Arg-Arg-Arg-Arg-$NH_2$ (SEQ ID NO:76) | 0.726 | 0.474 | 0.209 | 5.12 |
| Ac-c(Cys-Glu-His-D-2-Nal-Arg-Trp-Ala-Cys)-Pro-Pro-Lys-Asp-ß-Ala-Tyr-Gly-Arg-Lys-Gln-Lys-Arg-Arg-Arg-Arg-Arg-$NH_2$ (SEQ ID NO:77) | 0.857 | 0.580 | 0.209 | 4.42 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Arg-Arg-Arg-Arg-Gln-Arg-$NH_2$ (SEQ ID NO:78) | 0.813 | 0.675 | 0.269 | 4.20 |
| Ac-c(Cys-Glu-His-D-2-Nal-Arg-Bal-Ala-Cys)-Pro-Pro-Lys-Asp-ß-Ala-Tyr-Aib-Arg-Lys-Lys-Arg-Arg-Gln-Arg-Arg-Arg-$NH_2$ (SEQ ID NO:79) | 7.84 | 10.2 | 0.783 | 91.8 |

(continued)

| Compound | Ki hMC1-R | Ki hMC3-R | Ki hMC4-R | Ki hMC5-R |
|---|---|---|---|---|
| Ac-c(Cys-Glu-His-D-2-Nal-Arg-1-Nal-Ala-Cys)-Pro-Pro-Arg-Asp-ß-Ala-Arg-Arg-Arg-Arg-Arg-Gln-Arg-Arg-Arg-$NH_2$ (SEQ ID NO:80) | 2.93 | 9.07 | 0.293 | 59.0 |
| Ac-c(Cys-Glu-His-D-2-Nal-Arg-1-Nal-Ala-Cys)-Pro-Pro-Lys-Asp-ß-Ala-Arg-Arg-Arg-Arg-Arg-Gln-Arg-Arg-Arg-$NH_2$ (SEQ ID NO:80) | 2.42 | 6.56 | 0.238 | 41.7 |
| Ac-c(Cys-Glu-His-D-2-Nal-Arg-1-Nal-Ala-Cys)-Pro-Pro-Lys-Asp-ß-Ala-Arg-Arg-Arg-Arg-Arg-Arg-Gln-Arg-Arg-Arg-$NH_2$ (SEQ ID NO:81) | 6.66 | 19.3 | 0.819 | 88.8 |
| Ac-c(Cys-Glu-His-D-2-Nal-Arg-2-Nal-Ala-Cys)-Pro-Pro-Arg-Asp-ß-Ala-Arg-Arg-Arg-Arg-Arg-Gln-Arg-Arg-Arg-$NH_2$ (SEQ ID NO:82) | 2.63 | 2.09 | 0.0737 | 11.6 |
| Ac-c(Cys-Glu-His-D-2-Nal-Arg-2-Nal-Ala-Cys)-Pro-Pro-Lys-Asp-ß-Ala-Arg-Arg-Arg-Arg-Arg-Gln-Arg-Arg-Arg-$NH_2$ (SEQ ID NO:82) | 2.48 | 1.21 | 0.209 | 9.17 |
| Ac-c(Cys-Glu-His-D-2-Nal-Arg-2-Nal-Ala-Cys)-Pro-Pro-Lys-Asp-ß-Ala-Arg-Arg-Arg-Arg-Arg-Arg-Gln-Arg-Arg-Arg-$NH_2$ (SEQ ID NO:81) | 3.65 | 2.26 | 0.261 | 12.1 |
| Ac-c(Cys-Glu-His-D-2-Nal-Arg-Bal-Ala-Cys)-Pro-Pro-Arg-Asp-ß-Ala-Arg-Arg-Arg-Arg-Arg-Arg-Gln-Arg-Arg-Arg-$NH_2$(SEQ ID NO:83) | 7.32 | 11.0 | 0.659 | 78.0 |
| Ac-c(Cys-Glu-His-D-2-Nal-Arg-Bal-Ala-Cys)-Pro-Pro-Arg-Asp-ß-Ala-Arg-Arg-Arg-Arg-Arg-Gln-Arg-Arg-Arg-$NH_2$ (SEQ ID NO:84) | 4.11 | 7.26 | 0.302 | 48.3 |
| Ac-c(Cys-Glu-His-D-2-Nal-Arg-Bal-Ala-Cys)-Pro-Pro-Lys-Asp-ß-Ala-Arg-Arg-Arg-Arg-Arg-Arg-Gln-Arg-Arg-Arg-$NH_2$(SEQ ID NO:283) | 6.77 | 14.3 | 0.781 | 84.0 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Arg-Arg-Arg-Gln-Arg-Arg-$NH_2$ (SEQ ID NO:2685) | 3.04 | 3.22 | 0.230 | 3.85 |

(continued)

| Compound | Ki hMC1-R | Ki hMC3-R | Ki hMC4-R | Ki hMC5-R |
|---|---|---|---|---|
| Ac-c(Cys-Glu-His-D-2-Nal-Arg-Trp-Ala-Cys)-Pro-Pro-Lys-Asp-ß-Ala-Tyr-Gly-Arg-Gln-Lys-Lys-Arg-Arg-Arg-Arg-Arg-$NH_2$ (SEQ ID NO:86) | 3.24 | 2.66 | 0.208 | 5.96 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Arg-Arg-Arg-Arg-Arg-Gln-$NH_2$ (SEQ ID NO:87) | 1.58 | 1.43 | 0.275 | 2.97 |
| Ac-c(Cys-Glu-His-D-2-Nal-Arg-1-Nal-Ala-Cys)-Pro-Pro-Lys-Asp-ß-Ala-Tyr-Gly-Arg-Lys-Lys-Arg-Arg-Gln-Arg-Arg-Arg-$NH_2$ (SEQ ID NO:71) | 4.59 | 6.28 | 0.588 | 22.6 |
| Ac-c(Cys-Glu-His-D-2-Nal-Arg-Bal-Ala-Cys)-Pro-Pro-Lys-Asp-ß-Ala-Tyr-Gly-Arg-Lys-Lys-Arg-Arg-Gln-Arg-Arg-Arg-$NH_2$ (SEQ ID NO:71) | 6.46 | 5.22 | 0.380 | 15.3 |
| Ac-c(Cys-Glu-His-D-2-Nal-Arg-1-Nal-Ala-Cys)-Pro-Pro-Lys-Asp-ß-Ala-Arg-Arg-Lys-Arg-Arg-Gln-Arg-Arg-Arg-$NH_2$ (SEQ ID NO:88) | 4.62 | 5.68 | 0.505 | 45.3 |
| Ac-c(Cys-Glu-His-D-2-Nal-Arg-1-Nal-Ala-Cys)-Pro-Pro-Lys-Asp-ß-Ala-Arg-Lys-Arg-Arg-Arg-Gln-Arg-Arg-Arg-$NH_2$ (SEQ ID NO:89) | 2.12 | 3.99 | 0.352 | 27.5 |
| Ac-c(Cys-Glu-His-D-2-Nal-Arg-2-Nal-Ala-Cys)-Pro-Pro-Lys-Asp-ß-Ala-Arg-Arg-Lys-Arg-Arg-Gln-Arg-Arg-Arg-$NH_2$ (SEQ ID NO:88) | 3.41 | 0.975 | 0.549 | 11.3 |
| Ac-c(Cys-Glu-His-D-2-Nal-Arg-2-Nal-Ala-Cys)-Pro-Pro-Lys-Asp-ß-Ala-Tyr-Gly-Arg-Arg-Lys-Arg-Arg-Gln-Arg-Arg-Arg-$NH_2$ (SEQ ID NO:90) | 4.18 | 1.12 | 0.223 | 15.3 |
| Ac-c(Cys-Glu-His-D-2-Nal-Arg-2-Nal-Ala-Cys)-Pro-Pro-Lys-Asp-ß-Ala-Gly-Arg-Arg-Lys-Arg-Arg-Gln-Arg-Arg-Arg-$NH_2$ (SEQ ID NO:91) | 2.71 | 0.732 | 0.202 | 5.53 |
| Ac-c(Cys-Glu-His-D-2-Nal-Arg-2-Nal-Ala-Cys)-Pro-Pro-Lys-Asp-ß-Ala-Gly-Arg-Lys-Arg-Arg-Arg-Gln-Arg-Arg-Arg-$NH_2$ (SEQ ID NO:92) | 5.66 | 1.40 | 0.446 | 6.23 |

(continued)

| Compound | Ki hMC1-R | Ki hMC3-R | Ki hMC4-R | Ki hMC5-R |
|---|---|---|---|---|
| Ac-Nle-c(Cys-D-Ala-His-D-Phe-Arg-Trp-Cys)-(Doc)2-Tyr-Gly-Arg-Lys-Lys-Arg-Arg-Gln-Arg-Arg-Arg-$NH_2$ (SEQ ID NO:136) | 0.211 | 0.665 | 0.635 | 118 |
| Ac-Nle-c(Cys-D-Ala-His-D-Phe-Arg-Trp-Cys)-*ß*-Ala-Tyr-Gly-Arg-Lys-Lys-Arg-Gln-Arg-Arg-Arg-Arg-$NH_2$ (SEQ ID NO:137) | 0.351 | 0.891 | 0.503 | 102 |
| Ac-Nle-c(Cys-D-Ala-His-D-Phe-Arg-Trp-Cys)-Doc-Tyr-Gly-Arg-Lys-Lys-Arg-Arg-Gln-Arg-Arg-Arg-$NH_2$(SEQ ID NO:136) | 0.209 | 0.699 | 0.596 | 137 |
| Ac-Nle-c(Cys-D-Ala-His-D-Phe-Arg-Trp-Cys)-*ß*-Ala-Gly-Arg-Arg-Arg-Arg-Arg-Gln-Arg-Arg-Arg-$NH_2$(SEQ ID NO:138) | 0.439 | 1.52 | 0.476 | 115 |
| Ac-Nle-c(Cys-D-Ala-His-D-Phe-Arg-Trp-Cys)-*ß*-Ala-Arg-Arg-Arg-Arg-Arg-Gln-Arg-Arg-Arg-$NH_2$(SEQ ID NO:138) | 0.821 | 2.50 | 0.700 | 148 |
| Ac-Nle-c(Cys-D-Ala-His-D-Phe-Arg-Trp-Cys)-*ß*-Ala-Tyr-Gly-Arg-Arg-Arg-Arg-Arg-Gln-Arg-Arg-Arg-$NH_2$ (SEQ ID NO:139) | 0.406 | 1.11 | 0.602 | 131 |
| Ac-Nle-c(Cys-D-Ala-His-D-Phe-Arg-Trp-Cys)-*ß*-Ala-Gly-Arg-Arg-Arg-Arg-Arg-Gln-Arg-Arg-Arg-Arg-$NH_2$ (SEQ ID NO:139) | 1.27 | 4.63 | 1.51 | 220 |

Table 6B

| Compound | Ki hMC1-R | Ki hMC3-R | Ki hMC4-R | Ki hMC5-R |
|---|---|---|---|---|
| Ac-c(Cys-Glu-His-D-2-Nal-Arg-2-Nal-*ß*-Ala-Cys)-Pro-Pro-Lys-Asp-$NH_2$ (SEQ ID NO:270) | 2058 | 113 | 10.7 | 239 |
| Ac-c(Cys-Glu-His-D-2-Nal-Arg-2-Nal-Aib-Cys)-Pro-Pro-Lys-Asp-$NH_2$ (SEQ ID NO:270) | 1818 | 306 | 5.87 | 979 |
| Ac-c(Cys-Glu-His-D-2-Nal-Arg-1-Nal-Ala-Cys)-Pro-Pro-Lys-Asp-*ß*-Ala-Tyr-Gly-Arg-Arg-Lys-Arg-Arg-Gln-Arg-Arg-Arg-$NH_2$ (SEQ ID NO:95) | 1.75 | 1.74 | 0.15 | 16.8 |
| Ac-c(Cys-Glu-His-D-2-Nal-Arg-1-Nal-Ala-Cys)-Pro-Pro-Lys-Asp-*ß*-Ala-Tyr-Gly-Arg-Lys-Arg-Arg-Arg-Gln-Arg-Arg-Arg-$NH_2$ (SEQ ID NO:96) | 1.50 | 1.61 | 0.301 | 10.4 |

(continued)

| Compound | Ki hMC1-R | Ki hMC3-R | Ki hMC4-R | Ki hMC5-R |
|---|---|---|---|---|
| Ac-c(Cys-Glu-His-D-2-Nal-Arg-1-Nal-Ala-Cys)-Pro-Pro-Lys-Asp-ß-Ala-Gly-Arg-Arg-Lys-Arg-Arg-Gln-Arg-Arg-Arg-$NH_2$ (SEQ ID NO:97) | 1.81 | 2.08 | 0.305 | 19.3 |
| Ac-c(Cys-Glu-His-D-2-Nal-Arg-1-Nal-Ala-Cys)-Pro-Pro-Lys-Asp-ß-Ala-Gly-Arg-Lys-Arg-Arg-Arg-Gln-Arg-Arg-Arg-$NH_2$ (SEQ ID NO:92) | 2.69 | 2.59 | 0.243 | 19.2 |
| Ac-c(Cys-Glu-His-D-2-Nal-Arg-2-Nal-Ala-Cys)-Pro-Pro-Lys-Asp-ß-Ala-Arg-Lys-Arg-Arg-Arg-Gln-Arg-Arg-Arg-$NH_2$ (SEQ ID NO:99) | 2.25 | 0.62 | 0.303 | 2.77 |
| Ac-c(Cys-Glu-His-D-2-Nal-Arg-2-Nal-Ala-Cys)-Pro-Pro-Lys-Asp-ß-Ala-Tyr-Gly-Arg-Lys-Arg-Arg-Arg-Gln-Arg-Arg-Arg-$NH_2$(SEQ ID NO:101) | 1.49 | 0.604 | 0.865 | 3.13 |
| Ac-c(Cys-Glu-His-D-2-Nal-Arg-Bal-Ala-Cys)-Pro-Pro-Lys-Asp-ß-Ala-Arg-Lys-Arg-Arg-Arg-Gln-Arg-Arg-Arg-$NH_2$(SEQ ID NO:105) | 3.28 | 1.95 | 0.575 | 15.5 |
| Ac-c(Cys-Glu-His-D-2-Nal-Arg-Bal-Ala-Cys)-Pro-Pro-Lys-Asp-ß-Ala-Tyr-Gly-Arg-Arg-Lys-Arg-Arg-Gln-Arg-Arg-Arg-$NH_2$ (SEQ ID NO:100) | 2.24 | 1.57 | 0.437 | 16.4 |
| Ac-c(Cys-Glu-His-D-2-Nal-Arg-Bal-Ala-Cys)-Pro-Pro-Lys-Asp-ß-Ala-Tyr-Gly-Arg-Lys-Arg-Arg-Arg-Gln-Arg-Arg-Arg-$NH_2$ (SEQ ID NO:101) | 2.14 | 1.12 | 0.624 | 11.9 |
| Ac-c(Cys-Glu-His-D-2-Nal-Arg-Bal-Ala-Cys)-Pro-Pro-Lys-Asp-ß-Ala-Gly-Arg-Arg-Lys-Arg-Arg-Gln-Arg-Arg-Arg-$NH_2$ (SEQ ID NO:102) | 2.50 | 1.59 | 0.573 | 15.7 |
| Ac-c(Cys-Glu-His-D-2-Nal-Arg-Bal-Ala-Cys)-Pro-Pro-Lys-Asp-ß-Ala-Arg-Arg-Lys-Arg-Arg-Gln-Arg-Arg-Arg-$NH_2$ (SEQ ID NO:104) | 3.00 | 1.70 | 0.442 | 15.5 |
| Ac-c(Cys-Glu-His-D-2-Nal-Arg-Bal-Ala-Cys)-Pro-Pro-Lys-Asp-ß-Ala-Gly-Arg-Lys-Arg-Arg-Arg-Gln-Arg-Arg-Arg-$NH_2$ (SEQ ID NO:103) | 4.29 | 2.15 | 0.425 | 15.5 |

(continued)

| Compound | Ki hMC1-R | Ki hMC3-R | Ki hMC4-R | Ki hMC5-R |
|---|---|---|---|---|
| Ac-Nle-c(Cys-D-Ala-His-D-Phe-Arg-Trp-Cys)-ß-Ala-Tyr-Gly-Arg-Arg-Lys-Arg-Arg-Gln-Arg-Arg-Arg-$NH_2$ (SEQ ID NO:141) | 0.410 | 0.837 | 0.246 | 56.3 |
| Ac-Nle-c(Cys-D-Ala-His-D-Phe-Arg-Trp-Cys)-ß-Ala-Tyr-Gly-Arg-Lys-Arg-Arg-Arg-Gln-Arg-Arg-Arg-$NH_2$ (SEQ ID NO:142) | 0.572 | 1.07 | 0.210 | 63.6 |
| Ac-Nle-c(Cys-D-Ala-His-D-Phe-Arg-Trp-Cys)-ß-Ala-Gly-Arg-Arg-Lys-Arg-Arg-Gln-Arg-Arg-Arg-$NH_2$ (SEQ ID NO:141) | 0.475 | 0.800 | 0.196 | 53.8 |
| Ac-Nle-c(Cys-D-Ala-His-D-Phe-Arg-Trp-Cys)-ß-Ala-Gly-Arg-Lys-Arg-Arg-Arg-Gln-Arg-Arg-Arg-$NH_2$ (SEQ ID NO:142) | 0.779 | 1.21 | 0.293 | 56.0 |
| Ac-Nle-c(Cys-D-Ala-His-D-Phe-Arg-Trp-Cys)-ß-Ala-Arg-Arg-Lys-Arg-Arg-Gln-Arg-Arg-Arg-$NH_2$ (SEQ ID NO:143) | 0.212 | 1.23 | 0.484 | 58.5 |
| Ac-Nle-c(Cys-D-Ala-His-D-Phe-Arg-Trp-Cys)-ß-Ala-Arg-Lys-Arg-Arg-Arg-Gln-Arg-Arg-Arg-$NH_2$ (SEQ ID NO:144) | 0.778 | 1.22 | 0.468 | 47.0 |

TABLE 7 - Binding Constants for Formula (V) Examples

Table 7A

| Formula (V) Compounds | Ki hMC1 | Ki hMC3 | Ki hMC4 | Ki hMC5 |
|---|---|---|---|---|
| c[Hydantoin(C(O)-(Cys-D-Ala))-His-D-Phe-Arg-Trp-Cys]-$NH_2$ (SEQ ID NO:271) | 230 | 7590 | 126 | 7020 |
| c[Hydantoin(C(O)-(Glu-D-Ala))-His-D-Phe-Arg-Trp-Lys]-$NH_2$ (SEQ ID NO:276) | 72.6 | 1920 | 45.2 | >10000 |
| c[Hydantoin(C(O)-(Glu-D-Ala))-His-D-Phe-Arg-Trp-Orn]-$NH_2$ (SEQ ID NO:276) | 60.4 | 2840 | 52.4 | >10000 |
| c[Hydantoin(C(O)-(Glu-D-Ala))-His-D-Phe-Arg-Trp-Dab]-$NH_2$ (SEQ ID NO:276) | 28 | 90.5 | 12.7 | 877 |
| c[Hydantoin(C(O)-(Glu-D-Ala))-His-D-Phe-Arg-Trp-Dap]-$NH_2$ (SEQ ID NO:276) | 16.4 | 863 | 4.97 | >10000 |
| c[Hydantoin(C(O)-(Asp-D-Ala))-His-D-Phe-Arg-Trp-Orn]-$NH_2$ (SEQ ID NO:273) | 37.7 | 576 | 7.81 | 6400 |
| c[Hydantoin(C(O)-(Asp-D-Ala))-His-D-Phe-Arg-Trp-Dap]-$NH_2$ (SEQ ID NO:273) | 66.6 | 1820 | 19.9 | >10000 |

(continued)

| Formula (V) Compounds | Ki hMC1 | Ki hMC3 | Ki hMC4 | Ki hMC5 |
|---|---|---|---|---|
| c[Hydantoin(C(O)-(Asp-His))-D-2-Nal-Arg-Trp-Lys]-$NH_2$ (SEQ ID NO:275) | 200 | 68.8 | 6.63 | 142 |
| c[Hydantoin(C(O)-(Asp-Aic))-D-2-Nal-Arg-Trp-Lys]-$NH_2$ (SEQ ID NO:275) | 9028 | 2628 | 35.8 | 1156 |
| c[Hydantoin(C(O)-(Asp-A5c))-D-2-Nal-Arg-Trp-Lys]-$NH_2$ (SEQ ID NO:275) | 9938 | 2390 | 44.6 | 1103 |
| c[Hydantoin(C(O)-(Asp-A6c))-D-2-Nal-Arg-Trp-Lys]-$NH_2$ (SEQ ID NO:275) | 2170 | 1479 | 16.5 | 451 |
| c[Hydantoin(C(O)-(Asp-Apc))-D-2-Nal-Arg-Trp-Lys]-$NH_2$ (SEQ ID NO:275) | 1276 | 2756 | 266 | 1096 |
| c[Hydantoin(C(O)-(Asp-A3c))-D-2-Nal-Arg-Trp-Lys]-$NH_2$ (SEQ ID NO:275) | 7567 | 1922 | 420 | 2879 |

TABLE 7B - Binding Constants for Formula (VI) Examples

| Formula (VI) Compounds | Ki hMC1 | Ki hMC3 | Ki hMC4 | Ki hMC5 |
|---|---|---|---|---|
| Hydantoin(C(O)-(Nle-Gly))-c(Cys-Glu-His-D-Phe-Arg-Trp-Cys)-$NH_2$ (SEQ ID NO:278) | 14.3 | 198 | 5.76 | 67.8 |
| Hydantoin(C(O)-(Gly-Gly))-c(Cys-Glu-His-D-Phe-Arg-Trp-Cys)-$NH_2$ (SEQ ID NO:278) | 11.9 | 311 | 5.41 | 73.9 |
| Hydantoin(C(O)-(A6c-Nle))-c(Cys-D-Ala-His-D-Phe-Arg-Trp-Cys)-$NH_2$ (SEQ ID NO:284) | 31.6 | 224 | 19.6 | 2500 |
| Hydantoin(C(O)-(D-Ala-Nle))-c(Cys-D-Ala-His-D-Phe-Arg-Trp-Cys)-$NH_2$ (SEQ ID NO:284) | 16.0 | 63.9 | 8.64 | 1820 |
| Hydantoin(C(O)-(Val-Nle))-c(Cys-D-Ala-His-D-Phe-Arg-Trp-Cys)-$NH_2$ (SEQ ID NO:284) | 33.7 | 132 | 40.2 | 3210 |
| Hydantoin(C(O)-(Leu-Nle))-c(Cys-D-Ala-His-D-Phe-Arg-Trp-Cys)-$NH_2$ (SEQ ID NO:284) | 48.3 | 534 | 74.1 | 3290 |
| Hydantoin(C(O)-(Cha-Nle))-c(Cys-D-Ala-His-D-Phe-Arg-Trp-Cys)-$NH_2$ (SEQ ID NO:284) | 40.8 | 870 | 137 | 3560 |
| Hydantoin(C(O)-(Aib-Nle))-c(Cys-D-Ala-His-D-Phe-Arg-Trp-Cys)-$NH_2$ (SEQ ID NO:284) | 17.7 | 73.6 | 8.40 | 2120 |
| Hydantoin(C(O)-(Nle-Gly))-c(Cys-D-Ala-His-D-Phe-Arg-Trp-Cys)-$NH_2$ (SEQ ID NO:279) | 7.92 | 46.4 | 6.70 | 21.3 |
| Hydantoin(C(O)-(Gly-Gly))-c(Cys-D-Ala-His-D-Phe-Arg-Trp-Cys)-$NH_2$ (SEQ ID NO:279) | 20.9 | 69.7 | 8.32 | 50.0 |

(continued)

| Formula (VI) Compounds | Ki hMC1 | Ki hMC3 | Ki hMC4 | Ki hMC5 |
|---|---|---|---|---|
| Hydantoin(C(O)-(Nle-Gly))-c(Cys-D-Ala-His-D-Phe-Arg-Trp-Pen)-$NH_2$ (SEQ ID NO:280) | 12.9 | 38.5 | 3.53 | 27.1 |
| Hydantoin(C(O)-(Gly-Gly))-c(Cys-D-Ala-His-D-Phe-Arg-Trp-Pen)-$NH_2$ (SEQ ID NO:280) | 127 | 811 | 10.4 | 381 |
| Hydantoin(C(O)-(Ala-Gly))-c(Cys-D-Ala-His-D-Phe-Arg-Trp-Cys)-$NH_2$ (SEQ ID NO:279) | 13.9 | 38.4 | 5.73 | 18.9 |
| Hydantoin(C(O)-(D-Ala-Gly))-c(Cys-D-Ala-His-D-Phe-Arg-Trp-Cys)-$NH_2$ (SEQ ID NO:279) | 11.7 | 73.1 | 4.28 | 34.7 |
| Hydantoin(C(O)-(Aib-Gly))-c(Cys-D-Ala-His-D-Phe-Arg-Trp-Cys)-$NH_2$ (SEQ ID NO:279) | 36.8 | 290 | 13.7 | 133 |
| Hydantoin(C(O)-(Val-Gly))-c(Cys-D-Ala-His-D-Phe-Arg-Trp-Cys)-$NH_2$ (SEQ ID NO:279) | 15.3 | 160 | 8.66 | 33.4 |
| Hydantoin(C(O)-(Ile-Gly))-c(Cys-D-Ala-His-D-Phe-Arg-Trp-Cys)-$NH_2$ (SEQ ID NO:279) | 11.6 | 194 | 11.5 | 28.9 |
| Hydantoin(C(O)-(Leu-Gly))-c(Cys-D-Ala-His-D-Phe-Arg-Trp-Cys)-$NH_2$ (SEQ ID NO:279) | 19.3 | 331 | 26.7 | 44.6 |
| Hydantoin(C(O)-(D-Arg-Gly))-c(Cys-Glu-His-D-Phe-Arg-Trp-Cys)-$NH_2$ (SEQ ID NO:278) | 9.49 | 124 | 2.95 | 2260 |
| Hydantoin(C(O)-(Gly-D-Arg))-c(Cys-Glu-His-D-Phe-Arg-Trp-Cys)-$NH_2$ (SEQ ID NO:287) | 4.30 | 78.0 | 1.77 | 4540 |
| Hydantoin(C(O)-(Arg-Gly))-c(Cys-Glu-His-D-Phe-Arg-Trp-Cys)-$NH_2$ (SEQ ID NO:278) | 8.59 | 94.1 | 2.44 | 7760 |
| Hydantoin(C(O)-(Gly-Arg))-c(Cys-Glu-His-D-Phe-Arg-Trp-Cys)-$NH_2$ (SEQ ID NO:285) | 5.68 | 55.5 | 2.44 | 4220 |
| Hydantoin(C(O)-(Arg-Gly))-c(Cys-D-Ala-His-D-Phe-Arg-Trp-Cys)-$NH_2$ (SEQ ID NO:279) | 2.65 | 41.3 | 4.17 | 650 |
| Hydantoin(C(O)-(D-Arg-Gly))-c(Cys-D-Ala-His-D-Phe-Arg-Trp-Cys)-$NH_2$ (SEQ ID NO:279) | 3.52 | 48.7 | 5.78 | 872 |
| Hydantoin(C(O)-(Gly-D-Arg))-c(Cys-D-Ala-His-D-Phe-Arg-Trp-Cys)-$NH_2$ (SEQ ID NO288) | 3.51 | 29.2 | 6.04 | 914 |
| Hydantoin(C(O)-(Gly-Arg))-c(Cys-D-Ala-His-D-Phe-Arg-Trp-Cys)-$NH_2$ (SEQ ID NO:286) | 1.14 | 01.7 | 4.53 | 783 |
| Hydantoin(C(O)-(Arg-Gly))-c(Cys-D-Ala-His-D-2-Nal-Arg-Trp-Cys)-$NH_2$ (SEQ ID NO:282) | 11.9 | 7.43 | 0.195 | 14.6 |

TABLE 7C - Binding Constants for Formula (VII) Examples

| Formula (VII) Compounds | Ki hMC1 | Ki hMC3 | Ki hMC4 | Ki hMC5 |
|---|---|---|---|---|
| c[Hydantoin(C(O)-(Aib-Cys))-D-Ala-His-D-Phe-Arg-Trp-Cys]-$NH_2$ (SEQ ID NO:290) | 47.6 | 1100 | 47.1 | >10000 |
| c[Hydantoin(C(O)-(Val-Cys))-D-Ala-His-D-Phe-Arg-Trp-Cys]-$NH_2$ (SEQ ID NO:290) | 21.2 | 730 | 34.5 | >10000 |
| c[Hydantoin(C(O)-(Leu-Cys))-D-Ala-His-D-Phe-Arg-Trp-Cys]-$NH_2$ (SEQ ID NO:290) | 47.4 | 1550 | 27.9 | >10000 |
| c[Hydantoin(C(O)-(Ile-Cys))-D-Ala-His-D-Phe-Arg-Trp-Cys]-$NH_2$ (SEQ ID NO:290) | 53.4 | 1760 | 41.6 | >10000 |
| c[Hydantoin(C(O)-(A6c-Cys))-D-Ala-His-D-Phe-Arg-Trp-Cys]-$NH_2$ (SEQ ID NO:290) | 38.5 | 1760 | 53.2 | 9270 |
| c[Hydantoin(C(O)-(Gly-Cys))-Glu-His-D-Phe-Arg-Trp-Cys]-$NH_2$ (SEQ ID NO:291) | 15.6 | 305 | 8.92 | 3070 |

TABLE 8-Radioligand Binding Assay Data for Selected Compounds

| Compound | Ki hMC1-R | Ki hMC3-R | Ki hMC4-R | Ki hMC5-R |
|---|---|---|---|---|
| Ac-Tyr-Arg-c(Cys-D-Ala-His-D-Phe-Arg-Trp-Cys)-$NH_2$ (SEQ ID NO:292) | 8.53 | 21.2 | 3.72 | 714 |
| Ac-2-Nal-Arg-c(Cys-D-Ala-His-D-Phe-Arg-Trp-Cys)-$NH_2$ (SEQ ID NO:292) | 6.09 | 34.9 | 2.02 | 864 |
| Ac-1-Nal-Arg-c(Cys-D-Ala-His-D-Phe-Arg-Trp-Cys)-$NH_2$ (SEQ ID NO:292) | 6.27 | 36.4 | 1.53 | 888 |
| Ac-Phe-Arg-c(Cys-D-Ala-His-D-Phe-Arg-Trp-Cys)-$NH_2$ (SEQ ID NO:292) | 1.48 | 14.8 | 2.34 | 491 |
| Ac-Trp-Arg-c(Cys-D-Ala-His-D-Phe-Arg-Trp-Cys)-$NH_2$ (SEQ ID NO:292) | 4.7 | 42 | 2.25 | 1470 |
| Ac-Pff-Arg-c(Cys-D-Ala-His-D-Phe-Arg-Trp-Cys)-$NH_2$ (SEQ ID NO:292) | 0.323 | 1.33 | 1.95 | 786 |

***Melanocortin Functional Activity and Selectivity***

The compounds described herein will interact preferentially (i.e., selectively) to MC-4 relative to the other melanocortin receptors. Selectivity is particularly important when the compounds are administered to humans or other animals to minimize the number of side effects associated with their administration. MC-4 selectivity of a compound is defined herein as the ratio of the $EC_{50}$ of the compound for an MC-1 receptor ($EC_{50}$ -MC-1) over the $EC_{50}$ of the compound for the MC-3 ($EC_{50}$ -MC-3)/MC-4 ($EC_{50}$ -MC-4) receptor, the $EC_{50}$ values being measured as described above. The formulas are as follows:

$$MC\text{-}3\ selectivity = [EC_{50}\text{-}MC\text{-}1]/[EC_{50}\text{-}MC\text{-}3]$$

$$MC\text{-}4\ selectivity = [EC_{50}\text{-}MC\text{-}1]/[EC_{50}\text{-}MC\text{-}4]$$

A compound is defined herein as being "selective for the MC-3 receptor" when the above mentioned ratio "MC-3-selectivity" is at least about 10, preferably at least about 100, and more preferably at least about 500.

A compound is defined herein as being "selective for the MC-4 receptor" when the above mentioned ratio "MC-4-selectivity" is at least about 10, preferably at least about 100, and more preferably at least about 500.

One skilled in the art would know that procedures similar to those described herein may be used to assay the binding activities of the compounds to melanocortin receptor molecules.

***cyclic AMP Bioassay***

Intracellular cyclic AMP (cAMP) levels were determined by an electrochemiluminescence (ECL) assay (Meso Scale Discovery®, Gaithersburg, MD; referred to hereinafter as MSD). CHO-K1 cells stably expressing the hMC receptor subtypes were suspended in RMPI 1640® assay buffer (RMPI 1640 buffer contains 0.5 mM isobutylmethylxanthine (IBMX), and 0.2% protein cocktail (MSD blocker A)). Transgenic CHO-K1 cells stably expressing hMC receptor subtypes 1, 3, 4 or 5 were dispensed at a density of approximately 7,000 cells/well in 384-well Multi-Array® plates (MSD) containing integrated carbon electrodes and coated with anti-cAMP antibody. Increasing concentrations of the test compounds were added and the cells were incubated for approximately 40 minutes at approximately 37°C. Following this incubation, lysis buffer (HEPES-buffered saline solution with MgCh and Triton X-100® at ph 7.3) containing 0.2% protein cocktail and 2.5 nM TAG™ ruthenium-labeled cAMP (MSD) was added and the cells were incubated for approximately 90 minutes at room temperature. At the end of the second incubation period read buffer (Tris-buffered solution containing an ECL co-reactant and Triton X-100 at ph 7.8) was added and the cAMP levels in the cell lysates were immediately determined by ECL detection with a Sector Imager 6000 reader® (MSD). Data were analyzed using a computer-assisted non-linear regression analysis (XL fit; IDBS) and reported as either an $EC_{50}$ value or a Kb value.

$EC_{50}$ represents the concentration of an agonist compound needed to obtain 50% of the maximum reaction response, *e.g.,* 50% of the maximum level of cAMP as determined using the assay described above. The Kb value reflects the potency of an antagonist and is determined by Schild analysis. In brief, concentration-response curves of an agonist are carried out in the presence of increasing concentrations of an antagonist. The Kb value is the concentration of antagonist which would produce a 2-fold shift in the concentration-response curve for an agonist. It is calculated by extrapolating the line on a Schild plot to zero on the y-axis.

A selection of compounds was tested using the above-discussed assays and the results are reported in Tables 9, 10, 11, and 12.

TABLE 9 - cAMP Bioassay Data for Selected Compounds

Table 9A

| Compound | $EC_{50}$ hMC1-R | $EC_{50}$ hMC3-R | $EC_{50}$ hMC4-R | $EC_{50}$ hMC5-R | $EC_{50}$ hMC1-R /MC4-R | SEQ ID NO: |
|---|---|---|---|---|---|---|
| Ac-Arg-c(Cys-D-Ala-His-D-Phe-Arg-Trp-Cys)-$NH_2$ | 5.79 | 5.25 | 0.313 | 1630 | 18.0 | SEQ ID NO:50 |
| Ac-D-Arg-c(Cys-D-Ala-His-D-Phe-Arg-Trp-Cys)-$NH_2$ | 6.17 | 5.6 | 0.397 | 1020 | 16.0 | SEQ ID NO:50 |
| Ac-D-Arg-c(Cys-D-Ala-His-D-Phe-Arg-Trp-Pen)-$NH_2$ | 26.5 | 10.5 | 0.493 | 2440 | 54.0 | SEQ ID NO:51 |

(continued)

| Compound | $EC_{50}$ hMC1-R | $EC_{50}$ hMC3-R | $EC_{50}$ hMC4-R | $EC_{50}$ hMC5-R | $EC_{50}$ hMC1-R /MC4-R | SEQ ID NO: |
|---|---|---|---|---|---|---|
| Ac-D-Arg-c(Cys-His-D-Phe-Arg-Trp-Gaba-Pen)-$NH_2$ | 8.43 | 32.4 | 0.959 | 2140 | 9.0 | SEQ ID NO:52 |
| Ac-Arg-c(Cys-His-D-Phe-Arg-Trp-Gaba-Pen)-$NH_2$ | 4.23 | 8.09 | 0.719 | 23.2 | 6.0 | SEQ ID NO:52 |
| Ac-Arg-c(Cys-D-Ala-His-D-Phe-Arg-Trp-Pen)-$NH_2$ | 48.3 | 13.3 | 0.79 | 10000 | 61.0 | SEQ ID NO:51 |
| Ac-D-Arg-c(Asp-His-D-Phe-Arg-Trp-Ala-Lys)-$NH_2$ | 1.48 | 5.76 | 0.078 | 297 | 19.0 | SEQ ID NO:53 |
| Ac-Arg-c(Asp-His-D-Phe-Arg-Trp-Ala-Lys)-$NH_2$ | 1.39 | 2.89 | 0.055 | 467 | 25.0 | SEQ ID NO:53 |
| ND = not determined | | | | | | |

Table 9B

| Compound | $EC_{50}$ hMC 1-R | $EC_{50}$ hMC 3-R | $EC_{50}$ hMC4-R | $EC_{50}$ hMC5-R | $EC_{50}$ hMC1-R /MC4-R | SEQ ID NO: |
|---|---|---|---|---|---|---|
| Ac-Nle-c(Cys-D-Ala-His-D-Phe-Arg-Trp-Cys)-$NH_2$ | 2.4 | 0.33 | 0.078 | 420 | 31 | SEQ ID NO:7 |
| D-Phe-c(Cys-His-D-(Et)Tyr-Arg-Trp-β-Ala-D-Cys)-Thr-$NH_2$ | 0.35 | 1.1 | 0.11 | 0.37 | 3 | SEQ ID NO:24 |
| Nle-c(Cys-His-D-Phe-Arg-Trp-Apn-Cys)-$NH_2$ | 0.31 | 0.27 | 0.018 | 3.1 | 17 | SEQ ID NO:27 |
| Ac-Nle-c(Cys-His-D-Phe-Arg-Trp-Gaba-Pen)-$NH_2$ | 0.28 | 0.24 | 0.028 | 3.9 | 10 | SEQ ID NO:32 |
| Nle-c(Cys-His-D-Phe-Arg-Trp-Gaba-Cys)-$NH_2$ | 0.37 | 0.1 | 0.021 | 1.7 | 18 | SEQ ID NO:34 |
| Ac-Nle-c(Asp-His-D-Phe-Arg-Trp-β-Ala-Lys)-$NH_2$ | 0.834 | 0.145 | 0.128 | 2.79 | 6.52 | SEQ ID NO:1 |

(continued)

| Compound | $EC_{50}$ hMC 1-R | $EC_{50}$ hMC 3-R | $EC_{50}$ hMC4-R | $EC_{50}$ hMC5-R | $EC_{50}$ hMC1-R /MC4-R | SEQ ID NO: |
|---|---|---|---|---|---|---|
| Ac-Nle-c(Cys-His-D-Phe-Arg-Trp-Apn-Cys)-$NH_2$ | 0.76 | 0.199 | 0.0492 | 1.73 | 15.45 | SEQ ID NO:2 |
| Ac-Cha-c(Asp-His-D-Phe-Arg-Trp-Gaba-Lys)-$NH_2$ | 3.26 | 0.189 | 0.0949 | 30.2 | 34.35 | SEQ ID NO:6 |
| Ac-Nle-c(Asp-His-D-Phe-Arg-Trp-Gaba-Lys)-$NH_2$ | 1.37 | 0.628 | 0.131 | 3.48 | 10.46 | SEQ ID NO:6 |
| Ac-hCha-c(Asp-His-D-Phe-Arg-Trp-Gaba-Lys)-$NH_2$ | 2.27 | 3.32 | 7.24 | 415 | 0.31 | SEQ ID NO:11 |
| Ac-Nle-c(Pen-D-Ala-His-D-Phe-Arg-Trp-Cys)-$NH_2$ | ND | 1.89 | 0.531 | ND | ND | SEQ ID NO:21 |
| Ac-Nle-c(Cys-D-Ala-His-D-Phe-Arg-Trp-Pen)-$NH_2$ | 14.3 | 2.03 | 0.183 | 2240 | 78.14 | SEQ ID NO:22 |
| D-Phe-c(Cys-His-D-(Et)Tyr-hArg-Trp-β-Ala-D-Cys)-Thr-$NH_2$ | 0.345 | 2.71 | 5376 | 2.38 | 0.06 | SEQ ID NO:24 |
| D-Phe-c(Cys-His-D-(Et)Tyr-hArg-Bip-β-Ala-D-Cys)-Thr-$NH_2$ | 0.685 | 81.8 | 86.9 | 31.8 | 0.01 | SEQ ID NO:26 |
| Ac-Nle-c(Asp-D-Ala-His-D-Phe-Arg-Bal-Lys)-$NH_2$ | 0.931 | 3.22 | 1.65 | >10000 | 0.56 | SEQ ID NO:28 |
| Ac-Nle-c(Cys-D-Leu-His-D-Phe-Arg-Trp-Cys)-$NH_2$ | 3.24 | 0.465 | 0.0915 | 78.5 | 35.41 | SEQ ID NO:30 |
| Ac-Nle-c(Cys-D-Cha-His-D-Phe-Arg-Trp-Cys)-$NH_2$ | 0.819 | 0.541 | 0.453 | 45.3 | 1.81 | SEQ ID NO:30 |
| ND = not determined | | | | | | |

Table 9C

| Compound | EC50 hMC 1-R | Kb hMC3-R | Kb MC4-R | EC50 hMC5-R | SEQ ID NO: |
|---|---|---|---|---|---|
| Ac-Nle-c(Cys-D-Ala-His-D-2-Nal-Arg-Trp-Cys)-$NH_2$ | 17.6 | 12.4 | 38.8 | 11.8 | SEQ ID NO:16 |
| Ac-Nle-c(Asp-His-D-2-Nal-Arg-Trp-Ala-Lys)-$NH_2$ | 0.619 | 2.98 | 0.109 | 0.189 | SEQ ID NO:38 |
| Ac-Nle-c(Asp-His-D-2-Nal-Arg-Trp-β-Ala-Lys)-$NH_2$ | 0.913 | 0.536 | 0.346 | 0.489 | SEQ ID NO:38 |
| Ac-Nle-c(Cys-His-D-2-Nal-Arg-Trp-Gaba-Cys)-$NH_2$ | 0.231 | 18.4 | 0.782 | 0.153 | SEQ ID NO:39 |
| Ac-Nle-c(Cys-His-D-2-Nal-Arg-Trp-Ahx-Cys)-$NH_2$ | 0.581 | 10.8 | 0.967 | 0.126 | SEQ ID NO:39 |
| Ac-hPhe-c(Asp-His-D-2-Nal-Arg-Trp-Gaba-Lys)-$NH_2$ | 0.413 | 9.32 | 0.824 | 0.307 | SEQ ID NO:40 |
| Ac-Cha-c(Asp-His-D-2-Nal-Arg-Trp-Gaba-Lys)-NH2 | 1.27 | 3.02 | 0.442 | 0.736 | SEQ ID NO:40 |
| Ac-Nle-c(Cys-D-Ala-His-D-2-Nal-Arg-1-Nal-Cys)-$NH_2$ | 383 | 61.5 | 53.6 | 2842 | SEQ ID NO:16 |

Table 9D

| Compound | EC50 hMC 1-R | Kb hMC3-R | Kb MC4-R | EC50 hMC5-R | SEQ ID NO: |
|---|---|---|---|---|---|
| Ac-Arg-c(Cys-D-Ala-His-D-2-Nal-Arg-Trp-Cys)-$NH_2$ | 193 | 5.72 | 1.58 | 1111 | SEQ ID NO:49 |

Table 10A

| Compound | $EC_{50}$ hMC1-R | Kb hMC3-R | Kb hMC4-R | $EC_{50}$ hMC5-R |
|---|---|---|---|---|
| Ac-c(Cys-Glu-His-D-2-Nal-Arg-Trp-Ala-Cys)-Pro-Pro-Lys-Asp-$NH_2$ (SEQ ID NO:269) | 66.1 | 33.4 | 0.687 | 6.84 |
| Tyr-Gly-Arg-Lys-Lys-Arg-Arg-Gln-Arg-Arg-Arg-Nle-c(Asp-His-D-2-Nal-Arg-Trp-Lys)-$NH_2$ (SEQ ID NO:60) | ND | 4500 | 105 | ND |
| Tyr-Gly-Arg-Lys-Lys-Arg-Arg-Gln-Arg-Arg-Arg-Doc-Nle-c(Asp-His-D-2-Nal-Arg-Trp-Lys)-$NH_2$ (SEQ ID NO:61) | ND | 395 | 16.8 | ND |
| Nle-c(Asp-His-D-2-Nal-Arg-Trp-Lys)-ß-Ala-Tyr-Gly-Arg-Lys-Lys-Arg-Arg-Gln-Arg-Arg-Arg-$NH_2$ (SEQ ID NO:62) | ND | 207 | 18.5 | ND |

(continued)

| Compound | $EC_{50}$ hMC1-R | Kb hMC3-R | Kb hMC4-R | $EC_{50}$ hMC5-R |
|---|---|---|---|---|
| Ac-Nle-c(Asp-His-D-2-Nal-Arg-Trp-Lys)-ß-Ala-Tyr-Gly-Arg-Lys-Lys-Arg-Arg-Gln-Arg-Arg-Arg-$NH_2$ (SEQ ID NO:62) | ND | 220 | 4.07 | ND |
| H-Nle-c(Asp-His-D-2-Nal-Arg-Trp-Lys)-Doc-Doc-Tyr-Gly-Arg-Lys-Lys-Arg-Arg-Gln-Arg-Arg-Arg-$NH_2$ (SEQ ID NO:63) | ND | 261 | 3.11 | ND |
| Ac-Nle-c(Asp-His-D-2-Nal-Arg-Trp-Lys)-Pro-Pro-Lys-Asp-Tyr-Gly-Arg-Lys-Lys-Arg-Arg-Gln-Arg-Arg-Arg-$NH_2$ (SEQ ID NO:64) | ND | 14.1 | 22.8 | ND |
| Ac-c(Cys-Glu-His-D-2-Nal-Arg-Trp-Gly-Cys)-Pro-Pro-Lys-Asp-Tyr-Gly-Arg-Lys-Lys-Arg-Arg-Gln-Arg-Arg-Arg-$NH_2$ (SEQ ID NO:65) | ND | 233 | 26.0 | ND |
| Ac-Nle-c(Asp-His-D-2-Nal-Arg-Trp-Lys)-ß-Ala-ß-Ala-Tyr-Gly-Arg-Lys-Lys-Arg-Arg-Gln-Arg-Arg-Arg-$NH_2$ (SEQ ID NO:66) | 1.39 | 16.2 | 7.94 | 0.839 |
| Ac-Nle-c(Asp-His-D-2-Nal-Arg-Trp-Lys)-Pro-Pro-Lys-Asp-Doc-Tyr-Gly-Arg-Lys-Lys-Arg-Arg-Gln-Arg-Arg-Arg-$NH_2$ (SEQ ID NO:67) | 3.65 | 19.4 | 3.73 | 1.61 |
| Ac-c(Cys-Glu-His-D-2-Nal-Arg-Trp-Gly-Cys)-Pro-Pro-Lys-Asp-Doc-Tyr-Gly-Arg-Lys-Lys-Arg-Arg-Gln-Arg-Arg-Arg-$NH_2$ (SEQ ID NO:68) | ND | 17.7 | 1.49 | ND |
| Ac-c(Cys-Glu-His-D-2-Nal-Arg-Trp-Ala-Cys)-Pro-Pro-Lys-Asp-ß-Ala-Tyr-Gly-Arg-Lys-Lys-Arg-Arg-Gln-Arg-Arg-Arg-$NH_2$(SEQ ID NO:69) | 6.3 | 70.0 | 1.66 | 38.2 |
| Ac-c(Cys-Glu-His-D-2-Nal-Arg-Trp-Ala-Cys)-Pro-Pro-Lys-Asp-Doc-Tyr-Gly-Arg-Lys-Lys-Arg-Arg-Gln-Arg-Arg-Arg-$NH_2$ (SEQ ID NO:69) | 12.1 | 30.3 | 1.81 | 70.0 |
| Ac-c(Cys-Glu-His-D-2-Nal-Arg-2-Nal-Ala-Cys)-Pro-Pro-Lys-Asp-$NH_2$ (SEQ ID NO:269) | 33.6 | 140 | 12.2 | 66.9 |
| Ac-c(Cys-Glu-His-D-2-Nal-Arg-1-Nal-Ala-Cys)-Pro-Pro-Lys-Asp-$NH_2$ (SEQ ID NO:269) | 269 | 105 | 5.92 | 104 |

(continued)

| Compound | $EC_{50}$ hMC1-R | Kb hMC3-R | Kb hMC4-R | $EC_{50}$ hMC5-R |
|---|---|---|---|---|
| Ac-c Cys-Glu-His-D-2-Nal-Arg-Bal-Ala-Cys)-Pro-Pro-Lys-Asp-$NH_2$ (SEQ ID NO:269) | 690 | 70.7 | 4.56 | 177 |
| Ac-Nle-c(Asp-His-D-2-Nal-Arg-Trp-Lys)-Doc-Doc-Tyr-Gly-Arg-Lys-Lys-Arg-Arg-Gln-Arg-Arg-Arg-$NH_2$ (SEQ ID NO:70) | 3.23 | 8.97 | 4.61 | 2.86 |
| Ac-c(Cys-Glu-His-D-2-Nal-Arg-Bal-Ala-Cys)-Pro-Pro-Lys-Asp-ß-Ala-Arg-Arg-Arg-Arg-Arg-Gln-Arg-Arg-Arg-$NH_2$ (SEQ ID NO:72) | 52.0 | 170 | 6.14 | 328 |
| Ac-c(Cys-Glu-His-D-2-Nal-Arg-Bal-Ala-Cys)-Pro-Pro-Lys-Asp-ß-Ala-Gly-Arg-Arg-Arg-Arg-Arg-Gln-Arg-Arg-Arg-$NH_2$ (SEQ ID NO:73) | 146 | 104 | 32.0 | 1400 |
| Ac-c(Cys-Glu-His-D-2-Nal-Arg-Bal-Ala-Cys)-Pro-Pro-Lys-Asp-ß-Ala-Tyr-Gly-Arg-Arg-Arg-Arg-Arg-Gln-Arg-Arg-Arg-$NH_2$ (SEQ ID NO:74) | 114 | 44.6 | 28.4 | 879 |
| Ac-c(Cys-Glu-His-D-2-Nal-Arg-Bal-Ala-Cys)-Pro-Pro-Lys-Asp-ß-Ala-Tyr-Aib-Arg-Lys-Lys-Arg-Arg-Gln-Arg-Arg-Arg-$NH_2$ (SEQ ID NO:79) | 67.1 | 439 | 46.5 | 582 |
| Ac-c(Cys-Glu-His-D-2-Nal-Arg-1-Nal-Ala-Cys)-Pro-Pro-Arg-Asp-ß-Ala-Arg-Arg-Arg-Arg-Arg-Gln-Arg-Arg-Arg-$NH_2$ (SEQ ID NO:80) | 144 | 116 | 8.93 | 819 |
| Ac-c(Cys-Glu-His-D-2-Nal-Arg-1-Nal-Ala-Cys)-Pro-Pro-Lys-Asp-ß-Ala-Arg-Arg-Arg-Arg-Arg-Gln-Arg-Arg-Arg-$NH_2$ (SEQ ID NO:80) | 36.0 | 46.5 | 11.4 | 56.1 |
| Ac-c(Cys-Glu-His-D-2-Nal-Arg-1-Nal-Ala-Cys)-Pro-Pro-Lys-Asp-ß-Ala-Arg-Arg-Arg-Arg-Arg-Arg-Gln-Arg-Arg-Arg-$NH_2$ (SEQ ID NO:81) | 93.0 | 71 | 15.9 | > 10000 |
| Ac-c(Cys-Glu-His-D-2-Nal-Arg-2-Nal-Ala-Cys)-Pro-Pro-Arg-Asp-ß-Ala-Arg-Arg-Arg-Arg-Arg-Gln-Arg-Arg-Arg-$NH_2$ (SEQ ID NO:82) | 39.7 | 30.9 | 6.66 | 501 |

(continued)

| Compound | $EC_{50}$ hMC1-R | Kb hMC3-R | Kb hMC4-R | $EC_{50}$ hMC5-R |
|---|---|---|---|---|
| Ac-c(Cys-Glu-His-D-2-Nal-Arg-2-Nal-Ala-Cys)-Pro-Pro-Lys-Asp-ß-Ala-Arg-Arg-Arg-Arg-Arg-Gln-Arg-Arg-Arg-$NH_2$ (SEQ ID NO:82) | 35.2 | 22.9 | 12.6 | 199 |
| Ac-c(Cys-Glu-His-D-2-Nal-Arg-2-Nal-Ala-Cys)-Pro-Pro-Lys-Asp-ß-Ala-Arg-Arg-Arg-Arg-Arg-Arg-Gln-Arg-Arg-Arg-$NH_2$ (SEQ ID NO:81) | 29.1 | 13.6 | 13.4 | 204 |
| Ac-c(Cys-Glu-His-D-2-Nal-Arg-Bal-Ala-Cys)-Pro-Pro-Arg-Asp-ß-Ala-Arg-Arg-Arg-Arg-Arg-Arg-Gln-Arg-Arg-Arg-$NH_2$ (SEQ ID NO:83) | 86.1 | 41.7 | 19.4 | 2360 |
| Ac-c(Cys-Glu-His-D-2-Nal-Arg-Bal-Ala-Cys)-Pro-Pro-Arg-Asp-ß-Ala-Arg-Arg-Arg-Arg-Arg-Gln-Arg-Arg-Arg-$NH_2$ (SEQ ID NO:84) | 38.3 | 20.2 | 21.2 | > 10000 |
| Ac-c(Cys-Glu-His-D-2-Nal-Arg-Bal-Ala-Cys)-Pro-Pro-Lys-Asp-ß-Ala-Arg-Arg-Arg-Arg-Arg-Arg-Gln-Arg-Arg-Arg-$NH_2$ (SEQ ID NO:83) | 68.6 | 153 | 33.2 | > 10000 |
| Ac-c(Cys-Glu-His-D-2-Nal-Arg-1-Nal-Ala-Cys)-Pro-Pro-Lys-Asp-ß-Ala-Arg-Arg-Lys-Arg-Arg-Gln-Arg-Arg-Arg-$NH_2$ (SEQ ID NO:88) | 70.4 | 286 | 18.6 | > 10000 |
| Ac-c(Cys-Glu-His-D-2-Nal-Arg-1-Nal-Ala-Cys)-Pro-Pro-Lys-Asp-ß-Ala-Arg-Lys-Arg-Arg-Arg-Gln-Arg-Arg-Arg-$NH_2$ (SEQ ID NO:89) | 33.1 | 65.1 | 15.3 | 1720 |
| Ac-c(Cys-Glu-His-D-2-Nal-Arg-2-Nal-Ala-Cys)-Pro-Pro-Lys-Asp-ß-Ala-Arg-Arg-Lys-Arg-Arg-Gln-Arg-Arg-Arg-$NH_2$ (SEQ ID NO:88) | 88.2 | 10.6 | 17.4 | 514 |
| Ac-c(Cys-Glu-His-D-2-Nal-Arg-2-Nal-Ala-Cys)-Pro-Pro-Lys-Asp-ß-Ala-Tyr-Gly-Arg-Arg-Lys-Arg-Arg-Gln-Arg-Arg-Arg-$NH_2$ (SEQ ID NO:90) | 58.7 | 39.3 | 10.3 | 460 |
| Ac-c(Cys-Glu-His-D-2-Nal-Arg-2-Nal-Ala-Cys)-Pro-Pro-Lys-Asp-ß-Ala-Gly-Arg-Arg-Lys-Arg-Arg-Gln-Arg-Arg-Arg-$NH_2$ (SEQ ID NO:91) | 45.4 | 12.7 | 12.7 | 162 |

(continued)

| Compound | $EC_{50}$ hMC1-R | Kb hMC3-R | Kb hMC4-R | $EC_{50}$ hMC5-R |
|---|---|---|---|---|
| Ac-c(Cys-Glu-His-D-2-Nal-Arg-2-Nal-Ala-Cys)-Pro-Pro-Lys-Asp-ß-Ala-Gly-Arg-Lys-Arg-Arg-Arg-Gln-Arg-Arg-Arg-$NH_2$ (SEQ ID NO:92) | 309 | 22.8 | 17.1 | 570 |
| Ac-Nle-c(Cys-D-Ala-His-D-Phe-Arg-Trp-Cys)-ß-Ala-Tyr-Gly-Arg-Arg-Arg-Arg-Arg-Gln-Arg-Arg-Arg-$NH_2$ (SEQ ID NO:139) | 7.86 | 10.5 | 0.843 | 4900 |
| Ac-c(Cys-Glu-His-D-2-Nal-Arg-2-Nal-Ala-Cys)-Pro-Pro-Lys-Asp-ß-Ala-Tyr-Gly-Arg-Lys-Lys-Arg-Arg-Gln-Arg-Arg-Arg-$NH_2$ (SEQ ID NO:71) | 29.7 | 25.6 | 7.37 | 82.9 |
| Ac-c(Cys-Glu-His-D-2-Nal-Arg-Trp-Ala-Cys)-Pro-Pro-Lys-Asp-βAla-Tyr-Gly-Arg-Lys-Lys-Arg-Gln-Arg-Arg-Arg-Arg-$NH_2$ (SEQ ID NO:75) | 15.2 | 14.6 | 4.52 | 36.8 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Gln-Arg-Arg-Arg-Arg-Arg-$NH_2$ (SEQ ID NO:76) | 6.7 | 9.38 | 11.7 | 46.2 |
| Ac-c(Cys-Glu-His-D-2-Nal-Arg-Trp-Ala-Cys)-Pro-Pro-Lys-Asp-ß-Ala-Tyr-Gly-Arg-Lys-Gln-Lys-Arg-Arg-Arg-Arg-Arg-$NH_2$ (SEQ ID NO:77) | 7.9 | 41.7 | 10.9 | 62.4 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Arg-Arg-Arg-Arg-Gln-Arg-$NH_2$ (SEQ ID NO:78) | 16.9 | 36.0 | 7.12 | 58.9 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Arg-Arg-Arg-Gln-Arg-Arg-$NH_2$ (SEQ ID NO:85) | 16.4 | 20.8 | 7.31 | 44.2 |
| Ac-c(Cys-Glu-His-D-2-Nal-Arg-Trp-Ala-Cys)-Pro-Pro-Lys-Asp-ß-Ala-Tyr-Gly-Arg-Gln-Lys-Lys-Arg-Arg-Arg-Arg-Arg-$NH_2$ (SEQ ID NO:86) | 12.0 | 13.7 | 9.38 | 54.2 |
| Ac-c(Cys-Glu-His-D-2-Nal-Arg-Trp-Ala-Cys)-Pro-Pro-Lys-Asp-ß-Ala-Tyr-Gly-Arg-Lys-Lys-Arg-Arg-Arg-Arg-Arg-Gln-$NH_2$(SEQ ID NO:87) | 7.5 | 12.2 | 7.61 | 51.7 |

(continued)

| Compound | $EC_{50}$ hMC1-R | Kb hMC3-R | Kb hMC4-R | $EC_{50}$ hMC5-R |
|---|---|---|---|---|
| Ac-c(Cys-Glu-His-D-2-Nal-Arg-1-Nal-Ala-Cys)-Pro-Pro-Lys-Asp-ß-Ala-Tyr-Gly-Arg-Lys-Lys-Arg-Arg-Gln-Arg-Arg-Arg-$NH_2$ (SEQ ID NO:71) | 43.3 | 215 | 5.87 | 1286 |
| Ac-c(Cys-Glu-His-D-2-Nal-Arg-Bal-Ala-Cys)-Pro-Pro-Lys-Asp-ß-Ala-Tyr-Gly-Arg-Lys-Lys-Arg-Arg-Gln-Arg-Arg-Arg-$NH_2$ (SEQ ID NO:71) | 37.9 | 112 | 41.1 | 1798 |
| ND = not determined | | | | |

Table 10B

| **Compound** | **EC50 hMC1-R** | **EC50 hMC3-R** | **EC50 hMC4-R** | **EC50 hMC5-R** |
|---|---|---|---|---|
| Ac-Nle-c(Cys-D-Ala-His-D-Phe-Arg-Trp-Cys)-(Doc)2-Tyr-Gly-Arg-Lys-Lys-Arg-Arg-Gln-Arg-Arg-Arg-$NH_2$ (SEQ ID NO:136) | 4.70 | 4.56 | 0.634 | 147 |
| Ac-Nle-c(Cys-D-Ala-His-D-Phe-Arg-Trp-Cys)-ß-Ala-Tyr-Gly-Arg-Lys-Lys-Arg-Gln-Arg-Arg-Arg-Arg-$NH_2$ (SEQ ID NO:137) | 5.90 | 7.73 | 1.02 | 2890 |
| Ac-Nle-c(Cys-D-Ala-His-D-Phe-Arg-Trp-Cys)-ß-Ala-Gly-Arg-Arg-Arg-Arg-Arg-Gln-Arg-Arg-Arg-$NH_2$ (SEQ ID NO:136) | 0.481 | 7.32 | 0.964 | 2010 |
| Ac-Nle-c(Cys-D-Ala-His-D-Phe-Arg-Trp-Cys)-ß-Ala-Arg-Arg-Arg-Arg-Arg-Gln-Arg-Arg-Arg-$NH_2$ (SEQ ID NO:138) | 7.15 | 9.37 | 1.25 | 1570 |

Table 10C

| **Compound** | **$EC_{50}$ hMC1-R** | **Kb hMC3-R** | **Kb hMC4-R** | **$EC_{50}$ hMC5-R** |
|---|---|---|---|---|
| Ac-c(Cys-Glu-His-D-2-Nal-Arg-2-Nal-ß-Ala-Cys)-Pro-Pro-Lys-Asp-$NH_2$ (SEQ ID NO:270) | ND | ND | ND | ND |
| Ac-c(Cys-Glu-His-D-2-Nal-Arg-2-Nal-Aib-Cys)-Pro-Pro-Lys-Asp-$NH_2$ (SEQ ID NO:270) | 770 | 221 | 4.52 | 869 |

(continued)

| Compound | $EC_{50}$ hMC1-R | Kb hMC3-R | Kb hMC4-R | $EC_{50}$ hMC5-R |
|---|---|---|---|---|
| Ac-c(Cys-Glu-His-D-2-Nal-Arg-1-Nal-Ala-Cys)-Pro-Pro-Lys-Asp-ß-Ala-Tyr-Gly-Arg-Arg-Lys-Arg-Arg-Gln-Arg-Arg-Arg-$NH_2$ (SEQ ID NO:95) | 29 | 22.6 | 16.7 | 173 |
| Ac-c(Cys-Glu-His-D-2-Nal-Arg-1-Nal-Ala-Cys)-Pro-Pro-Lys-Asp-ß-Ala-Tyr-Gly-Arg-Lys-Arg-Arg-Arg-Gln-Arg-Arg-Arg-$NH_2$ (SEQ ID NO:96) | 102 | 26.3 | 14.6 | 261 |
| Ac-c(Cys-Glu-His-D-2-Nal-Arg-1-Nal-Ala-Cys)-Pro-Pro-Lys-Asp-ß-Ala-Gly-Arg-Arg-Lys-Arg-Arg-Gln-Arg-Arg-Arg-$NH_2$ (SEQ ID NO:97) | 26.6 | 101 | 9.34 | 351 |
| Ac-c(Cys-Glu-His-D-2-Nal-Arg-1-Nal-Ala-Cys)-Pro-Pro-Lys-Asp-ß-Ala-Gly-Arg-Lys-Arg-Arg-Arg-Gln-Arg-Arg-Arg-$NH_2$(SEQ ID NO:92) | 45.5 | 181 | 6.35 | 149 |
| Ac-c(Cys-Glu-His-D-2-Nal-Arg-2-Nal-Ala-Cys)-Pro-Pro-Lys-Asp-ß-Ala-Arg-Lys-Arg-Arg-Arg-Gln-Arg-Arg-Arg-$NH_2$ (SEQ ID NO:99) | 23.7 | 9.22 | 5.87 | 39.7 |
| Ac-c(Cys-Glu-His-D-2-Nal-Arg-2-Nal-Ala-Cys)-Pro-Pro-Lys-Asp-ß-Ala-Tyr-Gly-Arg-Lys-Arg-Arg-Arg-Gln-Arg-Arg-Arg-$NH_2$ (SEQ ID NO:101) | 34.7 | 15.0 | 8.68 | 28.2 |
| Ac-c(Cys-Glu-His-D-2-Nal-Arg-Bal-Ala-Cys)-Pro-Pro-Lys-Asp-ß-Ala-Arg-Lys-Arg-Arg-Arg-Gln-Arg-Arg-Arg-$NH_2$ (SEQ ID NO:105) | 19.1 | 106 | 4.59 | 100 |
| Ac-c(Cys-Glu-His-D-2-Nal-Arg-Bal-Ala-Cys)-Pro-Pro-Lys-Asp-ß-Ala-Tyr-Gly-Arg-Arg-Lys-Arg-Arg-Gln-Arg-Arg-Arg-$NH_2$(SEQ ID NO:100) | 19.8 | 37.8 | 8.43 | 158 |
| Ac-c(Cys-Glu-His-D-2-Nal-Arg-Bal-Ala-Cys)-Pro-Pro-Lys-Asp-ß-Ala-Tyr-Gly-Arg-Lys-Arg-Arg-Arg-Gln-Arg-Arg-Arg-$NH_2$ (SEQ ID NO:101) | 11.2 | 52.1 | 9.45 | 95.7 |

(continued)

| Compound | $EC_{50}$ hMC1-R | Kb hMC3-R | Kb hMC4-R | $EC_{50}$ hMC5-R |
|---|---|---|---|---|
| Ac-c(Cys-Glu-His-D-2-Nal-Arg-Bal-Ala-Cys)-Pro-Pro-Lys-Asp-ß-Ala-Gly-Arg-Arg-Lys-Arg-Arg-Gln-Arg-Arg-Arg-$NH_2$ (SEQ ID NO:102) | 33.8 | 93.6 | 4.42 | 89.5 |
| Ac-c(Cys-Glu-His-D-2-Nal-Arg-Bal-Ala-Cys)-Pro-Pro-Lys-Asp-ß-Ala-Arg-Arg-Lys-Arg-Arg-Gln-Arg-Arg-Arg-$NH_2$(SEQ ID NO:104) | 232 | 68.8 | 10.0 | 250 |
| Ac-c(Cys-Glu-His-D-2-Nal-Arg-Bal-Ala-Cys)-Pro-Pro-Lys-Asp-ß-Ala-Gly-Arg-Lys-Arg-Arg-Arg-Gln-Arg-Arg-Arg-$NH_2$ (SEQ ID NO:103) | 32.2 | 98.3 | 5.23 | 194 |
| ND = not determined | | | | |

Table 10D

| Compound | EC50 hMC1-R | EC50 hMC3-R | EC50 hMC4-R | EC50 hMC5-R |
|---|---|---|---|---|
| Ac-Nle-c(Cys-D-Ala-His-D-Phe-Arg-Trp-Cys)-ß-Ala-Tyr-Gly-Arg-Arg-Lys-Arg-Arg-Gln-Arg-Arg-Arg-$NH_2$ (SEQ ID NO:141) | 5.66 | 4.70 | 0.422 | 1551 |
| Ac-Nle-c(Cys-D-Ala-His-D-Phe-Arg-Trp-Cys)-ß-Ala-Tyr-Gly-Arg-Lys-Arg-Arg-Arg-Gln-Arg-Arg-Arg-$NH_2$ (SEQ ID NO:142) | 7.57 | 4.18 | 0.600 | 1792 |
| Ac-Nle-c(Cys-D-Ala-His-D-Phe-Arg-Trp-Cys)-ß-Ala-Gly-Arg-Arg-Lys-Arg-Arg-Gln-Arg-Arg-Arg-$NH_2$ (SEQ ID NO:141) | 2.36 | 2.74 | 0.260 | 500 |
| Ac-Nle-c(Cys-D-Ala-His-D-Phe-Arg-Trp-Cys)-ß-Ala-Gly-Arg-Lys-Arg-Arg-Arg-Gln-Arg-Arg-Arg-$NH_2$ (SEQ ID NO:142) | 2.81 | 3.29 | 0.298 | 566 |
| Ac-Nle-c(Cys-D-Ala-His-D-Phe-Arg-Trp-Cys)-ß-Ala-Arg-Arg-Lys-Arg-Arg-Gln-Arg-Arg-Arg-$NH_2$ (SEQ ID NO:143) | 1.86 | 1.39 | 0.367 | 165 |
| Ac-Nle-c(Cys-D-Ala-His-D-Phe-Arg-Trp-Cys)-ß-Ala-Arg-Lys-Arg-Arg-Arg-Gln-Arg-Arg-Arg-$NH_2$ (SEQ ID NO:144) | 2.06 | 1.61 | 0.394 | 199 |

TABLE 11 - Intracellular Cyclic AMP (cAMP) Levels for Formula (I) Examples

Table 11A

| Formula (V) Compounds | $EC_{50}$ hMC1 | $EC_{50}$ hMC3 | $EC_{50}$ hMC4 | $EC_{50}$ hMC5 |
|---|---|---|---|---|
| c[Hydantoin(C(O)-(Cys-D-Ala))-His-D-Phe-Arg-Trp-Cys]-$NH_2$ (SEQ ID NO:271) | - | 218 | 5.42 | - |
| c[Hydantoin(C(O)-(Glu-D-Ala))-His-D-Phe-Arg-Trp-Lys]-$NH_2$ (SEQ ID NO:276) | - | 22.3 | 3.62 | - |
| c[Hydantoin(C(O)-(Glu-D-Ala))-His-D-Phe-Arg-Trp-Orn]-$NH_2$ (SEQ ID NO:276) | - | 39.2 | 4.94 | - |
| c[Hydantoin(C(O)-(Glu-D-Ala))-His-D-Phe-Arg-Trp-Dap]-$NH_2$ (SEQ ID NO:276) | 56.7 | 18.2 | 0.182 | >10000 |
| c[Hydantoin(C(O)-(Asp-D-Ala))-His-D-Phe-Arg-Trp-Orn]-$NH_2$ (SEQ ID NO:273) | 56.6 | 88.6 | 4.50 | 9300 |
| c[Hydantoin(C(O)-(Asp-D-Ala))-His-D-Phe-Arg-Trp-Dap]-$NH_2$ (SEQ ID NO:273) | - | 49.3 | 2.12 | - |

TABLE 11B -

| Formula (VI) Compounds | $EC_{50}$ hMC1 | $EC_{50}$ hMC3 | $EC_{50}$ hMC4 | $EC_{50}$ hMC5 |
|---|---|---|---|---|
| Hydantoin(C(O)-(Nle-Gly))-c(Cys-Glu-His-D-Phe-Arg-Trp-Cys)-$NH_2$ (SEQ ID NO:278) | 54.3 | 12.2 | 0.177 | >10000 |
| Hydantoin(C(O)-(Gly-Gly))-c(Cys-Glu-His-D-Phe-Arg-Trp-Cys)-$NH_2$ (SEQ ID NO:278) | 124 | 8.05 | 0.214 | >10000 |
| Hydantoin(C(O)-(A6c-Nle))-c(Cys-D-Ala-His-D-Phe-Arg-Trp-Cys)-$NH_2$ (SEQ ID NO:284) | - | 4.89 | 1.80 | - |
| Hydantoin(C(O)-(D-Ala-Nle))-c(Cys-D-Ala-His-D-Phe-Arg-Trp-Cys)-$NH_2$ (SEQ ID NO:284) | - | 2.56 | 1.47 | - |
| Hydantoin(C(O)-(Val-Nle))-c(Cys-D-Ala-His-D-Phe-Arg-Trp-Cys)-$NH_2$ (SEQ ID NO:284) | - | 4.61 | 0.977 | - |
| Hydantoin(C(O)-(Leu-Nle))-c(Cys-D-Ala-His-D-Phe-Arg-Trp-Cys)-$NH_2$ (SEQ ID NO:284) | - | 9.68 | 1.83 | - |
| Hydantoin(C(O)-(Cha-Nle))-c(Cys-D-Ala-His-D-Phe-Arg-Trp-Cys)-$NH_2$ (SEQ ID NO:284) | - | 9.97 | 13.9 | - |

(continued)

| Formula (VI) Compounds | $EC_{50}$ hMC1 | $EC_{50}$ hMC3 | $EC_{50}$ hMC4 | $EC_{50}$ hMC5 |
|---|---|---|---|---|
| Hydantoin(C(O)-(Gly-Gly))-c(Cys-D-Ala-His-D-Phe-Arg-Trp-Cys)-$NH_2$ (SEQ ID NO:279) | 14.2 | 2.46 | 0.336 | 201 |
| Hydantoin(C(O)-(Nle-Gly))-c(Cys-D-Ala-His-D-Phe-Arg-Trp-Pen)-$NH_2$ (SEQ ID NO:280) | 17.0 | 21.5 | 0.584 | 352 |
| Hydantoin(C(O)-(Gly-Gly))-c(Cys-D-Ala-His-D-Phe-Arg-Trp-Pen)-$NH_2$ (SEQ ID NO:280) | 40.2 | 8.90 | 0.495 | 8300 |
| Hydantoin(C(O)-(Ala-Gly))-c(Cys-D-Ala-His-D-Phe-Arg-Trp-Cys)-$NH_2$ (SEQ ID NO:279) | 17.6 | 2.23 | 0.241 | 516 |
| Hydantoin(C(O)-(D-Ala-Gly))-c(Cys-D-Ala-His-D-Phe-Arg-Trp-Cys)-$NH_2$ (SEQ ID NO:279) | 4.70 | 2.22 | 0.309 | 355 |
| Hydantoin(C(O)-(D-Arg-Gly))-c(Cys-Glu-His-D-Phe-Arg-Trp-Cys)-$NH_2$ (SEQ ID NO:278) | 18.0 | 17.1 | 0.160 | 2710 |
| Hydantoin(C(O)-(Gly-D-Arg))-c(Cys-Glu-His-D-Phe-Arg-Trp-Cys)-$NH_2$ (SEQ ID NO:287) | 12.9 | 10.3 | 0.125 | 7440 |
| Hydantoin(C(O)-(Arg-Gly))-c(Cys-Glu-His-D-Phe-Arg-Trp-Cys)-$NH_2$ (SEQ ID NO:278) | 8.83 | 7.86 | 0.0979 | 4010 |
| Hydantoin(C(O)-(Gly-Arg))-c(Cys-Glu-His-D-Phe-Arg-Trp-Cys)-$NH_2$ (SEQ ID NO:285) | 9.97 | 3.63 | 0.0687 | 335 |
| Hydantoin(C(O)-(Arg-Gly))-c(Cys-D-Ala-His-D-Phe-Arg-Trp-Cys)-$NH_2$ (SEQ ID NO:279) | 8.81 | 18.2 | 0.503 | 3560 |
| Hydantoin(C(O)-(D-Arg-Gly))-c(Cys-D-Ala-His-D-Phe-Arg-Trp-Cys)-$NH_2$ (SEQ ID NO:279) | 11.5 | 23.2 | 0.513 | 3950 |
| Hydantoin(C(O)-(Gly-D-Arg))-c(Cys-D-Ala-His-D-Phe-Arg-Trp-Cys)-$NH_2$ (SEQ ID NO:288) | 7.53 | 11.6 | 0.435 | 9840 |
| Hydantoin(C(O)-(Gly-Arg))-c(Cys-D-Ala-His-D-Phe-Arg-Trp-Cys)-$NH_2$ (SEQ ID NO:286) | 8.85 | 5.17 | 0.599 | 3610 |

(continued)

| Formula (VI) Compounds | $EC_{50}$ hMC1 | $EC_{50}$ hMC3 | $EC_{50}$ hMC4 | $EC_{50}$ hMC5 |
|---|---|---|---|---|
| Hydantoin(C(O)-(Arg-Gly))-c(Cys-D-Ala-His-D-2-Nal-Arg-Trp-Cys)-$NH_2$ (SEQ ID NO:282) | 96.6 | 13.1 | 21.2 | 103 |

TABLE 11C

| Formula (VII) Compounds | $EC_{50}$ hMC1 | $EC_{50}$ hMC3 | $EC_{50}$ hMC4 | $EC_{50}$ hMC5 |
|---|---|---|---|---|
| c[Hydantoin(C(O)-(Aib-Cys))-D-Ala-His-D-Phe-Arg-Trp-Cys]-$NH_2$ (SEQ ID NO:290) | - | 6.28 | 0.407 | - |
| c[Hydantoin(C(O)-(Val-Cys))-D-Ala-His-D-Phe-Arg-Trp-Cys]-$NH_2$ (SEQ ID NO:290) | - | 3.77 | 0.214 | - |
| c[Hydantoin(C(O)-(Leu-Cys))-D-Ala-His-D-Phe-Arg-Trp-Cys]-$NH_2$ (SEQ ID NO:290) | - | 4.72 | 0.428 | - |
| c[Hydantoin(C(O)-(Ile-Cys))-D-Ala-His-D-Phe-Arg-Trp-Cys]-$NH_2$ (SEQ ID NO:290) | - | 8.51 | 1.85 | - |
| c[Hydantoin(C(O)-(A6c-Cys))-D-Ala-His-D-Phe-Arg-Trp-Cys]-$NH_2$ (SEQ ID NO:290) | - | 5.66 | 1.72 | - |
| c[Hydantoin(C(O)-(Gly-Cys))-Glu-His-D-Phe-Arg-Trp-Cys]-$NH_2$ (SEQ ID NO:291) | 14.5 | 21.8 | 0.576 | 1780 |

TABLE 12-cAMP Bioassay Data for Selected Compounds

| Compound | $EC_{50}$ hMC1-R | $EC_{50}$ hMC3-R | $EC_{50}$ hMC4-R | $EC_{50}$ hMC5-R |
|---|---|---|---|---|
| Ac-Tyr-Arg-c(Cys-D-Ala-His-D-Phe-Arg-Trp-Cys)-$NH_2$ (SEQ ID NO:292) | 6.42 | 2.39 | 0.194 | 1540 |
| Ac-2-Nal-Arg-c(Cys-D-Ala-His-D-Phe-Arg-Trp-Cys)-$NH_2$ (SEQ ID NO:292) | 9.66 | 6.11 | 0.275 | 1730 |
| Ac-1-Nal-Arg-c(Cys-D-Ala-His-D-Phe-Arg-Trp-Cys)-$NH_2$ (SEQ ID NO:292) | 8.67 | 4.21 | 0.363 | 1320 |
| Ac-Trp-Arg-c(Cys-D-Ala-His-D-Phe-Arg-Trp-Cys)-$NH_2$ (SEQ ID NO:292) | 5.78 | 3.95 | 0.219 | 2580 |

## *In vivo* studies

Compounds herein can be and were tested for an effect upon insulin resistance and/or body weight according to the following procedures. One skilled in the art would know that procedures similar to those described herein may be

used to assay the effect of the compounds upon insulin resistance and/or body weight.

Ligand compounds activating melanocortin receptors tested in the *in vivo* studies were as follows (Table 13):

Table 13

| Ligand Code | Structure |
|---|---|
| Compound A | Ac-Arg-c(Cys-D-Ala-His-D-Phe-Arg-Trp-Cys)-$NH_2$ SEQ ID NO:50 |
| Compound B | Hydantoin(C(O)-(Arg-Gly))-c(Cys-Glu-His-D-Phe-Arg-Trp-Cys)-$NH_2$ (SEQ ID NO:278) |

**Acute feeding experiments (fasting)**

Male Sprague Dawley rats (250g) were housed in individual cages and maintained under 12:12 hour light:dark conditions. The rats were fasted for 18 hours prior to the start of the experiment with water available *ad libitum.* At time 0, the rats were injected subcutaneously (sc) with selected compounds at doses of 100 nmole/kg, or with vehicle, and were provided with food. Individual food consumption was measured at about 2, 4 and 6 hours after injection. Data for selected compounds are reported in Figure 1.

**Chronic feeding experiments**

Male, Sprague Dawley rats that had been fed either a normal diet (300g; Research Diets 12450) or a high fat diet (400g; Research Diets 12451) for 10 weeks prior to the start of the experiment were housed in individual cages and maintained under 12:12 hour light:dark conditions with both food and water available ad libitum. The rats were anesthetized and implanted subcutaneously with an osmotic mini pump (Alzet, Cupertino, CA). The pumps delivered either Compound A or Compound B at doses of 75, 300 or 1200 nmole/kg/day, or vehicle for 7 days. Individual body weight and food consumption were measured daily.

On day 7 rats were anesthetized and fit with a jugular-right atrial cannula. On day 8 an iv glucose tolerance test was performed and blood samples were withdrawn into heparinized syringes at time -10 and 0. Immediately after the time 0 blood sample, the rats were injected with glucose (1g/kg) via the indwelling cannula. Subsequent blood samples were withdrawn at 2.5, 5, 10, 20 and 40 minutes later. Plasma levels of glucose (Diagnostic Chemicals Limited) and insulin (Alpco) were determined by commercially available kits. Results are showin in Figures 2A-D and 3A-D.

**Glucose tolerance tests**

Male, C57BL/6 mice that had been fed either a normal diet (30g; Research Diets 12450) or a high fat diet (45g; Research Diets 12452) for 12 weeks prior to the start of the experiment were housed in individual cages and maintained under 12:12 hour light:dark conditions with both food and water available ad libitum. The mice were anesthetized and implanted subcutaneously with an osmotic mini pump (Alzet, Cupertino, CA). The pumps delivered Compound A at doses of 200, 600 or 1800 nmole/kg/day, or vehicle for 14 days. On day 14 the mice were fasted for 18 hours or overnight. On day 15 an glucose tolerance test was performed by injecting the mice with glucose (2g/kg) ip. Blood samples were taken by tail stick at 0, 15, 30, 60 and 180 minutes after the glucose injection and blood glucose level was measured using an Accu-Chek glucometer. Results are shown in Figure 4.

Male, $Lep^{ob}/Lep^{ob}$ mice (50g) were group housed maintained under 12:12 hour light:dark conditions with both food and water available ad libitum. The mice were fasted for 18 hours or overnight and an ip glucose tolerance test was performed. Mice were injected with Compound A ip at a dose of 6.4$\mu$mole/kg at -15 minutes and a blood sample was taken by tail stick. At time 0 mice were injected ip with glucose (1g/kg) and blood samples were taken by tail stick at 0, 15, 30, 60 and 90 minutes later and blood glucose level was measured using Glucometer Elite XL (Bayer Corporation). Results are shown in Figure 5.

***Administration and Use***

The peptides herein can be provided in the form of pharmaceutically acceptable salts. Examples of such salts include, but are not limited to, those formed with organic acids (e.g., acetic, lactic, maleic, citric, malic, ascorbic, succinic, benzoic, methane sulfonic, toluene sulfonic, or pamoic acid), inorganic acids (e.g., hydrochloric acid, sulfuric acid, or phosphoric acid), and polymeric acids (e.g., tannic acid, carboxymethyl cellulose, polylactic, polyglycolic, or copolymers of polylactic-glycolic acids). A typical method of making a salt of a peptide oherein is well known in the art and can be accomplished by standard methods of salt exchange. Accordingly, the TFA salt of a peptide herein (the TFA salt results from the purification of the peptide by using preparative HPLC, eluting with TFA containing buffer solutions) can be

converted into another salt, such as an acetate salt, by dissolving the peptide in a small amount of 0.25 N acetic acid aqueous solution. The resulting solution is applied to a semi-prep HPLC column (Zorbax®, 300 SB, C-8). The column is eluted with: (1) 0.1N ammonium acetate aqueous solution for 0.5 hours; (2) 0.25N acetic acid aqueous solution for 0.5 hours; and (3) a linear gradient (20% to 100% of solution B over 30 minutes) at a flow rate of 4 ml/min (solution A is 0.25N acetic acid aqueous solution; solution B is 0.25N acetic acid in acetonitrile/water, 80:20). The fractions containing the peptide are collected and lyophilized to dryness.

As is well known to those skilled in the art, the known and potential uses of peptides with melanocortin receptor (MC-R) agonist or antagonist activity is varied and multitudinous, thus the administration of the compounds herein for purposes of eliciting an agonist effect can have the same effects and uses as melanocortin itself.

Accordingly, the present disclosure includes within its scope pharmaceutical compositions comprising, as an active ingredient, at least one of the compounds herein in association with a pharmaceutically acceptable carrier.

The dosage of active ingredient in the compositions herein may be varied; however, it is necessary that the amount of the active ingredient be such that a suitable dosage form is obtained. The selected dosage depends upon the desired therapeutic effect, on the route of administration, and on the duration of the treatment. In general, an effective dosage for the activities described herein is in the range of $1x10^{-7}$ to 200 mg/kg/day, preferably $1x10^{-4}$ to 100 mg/kg/day which can be administered as a single dose or divided into multiple doses.

The compounds herein can be administered by oral, parenteral (e.g., intramuscular, intraperitoneal, intravenous or subcutaneous injection, or implant), nasal, vaginal, rectal, sublingual or topical routes of administration and can be formulated with pharmaceutically acceptable carriers to provide dosage forms appropriate for each route of administration.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups, the elixirs containing inert diluents commonly used in the art, such as water. Besides such inert diluents, compositions can also include adjuvants, such as wetting agents, emulsifying and suspending agents, and sweetening, flavoring and perfuming agents.

Preparations described herein for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. Preparations may be sterilized by, for example, filtration through a bacteria-retaining filter, by incorporating sterilizing agents into the compositions, by irradiating the compositions, or by heating the compositions. Preparations can also be manufactured in the form of sterile solid compositions which can be dissolved in sterile water or some other sterile injectable medium immediately before use.

Compositions for rectal or vaginal administration are preferably suppositories which may contain, in addition to the active substance, excipients such as cocoa butter or a suppository wax.

Compositions for nasal or sublingual administration are also prepared with standard excipients well known in the art.

Further, a compound herein can be administered in a sustained release composition such as those described in the following patents and patent applications. U.S. Patent No. 5,672,659 teaches sustained release compositions comprising a bioactive agent and a polyester. U.S. Patent No. 5,595,760 teaches sustained release compositions comprising a bioactive agent in a gelable form. U.S. Patent No. 5,821,221 teaches polymeric sustained release compositions comprising a bioactive agent and chitosan. U.S. Patent No. 5,916,883 teaches sustained release compositions comprising a bioactive agent and cyclodextrin. The teachings of the foregoing patents and applications are incorporated herein by reference.

## Claims

1. A therapeutically effective amount of a melanocortin receptor 4 agonist for use in the treatment of insulin resistance in a subject by peripheral administration wherein said melanocortin receptor 4 agonist is:

   Ac-Arg-c(Cys-D-Ala-His-D-Phe-Arg-Trp-Cys)-$NH_2$; SEQ ID NO:50;

   or a pharmaceutically acceptable salt thereof.

2. The therapeutically effective amount of a melanocortin receptor 4 agonist for use according to claim 1, wherein said subject is obese or overweight.

3. The therapeutically effective amount of a melanocortin receptor 4 agonist for use according to claim 1, wherein said subject is normal weight or lean.

4. The therapeutically effective amount of a melanocortin receptor 4 agonist for use according to claim 2 or 3, wherein said obese, overweight, normal weight or lean subject suffers from type II diabetes.

5. The therapeutically effective amount of a melanocortin receptor 4 agonist for use according to any one of claims 1-4, wherein said peripheral administration is oral, subcutaneous, intraperitoneal, intramuscular, intravenous, rectal, transdermal or intranasal.

6. The therapeutically effective amount of a melanocortin receptor 4 agonist for use according to claim 5, wherein said administration is continuous, hourly, four times daily, three time daily, twice daily, once daily, once every other day, twice weekly, once weekly, once every two weeks, once a month, or once every two months.

7. The therapeutically effective amount of a melanocortin receptor 4 agonist for use according to claim 6, wherein said administration is continuous.

8. The therapeutically effective amount of a melanocortin receptor 4 agonist for use according to claim 6, wherein said administration is once daily.

9. The therapeutically effective amount of a melanocortin receptor 4 agonist for use according to claim 6, wherein said administration is once weekly

10. The therapeutically effective amount of a melanocortin receptor 4 agonist for use according to claim 6, wherein said administration is once every two weeks.

11. The therapeutically effective amount of a melanocortin receptor 4 agonist for use according to claim 6, wherein said administration is once a month or once every two months.

12. The therapeutically effective amount of a melanocortin receptor 4 agonist for use according to any one of claims 1-11, wherein said peripheral administration of an effective amount of a melanocortin receptor 4 agonist to treat said insulin resistance also reduces the body weight of said subject in need thereof.

## Patentansprüche

1. Therapeutisch wirksame Menge eines Agonisten eines Melanocortin-4-Rezeptors zur Verwendung in der Behandlung von Insulinresistenz in einem Subjekt durch periphere Verabreichung, wobei der Agonist eines Melanocortin-4-Rezeptors folgendes ist: Ac-Arg-c(Cys-D-Ala-His-D-Phe-Arg-Trp-Cys)-$NH_2$; SEQ ID NO: 50; oder ein pharmazeutisch verträgliches Salz davon.

2. Therapeutisch wirksame Menge eines Agonisten eines Melanocortin-4-Rezeptors zur Verwendung nach Anspruch 1, wobei das Subjekt fettleibig oder übergewichtig ist.

3. Therapeutisch wirksame Menge eines Agonisten eines Melanocortin-4-Rezeptors zur Verwendung nach Anspruch 1, wobei das Subjekt normalgewichtig oder schlank ist.

4. Therapeutisch wirksame Menge eines Agonisten eines Melanocortin-4-Rezeptors zur Verwendung nach Anspruch 2 oder 3, wobei das fettleibige, übergewichtige, normalgewichtige oder schlanke Subjekt an Typ-2-Diabetes leidet.

5. Therapeutisch wirksame Menge eines Agonisten eines Melanocortin-4-Rezeptors zur Verwendung nach einem der Ansprüche 1 bis 4, wobei die periphere Verabreichung oral, subkutan, intraperitoneal, intramuskulär, intravenös, rektal, transdermal oder intranasal erfolgt.

6. Therapeutisch wirksame Menge eines Agonisten eines Melanocortin-4-Rezeptors zur Verwendung nach Anspruch 5, wobei die Verabreichung kontinuierlich, stündlich, viermal täglich, dreimal täglich, zweimal täglich, einmal täglich, einmal täglich jeden zweiten Tag, zweimal in der Woche, einmal wöchentlich, einmal alle zwei Wochen, einmal im Monat oder einmal alle zwei Monate erfolgt.

7. Therapeutisch wirksame Menge eines Agonisten eines Melanocortin-4-Rezeptors zur Verwendung nach Anspruch 6, wobei die Verabreichung kontinuierlich erfolgt.

8. Therapeutisch wirksame Menge eines Agonisten eines Melanocortin-4-Rezeptors zur Verwendung nach Anspruch 6, wobei die Verabreichung einmal täglich erfolgt.

9. Therapeutisch wirksame Menge eines Agonisten eines Melanocortin-4-Rezeptors zur Verwendung nach Anspruch 6, wobei die Verabreichung einmal pro Woche erfolgt.

10. Therapeutisch wirksame Menge eines Agonisten eines Melanocortin-4-Rezeptors zur Verwendung nach Anspruch 6, wobei die Verabreichung alle zwei Wochen erfolgt.

11. Therapeutisch wirksame Menge eines Agonisten eines Melanocortin-4-Rezeptors zur Verwendung nach Anspruch 6, wobei die Verabreichung einmal im Monat oder einmal alle zwei Monate erfolgt.

12. Therapeutisch wirksame Menge eines Agonisten eines Melanocortin-4-Rezeptors zur Verwendung nach einem der Ansprüche 1 bis 11, wobei die periphere Verabreichung einer wirksamen Menge eines Melanocortin-4-Rezeptors zur Behandlung von Insulinresistenz auch das Körpergewicht des die Behandlung bedürfenden Subjekts reduziert.

## Revendications

1. Quantité thérapeutiquement efficace d'un agoniste du récepteur 4 de la mélanocortine destiné à une utilisation dans le traitement de la résistance à l'insuline chez un sujet par administration périphérique dans laquelle l'agoniste du récepteur 4 de la mélanocortine est :

   Ac-Arg-c(Cys-D-Ala-His-D-Phe-Arg-Trp-Cys)-$NH_2$ ; SEQ ID NO : 50 ;

   ou un sel pharmaceutiquement acceptable de celle-ci.

2. Quantité thérapeutiquement efficace d'un agoniste du récepteur 4 de la mélanocortine destiné à une utilisation selon la revendication 1, dans laquelle ledit sujet est obèse ou en surpoids.

3. Quantité thérapeutiquement efficace d'un agoniste du récepteur 4 de la mélanocortine destiné à une utilisation selon la revendication 1, dans laquelle ledit sujet est de poids normal ou est mince.

4. Quantité thérapeutiquement efficace d'un agoniste du récepteur 4 de la mélanocortine destiné à une utilisation selon la revendication 2 ou la revendication 3, dans laquelle ledit sujet obèse, en surpoids, de poids normal ou mince souffre du diabète de type II.

5. Quantité thérapeutiquement efficace d'un agoniste du récepteur 4 de la mélanocortine destiné à une utilisation selon l'une quelconque des revendications 1 à 4, dans laquelle ladite administration périphérique est orale, sous-cutanée, intrapéritonéale, intramusculaire, intraveineuse, rectale, transdermique ou intranasale.

6. Quantité thérapeutiquement efficace d'un agoniste du récepteur 4 de la mélanocortine destiné à une utilisation selon la revendication 5, dans laquelle ladite administration est continue, horaire, quatre fois par jour, trois fois par jour, deux fois par jour, une fois par jour, une fois tous les deux jours, deux fois par semaine, une fois par semaine, toutes les deux semaines, une fois par mois, ou une fois tous les deux mois.

7. Quantité thérapeutiquement efficace d'un agoniste du récepteur 4 de la mélanocortine destiné à une utilisation selon la revendication 6, dans laquelle ladite administration est continue.

8. Quantité thérapeutiquement efficace d'un agoniste du récepteur 4 de la mélanocortine destiné à une utilisation selon la revendication 6, dans laquelle ladite administration est une fois par jour.

9. Quantité thérapeutiquement efficace d'un agoniste du récepteur 4 de la mélanocortine destiné à une utilisation selon la revendication 6, dans laquelle ladite administration est une fois par semaine.

10. Quantité thérapeutiquement efficace d'un agoniste du récepteur 4 de la mélanocortine destiné à une utilisation selon la revendication 6, dans laquelle ladite administration est une fois toutes les deux semaines.

11. Quantité thérapeutiquement efficace d'un agoniste du récepteur 4 de la mélanocortine destiné à une utilisation selon la revendication 6, dans laquelle ladite administration est une fois par mois ou une fois tous les deux mois.

**12.** Quantité thérapeutiquement efficace d'un agoniste du récepteur 4 de la mélanocortine destiné à une utilisation selon l'une quelconque des revendications 1 à 11, dans laquelle ladite administration périphérique d'une quantité efficace d'un agoniste du récepteur 4 de la mélanocortine pour traiter ladite résistance à l'insuline réduit également la masse corporelle chez ledit sujet qui en a besoin.

Figure 1

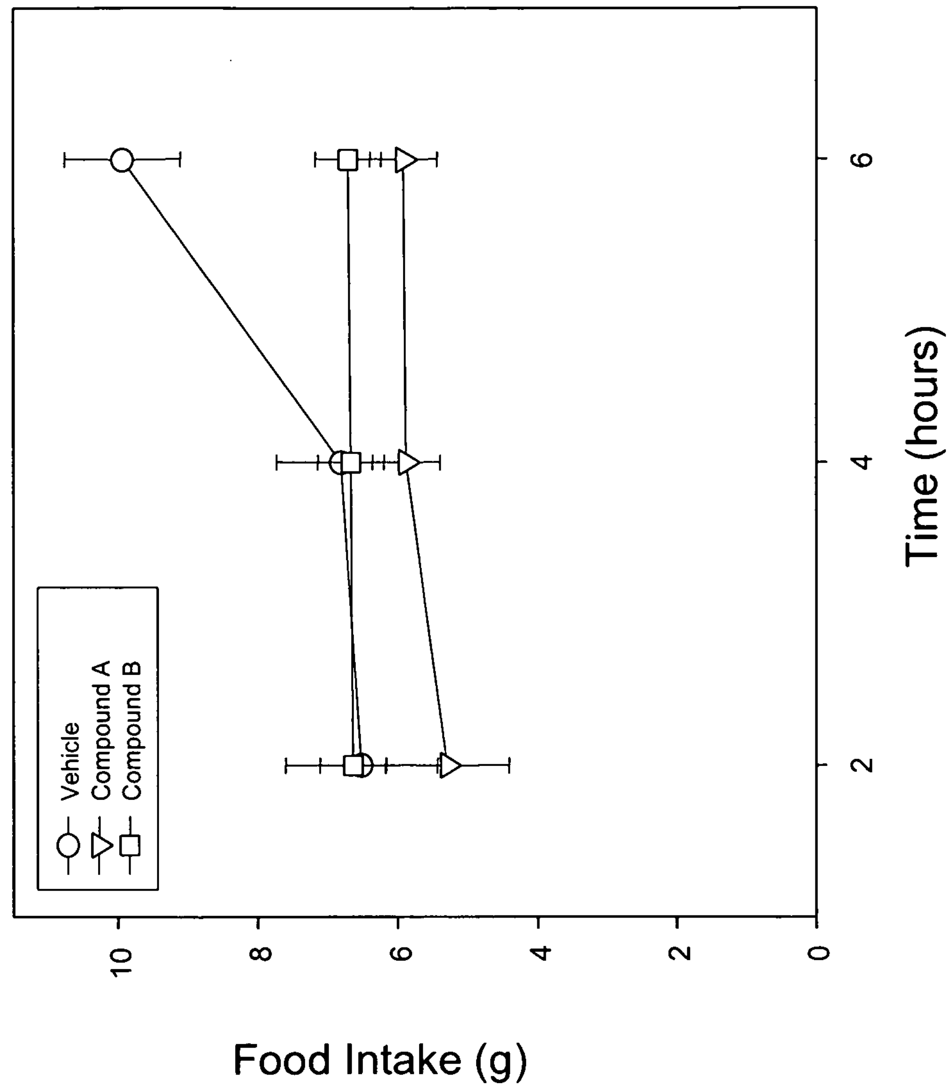
Vehicle
Compound A
Compound B
Food Intake (g)
Time (hours)
0
2
4
6
8
10
2
4
6

Figure 2A

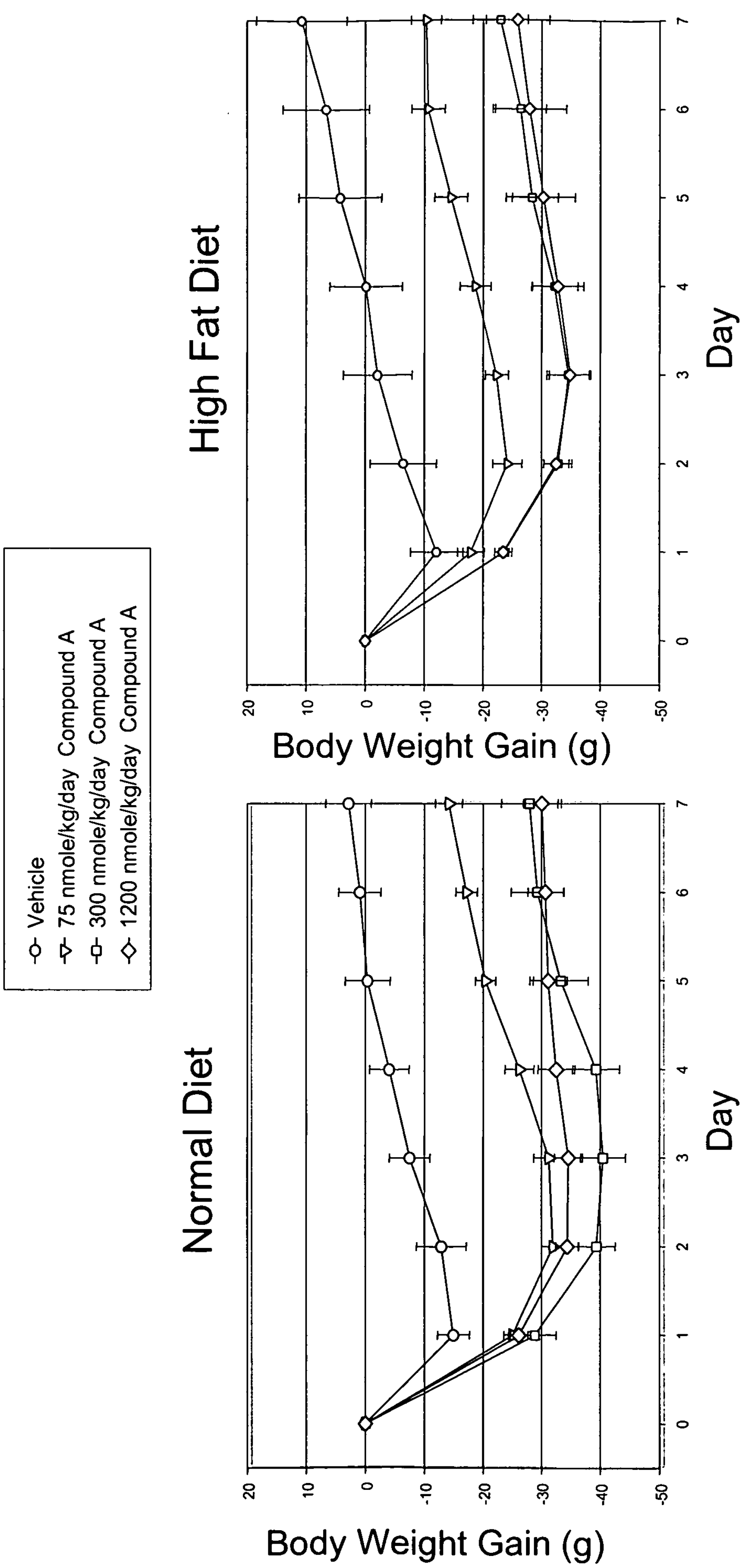
Vehicle
75 nmole/kg/day Compound A
300 nmole/kg/day Compound A
1200 nmole/kg/day Compound A
Normal Diet
High Fat Diet
Body Weight Gain (g)
Day
20
10
0
-10
-20
-30
-40
-50
0
1
2
3
4
5
6
7

Figure 2B

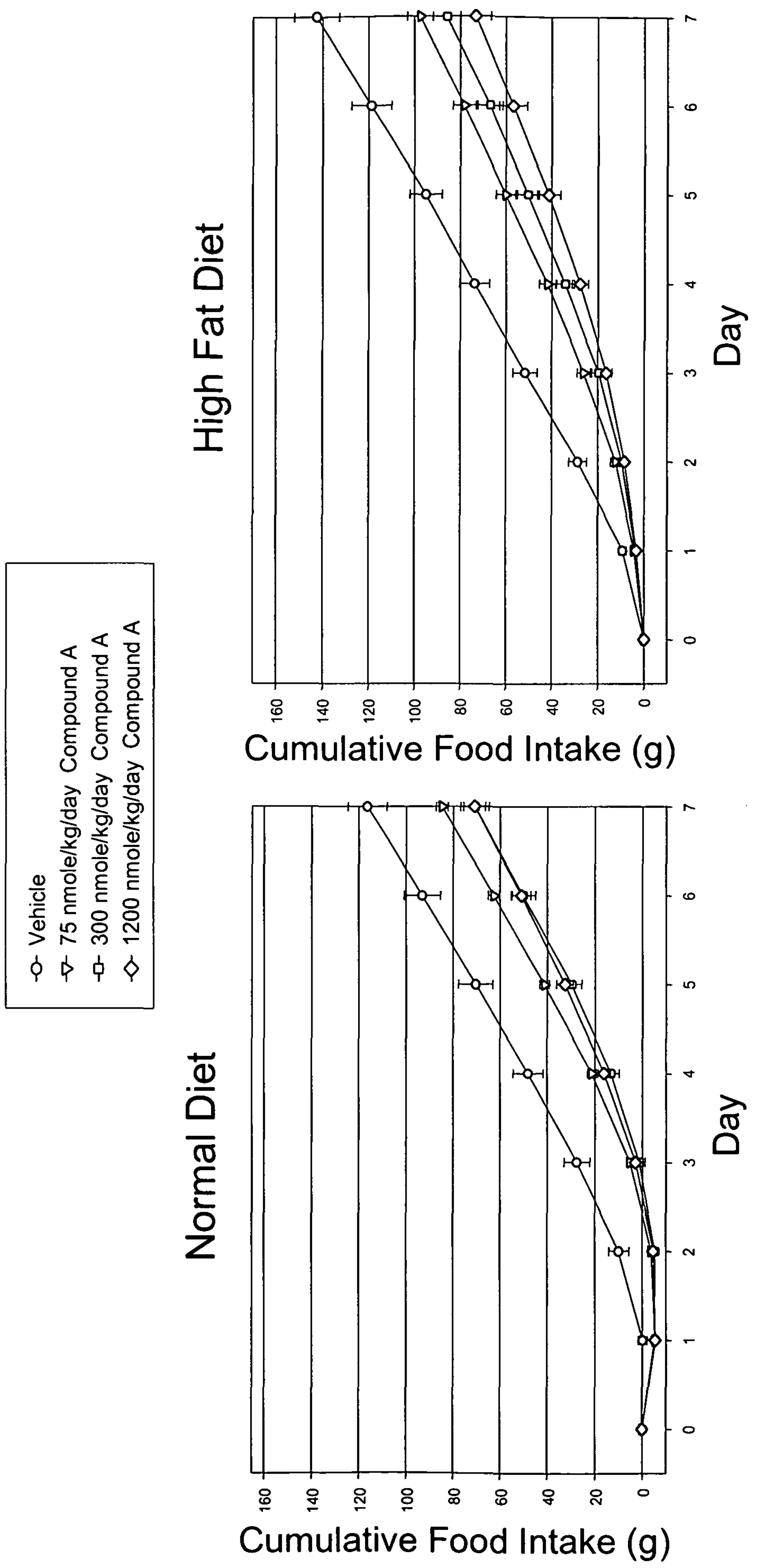
Vehicle
75 nmole/kg/day Compound A
300 nmole/kg/day Compound A
1200 nmole/kg/day Compound A
Normal Diet
High Fat Diet
Cumulative Food Intake (g)
Day
0
20
40
60
80
100
120
140
160
0
1
2
3
4
5
6
7

Figure 2C

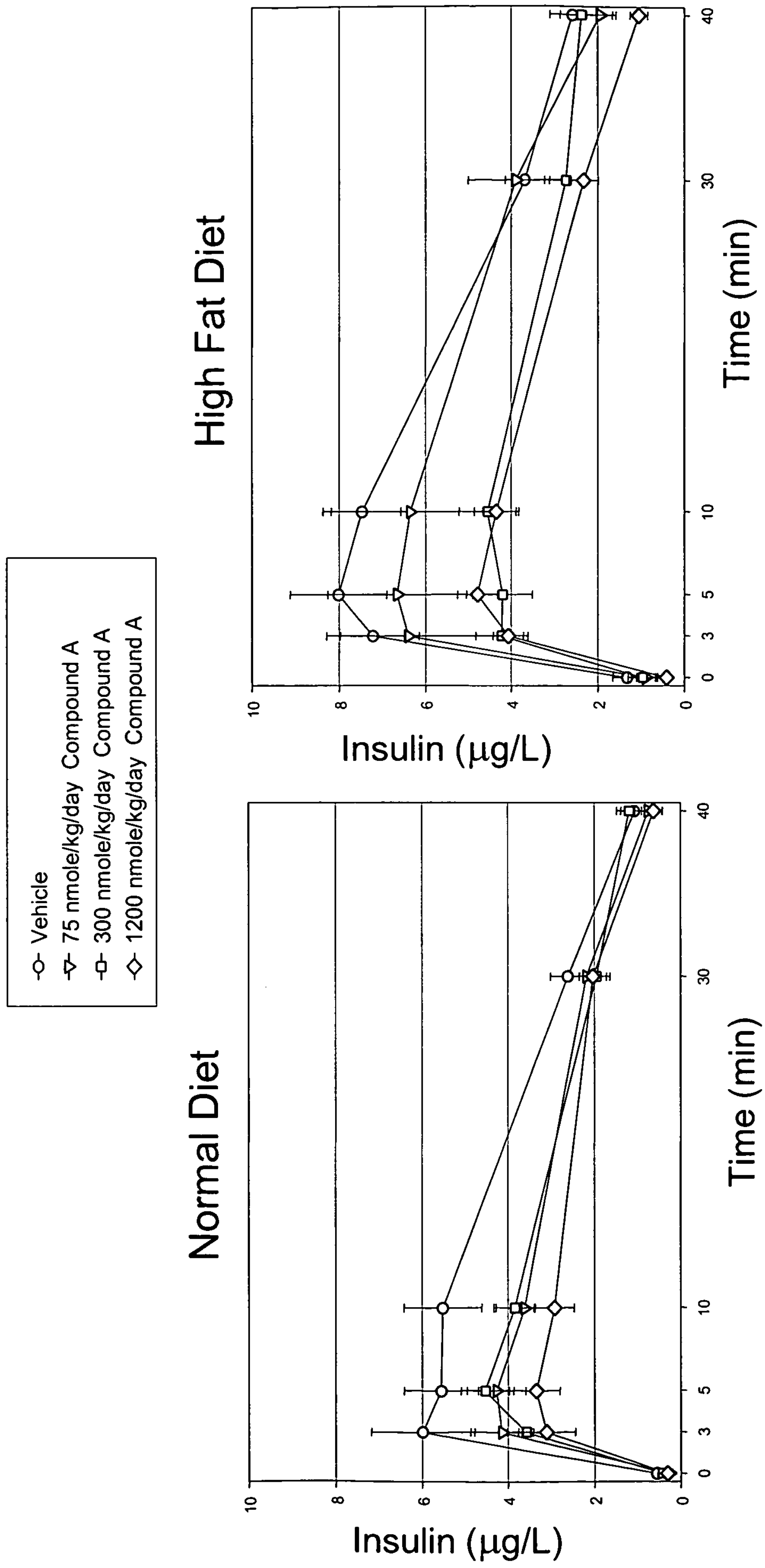
Vehicle
75 nmole/kg/day Compound A
300 nmole/kg/day Compound A
1200 nmole/kg/day Compound A
Normal Diet
High Fat Diet
Insulin (μg/L)
Time (min)
0
2
4
6
8
10
0
3
5
10
30
40

Figure 2D

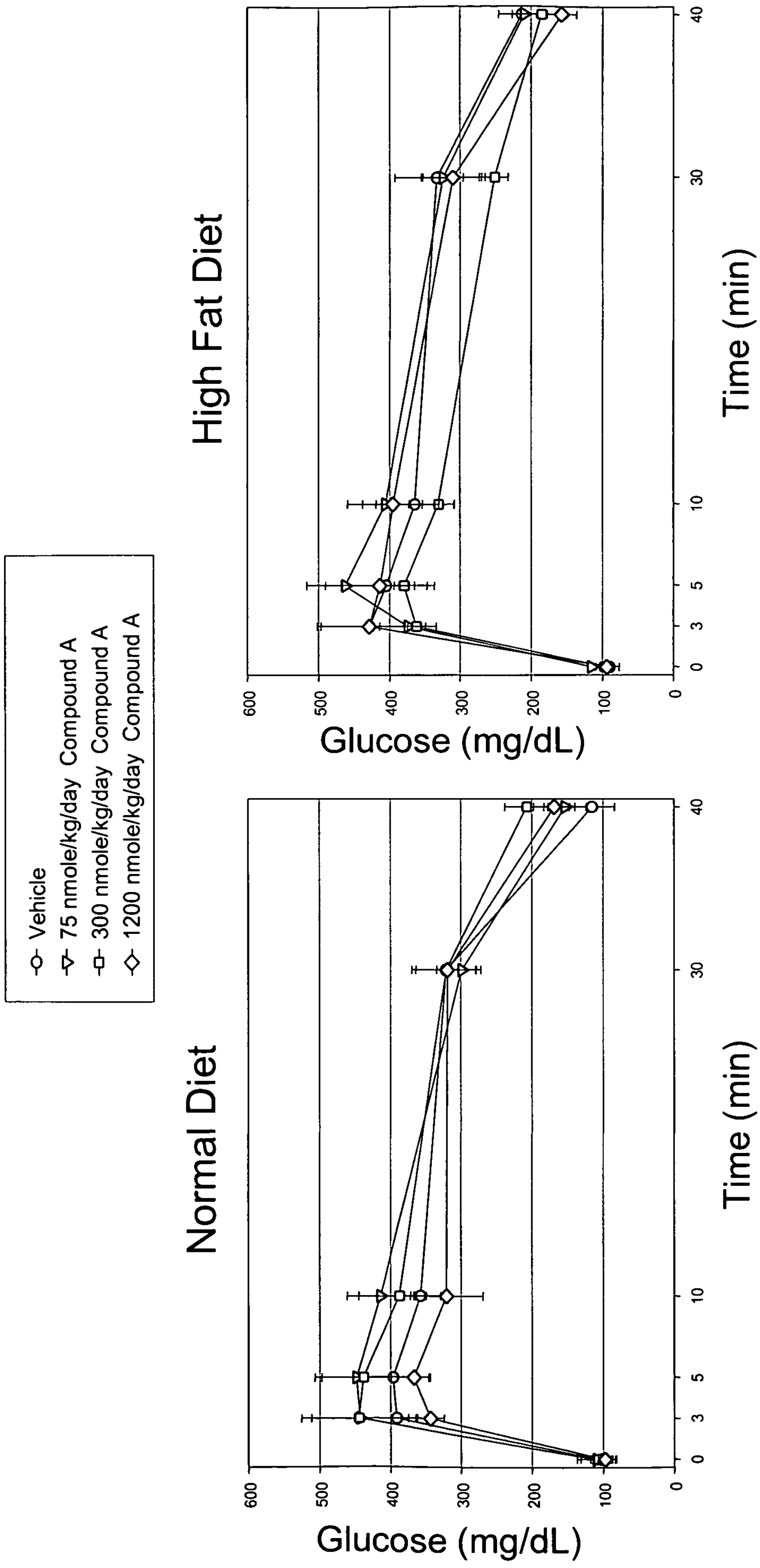
Vehicle
75 nmole/kg/day Compound A
300 nmole/kg/day Compound A
1200 nmole/kg/day Compound A
Normal Diet
High Fat Diet
Glucose (mg/dL)
Glucose (mg/dL)
600
500
400
300
200
100
0
0
3
5
10
30
40
Time (min)
Time (min)

Figure 3A

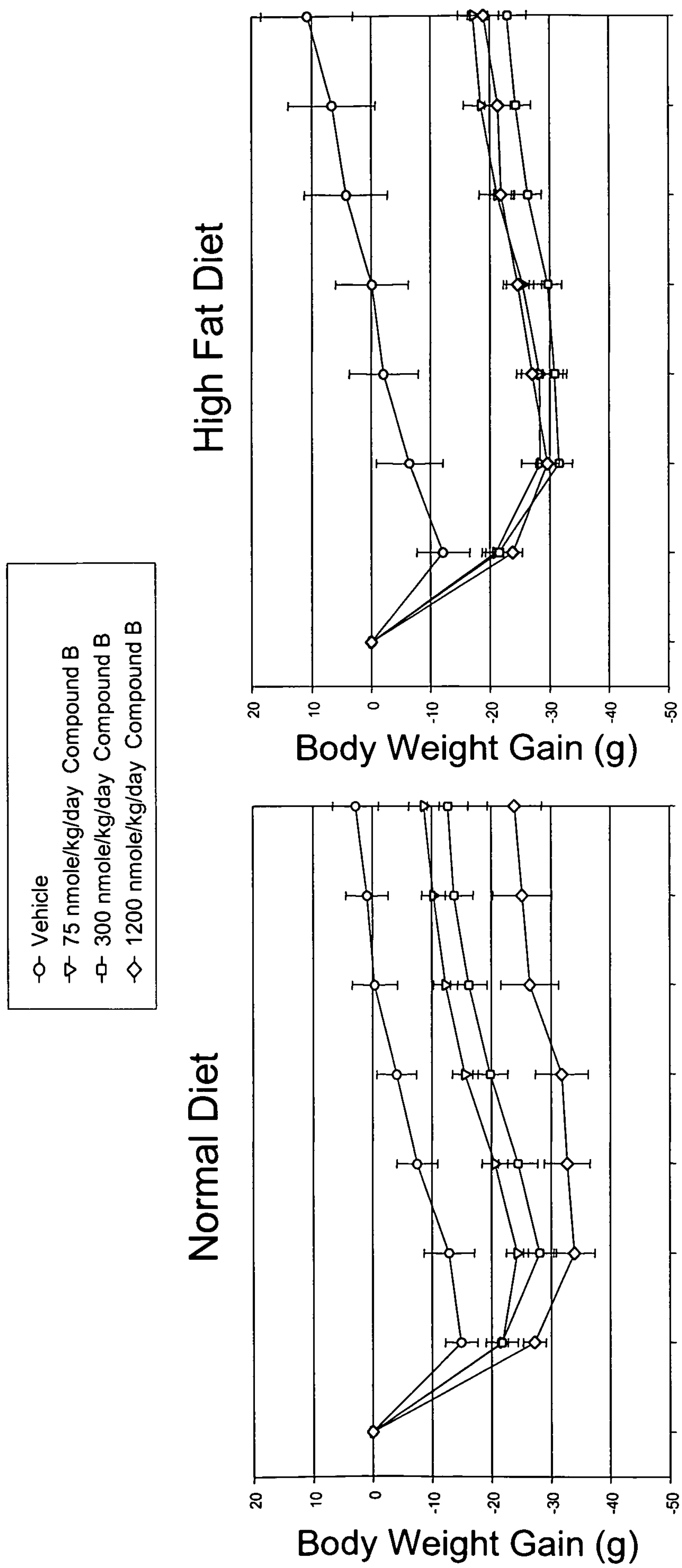
Vehicle
75 nmole/kg/day Compound B
300 nmole/kg/day Compound B
1200 nmole/kg/day Compound B
Normal Diet
High Fat Diet
Body Weight Gain (g)
Day
20
10
0
-10
-20
-30
-40
-50
0
1
2
3
4
5
6
7

Figure 3B

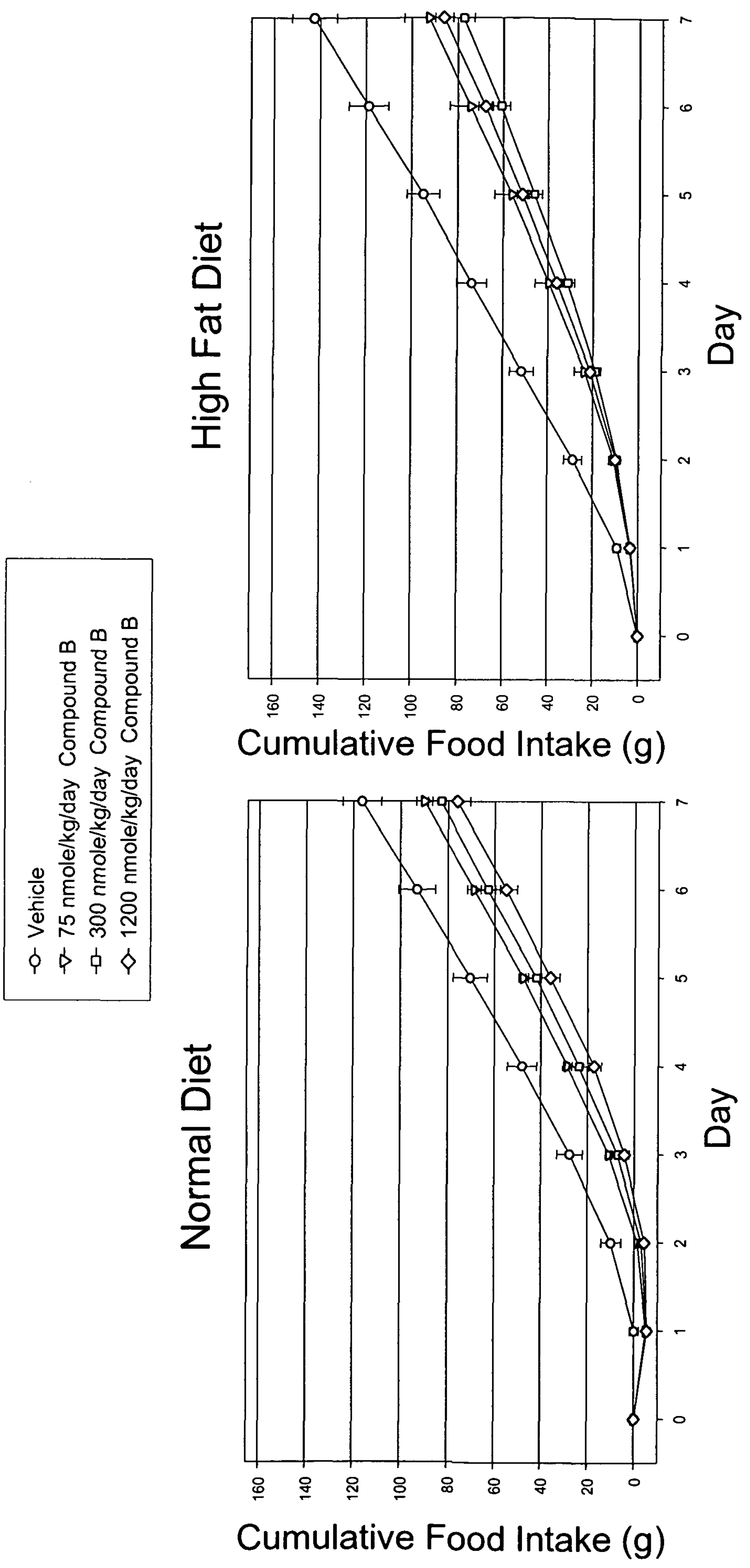
Vehicle
75 nmole/kg/day Compound B
300 nmole/kg/day Compound B
1200 nmole/kg/day Compound B
Normal Diet
High Fat Diet
Cumulative Food Intake (g)
0
20
40
60
80
100
120
140
160
Day
0
1
2
3
4
5
6
7

Figure 3C

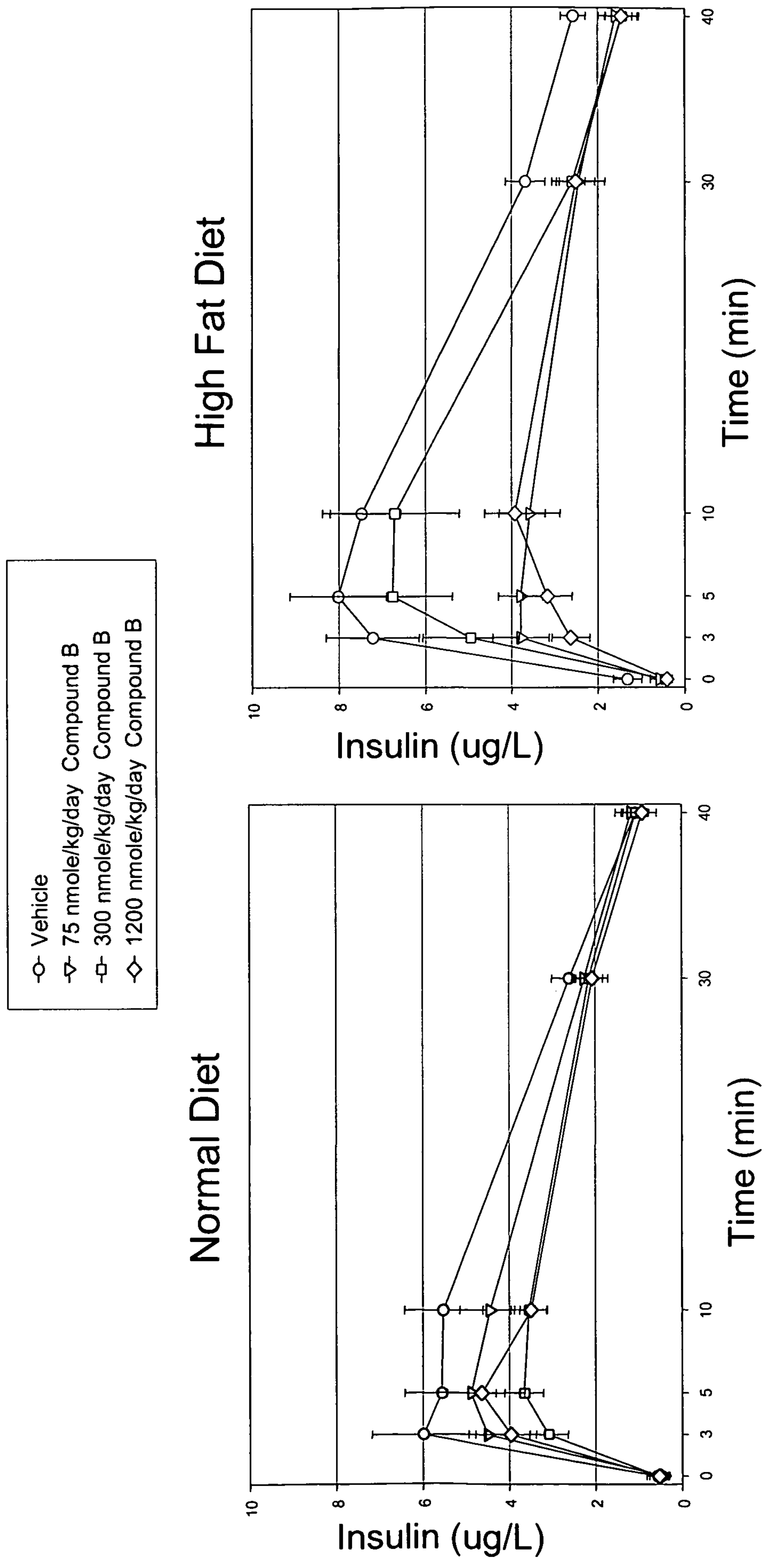

Vehicle
75 nmole/kg/day Compound B
300 nmole/kg/day Compound B
1200 nmole/kg/day Compound B
Normal Diet
High Fat Diet
Insulin (ug/L)
Time (min)
0
2
4
6
8
10
0
3
5
10
30
40

Figure 3D

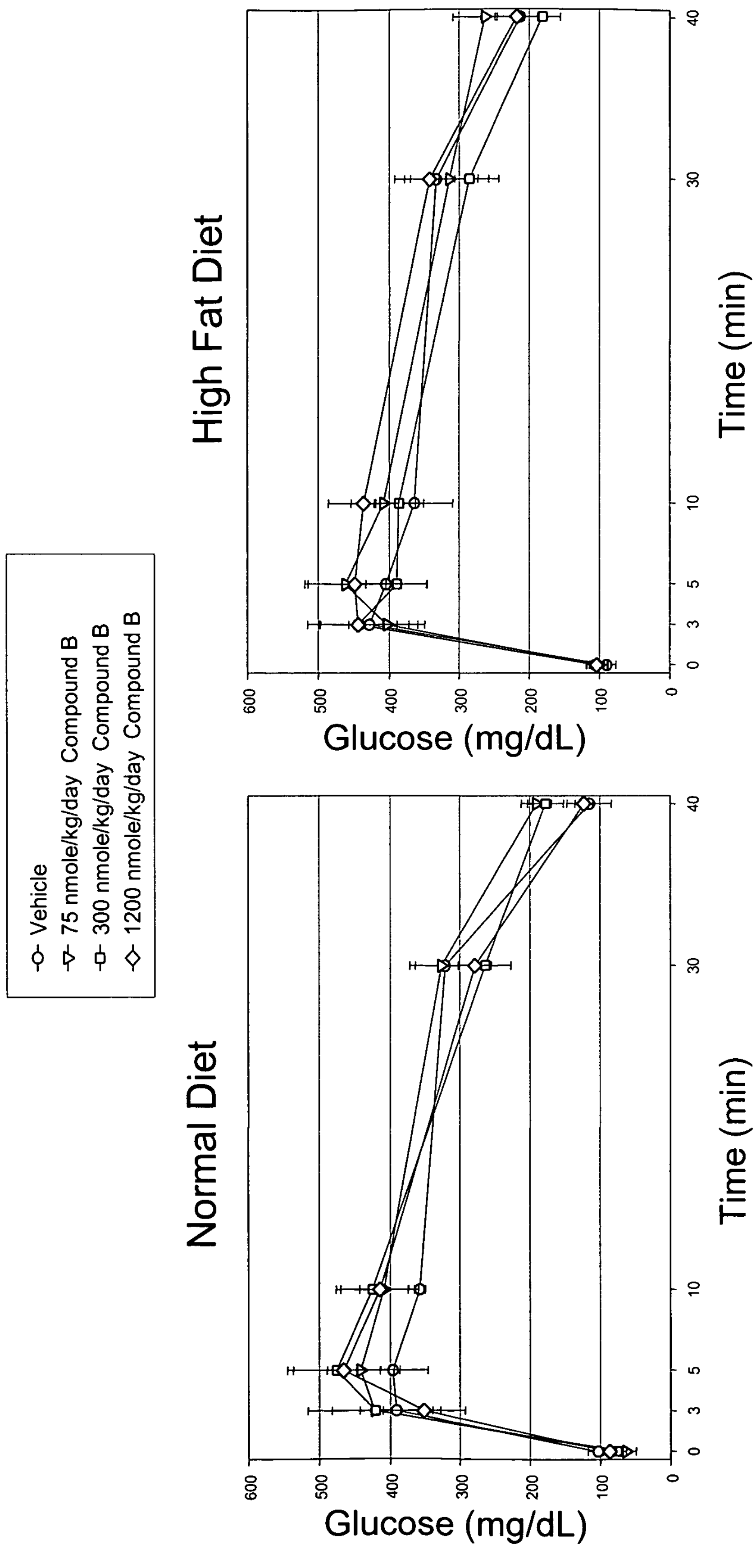
Vehicle
75 nmole/kg/day Compound B
300 nmole/kg/day Compound B
1200 nmole/kg/day Compound B
Normal Diet
High Fat Diet
Glucose (mg/dL)
Time (min)
0
100
200
300
400
500
600
0
3
5
10
30
40

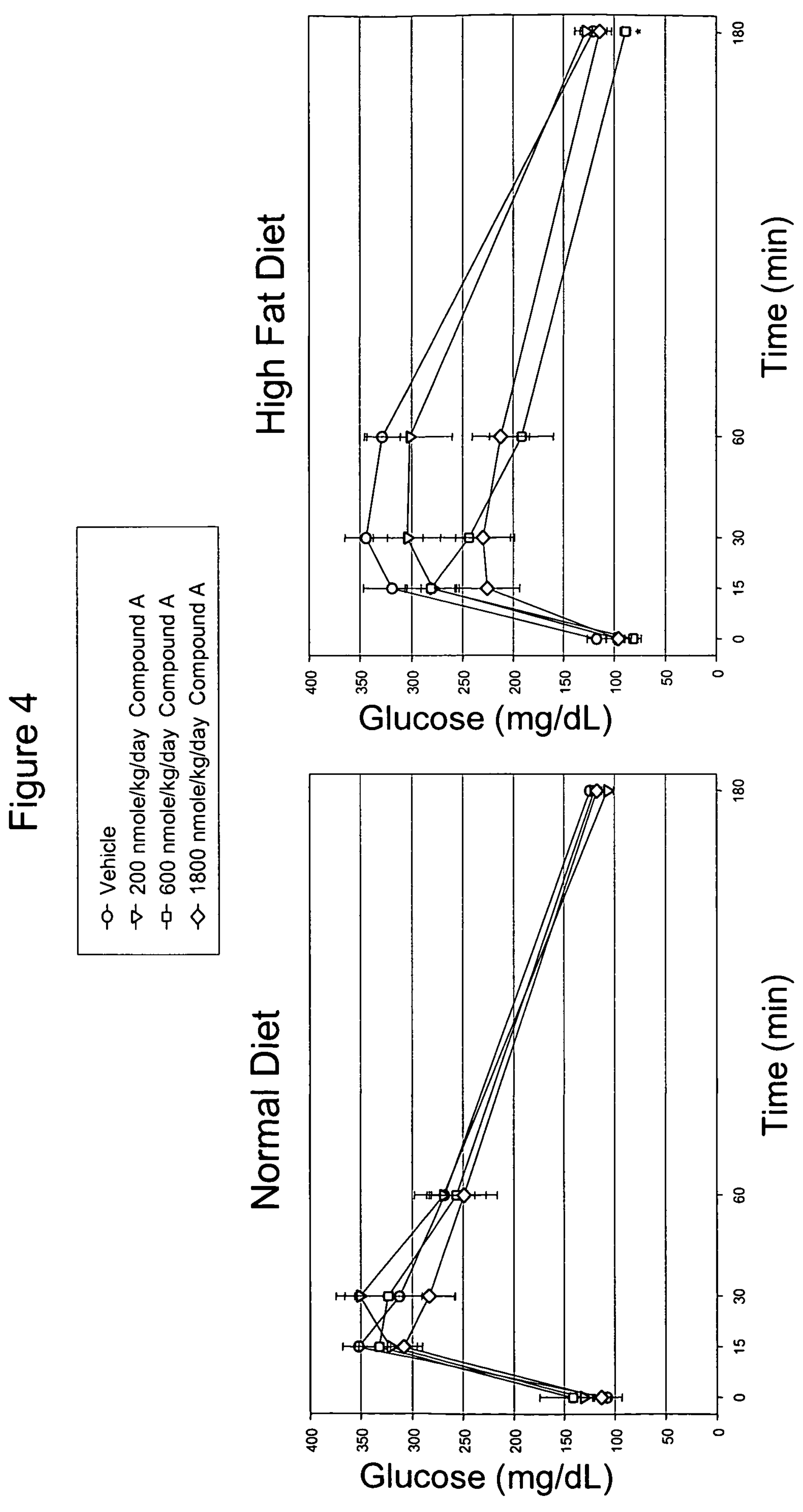
Figure 4
Vehicle
200 nmole/kg/day Compound A
600 nmole/kg/day Compound A
1800 nmole/kg/day Compound A
Normal Diet
High Fat Diet
Glucose (mg/dL)
0
50
100
150
200
250
300
350
400
Time (min)
0
15
30
60
180

Figure 5

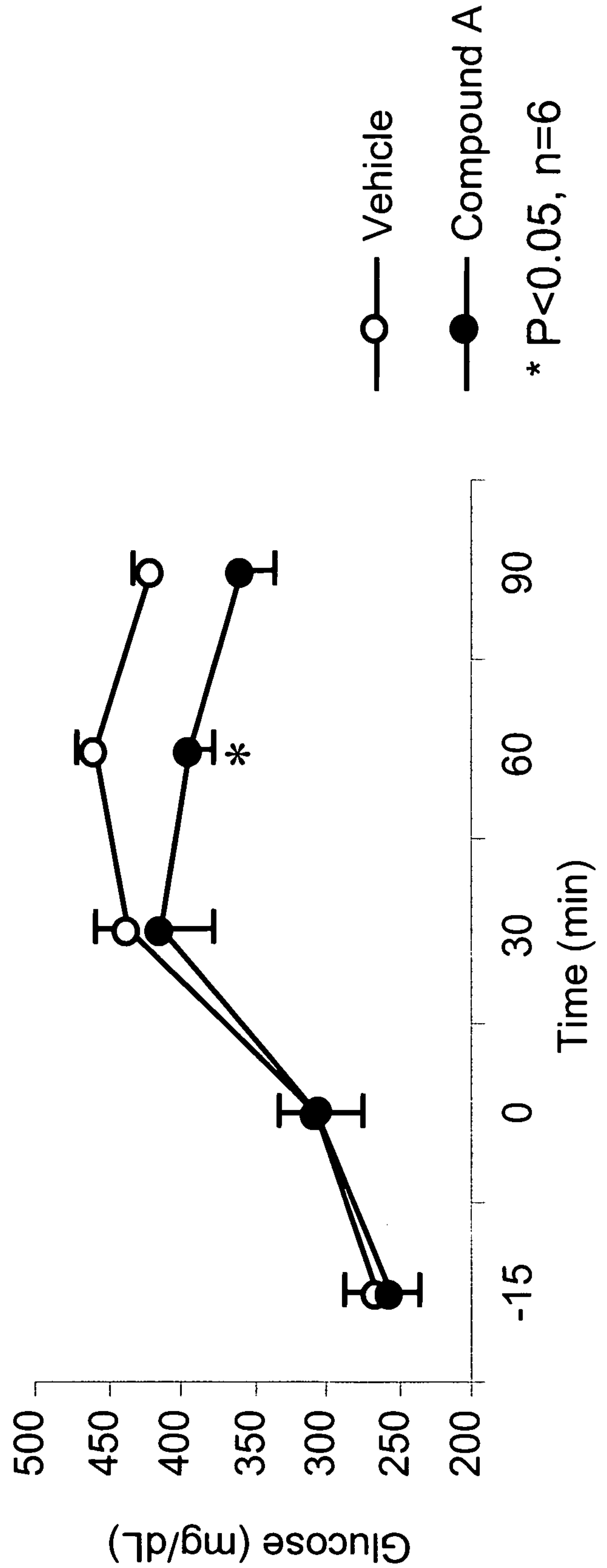
Glucose (mg/dL)
500
450
400
350
300
250
200
-15
0
30
60
90
Time (min)
*
Vehicle
Compound A
* P<0.05, n=6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5672659 A
- US 5595760 A
- US 5821221 A
- US 5916883 A
- US 61001933 B


### Non-patent literature cited in the description


- **CONE et al.** *Recent Prog. Horm. Res.,* 1996, vol. 51, 287-317
- **CONE et al.** *Ann. N.Y. Acad. Sci.,* 1993, vol. 31, 342-363
- **CONE et al.** *Ann. N.Y. Acad. Sci.,* 1993, vol. 680, 342-363
- **CONE et al.** *Recent Prog. Horm. Res.,* 1996, vol. 51, 287-318
- **GIRAUDO et al.** *Brain Res.,* 1998, vol. 809, 302-306
- **FAROOQI et al.** *NE J Med.,* 2003, vol. 348, 1085-1095
- **MACNEIL et al.** *Eu. J. Pharm.,* 2002, vol. 44, 141-157
- **MACNEIL et al.** *Eu. J. Pharm.,* 2002, vol. 450, 93-109
- **KASK et al.** *NeuroReport,* 1999, vol. 10, 707-711
- **CHEN et al.** *Transgenic Res.,* 2000, vol. 9, 145-54
- **MARSH et al.** *Nat Genet.,* 1995, vol. 21, 119-22
- **BALTHASAR et al.** *Cell,* 2005, vol. 123, 493-505
- **KOURIS-BLAZOS et al.** *Asia Pac. J. Clin. Nutr.,* 2007, vol. 16, 329-338
- **KOSTI et al.** *Cent. Eur. J. Public Health,* 2006, vol. 14, 151-159
- **BRAY et al.** *Diabetes/Metabolism Review,* 1988, vol. 4, 653-679
- **FLYNN et al.** *Proc. Nutritional Society,* 1991, vol. 50, 413
- **RICCARDI et al.** *Clin. Nutr.,* 2004, vol. 23, 447-456
- **CORNELL.** *Manag. Care Pharm.,* 2007, vol. 13, 11-5
- **MUDALIAR.** *Indian J. Med. Res.,* 2007, vol. 125, 275-296
- **OBICI et al.** *J. Clin. Inv.,* 2001, vol. 108, 1079-1085
- **BANNO et al.** *FEBS letters,* 2007, vol. 581, 1131-1136
- **SCHWARTZ et al.** *Nature,* 2000, vol. 404, 661-671
- **SEELEY et al.** *Ann. Rev. Nutr.,* 2004, vol. 24, 133-149
- **HEIJBOR et al.** *Diabetologia,* 2005, vol. 48, 1621-1626
- **BERGE, S. M. et al.** *J. Pharm. Sci.,* 1977, vol. 66, 1-19
- **GOULD, P.L.** *Int'l J. Pharmaceutics,* 1986, vol. 33, 201-17
- **BIGHLEY, L.D et al.** Encyclo. Parma. Tech. Marcel Dekker Inc, 1996, vol. 13, 453-97